(19) Europäisches Patentamt
European Patent Office
Office européen des brevets

(11) EP 2 949 672 A1

(12) # EUROPEAN PATENT APPLICATION

(43) Date of publication:
**02.12.2015 Bulletin 2015/49**

(51) Int Cl.:
***C07K 16/28*** $^{(2006.01)}$ ***A61K 39/395*** $^{(2006.01)}$
***A61P 29/00*** $^{(2006.01)}$ ***C12N 15/09*** $^{(2006.01)}$

(21) Application number: **15172595.9**

(22) Date of filing: **04.12.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **05.12.2008 PCT/JP2008/072152**
**13.03.2009 PCT/JP2009/054941**

(62) Document number(s) of the earlier application(s) in accordance with Art. 76 EPC:
**09830463.7 / 2 354 161**

(71) Applicant: **CHUGAI SEIYAKU KABUSHIKI KAISHA**
**Tokyo 115 (JP)**

(72) Inventors:
- **Kuramochi, Taichi**
**Gotemba-shi, Shizuoka 412-8513 (JP)**
- **Kasutani, Keiko**
**Gotemba-shi, Shizuoka 412-8513 (JP)**
- **Ohyama, Souhei**
**Gotemba-shi, Shizuoka 412-8513 (JP)**
- **Tsunoda, Hiroyuki**
**Gotemba-shi, Shizuoka 412-8513 (JP)**
- **Igawa, Tomoyuki**
**Gotemba-shi, Shizuoka 412-8513 (JP)**
- **Tachibana, Tatsuhiko**
**Gotemba-shi, Shizuoka 412-8513 (JP)**
- **Shiraiwa, Hirotake**
**Gotemba-shi, Shizuoka 412-8513 (JP)**
- **Esaki, Keiko**
**Gotemba-shi, Shizuoka 412-8513 (JP)**

(74) Representative: **Vossius & Partner**
**Patentanwälte Rechtsanwälte mbB**
**Siebertstrasse 3**
**81675 München (DE)**

Remarks:
•A request for correction of the description has been filed pursuant to Rule 139 EPC. A decision on the request will be taken during the proceedings before the Examining Division (Guidelines for Examination in the EPO, A-V, 3.).
•This application was filed on 17-06-2015 as a divisional application to the application mentioned under INID code 62.

(54) **ANTI-NR10 ANTIBODY, AND USE THEREOF**

(57) The present inventors successfully obtained anti-NR10 antibodies having an effective neutralizing activity against NR10. The anti-NR10 antibodies provided by the present disclosure are useful as, for example, pharmaceuticals for treating or preventing inflammatory diseases.

EP 2 949 672 A1

SEQUENCE LISTING

<110>  CHUGAI SEIYAKU KABUSHIKI KAISHA

<120>  ANTI-NR10 ANTIBODY AND USE THEREOF

<130>  S2132 EP/1 S3

<150>  EP 09 83 0463.7
<151>  2009-12-04

<150>  JP 2008-072152
<151>  2008-12-05

<150>  JP 2009-054941
<151>  2009-03-13

<160>  287

<170>  PatentIn version 3.4

<210>  1
<211>  5
<212>  PRT
<213>  Mus musculus

<400>  1

Gly Tyr Ile Met Asn
1               5

<210>  2
<211>  17
<212>  PRT
<213>  Mus musculus

<400>  2

Leu Ile Asn Pro Tyr Asn Gly Asp Thr Asn Tyr Asn Gln Lys Phe Lys
1               5                   10                  15

Gly

<210>  3
<211>  12
<212>  PRT
<213>  Mus musculus

<400>  3

Asp Gly Tyr Asp Asp Gly Pro Tyr Thr Met Asp Tyr
1               5                   10

<210>  4
<211>  121
<212>  PRT
<213>  Mus musculus

<400>  4

Glu Val Gln Leu Gln Gln Ser Gly Pro Glu Leu Val Lys Pro Gly Ala
1               5                   10                  15

Ser Met Lys Ile Ser Cys Lys Ala Ser Gly Tyr Ser Phe Thr Gly Tyr
20 25 30

Ile Met Asn Trp Val Lys Gln Ser His Gly Lys Asn Leu Glu Trp Ile
35 40 45

Gly Leu Ile Asn Pro Tyr Asn Gly Asp Thr Asn Tyr Asn Gln Lys Phe
50 55 60

Lys Gly Lys Ala Thr Leu Thr Val Asp Lys Ser Ser Ser Thr Ala Tyr
65 70 75 80

Met Glu Leu Leu Ser Leu Thr Ser Glu Asp Ser Ala Val Tyr Tyr Cys
85 90 95

Ala Arg Asp Gly Tyr Asp Asp Gly Pro Tyr Thr Met Asp Tyr Trp Gly
100 105 110

Gln Gly Thr Ser Val Thr Val Ser Ser
115 120

<210> 5
<211> 11
<212> PRT
<213> Mus musculus

<400> 5

Arg Ala Ser Glu Asn Ile Tyr Ser Phe Leu Ala
1 5 10

<210> 6
<211> 7
<212> PRT
<213> Mus musculus

<400> 6

Asn Ala Lys Thr Leu Ala Lys
1 5

<210> 7
<211> 9
<212> PRT
<213> Mus musculus

<400> 7

Gln His His Tyr Glu Ser Pro Leu Thr
1 5

<210> 8
<211> 107
<212> PRT
<213> Mus musculus

<400> 8

Asp Ile Gln Met Thr Gln Ser Pro Ala Ser Leu Ser Ala Ser Val Gly
1 5 10 15

Glu Thr Val Thr Ile Thr Cys Arg Ala Ser Glu Asn Ile Tyr Ser Phe

20 25 30

Leu Ala Trp Tyr Gln Gln Lys Gln Gly Lys Ser Pro His Leu Leu Val
35 40 45

Tyr Asn Ala Lys Thr Leu Ala Lys Gly Val Pro Ser Arg Phe Ser Gly
50 55 60

Ser Gly Ser Gly Thr Gln Phe Ser Leu Lys Ile Asn Ser Leu Gln Pro
65 70 75 80

Glu Asp Phe Gly Ser Tyr Tyr Cys Gln His His Tyr Glu Ser Pro Leu
85 90 95

Thr Phe Gly Gly Gly Thr Lys Leu Glu Ile Lys
100 105

<210> 9
<211> 5
<212> PRT
<213> Mus musculus

<400> 9

Gly Tyr Ile Met Asn
1 5

<210> 10
<211> 17
<212> PRT
<213> Mus musculus

<400> 10

Leu Ile Asn Pro Tyr Asn Gly Gly Thr Ser Tyr Asn Gln Lys Phe Lys
1 5 10 15

Gly

<210> 11
<211> 12
<212> PRT
<213> Mus musculus

<400> 11

Asp Gly Tyr Asp Asp Gly Pro Tyr Thr Met Asp Tyr
1 5 10

<210> 12
<211> 121
<212> PRT
<213> Mus musculus

<400> 12

Glu Val Gln Leu Gln Gln Ser Gly Pro Glu Leu Val Lys Pro Gly Ala
1 5 10 15

Ser Met Lys Ile Ser Cys Lys Ala Ser Gly Tyr Ser Phe Thr Gly Tyr
20 25 30

Ile Met Asn Trp Val Lys Gln Ser His Gly Lys Asn Leu Glu Trp Ile
35 40 45

Gly Leu Ile Asn Pro Tyr Asn Gly Gly Thr Ser Tyr Asn Gln Lys Phe
50 55 60

Lys Gly Lys Ala Thr Leu Thr Val Asp Lys Ser Ser Ser Thr Ala Tyr
65 70 75 80

Met Glu Leu Leu Ser Leu Thr Ser Glu Asp Ser Ala Val Tyr Tyr Cys
85 90 95

Ala Arg Asp Gly Tyr Asp Asp Gly Pro Tyr Thr Met Asp Tyr Trp Gly
100 105 110

Gln Gly Thr Ser Val Thr Val Ser Ser
115 120

<210> 13
<211> 11
<212> PRT
<213> Mus musculus

<400> 13

Arg Thr Ser Glu Asn Ile Tyr Ser Phe Leu Ala
1 5 10

<210> 14
<211> 7
<212> PRT
<213> Mus musculus

<400> 14

Asn Ala Lys Thr Leu Ala Lys
1 5

<210> 15
<211> 9
<212> PRT
<213> Mus musculus

<400> 15

Gln His His Tyr Glu Ser Pro Leu Thr
1 5

<210> 16
<211> 107
<212> PRT
<213> Mus musculus

<400> 16

Asp Ile Gln Met Thr Gln Ser Pro Ala Ser Leu Ser Ala Ser Val Gly
1 5 10 15

Glu Thr Val Thr Ile Thr Cys Arg Thr Ser Glu Asn Ile Tyr Ser Phe
20 25 30

Leu Ala Trp Tyr Gln Gln Lys Gln Gly Lys Ser Pro His Leu Leu Val
35 40 45

Tyr Asn Ala Lys Thr Leu Ala Lys Gly Val Pro Ser Arg Phe Ser Gly
    50                  55                  60

Ser Gly Ser Gly Thr Gln Phe Ser Leu Lys Ile Asn Ser Leu Gln Pro
65                  70                  75                  80

Glu Asp Phe Gly Ser Tyr Phe Cys Gln His His Tyr Glu Ser Pro Leu
                85                  90                  95

Thr Phe Gly Gly Gly Thr Lys Leu Glu Ile Lys
            100                 105

<210>  17
<211>  5
<212>  PRT
<213>  Mus musculus

<400>  17

Gly Tyr Ile Met Asn
1               5

<210>  18
<211>  17
<212>  PRT
<213>  Mus musculus

<400>  18

Leu Ile Asn Pro Tyr Asn Gly Gly Ala Glu Tyr Asn Gln Lys Phe Lys
1               5                   10                  15

Asp

<210>  19
<211>  12
<212>  PRT
<213>  Mus musculus

<400>  19

Asp Gly Tyr Asp Asp Gly Pro Tyr Thr Met Asp Tyr
1               5                   10

<210>  20
<211>  121
<212>  PRT
<213>  Mus musculus

<400>  20

Glu Val Gln Leu Gln Gln Ser Gly Pro Glu Leu Val Lys Pro Gly Thr
1               5                   10                  15

Ser Met Lys Ile Ser Cys Lys Ala Ser Gly Tyr Ser Phe Thr Gly Tyr
            20                  25                  30

Ile Met Asn Trp Val Lys Gln Ser His Gly Lys Asn Leu Glu Trp Ile
        35                  40                  45

Gly Leu Ile Asn Pro Tyr Asn Gly Gly Ala Glu Tyr Asn Gln Lys Phe

50 55 60

Lys Asp Lys Ala Thr Phe Thr Val Asp Lys Ser Ser Ser Thr Ala Tyr
65 70 75 80

Met Glu Leu Leu Ser Leu Thr Ser Glu Asp Ser Ala Val Tyr Tyr Cys
85 90 95

Ala Arg Asp Gly Tyr Asp Asp Gly Pro Tyr Thr Met Asp Tyr Trp Gly
100 105 110

Gln Gly Thr Ser Val Thr Val Ser Ser
115 120

<210> 21
<211> 11
<212> PRT
<213> Mus musculus

<400> 21

Arg Ala Asn Glu Asn Ile Tyr Ser Tyr Leu Ala
1 5 10

<210> 22
<211> 7
<212> PRT
<213> Mus musculus

<400> 22

Asn Ala Lys Thr Leu Ala Glu
1 5

<210> 23
<211> 9
<212> PRT
<213> Mus musculus

<400> 23

Gln His His Tyr Gly Thr Pro Pro Thr
1 5

<210> 24
<211> 107
<212> PRT
<213> Mus musculus

<400> 24

Asp Ile Gln Met Thr Gln Ser Pro Ala Ser Leu Ser Ala Ser Val Gly
1 5 10 15

Glu Thr Val Thr Phe Thr Cys Arg Ala Asn Glu Asn Ile Tyr Ser Tyr
20 25 30

Leu Ala Trp Tyr Gln Gln Lys Gln Gly Lys Ser Pro Gln Leu Leu Val
35 40 45

Tyr Asn Ala Lys Thr Leu Ala Glu Gly Val Pro Ser Arg Phe Ser Gly
50 55 60

Ser Gly Ser Gly Thr Gln Phe Ser Leu Lys Ile Asn Ser Leu Gln Pro
65 70 75 80

Glu Asp Phe Gly Ser Tyr Tyr Cys Gln His His Tyr Gly Thr Pro Pro
85 90 95

Thr Phe Gly Gly Gly Thr Lys Leu Glu Ile Lys
100 105

<210> 25
<211> 5
<212> PRT
<213> Mus musculus

<400> 25

Asn Tyr Trp Met His
1 5

<210> 26
<211> 17
<212> PRT
<213> Mus musculus

<400> 26

Ala Ile Tyr Pro Gly Asn Ser Asp Thr Asp Tyr Asn Gln Lys Phe Lys
1 5 10 15

Gly

<210> 27
<211> 8
<212> PRT
<213> Mus musculus

<400> 27

Asp Gly Tyr Asp Asp Phe Asp His
1 5

<210> 28
<211> 116
<212> PRT
<213> Mus musculus

<400> 28

Glu Val Gln Leu Gln Gln Ser Gly Thr Val Leu Ala Arg Pro Gly Ala
1 5 10 15

Ser Val Lys Met Ser Cys Lys Ala Ser Gly Tyr Thr Phe Thr Asn Tyr
20 25 30

Trp Met His Trp Val Lys Gln Arg Pro Gly Gln Gly Leu Glu Trp Ile
35 40 45

Gly Ala Ile Tyr Pro Gly Asn Ser Asp Thr Asp Tyr Asn Gln Lys Phe
50 55 60

Lys Gly Lys Ala Lys Leu Thr Ala Val Thr Ser Ala Ser Thr Ala Tyr
65 70 75 80

Met Glu Leu Ser Ser Leu Thr Asn Glu Asp Ser Ala Val Phe Phe Cys
85 90 95

Thr Thr Gly Tyr Asp Asp Phe Asp His Trp Gly Gln Gly Thr Thr Leu
100 105 110

Thr Val Ser Ser
115

<210> 29
<211> 12
<212> PRT
<213> Mus musculus

<400> 29

Arg Ala Ser Ser Ser Val Ser Ser Ser Tyr Leu His
1 5 10

<210> 30
<211> 7
<212> PRT
<213> Mus musculus

<400> 30

Ser Thr Ser Asn Leu Ala Ser
1 5

<210> 31
<211> 9
<212> PRT
<213> Mus musculus

<400> 31

Gln Gln Tyr Ser Gly Tyr Pro Leu Thr
1 5

<210> 32
<211> 108
<212> PRT
<213> Mus musculus

<400> 32

Glu Asn Val Leu Thr Gln Ser Pro Ala Ile Met Ser Ala Ser Pro Gly
1 5 10 15

Glu Lys Val Thr Met Thr Cys Arg Ala Ser Ser Ser Val Ser Ser Ser
20 25 30

Tyr Leu His Trp Tyr Gln Gln Lys Ser Gly Ala Ser Pro Lys Leu Trp
35 40 45

Ile Tyr Ser Thr Ser Asn Leu Ala Ser Gly Val Pro Ala Arg Phe Ser
50 55 60

Gly Ser Gly Ser Gly Thr Ser Tyr Tyr Phe Thr Ile Ser Ser Val Glu
65 70 75 80

Ala Glu Asp Ala Ala Thr Tyr Tyr Cys Gln Gln Tyr Ser Gly Tyr Pro

85 90 95

Leu Thr Phe Gly Gly Gly Thr Lys Leu Glu Ile Lys
100 105

<210> 33
<211> 1406
<212> DNA
<213> Mus musculus

<400> 33
atgggatgga gctggatctt tctcttcctc ctgtcaggaa ctgcaggtgt ccactctgag 60

gtccagctgc aacagtctgg acctgagctg gtgaagcctg gagcttcaat gaagatctcc 120

tgcaaggctt ctggttactc attcactggc tacatcatga actgggtgaa gcagagccat 180

ggaaagaacc ttgagtggat tggacttatt aatccttaca atggtgatac taactacaac 240

cagaagttca agggcaaggc cacattaact gtagacaagt catccagcac agcctacatg 300

gaactcctca gtctgacatc agaggactct gcagtctatt actgtgcaag ggatggttac 360

gacgacggac cctatactat ggactactgg ggtcaaggaa cctcagtcac cgtctcctca 420

gccaaaacga cacccccatc tgtctatcca ctggcccctg gatctgctgc ccaaactaac 480

tccatggtga ccctgggatg cctggtcaag ggctatttcc ctgagccagt gacagtgacc 540

tggaactctg gatccctgtc cagcggtgtg cacaccttcc cagctgtcct gcagtctgac 600

ctctacactc tgagcagctc agtgactgtc ccctccagca cctggcccag cgagaccgtc 660

acctgcaacg ttgcccaccc ggccagcagc accaaggtgg acaagaaaat tgtgcccagg 720

gattgtggtt gtaagccttg catatgtaca gtcccagaag tatcatctgt cttcatcttc 780

cccccaaagc ccaaggatgt gctcaccatt actctgactc ctaaggtcac gtgtgttgtg 840

gtagacatca gcaaggatga tcccgaggtc cagttcagct ggtttgtaga tgatgtggag 900

gtgcacacag ctcagacgca accccgggag gagcagttca acagcacttt ccgctcagtc 960

agtgaacttc ccatcatgca ccaggactgg ctcaatggca aggagttcaa atgcagggtc 1020

aacagtgcag ctttccctgc ccccatcgag aaaaccatct ccaaaaccaa aggcagaccg 1080

aaggctccac aggtgtacac cattccacct cccaaggagc agatggccaa ggataaagtc 1140

agtctgacct gcatgataac agacttcttc cctgaagaca ttactgtgga gtggcagtgg 1200

aatgggcagc cagcggagaa ctacaagaac actcagccca tcatggacac agatggctct 1260

tacttcgtct acagcaagct caatgtgcag aagagcaact gggaggcagg aaatactttc 1320

acctgctctg tgttacatga gggcctgcac aaccaccata ctgagaagag cctctcccac 1380

tctcctggta aataatgagc ggccgc 1406

<210> 34
<211> 445
<212> PRT
<213> Mus musculus

<400> 34

Glu Val Gln Leu Gln Gln Ser Gly Pro Glu Leu Val Lys Pro Gly Ala
1               5                   10                  15

Ser Met Lys Ile Ser Cys Lys Ala Ser Gly Tyr Ser Phe Thr Gly Tyr
            20                  25                  30

Ile Met Asn Trp Val Lys Gln Ser His Gly Lys Asn Leu Glu Trp Ile
        35                  40                  45

Gly Leu Ile Asn Pro Tyr Asn Gly Asp Thr Asn Tyr Asn Gln Lys Phe
    50                  55                  60

Lys Gly Lys Ala Thr Leu Thr Val Asp Lys Ser Ser Ser Thr Ala Tyr
65                  70                  75                  80

Met Glu Leu Leu Ser Leu Thr Ser Glu Asp Ser Ala Val Tyr Tyr Cys
                85                  90                  95

Ala Arg Asp Gly Tyr Asp Asp Gly Pro Tyr Thr Met Asp Tyr Trp Gly
            100                 105                 110

Gln Gly Thr Ser Val Thr Val Ser Ser Ala Lys Thr Thr Pro Pro Ser
        115                 120                 125

Val Tyr Pro Leu Ala Pro Gly Ser Ala Ala Gln Thr Asn Ser Met Val
    130                 135                 140

Thr Leu Gly Cys Leu Val Lys Gly Tyr Phe Pro Glu Pro Val Thr Val
145                 150                 155                 160

Thr Trp Asn Ser Gly Ser Leu Ser Ser Gly Val His Thr Phe Pro Ala
                165                 170                 175

Val Leu Gln Ser Asp Leu Tyr Thr Leu Ser Ser Ser Val Thr Val Pro
            180                 185                 190

Ser Ser Thr Trp Pro Ser Glu Thr Val Thr Cys Asn Val Ala His Pro
        195                 200                 205

Ala Ser Ser Thr Lys Val Asp Lys Lys Ile Val Pro Arg Asp Cys Gly
    210                 215                 220

Cys Lys Pro Cys Ile Cys Thr Val Pro Glu Val Ser Ser Val Phe Ile
225                 230                 235                 240

Phe Pro Pro Lys Pro Lys Asp Val Leu Thr Ile Thr Leu Thr Pro Lys
                245                 250                 255

Val Thr Cys Val Val Val Asp Ile Ser Lys Asp Asp Pro Glu Val Gln
            260                 265                 270

Phe Ser Trp Phe Val Asp Asp Val Glu Val His Thr Ala Gln Thr Gln
        275                 280                 285

Pro Arg Glu Glu Gln Phe Asn Ser Thr Phe Arg Ser Val Ser Glu Leu
    290                 295                 300

Pro Ile Met His Gln Asp Trp Leu Asn Gly Lys Glu Phe Lys Cys Arg
305                 310                 315                 320

Val Asn Ser Ala Ala Phe Pro Ala Pro Ile Glu Lys Thr Ile Ser Lys
325 330 335

Thr Lys Gly Arg Pro Lys Ala Pro Gln Val Tyr Thr Ile Pro Pro Pro
340 345 350

Lys Glu Gln Met Ala Lys Asp Lys Val Ser Leu Thr Cys Met Ile Thr
355 360 365

Asp Phe Phe Pro Glu Asp Ile Thr Val Glu Trp Gln Trp Asn Gly Gln
370 375 380

Pro Ala Glu Asn Tyr Lys Asn Thr Gln Pro Ile Met Asp Thr Asp Gly
385 390 395 400

Ser Tyr Phe Val Tyr Ser Lys Leu Asn Val Gln Lys Ser Asn Trp Glu
405 410 415

Ala Gly Asn Thr Phe Thr Cys Ser Val Leu His Glu Gly Leu His Asn
420 425 430

His His Thr Glu Lys Ser Leu Ser His Ser Pro Gly Lys
435 440 445

<210> 35
<211> 716
<212> DNA
<213> Mus musculus

<400> 35
atgagtgtgc ccactcaggt cctggggttg ctgctgctgt ggcttacagg tgccagatgt 60

gacatccaga tgactcagtc tccagcctcc ctatctgcat ctgtgggaga aactgtcacc 120

atcacatgtc gagcaagtga gaatatttac agtttttag catggtatca gcagaaacag 180

ggaaaatctc ctcacctcct ggtctataat gcaaaaacct tagcaaaagg tgtgccatca 240

aggttcagtg gcagtggatc tggcacacag ttttctctga agatcaacag cctgcagcct 300

gaagattttg ggagttatta ctgtcaacat cattatgaga gtcctctgac gttcggtgga 360

ggcaccaagc tggaaatcaa acgggctgat gctgcaccaa ctgtatccat cttcccacca 420

tccagtgagc agttaacatc tggaggtgcc tcagtcgtgt gcttcttgaa caacttctac 480

cccaaagaca tcaatgtcaa gtggaagatt gatggcagtg aacgacaaaa tggcgtcctg 540

aacagttgga ctgatcagga cagcaaagac agcacctaca gcatgagcag caccctcacg 600

ttgaccaagg acgagtatga acgacataac agctatacct gtgaggccac tcacaagaca 660

tcaacttcac ccattgtcaa gagcttcaac aggaatgagt gttaatgagc ggccgc 716

<210> 36
<211> 214
<212> PRT
<213> Mus musculus

<400> 36

Asp Ile Gln Met Thr Gln Ser Pro Ala Ser Leu Ser Ala Ser Val Gly
1 5 10 15

Glu Thr Val Thr Ile Thr Cys Arg Ala Ser Glu Asn Ile Tyr Ser Phe
20 25 30

Leu Ala Trp Tyr Gln Gln Lys Gln Gly Lys Ser Pro His Leu Leu Val
35 40 45

Tyr Asn Ala Lys Thr Leu Ala Lys Gly Val Pro Ser Arg Phe Ser Gly
50 55 60

Ser Gly Ser Gly Thr Gln Phe Ser Leu Lys Ile Asn Ser Leu Gln Pro
65 70 75 80

Glu Asp Phe Gly Ser Tyr Tyr Cys Gln His His Tyr Glu Ser Pro Leu
85 90 95

Thr Phe Gly Gly Gly Thr Lys Leu Glu Ile Lys Arg Ala Asp Ala Ala
100 105 110

Pro Thr Val Ser Ile Phe Pro Pro Ser Ser Glu Gln Leu Thr Ser Gly
115 120 125

Gly Ala Ser Val Val Cys Phe Leu Asn Asn Phe Tyr Pro Lys Asp Ile
130 135 140

Asn Val Lys Trp Lys Ile Asp Gly Ser Glu Arg Gln Asn Gly Val Leu
145 150 155 160

Asn Ser Trp Thr Asp Gln Asp Ser Lys Asp Ser Thr Tyr Ser Met Ser
165 170 175

Ser Thr Leu Thr Leu Thr Lys Asp Glu Tyr Glu Arg His Asn Ser Tyr
180 185 190

Thr Cys Glu Ala Thr His Lys Thr Ser Thr Ser Pro Ile Val Lys Ser
195 200 205

Phe Asn Arg Asn Glu Cys
210

<210> 37
<211> 1406
<212> DNA
<213> Mus musculus

<400> 37
atgggatgga gctggatctt tctcttcctc ctgtcaggaa ctgcaggtgt ccactctgag 60

gtccagctgc aacagtctgg acctgagctg gtgaagcctg gagcttcaat gaagatctcc 120

tgcaaggctt ctggttactc attcactggc tacatcatga actgggtgaa gcagagccat 180

ggaaagaacc ttgagtggat tggacttatt aatccttaca atggtggtac tagctacaac 240

cagaagttca agggcaaggc cacattaact gtagacaagt catccagtac agcctacatg 300

gaactcctca gtctgacatc agaggactct gcagtctatt actgtgcaag ggatggttac 360

gacgacggac cctatactat ggactactgg ggtcaaggaa cctcagtcac cgtctcctca 420

gccaaaacga cacccccatc tgtctatcca ctggcccctg gatctgctgc ccaaactaac 480

tccatggtga ccctgggatg cctggtcaag ggctatttcc ctgagccagt gacagtgacc 540

tggaactctg gatccctgtc cagcggtgtg cacaccttcc cagctgtcct gcagtctgac 600

ctctacactc tgagcagctc agtgactgtc ccctccagca cctggcccag cgagaccgtc 660

acctgcaacg ttgcccaccc ggccagcagc accaaggtgg acaagaaaat tgtgcccagg 720

gattgtggtt gtaagccttg catatgtaca gtcccagaag tatcatctgt cttcatcttc 780

cccccaaagc ccaaggatgt gctcaccatt actctgactc ctaaggtcac gtgtgttgtg 840

gtagacatca gcaaggatga tcccgaggtc cagttcagct ggtttgtaga tgatgtggag 900

gtgcacacag ctcagacgca accccgggag gagcagttca acagcacttt ccgctcagtc 960

agtgaacttc ccatcatgca ccaggactgg ctcaatggca aggagttcaa atgcagggtc 1020

aacagtgcag ctttccctgc ccccatcgag aaaaccatct ccaaaaccaa aggcagaccg 1080

aaggctccac aggtgtacac cattccacct cccaaggagc agatggccaa ggataaagtc 1140

agtctgacct gcatgataac agacttcttc cctgaagaca ttactgtgga gtggcagtgg 1200

aatgggcagc cagcggagaa ctacaagaac actcagccca tcatggacac agatggctct 1260

tacttcgtct acagcaagct caatgtgcag aagagcaact gggaggcagg aaatactttc 1320

acctgctctg tgttacatga gggcctgcac aaccaccata ctgagaagag cctctcccac 1380

tctcctggta aataatgagc ggccgc 1406

<210> 38
<211> 445
<212> PRT
<213> Mus musculus

<400> 38

Glu Val Gln Leu Gln Gln Ser Gly Pro Glu Leu Val Lys Pro Gly Ala
1 5 10 15

Ser Met Lys Ile Ser Cys Lys Ala Ser Gly Tyr Ser Phe Thr Gly Tyr
20 25 30

Ile Met Asn Trp Val Lys Gln Ser His Gly Lys Asn Leu Glu Trp Ile
35 40 45

Gly Leu Ile Asn Pro Tyr Asn Gly Gly Thr Ser Tyr Asn Gln Lys Phe
50 55 60

Lys Gly Lys Ala Thr Leu Thr Val Asp Lys Ser Ser Ser Thr Ala Tyr
65 70 75 80

Met Glu Leu Leu Ser Leu Thr Ser Glu Asp Ser Ala Val Tyr Tyr Cys
85 90 95

Ala Arg Asp Gly Tyr Asp Asp Gly Pro Tyr Thr Met Asp Tyr Trp Gly
100 105 110

Gln Gly Thr Ser Val Thr Val Ser Ser Ala Lys Thr Thr Pro Pro Ser
115 120 125

Val Tyr Pro Leu Ala Pro Gly Ser Ala Ala Gln Thr Asn Ser Met Val

```
    130                 135                 140

Thr Leu Gly Cys Leu Val Lys Gly Tyr Phe Pro Glu Pro Val Thr Val
145                 150                 155                 160

Thr Trp Asn Ser Gly Ser Leu Ser Ser Gly Val His Thr Phe Pro Ala
                165                 170                 175

Val Leu Gln Ser Asp Leu Tyr Thr Leu Ser Ser Ser Val Thr Val Pro
            180                 185                 190

Ser Ser Thr Trp Pro Ser Glu Thr Val Thr Cys Asn Val Ala His Pro
        195                 200                 205

Ala Ser Ser Thr Lys Val Asp Lys Lys Ile Val Pro Arg Asp Cys Gly
    210                 215                 220

Cys Lys Pro Cys Ile Cys Thr Val Pro Glu Val Ser Ser Val Phe Ile
225                 230                 235                 240

Phe Pro Pro Lys Pro Lys Asp Val Leu Thr Ile Thr Leu Thr Pro Lys
                245                 250                 255

Val Thr Cys Val Val Val Asp Ile Ser Lys Asp Asp Pro Glu Val Gln
            260                 265                 270

Phe Ser Trp Phe Val Asp Asp Val Glu Val His Thr Ala Gln Thr Gln
        275                 280                 285

Pro Arg Glu Glu Gln Phe Asn Ser Thr Phe Arg Ser Val Ser Glu Leu
    290                 295                 300

Pro Ile Met His Gln Asp Trp Leu Asn Gly Lys Glu Phe Lys Cys Arg
305                 310                 315                 320

Val Asn Ser Ala Ala Phe Pro Ala Pro Ile Glu Lys Thr Ile Ser Lys
                325                 330                 335

Thr Lys Gly Arg Pro Lys Ala Pro Gln Val Tyr Thr Ile Pro Pro Pro
            340                 345                 350

Lys Glu Gln Met Ala Lys Asp Lys Val Ser Leu Thr Cys Met Ile Thr
        355                 360                 365

Asp Phe Phe Pro Glu Asp Ile Thr Val Glu Trp Gln Trp Asn Gly Gln
    370                 375                 380

Pro Ala Glu Asn Tyr Lys Asn Thr Gln Pro Ile Met Asp Thr Asp Gly
385                 390                 395                 400

Ser Tyr Phe Val Tyr Ser Lys Leu Asn Val Gln Lys Ser Asn Trp Glu
                405                 410                 415

Ala Gly Asn Thr Phe Thr Cys Ser Val Leu His Glu Gly Leu His Asn
            420                 425                 430

His His Thr Glu Lys Ser Leu Ser His Ser Pro Gly Lys
        435                 440                 445
```

<210> 39
<211> 716
<212> DNA
<213> Mus musculus

<400> 39
atgagtgtgc ccactcaggt cctggggttg ctgctgctgt ggcttacagg tgccagatgt 60

gacatccaga tgactcagtc tccagcctcc ctatctgcat ctgtgggaga aactgtcacc 120

atcacatgtc gaacaagtga gaatatttac agtttttag catggtatca gcagaaacag 180

ggaaaatctc ctcacctcct ggtctataat gcaaaaacct tagcaaaagg tgtgccatca 240

aggttcagtg gcagtggatc tggcacacag ttttctctga agatcaacag cctgcagcct 300

gaagattttg ggagttattt ctgtcaacat cattatgaga gtcctctgac gttcggtgga 360

ggcaccaagc tggaaatcaa acgggctgat gctgcaccaa ctgtatccat cttcccacca 420

tccagtgagc agttaacatc tggaggtgcc tcagtcgtgt gcttcttgaa caacttctac 480

cccaaagaca tcaatgtcaa gtggaagatt gatggcagtg aacgacaaaa tggcgtcctg 540

aacagttgga ctgatcagga cagcaaagac agcacctaca gcatgagcag caccctcacg 600

ttgaccaagg acgagtatga acgacataac agctatacct gtgaggccac tcacaagaca 660

tcaacttcac ccattgtcaa gagcttcaac aggaatgagt gttaatgagc ggccgc 716

<210> 40
<211> 214
<212> PRT
<213> Mus musculus

<400> 40

Asp Ile Gln Met Thr Gln Ser Pro Ala Ser Leu Ser Ala Ser Val Gly
1 5 10 15

Glu Thr Val Thr Ile Thr Cys Arg Thr Ser Glu Asn Ile Tyr Ser Phe
20 25 30

Leu Ala Trp Tyr Gln Gln Lys Gln Gly Lys Ser Pro His Leu Leu Val
35 40 45

Tyr Asn Ala Lys Thr Leu Ala Lys Gly Val Pro Ser Arg Phe Ser Gly
50 55 60

Ser Gly Ser Gly Thr Gln Phe Ser Leu Lys Ile Asn Ser Leu Gln Pro
65 70 75 80

Glu Asp Phe Gly Ser Tyr Phe Cys Gln His His Tyr Glu Ser Pro Leu
85 90 95

Thr Phe Gly Gly Gly Thr Lys Leu Glu Ile Lys Arg Ala Asp Ala Ala
100 105 110

Pro Thr Val Ser Ile Phe Pro Pro Ser Ser Glu Gln Leu Thr Ser Gly
115 120 125

Gly Ala Ser Val Val Cys Phe Leu Asn Asn Phe Tyr Pro Lys Asp Ile
130 135 140

Asn Val Lys Trp Lys Ile Asp Gly Ser Glu Arg Gln Asn Gly Val Leu
145 150 155 160

Asn Ser Trp Thr Asp Gln Asp Ser Lys Asp Ser Thr Tyr Ser Met Ser
165 170 175

Ser Thr Leu Thr Leu Thr Lys Asp Glu Tyr Glu Arg His Asn Ser Tyr
180 185 190

Thr Cys Glu Ala Thr His Lys Thr Ser Thr Ser Pro Ile Val Lys Ser
195 200 205

Phe Asn Arg Asn Glu Cys
210

<210> 41
<211> 1406
<212> DNA
<213> Mus musculus

<400> 41
atgggatgga gctggatctt tctcttcctc ctgtcaggaa ctgcaggtgt ccactctgag 60

gtccagctgc aacagtctgg acctgagctg gtgaagcctg gaacttcaat gaagatatcc 120

tgcaaggctt ctggttactc attcactggc tacatcatga actgggtgaa gcagagccat 180

ggaaagaacc ttgagtggat tggacttatt aatccttaca atggtggtgc tgagtacaac 240

cagaagttca aggacaaggc cacattcact gtagacaagt catccagcac agcctacatg 300

gagctcctca gtctgacatc tgaagactct gcagtctatt actgtgcaag ggatggttac 360

gacgacggac cctatactat ggactactgg ggtcaaggaa cctcagtcac cgtctcctca 420

gccaaaacga cacccccatc tgtctatcca ctggcccctg gatctgctgc ccaaactaac 480

tccatggtga ccctgggatg cctggtcaag ggctatttcc ctgagccagt gacagtgacc 540

tggaactctg gatccctgtc cagcggtgtg cacaccttcc cagctgtcct gcagtctgac 600

ctctacactc tgagcagctc agtgactgtc ccctccagca cctggcccag cgagaccgtc 660

acctgcaacg ttgcccaccc ggccagcagc accaaggtgg acaagaaaat tgtgcccagg 720

gattgtggtt gtaagccttg catatgtaca gtcccagaag tatcatctgt cttcatcttc 780

cccccaaagc ccaaggatgt gctcaccatt actctgactc ctaaggtcac gtgtgttgtg 840

gtagacatca gcaaggatga tcccgaggtc cagttcagct ggtttgtaga tgatgtggag 900

gtgcacacag ctcagacgca accccgggag gagcagttca acagcacttt ccgctcagtc 960

agtgaacttc ccatcatgca ccaggactgg ctcaatggca aggagttcaa atgcagggtc 1020

aacagtgcag ctttccctgc ccccatcgag aaaaccatct ccaaaaccaa aggcagaccg 1080

aaggctccac aggtgtacac cattccacct cccaaggagc agatggccaa ggataaagtc 1140

agtctgacct gcatgataac agacttcttc cctgaagaca ttactgtgga gtggcagtgg 1200

aatgggcagc cagcggagaa ctacaagaac actcagccca tcatggacac agatggctct 1260

tacttcgtct acagcaagct caatgtgcag aagagcaact gggaggcagg aaatactttc 1320

acctgctctg tgttacatga gggcctgcac aaccaccata ctgagaagag cctctcccac 1380

tctcctggta aataatgagc ggccgc 1406

<210> 42
<211> 445
<212> PRT
<213> Mus musculus

<400> 42

Glu Val Gln Leu Gln Gln Ser Gly Pro Glu Leu Val Lys Pro Gly Thr
1 5 10 15

Ser Met Lys Ile Ser Cys Lys Ala Ser Gly Tyr Ser Phe Thr Gly Tyr
20 25 30

Ile Met Asn Trp Val Lys Gln Ser His Gly Lys Asn Leu Glu Trp Ile
35 40 45

Gly Leu Ile Asn Pro Tyr Asn Gly Gly Ala Glu Tyr Asn Gln Lys Phe
50 55 60

Lys Asp Lys Ala Thr Phe Thr Val Asp Lys Ser Ser Ser Thr Ala Tyr
65 70 75 80

Met Glu Leu Leu Ser Leu Thr Ser Glu Asp Ser Ala Val Tyr Tyr Cys
85 90 95

Ala Arg Asp Gly Tyr Asp Asp Gly Pro Tyr Thr Met Asp Tyr Trp Gly
100 105 110

Gln Gly Thr Ser Val Thr Val Ser Ser Ala Lys Thr Thr Pro Pro Ser
115 120 125

Val Tyr Pro Leu Ala Pro Gly Ser Ala Ala Gln Thr Asn Ser Met Val
130 135 140

Thr Leu Gly Cys Leu Val Lys Gly Tyr Phe Pro Glu Pro Val Thr Val
145 150 155 160

Thr Trp Asn Ser Gly Ser Leu Ser Ser Gly Val His Thr Phe Pro Ala
165 170 175

Val Leu Gln Ser Asp Leu Tyr Thr Leu Ser Ser Ser Val Thr Val Pro
180 185 190

Ser Ser Thr Trp Pro Ser Glu Thr Val Thr Cys Asn Val Ala His Pro
195 200 205

Ala Ser Ser Thr Lys Val Asp Lys Lys Ile Val Pro Arg Asp Cys Gly
210 215 220

Cys Lys Pro Cys Ile Cys Thr Val Pro Glu Val Ser Ser Val Phe Ile
225 230 235 240

Phe Pro Pro Lys Pro Lys Asp Val Leu Thr Ile Thr Leu Thr Pro Lys
245 250 255

Val Thr Cys Val Val Val Asp Ile Ser Lys Asp Asp Pro Glu Val Gln
260 265 270

Phe Ser Trp Phe Val Asp Asp Val Glu Val His Thr Ala Gln Thr Gln
275 280 285

Pro Arg Glu Glu Gln Phe Asn Ser Thr Phe Arg Ser Val Ser Glu Leu
    290                 295                 300

Pro Ile Met His Gln Asp Trp Leu Asn Gly Lys Glu Phe Lys Cys Arg
305                 310                 315                 320

Val Asn Ser Ala Ala Phe Pro Ala Pro Ile Glu Lys Thr Ile Ser Lys
                325                 330                 335

Thr Lys Gly Arg Pro Lys Ala Pro Gln Val Tyr Thr Ile Pro Pro Pro
            340                 345                 350

Lys Glu Gln Met Ala Lys Asp Lys Val Ser Leu Thr Cys Met Ile Thr
        355                 360                 365

Asp Phe Phe Pro Glu Asp Ile Thr Val Glu Trp Gln Trp Asn Gly Gln
    370                 375                 380

Pro Ala Glu Asn Tyr Lys Asn Thr Gln Pro Ile Met Asp Thr Asp Gly
385                 390                 395                 400

Ser Tyr Phe Val Tyr Ser Lys Leu Asn Val Gln Lys Ser Asn Trp Glu
                405                 410                 415

Ala Gly Asn Thr Phe Thr Cys Ser Val Leu His Glu Gly Leu His Asn
            420                 425                 430

His His Thr Glu Lys Ser Leu Ser His Ser Pro Gly Lys
        435                 440                 445

<210> 43
<211> 716
<212> DNA
<213> Mus musculus

<400> 43
atgagtgtgc ccactcaggt cctggggttg ctgctgctgt ggcttacagg tgccagatgt 60

gacatccaga tgactcagtc tccagcctcc ctatctgcat ctgtgggaga aactgtcacc 120

ttcacatgtc gagcaaatga gaatatttac agttatttag catggtatca gcagaaacag 180

ggaaaatctc ctcagctcct ggtctataat gcaaaaacct tagcagaagg tgtgccatca 240

aggttcagtg gcagtggatc aggcacacag ttttctctga agatcaacag cctgcagcct 300

gaagattttg ggagttatta ctgtcaacat cattatggaa ctcctccgac gttcggtgga 360

ggcaccaagc tggaaatcaa acgggctgat gctgcaccaa ctgtatccat cttcccacca 420

tccagtgagc agttaacatc tggaggtgcc tcagtcgtgt gcttcttgaa caacttctac 480

cccaaagaca tcaatgtcaa gtggaagatt gatggcagtg aacgacaaaa tggcgtcctg 540

aacagttgga ctgatcagga cagcaaagac agcacctaca gcatgagcag caccctcacg 600

ttgaccaagg acgagtatga acgacataac agctatacct gtgaggccac tcacaagaca 660

tcaacttcac ccattgtcaa gagcttcaac aggaatgagt gttaatgagc ggccgc 716

<210> 44
<211> 214

<212> PRT
<213> Mus musculus

<400> 44

Asp Ile Gln Met Thr Gln Ser Pro Ala Ser Leu Ser Ala Ser Val Gly
1 5 10 15

Glu Thr Val Thr Phe Thr Cys Arg Ala Asn Glu Asn Ile Tyr Ser Tyr
20 25 30

Leu Ala Trp Tyr Gln Gln Lys Gln Gly Lys Ser Pro Gln Leu Leu Val
35 40 45

Tyr Asn Ala Lys Thr Leu Ala Glu Gly Val Pro Ser Arg Phe Ser Gly
50 55 60

Ser Gly Ser Gly Thr Gln Phe Ser Leu Lys Ile Asn Ser Leu Gln Pro
65 70 75 80

Glu Asp Phe Gly Ser Tyr Tyr Cys Gln His His Tyr Gly Thr Pro Pro
85 90 95

Thr Phe Gly Gly Gly Thr Lys Leu Glu Ile Lys Arg Ala Asp Ala Ala
100 105 110

Pro Thr Val Ser Ile Phe Pro Pro Ser Ser Glu Gln Leu Thr Ser Gly
115 120 125

Gly Ala Ser Val Val Cys Phe Leu Asn Asn Phe Tyr Pro Lys Asp Ile
130 135 140

Asn Val Lys Trp Lys Ile Asp Gly Ser Glu Arg Gln Asn Gly Val Leu
145 150 155 160

Asn Ser Trp Thr Asp Gln Asp Ser Lys Asp Ser Thr Tyr Ser Met Ser
165 170 175

Ser Thr Leu Thr Leu Thr Lys Asp Glu Tyr Glu Arg His Asn Ser Tyr
180 185 190

Thr Cys Glu Ala Thr His Lys Thr Ser Thr Ser Pro Ile Val Lys Ser
195 200 205

Phe Asn Arg Asn Glu Cys
210

<210> 45
<211> 1391
<212> DNA
<213> Mus musculus

<400> 45
atggaatgta actggatact tccttttatt ctgtcggtaa tttcaggggt ctactcagag 60

gttcagctcc agcagtctgg gactgtgctg gcaaggcctg gggcttccgt gaagatgtcc 120

tgcaaggctt ctggctacac ctttaccaac tactggatgc actgggtaaa acagaggcct 180

ggacagggtc tagaatggat tggtgctatt tatcctggaa atagtgatac tgactacaac 240

cagaagttca agggcaaggc caaactgact gcagtcacat ccgccagcac tgcctacatg 300

gaactcagca gcctgacaaa tgaggactct gcggtctttt tctgtaccac tggttacgac 360

gacttcgacc actggggcca aggcaccact ctcacagtct cctcagccaa aacgacaccc 420

ccatctgtct atccactggc ccctggatct gctgcccaaa ctaactccat ggtgaccctg 480

ggatgcctgg tcaagggcta tttccctgag ccagtgacag tgacctggaa ctctggatcc 540

ctgtccagcg gtgtgcacac cttcccagct gtcctgcagt ctgacctcta cactctgagc 600

agctcagtga ctgtcccctc cagcacctgg cccagcgaga ccgtcacctg caacgttgcc 660

cacccggcca gcagcaccaa ggtggacaag aaaattgtgc ccagggattg tggttgtaag 720

ccttgcatat gtacagtccc agaagtatca tctgtcttca tcttcccccc aaagcccaag 780

gatgtgctca ccattactct gactcctaag gtcacgtgtg ttgtggtaga catcagcaag 840

gatgatcccg aggtccagtt cagctggttt gtagatgatg tggaggtgca cacagctcag 900

acgcaacccc gggaggagca gttcaacagc actttccgct cagtcagtga acttcccatc 960

atgcaccagg actggctcaa tggcaaggag ttcaaatgca gggtcaacag tgcagctttc 1020

cctgccccca tcgagaaaac catctccaaa accaaaggca gaccgaaggc tccacaggtg 1080

tacaccattc cacctcccaa ggagcagatg gccaaggata aagtcagtct gacctgcatg 1140

ataacagact tcttccctga agacattact gtggagtggc agtggaatgg gcagccagcg 1200

gagaactaca agaacactca gcccatcatg gacacagatg gctcttactt cgtctacagc 1260

aagctcaatg tgcagaagag caactgggag gcaggaaata ctttcacctg ctctgtgtta 1320

catgagggcc tgcacaacca ccatactgag aagagcctct cccactctcc tggtaaataa 1380

tgagcggccg c 1391

<210> 46
<211> 440
<212> PRT
<213> Mus musculus

<400> 46

Glu Val Gln Leu Gln Gln Ser Gly Thr Val Leu Ala Arg Pro Gly Ala
1 5 10 15

Ser Val Lys Met Ser Cys Lys Ala Ser Gly Tyr Thr Phe Thr Asn Tyr
20 25 30

Trp Met His Trp Val Lys Gln Arg Pro Gly Gln Gly Leu Glu Trp Ile
35 40 45

Gly Ala Ile Tyr Pro Gly Asn Ser Asp Thr Asp Tyr Asn Gln Lys Phe
50 55 60

Lys Gly Lys Ala Lys Leu Thr Ala Val Thr Ser Ala Ser Thr Ala Tyr
65 70 75 80

Met Glu Leu Ser Ser Leu Thr Asn Glu Asp Ser Ala Val Phe Phe Cys
85 90 95

```
Thr Thr Gly Tyr Asp Asp Phe Asp His Trp Gly Gln Gly Thr Thr Leu
            100                 105                 110

Thr Val Ser Ser Ala Lys Thr Thr Pro Pro Ser Val Tyr Pro Leu Ala
        115                 120                 125

Pro Gly Ser Ala Ala Gln Thr Asn Ser Met Val Thr Leu Gly Cys Leu
    130                 135                 140

Val Lys Gly Tyr Phe Pro Glu Pro Val Thr Val Thr Trp Asn Ser Gly
145                 150                 155                 160

Ser Leu Ser Ser Gly Val His Thr Phe Pro Ala Val Leu Gln Ser Asp
                165                 170                 175

Leu Tyr Thr Leu Ser Ser Ser Val Thr Val Pro Ser Ser Thr Trp Pro
            180                 185                 190

Ser Glu Thr Val Thr Cys Asn Val Ala His Pro Ala Ser Ser Thr Lys
        195                 200                 205

Val Asp Lys Lys Ile Val Pro Arg Asp Cys Gly Cys Lys Pro Cys Ile
    210                 215                 220

Cys Thr Val Pro Glu Val Ser Ser Val Phe Ile Phe Pro Pro Lys Pro
225                 230                 235                 240

Lys Asp Val Leu Thr Ile Thr Leu Thr Pro Lys Val Thr Cys Val Val
                245                 250                 255

Val Asp Ile Ser Lys Asp Asp Pro Glu Val Gln Phe Ser Trp Phe Val
            260                 265                 270

Asp Asp Val Glu Val His Thr Ala Gln Thr Gln Pro Arg Glu Glu Gln
        275                 280                 285

Phe Asn Ser Thr Phe Arg Ser Val Ser Glu Leu Pro Ile Met His Gln
    290                 295                 300

Asp Trp Leu Asn Gly Lys Glu Phe Lys Cys Arg Val Asn Ser Ala Ala
305                 310                 315                 320

Phe Pro Ala Pro Ile Glu Lys Thr Ile Ser Lys Thr Lys Gly Arg Pro
                325                 330                 335

Lys Ala Pro Gln Val Tyr Thr Ile Pro Pro Pro Lys Glu Gln Met Ala
            340                 345                 350

Lys Asp Lys Val Ser Leu Thr Cys Met Ile Thr Asp Phe Phe Pro Glu
        355                 360                 365

Asp Ile Thr Val Glu Trp Gln Trp Asn Gly Gln Pro Ala Glu Asn Tyr
    370                 375                 380

Lys Asn Thr Gln Pro Ile Met Asp Thr Asp Gly Ser Tyr Phe Val Tyr
385                 390                 395                 400

Ser Lys Leu Asn Val Gln Lys Ser Asn Trp Glu Ala Gly Asn Thr Phe
                405                 410                 415

Thr Cys Ser Val Leu His Glu Gly Leu His Asn His His Thr Glu Lys
            420                 425                 430
```

Ser Leu Ser His Ser Pro Gly Lys
435 440

<210> 47
<211> 725
<212> DNA
<213> Mus musculus

<400> 47
atggattttc tggtgcagat tttcagcttc ttgctaatca gtgcctcagt tgcaatgtcc 60

agaggagaaa atgtgctcac ccagtctcca gcaatcatgt ctgcatctcc aggggaaaag 120

gtcaccatga cctgcagggc cagctcaagt gtaagttcca gttacttgca ctggtaccag 180

cagaagtcag gtgcctcccc caaactctgg atttatagca cttccaactt ggcttctgga 240

gtccctgctc gcttcagtgg cagtgggtct gggacctctt actatttcac aatcagcagt 300

gtggaggctg aagatgctgc cacttattac tgccagcaat acagtggtta cccactcacg 360

ttcggagggg ggaccaagct ggaaataaaa cgggctgatg ctgcaccaac tgtatccatc 420

ttcccaccat ccagtgagca gttaacatct ggaggtgcct cagtcgtgtg cttcttgaac 480

aacttctacc ccaaagacat caatgtcaag tggaagattg atggcagtga acgacaaaat 540

ggcgtcctga acagttggac tgatcaggac agcaaagaca gcacctacag catgagcagc 600

accctcacgt tgaccaagga cgagtatgaa cgacataaca gctatacctg tgaggccact 660

cacaagacat caacttcacc cattgtcaag agcttcaaca ggaatgagtg ttaatgagcg 720

gccgc 725

<210> 48
<211> 215
<212> PRT
<213> Mus musculus

<400> 48

Glu Asn Val Leu Thr Gln Ser Pro Ala Ile Met Ser Ala Ser Pro Gly
1 5 10 15

Glu Lys Val Thr Met Thr Cys Arg Ala Ser Ser Ser Val Ser Ser Ser
20 25 30

Tyr Leu His Trp Tyr Gln Gln Lys Ser Gly Ala Ser Pro Lys Leu Trp
35 40 45

Ile Tyr Ser Thr Ser Asn Leu Ala Ser Gly Val Pro Ala Arg Phe Ser
50 55 60

Gly Ser Gly Ser Gly Thr Ser Tyr Tyr Phe Thr Ile Ser Ser Val Glu
65 70 75 80

Ala Glu Asp Ala Ala Thr Tyr Tyr Cys Gln Gln Tyr Ser Gly Tyr Pro
85 90 95

Leu Thr Phe Gly Gly Gly Thr Lys Leu Glu Ile Lys Arg Ala Asp Ala
100 105 110

```
Ala Pro Thr Val Ser Ile Phe Pro Pro Ser Ser Glu Gln Leu Thr Ser
        115                 120                 125

Gly Gly Ala Ser Val Val Cys Phe Leu Asn Asn Phe Tyr Pro Lys Asp
    130                 135                 140

Ile Asn Val Lys Trp Lys Ile Asp Gly Ser Glu Arg Gln Asn Gly Val
145                 150                 155                 160

Leu Asn Ser Trp Thr Asp Gln Asp Ser Lys Asp Ser Thr Tyr Ser Met
                165                 170                 175

Ser Ser Thr Leu Thr Leu Thr Lys Asp Glu Tyr Glu Arg His Asn Ser
            180                 185                 190

Tyr Thr Cys Glu Ala Thr His Lys Thr Ser Thr Ser Pro Ile Val Lys
        195                 200                 205

Ser Phe Asn Arg Asn Glu Cys
    210                 215

<210>  49
<211>  425
<212>  DNA
<213>  Artificial

<220>
<223>  an artificially synthesized sequence

<400>  49
ccaccatgga ctggacctgg agggtcttct gcttgctggc tgtagctcca ggtgctcact      60

cccaggtgca gctggtgcag tctggggctg aggtgaagaa gcctggggcc tcagtgaagg     120

tttcctgcaa ggcatctgga tacaccttca ccggctacat catgaactgg gtgcgacagg     180

cccctggaca agggcttgag tggatgggac ttattaatcc ttacaatggt ggtactagct     240

acaaccagaa gttcaagggc agagtcacga ttaccgcgga cgaatccacg agcacagcct     300

acatggagct gagcagcctg agatctgagg acacggccgt gtattactgt gcgagagatg     360

gttacgacga cggaccctat actatggact actggggcca gggcaccctc gtcacagtct     420

cctca                                                                 425

<210>  50
<211>  121
<212>  PRT
<213>  Artificial

<220>
<223>  an artificially synthesized sequence

<400>  50

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ile Met Asn Trp Val Arg Gln Ala Pro Gly Gln Gly Leu Glu Trp Met
```

35 40 45

Gly Leu Ile Asn Pro Tyr Asn Gly Gly Thr Ser Tyr Asn Gln Lys Phe
50 55 60

Lys Gly Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Met Asp Tyr Trp Gly
100 105 110

Gln Gly Thr Leu Val Thr Val Ser Ser
115 120

<210> 51
<211> 398
<212> DNA
<213> Artificial

<220>
<223> an artificially synthesized sequence

<400> 51
ccaccatgga catgagggtc cccgctcagc tcctggggct cctgctactc tggctccgag 60

gtgccagatg tgacatccag atgacccagt ctccatcctc cctgtctgca tctgtaggag 120

acagagtcac catcacttgc cgaacaagtg agaatattta cagtttttta gcatggtatc 180

agcagaaacc agggaaagcc cctaagctcc tgatctataa tgcaaaaacc ttagcaaaag 240

gggtcccatc aaggttcagt ggcagtggat ctgggacaga tttcactctc accatcagca 300

gtctgcaacc tgaagatttt gcaacttact actgtcaaca tcattatgag agtcctctga 360

cgttcggcgg agggaccaag gtggagatca aacgtacg 398

<210> 52
<211> 107
<212> PRT
<213> Artificial

<220>
<223> an artificially synthesized sequence

<400> 52

Asp Ile Gln Met Thr Gln Ser Pro Ser Ser Leu Ser Ala Ser Val Gly
1 5 10 15

Asp Arg Val Thr Ile Thr Cys Arg Thr Ser Glu Asn Ile Tyr Ser Phe
20 25 30

Leu Ala Trp Tyr Gln Gln Lys Pro Gly Lys Ala Pro Lys Leu Leu Ile
35 40 45

Tyr Asn Ala Lys Thr Leu Ala Lys Gly Val Pro Ser Arg Phe Ser Gly
50 55 60

Ser Gly Ser Gly Thr Asp Phe Thr Leu Thr Ile Ser Ser Leu Gln Pro

65 70 75 80

Glu Asp Phe Ala Thr Tyr Tyr Cys Gln His His Tyr Glu Ser Pro Leu
85 90 95

Thr Phe Gly Gly Gly Thr Lys Val Glu Ile Lys
100 105

<210> 53
<211> 1422
<212> DNA
<213> Artificial

<220>
<223> an artificially synthesized sequence

<400> 53
gaattccacc atggactgga cctggagggt cttctgcttg ctggctgtag ctccaggtgc 60

tcactcccag gtgcagctgg tgcagtctgg ggctgaggtg aagaagcctg gggcctcagt 120

gaaggtttcc tgcaaggcat ctggatacac cttcaccggc tacatcatga actgggtgcg 180

acaggcccct ggacaagggc ttgagtggat gggacttatt aatccttaca atggtggtac 240

tagctacaac cagaagttca agggcagagt cacgattacc gcggacgaat ccacgagcac 300

agcctacatg gagctgagca gcctgagatc tgaggacacg gccgtgtatt actgtgcgag 360

agatggttac gacgacggac cctatactat ggactactgg ggccagggca ccctcgtcac 420

agtctcctca gctagcacca agggcccatc ggtcttcccc ctggcgccct cctccaagag 480

cacctccgag agcacagcgg ccctgggctg cctggtcaag gactacttcc ccgaaccggt 540

gacggtgtcg tggaactcag gcgctctgac cagcggcgtg cacaccttcc cggctgtcct 600

acagtcctca ggactctact ccctcagcag cgtggtgacc gtgccctcca gcaacttcgg 660

cacccagacc tacacctgca acgtagatca caagcccagc aacaccaagg tggacaagac 720

agttgagcgc aaatcttgtg tcgagtgccc accgtgccca gcaccacctg tggcaggacc 780

gtcagtcttc ctcttccccc caaaacccaa ggacaccctc atgatctccc ggacccctga 840

ggtcacgtgc gtggtggtgg acgtgagcca cgaagacccc gaggtccagt tcaactggta 900

cgtggacggc gtggaggtgc ataatgccaa gacaaagcca cgggaggagc agttcaacag 960

cacgttccgt gtggtcagcg tcctcaccgt cgtgcaccag gactggctga acggcaagga 1020

gtacaagtgc aaggtctcca acaaaggcct cccagccccc atcgagaaaa ccatctccaa 1080

aaccaaaggg cagccccgag aaccacaggt gtacaccctg cccccatccc gggaggagat 1140

gaccaagaac caggtcagcc tgacctgcct ggtcaaaggc ttctacccca gcgacatcgc 1200

cgtggagtgg gagagcaatg ggcagccgga gaacaactac aagaccacac ctcccatgct 1260

ggactccgac ggctccttct tcctctacag caagctcacc gtggacaaga gcaggtggca 1320

gcaggggaac gtcttctcat gctccgtgat gcatgaggct ctgcacaacc actacacaca 1380

gaagagcctc tccctgtctc cgggtaaatg ataagcggcc gc 1422

<210> 54
<211> 447
<212> PRT
<213> Artificial

<220>
<223> an artificially synthesized sequence

<400> 54

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ile Met Asn Trp Val Arg Gln Ala Pro Gly Gln Gly Leu Glu Trp Met
35 40 45

Gly Leu Ile Asn Pro Tyr Asn Gly Gly Thr Ser Tyr Asn Gln Lys Phe
50 55 60

Lys Gly Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Met Asp Tyr Trp Gly
100 105 110

Gln Gly Thr Leu Val Thr Val Ser Ser Ala Ser Thr Lys Gly Pro Ser
115 120 125

Val Phe Pro Leu Ala Pro Ser Ser Lys Ser Thr Ser Glu Ser Thr Ala
130 135 140

Ala Leu Gly Cys Leu Val Lys Asp Tyr Phe Pro Glu Pro Val Thr Val
145 150 155 160

Ser Trp Asn Ser Gly Ala Leu Thr Ser Gly Val His Thr Phe Pro Ala
165 170 175

Val Leu Gln Ser Ser Gly Leu Tyr Ser Leu Ser Ser Val Val Thr Val
180 185 190

Pro Ser Ser Asn Phe Gly Thr Gln Thr Tyr Thr Cys Asn Val Asp His
195 200 205

Lys Pro Ser Asn Thr Lys Val Asp Lys Thr Val Glu Arg Lys Ser Cys
210 215 220

Val Glu Cys Pro Pro Cys Pro Ala Pro Pro Val Ala Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser His Glu Asp Pro Glu
260 265 270

Val Gln Phe Asn Trp Tyr Val Asp Gly Val Glu Val His Asn Ala Lys
275 280 285

Thr Lys Pro Arg Glu Glu Gln Phe Asn Ser Thr Phe Arg Val Val Ser
290 295 300

Val Leu Thr Val Val His Gln Asp Trp Leu Asn Gly Lys Glu Tyr Lys
305 310 315 320

Cys Lys Val Ser Asn Lys Gly Leu Pro Ala Pro Ile Glu Lys Thr Ile
325 330 335

Ser Lys Thr Lys Gly Gln Pro Arg Glu Pro Gln Val Tyr Thr Leu Pro
340 345 350

Pro Ser Arg Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Met Leu Asp Ser
385 390 395 400

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Lys
435 440 445

<210> 55
<211> 719
<212> DNA
<213> Artificial

<220>
<223> an artificially synthesized sequence

<400> 55
ccaccatgga catgagggtc cccgctcagc tcctggggct cctgctactc tggctccgag 60

gtgccagatg tgacatccag atgacccagt ctccatcctc cctgtctgca tctgtaggag 120

acagagtcac catcacttgc cgaacaagtg agaatattta cagtttttta gcatggtatc 180

agcagaaacc agggaaagcc cctaagctcc tgatctataa tgcaaaaacc ttagcaaaag 240

gggtcccatc aaggttcagt ggcagtggat ctgggacaga tttcactctc accatcagca 300

gtctgcaacc tgaagatttt gcaacttact actgtcaaca tcattatgag agtcctctga 360

cgttcggcgg agggaccaag gtggagatca aacgtacggt ggctgcacca tctgtcttca 420

tcttcccgcc atctgatgag cagttgaaat ctggaactgc ctctgttgtg tgcctgctga 480

ataacttcta tcccagagag gccaaagtac agtggaaggt ggataacgcc ctccaatcgg 540

gtaactccca ggagagtgtc acagagcagg acagcaagga cagcacctac agcctcagca 600

gcaccctgac gctgagcaaa gcagactacg agaaacacaa agtctacgcc tgcgaagtca 660

cccatcaggg cctgagctcg cccgtcacaa agagcttcaa caggggagag tgttgataa 719

<210> 56
<211> 214
<212> PRT
<213> Artificial

<220>
<223> an artificially synthesized sequence

<400> 56

Asp Ile Gln Met Thr Gln Ser Pro Ser Ser Leu Ser Ala Ser Val Gly
1 5 10 15

Asp Arg Val Thr Ile Thr Cys Arg Thr Ser Glu Asn Ile Tyr Ser Phe
20 25 30

Leu Ala Trp Tyr Gln Gln Lys Pro Gly Lys Ala Pro Lys Leu Leu Ile
35 40 45

Tyr Asn Ala Lys Thr Leu Ala Lys Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln His His Tyr Glu Ser Pro Leu
85 90 95

Thr Phe Gly Gly Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 57
<211> 327
<212> DNA
<213> Homo sapiens

<400> 57
cgtacggtgg ctgcaccatc tgtcttcatc ttcccgccat ctgatgagca gttgaaatct 60

ggaactgcct ctgttgtgtg cctgctgaat aacttctatc ccagagaggc caaagtacag 120

tggaaggtgg ataacgccct ccaatcgggt aactcccagg agagtgtcac agagcaggac 180

agcaaggaca gcacctacag cctcagcagc accctgacgc tgagcaaagc agactacgag 240

aaacacaaag tctacgcctg cgaagtcacc catcagggcc tgagctcgcc cgtcacaaag 300

agcttcaaca ggggagagtg ttgataa 327

<210> 58
<211> 107
<212> PRT
<213> Homo sapiens

<400> 58

Arg Thr Val Ala Ala Pro Ser Val Phe Ile Phe Pro Pro Ser Asp Glu
1 5 10 15

Gln Leu Lys Ser Gly Thr Ala Ser Val Val Cys Leu Leu Asn Asn Phe
20 25 30

Tyr Pro Arg Glu Ala Lys Val Gln Trp Lys Val Asp Asn Ala Leu Gln
35 40 45

Ser Gly Asn Ser Gln Glu Ser Val Thr Glu Gln Asp Ser Lys Asp Ser
50 55 60

Thr Tyr Ser Leu Ser Ser Thr Leu Thr Leu Ser Lys Ala Asp Tyr Glu
65 70 75 80

Lys His Lys Val Tyr Ala Cys Glu Val Thr His Gln Gly Leu Ser Ser
85 90 95

Pro Val Thr Lys Ser Phe Asn Arg Gly Glu Cys
100 105

<210> 59
<211> 990
<212> DNA
<213> Homo sapiens

<400> 59
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa 990

<210> 60
<211> 330
<212> PRT
<213> Homo sapiens

<400> 60

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Gln Thr
65 70 75 80

Tyr Ile Cys Asn Val Asn His Lys Pro Ser Asn Thr Lys Val Asp Lys
85 90 95

Lys Val Glu Pro Lys Ser Cys Asp Lys Thr His Thr Cys Pro Pro Cys
100 105 110

Pro Ala Pro Glu Leu Leu Gly Gly Pro Ser Val Phe Leu Phe Pro Pro
115 120 125

Lys Pro Lys Asp Thr Leu Met Ile Ser Arg Thr Pro Glu Val Thr Cys
130 135 140

Val Val Val Asp Val Ser His Glu Asp Pro Glu Val Lys Phe Asn Trp
145 150 155 160

Tyr Val Asp Gly Val Glu Val His Asn Ala Lys Thr Lys Pro Arg Glu
165 170 175

Glu Gln Tyr Asn Ser Thr Tyr Arg Val Val Ser Val Leu Thr Val Leu
180 185 190

His Gln Asp Trp Leu Asn Gly Lys Glu Tyr Lys Cys Lys Val Ser Asn
195 200 205

Lys Ala Leu Pro Ala Pro Ile Glu Lys Thr Ile Ser Lys Ala Lys Gly
210 215 220

Gln Pro Arg Glu Pro Gln Val Tyr Thr Leu Pro Pro Ser Arg Asp Glu
225 230 235 240

Leu Thr Lys Asn Gln Val Ser Leu Thr Cys Leu Val Lys Gly Phe Tyr
245 250 255

Pro Ser Asp Ile Ala Val Glu Trp Glu Ser Asn Gly Gln Pro Glu Asn
260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 61
<211> 984
<212> DNA
<213> Homo sapiens

<400> 61
gctagcacca agggcccatc ggtcttcccc ctggcgccct cctccaagag cacctccgag 60

agcacagcgg ccctgggctg cctggtcaag gactacttcc ccgaaccggt gacggtgtcg 120

tggaactcag gcgctctgac cagcggcgtg cacaccttcc cggctgtcct acagtcctca 180

ggactctact ccctcagcag cgtggtgacc gtgccctcca gcaacttcgg cacccagacc 240

tacacctgca acgtagatca caagcccagc aacaccaagg tggacaagac agttgagcgc 300

aaatcttgtg tcgagtgccc accgtgccca gcaccacctg tggcaggacc gtcagtcttc 360

ctcttccccc caaaacccaa ggacaccctc atgatctccc ggacccctga ggtcacgtgc 420

gtggtggtgg acgtgagcca cgaagacccc gaggtccagt tcaactggta cgtggacggc 480

gtggaggtgc ataatgccaa gacaaagcca cgggaggagc agttcaacag cacgttccgt 540

gtggtcagcg tcctcaccgt cgtgcaccag gactggctga acggcaagga gtacaagtgc 600

aaggtctcca acaaaggcct cccagccccc atcgagaaaa ccatctccaa aaccaaaggg 660

cagccccgag aaccacaggt gtacaccctg cccccatccc gggaggagat gaccaagaac 720

caggtcagcc tgacctgcct ggtcaaaggc ttctacccca gcgacatcgc cgtggagtgg 780

gagagcaatg ggcagccgga gaacaactac aagaccacac ctcccatgct ggactccgac 840

ggctccttct tcctctacag caagctcacc gtggacaaga gcaggtggca gcaggggaac 900

gtcttctcat gctccgtgat gcatgaggct ctgcacaacc actacacaca gaagagcctc 960

tccctgtctc cgggtaaatg ataa 984

<210> 62

<211> 326
<212> PRT
<213> Homo sapiens

<400> 62

```
Ala Ser Thr Lys Gly Pro Ser Val Phe Pro Leu Ala Pro Ser Ser Lys
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Asn Phe Gly Thr Gln Thr
65                  70                  75                  80

Tyr Thr Cys Asn Val Asp His Lys Pro Ser Asn Thr Lys Val Asp Lys
                85                  90                  95

Thr Val Glu Arg Lys Ser Cys Val Glu Cys Pro Pro Cys Pro Ala Pro
            100                 105                 110

Pro Val Ala Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp
    130                 135                 140

Val Ser His Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ala Pro Ile Glu Lys Thr Ile Ser Lys Thr Lys Gly Gln Pro Arg Glu
    210                 215                 220

Pro Gln Val Tyr Thr Leu Pro Pro Ser Arg Glu Glu Met Thr Lys Asn
225                 230                 235                 240

Gln Val Ser Leu Thr Cys Leu Val Lys Gly Phe Tyr Pro Ser Asp Ile
                245                 250                 255

Ala Val Glu Trp Glu Ser Asn Gly Gln Pro Glu Asn Asn Tyr Lys Thr
            260                 265                 270

Thr Pro Pro Met Leu Asp Ser Asp Gly Ser Phe Phe Leu Tyr Ser Lys
        275                 280                 285

Leu Thr Val Asp Lys Ser Arg Trp Gln Gln Gly Asn Val Phe Ser Cys
    290                 295                 300
```

Ser Val Met His Glu Ala Leu His Asn His Tyr Thr Gln Lys Ser Leu
305                 310                 315                 320

Ser Leu Ser Pro Gly Lys
                325

<210>  63
<211>  995
<212>  DNA
<213>  Homo sapiens

<400>  63
gctagcacca agggcccatc cgtcttcccc ctggcgccct gctccaggag cacctccgag      60

agcacagccg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacgaagacc     240

tacacctgca acgtagatca caagcccagc aacaccaagg tggacaagag agttgagtcc     300

aaatatggtc ccccatgccc accatgccca gcacctgagt tcctgggggg accatcagtc     360

ttcctgttcc ccccaaaacc caaggacact ctcatgatct cccggacccc tgaggtcacg     420

tgcgtggtgg tggacgtgag ccaggaagac cccgaggtcc agttcaactg gtacgtggat     480

ggcgtggagg tgcataatgc caagacaaag ccgcgggagg agcagttcaa cagcacgtac     540

cgtgtggtca gcgtcctcac cgtcctgcac caggactggc tgaacggcaa ggagtacaag     600

tgcaaggtct ccaacaaagg cctcccgtcc tccatcgaga aaaccatctc caaagccaaa     660

gggcagcccc gagagccaca ggtgtacacc ctgcccccat cccaggagga gatgaccaag     720

aaccaggtca gcctgacctg cctggtcaaa ggcttctacc ccagcgacat cgccgtggag     780

tgggagagca atgggcagcc ggagaacaac tacaagacca cgcctcccgt gctggactcc     840

gacggctcct tcttcctcta cagcaggcta accgtggaca agagcaggtg gcaggagggg     900

aatgtcttct catgctccgt gatgcatgag gctctgcaca accactacac acagaagagc     960

ctctccctgt ctctgggtta atgataagcg gccgc                                995

<210>  64
<211>  326
<212>  PRT
<213>  Homo sapiens

<400>  64

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser

50 55 60

Leu Ser Ser Val Val Thr Val Pro Ser Ser Ser Leu Gly Thr Lys Thr
65 70 75 80

Tyr Thr Cys Asn Val Asp His Lys Pro Ser Asn Thr Lys Val Asp Lys
85 90 95

Arg Val Glu Ser Lys Tyr Gly Pro Pro Cys Pro Pro Cys Pro Ala Pro
100 105 110

Glu Phe Leu Gly Gly Pro Ser Val Phe Leu Phe Pro Pro Lys Pro Lys
115 120 125

Asp Thr Leu Met Ile Ser Arg Thr Pro Glu Val Thr Cys Val Val Val
130 135 140

Asp Val Ser Gln Glu Asp Pro Glu Val Gln Phe Asn Trp Tyr Val Asp
145 150 155 160

Gly Val Glu Val His Asn Ala Lys Thr Lys Pro Arg Glu Glu Gln Phe
165 170 175

Asn Ser Thr Tyr Arg Val Val Ser Val Leu Thr Val Leu His Gln Asp
180 185 190

Trp Leu Asn Gly Lys Glu Tyr Lys Cys Lys Val Ser Asn Lys Gly Leu
195 200 205

Pro Ser Ser Ile Glu Lys Thr Ile Ser Lys Ala Lys Gly Gln Pro Arg
210 215 220

Glu Pro Gln Val Tyr Thr Leu Pro Pro Ser Gln Glu Glu Met Thr Lys
225 230 235 240

Asn Gln Val Ser Leu Thr Cys Leu Val Lys Gly Phe Tyr Pro Ser Asp
245 250 255

Ile Ala Val Glu Trp Glu Ser Asn Gly Gln Pro Glu Asn Asn Tyr Lys
260 265 270

Thr Thr Pro Pro Val Leu Asp Ser Asp Gly Ser Phe Phe Leu Tyr Ser
275 280 285

Arg Leu Thr Val Asp Lys Ser Arg Trp Gln Glu Gly Asn Val Phe Ser
290 295 300

Cys Ser Val Met His Glu Ala Leu His Asn His Tyr Thr Gln Lys Ser
305 310 315 320

Leu Ser Leu Ser Leu Gly
325

<210> 65
<211> 2208
<212> DNA
<213> Macaca fascicularis

<400> 65
atgatgtgga cctgggcact gtggatgttc cctttactct gcaaattcgg cctggcagct 60

ctgccagcta agcctgagaa catttcctgt gtctactact ataggaaaaa tttaacctgc 120

acttggagtc caggaaagga aactagttat acccagtaca cagctaagag aacttacgct 180
tttggaaaaa aacatgataa ttgtacaacc agtagttcta caagtgaaaa tcgtgcttcg 240
tgctcttttt tccttccaag aataacgatc ccagataatt ataccattga ggtggaagct 300
gaaaatggag atggtgtaat taaatctgat atgacatgtt ggagattaga ggacatagcg 360
aaaactgaac cacctgagat tttcagtgtg aaaccagttt tgggcatcaa acgaatgatt 420
cggattgaat ggataaagcc tgagttggca cctgtttcat ctgatttaaa atatgcactt 480
cgattcagga cagtcaatag taccagctgg atggaagtca acttcgctaa gaaccgtaaa 540
gatacaaacc aaacctacaa ccttatgggg ctgcaggctt ttacagagta tgtcgtagct 600
ctgcgatgtg cggtcaagga gtcaaagttc tggagtgact ggagccaaga aaaaatggga 660
atgactgagg aagaagctcc atgtggcctg gaactgtgga gagtcctgaa accaactgag 720
gtggatggaa gaaggccagt gcggttgtta tggaagaagg caagaggagc cccagtccta 780
gagaaaacac ttggctacaa catatggtac tttccagaaa acaacactaa cctcacagag 840
acagtgaaca ccactaacca gcagcttgaa ctgcatctgg gaggcgagag ctattgggtg 900
tctatgattt cttataattc tcttgggaag tctccagtga ccaccctgag gattccagcc 960
attcaggaaa agtcatttcg gtgcattgag gtcatgcagg cctgccttgc tgaggaccag 1020
ctagtggtga agtggcaaag ctctgctcta gacgtgaaca cttggatgat tgaatggttt 1080
ccggacatgg actcagagca ccccactctt tcctgggaat ctgtgtctca ggccacgaac 1140
tggacaatcc agcaagataa attaaaacct ttctggtgct ataacatctc tgtgtatcca 1200
atgttgcacg acaaagttgg cgagccatat tccatccagg cttatgccaa agaaggcatt 1260
ccatcaaaag gtcctgagac caaggtggag aacattggcg tgaagacggt cacgatcaca 1320
tggaaagaga ttcccaagag tgagagaaag ggtatcatct gcaactacac catcttttac 1380
caagctgaag gtggaaaagg attctccaag acagtcaact ccagcatctt gcagtatggc 1440
ctggagtccc tgaaacgaaa gacctcttac actgttcggg tcatggccag caccagtgct 1500
gggggaatca acgggaccag cataaatttc aagacattgt cattcagtgt ttttgagatt 1560
atccttataa cttctctgat tggtggaggc cttcttattc tcattatcct gacggtggca 1620
tatggtctca aaaaacccaa caaattgact cacctgtgtt ggcccagtgt tcccaaccct 1680
gctgaaagta gtatagccac atggcgtgga gatgatttca aggataagct aaacctgaag 1740
gagtctgatg actctgtgaa cacagaagac aggatcttaa aaccatgttc cacccccagt 1800
gacaagttgg ttattgacaa gtcggtggtg aactttggga atgttctgca agaaatgttc 1860
acagatgaag ccagaacggg tcaggaaaac aatttaggag gggaaaagaa tgagtatgtg 1920
acccacccct tcagggctga ctgtcccctg ggaaaagtt ttgaggagct cccagtttca 1980
cctgagattc ctcccagaaa atcccaatac ctacgttcga ggatgccaga agggacctgc 2040

ctagaagccg aagagcagct tctcgtttct ggtcaaagtc tagaaagtct agcaccagac 2100

catgtgcggg aggcagcggc cccaaatccg tatttgaaaa attcagtgac aaccagggaa 2160

tttcttgtgt ctcaaaaact tccagagcac accaaaggag aagtctaa 2208

<210> 66
<211> 735
<212> PRT
<213> Macaca fascicularis

<400> 66

Met Met Trp Thr Trp Ala Leu Trp Met Phe Pro Leu Leu Cys Lys Phe
1 5 10 15

Gly Leu Ala Ala Leu Pro Ala Lys Pro Glu Asn Ile Ser Cys Val Tyr
20 25 30

Tyr Tyr Arg Lys Asn Leu Thr Cys Thr Trp Ser Pro Gly Lys Glu Thr
35 40 45

Ser Tyr Thr Gln Tyr Thr Ala Lys Arg Thr Tyr Ala Phe Gly Lys Lys
50 55 60

His Asp Asn Cys Thr Thr Ser Ser Ser Thr Ser Glu Asn Arg Ala Ser
65 70 75 80

Cys Ser Phe Phe Leu Pro Arg Ile Thr Ile Pro Asp Asn Tyr Thr Ile
85 90 95

Glu Val Glu Ala Glu Asn Gly Asp Gly Val Ile Lys Ser Asp Met Thr
100 105 110

Cys Trp Arg Leu Glu Asp Ile Ala Lys Thr Glu Pro Pro Glu Ile Phe
115 120 125

Ser Val Lys Pro Val Leu Gly Ile Lys Arg Met Ile Arg Ile Glu Trp
130 135 140

Ile Lys Pro Glu Leu Ala Pro Val Ser Ser Asp Leu Lys Tyr Ala Leu
145 150 155 160

Arg Phe Arg Thr Val Asn Ser Thr Ser Trp Met Glu Val Asn Phe Ala
165 170 175

Lys Asn Arg Lys Asp Thr Asn Gln Thr Tyr Asn Leu Met Gly Leu Gln
180 185 190

Ala Phe Thr Glu Tyr Val Val Ala Leu Arg Cys Ala Val Lys Glu Ser
195 200 205

Lys Phe Trp Ser Asp Trp Ser Gln Glu Lys Met Gly Met Thr Glu Glu
210 215 220

Glu Ala Pro Cys Gly Leu Glu Leu Trp Arg Val Leu Lys Pro Thr Glu
225 230 235 240

Val Asp Gly Arg Arg Pro Val Arg Leu Leu Trp Lys Lys Ala Arg Gly
245 250 255

Ala Pro Val Leu Glu Lys Thr Leu Gly Tyr Asn Ile Trp Tyr Phe Pro

260 265 270

Glu Asn Asn Thr Asn Leu Thr Glu Thr Val Asn Thr Thr Asn Gln Gln
275 280 285

Leu Glu Leu His Leu Gly Gly Glu Ser Tyr Trp Val Ser Met Ile Ser
290 295 300

Tyr Asn Ser Leu Gly Lys Ser Pro Val Thr Thr Leu Arg Ile Pro Ala
305 310 315 320

Ile Gln Glu Lys Ser Phe Arg Cys Ile Glu Val Met Gln Ala Cys Leu
325 330 335

Ala Glu Asp Gln Leu Val Val Lys Trp Gln Ser Ser Ala Leu Asp Val
340 345 350

Asn Thr Trp Met Ile Glu Trp Phe Pro Asp Met Asp Ser Glu His Pro
355 360 365

Thr Leu Ser Trp Glu Ser Val Ser Gln Ala Thr Asn Trp Thr Ile Gln
370 375 380

Gln Asp Lys Leu Lys Pro Phe Trp Cys Tyr Asn Ile Ser Val Tyr Pro
385 390 395 400

Met Leu His Asp Lys Val Gly Glu Pro Tyr Ser Ile Gln Ala Tyr Ala
405 410 415

Lys Glu Gly Ile Pro Ser Lys Gly Pro Glu Thr Lys Val Glu Asn Ile
420 425 430

Gly Val Lys Thr Val Thr Ile Thr Trp Lys Glu Ile Pro Lys Ser Glu
435 440 445

Arg Lys Gly Ile Ile Cys Asn Tyr Thr Ile Phe Tyr Gln Ala Glu Gly
450 455 460

Gly Lys Gly Phe Ser Lys Thr Val Asn Ser Ser Ile Leu Gln Tyr Gly
465 470 475 480

Leu Glu Ser Leu Lys Arg Lys Thr Ser Tyr Thr Val Arg Val Met Ala
485 490 495

Ser Thr Ser Ala Gly Gly Ile Asn Gly Thr Ser Ile Asn Phe Lys Thr
500 505 510

Leu Ser Phe Ser Val Phe Glu Ile Ile Leu Ile Thr Ser Leu Ile Gly
515 520 525

Gly Gly Leu Leu Ile Leu Ile Ile Leu Thr Val Ala Tyr Gly Leu Lys
530 535 540

Lys Pro Asn Lys Leu Thr His Leu Cys Trp Pro Ser Val Pro Asn Pro
545 550 555 560

Ala Glu Ser Ser Ile Ala Thr Trp Arg Gly Asp Asp Phe Lys Asp Lys
565 570 575

Leu Asn Leu Lys Glu Ser Asp Asp Ser Val Asn Thr Glu Asp Arg Ile
580 585 590

Leu Lys Pro Cys Ser Thr Pro Ser Asp Lys Leu Val Ile Asp Lys Ser

595 600 605

Val Val Asn Phe Gly Asn Val Leu Gln Glu Met Phe Thr Asp Glu Ala
610 615 620

Arg Thr Gly Gln Glu Asn Asn Leu Gly Gly Glu Lys Asn Glu Tyr Val
625 630 635 640

Thr His Pro Phe Arg Ala Asp Cys Pro Leu Gly Lys Ser Phe Glu Glu
645 650 655

Leu Pro Val Ser Pro Glu Ile Pro Pro Arg Lys Ser Gln Tyr Leu Arg
660 665 670

Ser Arg Met Pro Glu Gly Thr Cys Leu Glu Ala Glu Glu Gln Leu Leu
675 680 685

Val Ser Gly Gln Ser Leu Glu Ser Leu Ala Pro Asp His Val Arg Glu
690 695 700

Ala Ala Ala Pro Asn Pro Tyr Leu Lys Asn Ser Val Thr Thr Arg Glu
705 710 715 720

Phe Leu Val Ser Gln Lys Leu Pro Glu His Thr Lys Gly Glu Val
725 730 735

<210> 67
<211> 495
<212> DNA
<213> Macaca fascicularis

<400> 67
atggcctctc actcagcagg ccccgcgacg tccgtgctgt ttctgctctg ctgcctggga 60

ggctggctga cctcccacac gttgcccgtc catttcctac aaccaagtga tatacagaaa 120

atagtcgagg aattacagtc cctctcgaag atgcttttga aagatgtgaa ggaagacaag 180

ggggtgctcg tgtcccagaa ttacacgctg ccgtgtctca cccctgacgc ccagccgcca 240

aacatcatcc acagcccagc catccgggca tatctcaaga caatcagaca gttagacaac 300

aaatctgtta ttgatgagat catagagcac ctcgacaaac tcatatttca agatgcacca 360

gaaacaaaca tttctgtgcc aacagacacc catgaatgta aacgcttcat cctgactatt 420

tctcaacagt tttcagagtg catggacctt gcattaaaat cgttgacttc tggagcccag 480

caggccacca cttaa 495

<210> 68
<211> 164
<212> PRT
<213> Macaca fascicularis

<400> 68

Met Ala Ser His Ser Ala Gly Pro Ala Thr Ser Val Leu Phe Leu Leu
1 5 10 15

Cys Cys Leu Gly Gly Trp Leu Thr Ser His Thr Leu Pro Val His Phe
20 25 30

```
Leu Gln Pro Ser Asp Ile Gln Lys Ile Val Glu Glu Leu Gln Ser Leu
        35                  40                  45

Ser Lys Met Leu Leu Lys Asp Val Lys Glu Asp Lys Gly Val Leu Val
    50                  55                  60

Ser Gln Asn Tyr Thr Leu Pro Cys Leu Thr Pro Asp Ala Gln Pro Pro
65                  70                  75                  80

Asn Ile Ile His Ser Pro Ala Ile Arg Ala Tyr Leu Lys Thr Ile Arg
                85                  90                  95

Gln Leu Asp Asn Lys Ser Val Ile Asp Glu Ile Ile Glu His Leu Asp
            100                 105                 110

Lys Leu Ile Phe Gln Asp Ala Pro Glu Thr Asn Ile Ser Val Pro Thr
        115                 120                 125

Asp Thr His Glu Cys Lys Arg Phe Ile Leu Thr Ile Ser Gln Gln Phe
    130                 135                 140

Ser Glu Cys Met Asp Leu Ala Leu Lys Ser Leu Thr Ser Gly Ala Gln
145                 150                 155                 160

Gln Ala Thr Thr


<210>  69
<211>  2934
<212>  DNA
<213>  Macaca fascicularis

<400>  69
atggctctat ttgtagtctt tcagacaaca ttcttcttaa cattgctgtc cttgaggact      60

taccagagtg aagtcttggc tgaacgttta ccattgactc ctgtgtcact taaagtttcc     120

accaattcta tacatcagag tttgcattta caatggactg tccacaacct tccttatcat     180

caggaattga aaatggtatt tcagatccag atcagtagga ttgaaacatc caatgtcgtc     240

tgggtgggga attacagcac cactgtgaag tggaaccagg ttctgcattg gagctgggaa     300

tcggaactcc ctttggaatg tgccacacac tttgtaagaa tcaagagtgt gatagacgat     360

gccagtttcc ctgagccaaa tttctggagc aactggagtt cctgggagga agtcagtgta     420

caagattatc ttggacgggg cactttgttc gttttcccta aagataagct ggtggaagaa     480

ggctccaatg ttaccatttg ttatgtttct aggaacattc aaaataatgt atcctgttat     540

ttggaaggga aacagattca cggagaacaa cttgatccac atgtaactgc attcaacttg     600

aatagtgtgc ctttcattag gaatagaggg acaaatatct attgtgaggc gagtcaagga     660

aatgtcagta aaggcataga aggcatcgtt ctctttgtct caaaagtact tgaggagccc     720

aaggactttt cttgtgaatc ccaggacttc aacactttgc actgtacttg ggatcctggg     780

acggacactg ccttggggtg gtctaaacaa ccttcccaaa gctacacttt atttgaatca     840

ttttctgggg aaaagaaact ttgtacgcac aaaaactggt gtaattggca aataactcaa     900
```

gactcacaag aaatgtataa cttcacactc atagctgaaa attacttaag gaagagaagt 960
gtcaatatcc tttttaacct gactcatcga gtttatttaa tgaatccttt tagtgtcaac 1020
tttgaaaatg taaatgccac aaatgccatc atgacctgga aggtgcactc catgaggaat 1080
aatttcacat atttgtgtca gattgaactc catggtgaag gaaaaatgat gcaatacgat 1140
gtttctatca acgtgaacgg tgagtacttc ttaagtgaac tggaacctgc cacagaatat 1200
atggcccgag tacgctgtgc tgatgccagc cacttctgga aatggactga atggagtggt 1260
cagaacttca ccacacttga agctgctccg tcagaggccc ctgatgtctg gagaagtgtg 1320
aactcagagc caggaaatca tactgtgacc ttattctgga agccattatc aaaactgcat 1380
gccaatggaa agatcctgtt ctataatgta gttgtagaaa acctagacaa accgtccagg 1440
tcagagctcc gttccattcc ggcaccagcc aacagcacaa aactaatcct cgacaggtgt 1500
tcctaccaaa tctgcgtcac agctaacaac agtgtgggcg cttctcctgc ttctataata 1560
gtcatctctg cggaccctga aaacaaagag gttgaggaag aaagaattgc aggcacagag 1620
ggtggattct ctctgtcttg gaaaccccag cctggagatg ttataggcta tgttgtggac 1680
tggtgtgacc atccccagga tgtgctccag tggaagaatg taggtcccaa taccacaagc 1740
acagtcatta gcacagatgc ttttaggcca ggagttcgat acgacttcag aatctatggg 1800
ttatctacaa aaaggattgc ttgtttatta gagaaaaaaa caggatactc tcaggaactg 1860
gctccttcag acaaccctca cgtgctggta gatatgttga catcccactc cttcactctg 1920
agttggaaag attactctac tgaatctcaa cctggtttta tacaagggta ccatgtctat 1980
ctgaaatcca aggcgaggca gtgccaccca cgatttcaaa aggcagttct ttcagatggt 2040
tcagaatgtt gcaaatacaa aattgacaac ccagaagaaa aggcattgat tgtggacaac 2100
ctaaagccag aatccttcta tgagtttttc gttactccat tcactagtgc tggcgagggc 2160
cccaatgcta cgttcacgaa ggtcacgact ccggatgaac actcctccat gttgattcgt 2220
atcctactgc ccatggtttt ctgcgtcttg ctcatcatga tcgtgtgcta cttgaaaagt 2280
cagtggatca aggagacgtg ttatcctgac atccctgacc cttacaagag cagcatcctg 2340
tcgttaataa aattcaagga gaaccctcac ctaacaataa tgaatgtcag tgactgtatc 2400
ccagatgcta ttgaagttgt cagcaagcca gaagggacaa agatacagct cctaggcact 2460
aggaagtcac tcacagaaac tgagttaact aagcctaact acctttatct ccttccaaca 2520
gaaaagaatc actctggccc tggcccctgc atctgttttg agaactttac ctacaaccag 2580
gcagcttctg acgctggctc ttgtggccat gttccagtac cccccaaagc cccaccaagt 2640
atgctaggac taatgacctc acctgaaaat gtactaaagg cgctagaaaa aaactacatg 2700
aactccctgg gagaagtccc agctggagaa acaagtttga attatgtgtc ccagttggct 2760
tcacccatgt ctggagacaa ggacagtctc ccaacaaacc cagtggagcc accacactgt 2820

tcagagtata aaatgcaaat ggcagtcccc ctgcgtcttg ccctgcctcc cccgaccgag 2880

aatagcagcc tttcctcaat taccctttta gatccaggtg aacactaccg ctaa 2934

<210> 70
<211> 977
<212> PRT
<213> Macaca fascicularis

<400> 70

Met Ala Leu Phe Val Val Phe Gln Thr Thr Phe Phe Leu Thr Leu Leu
1 5 10 15

Ser Leu Arg Thr Tyr Gln Ser Glu Val Leu Ala Glu Arg Leu Pro Leu
20 25 30

Thr Pro Val Ser Leu Lys Val Ser Thr Asn Ser Ile His Gln Ser Leu
35 40 45

His Leu Gln Trp Thr Val His Asn Leu Pro Tyr His Gln Glu Leu Lys
50 55 60

Met Val Phe Gln Ile Gln Ile Ser Arg Ile Glu Thr Ser Asn Val Val
65 70 75 80

Trp Val Gly Asn Tyr Ser Thr Thr Val Lys Trp Asn Gln Val Leu His
85 90 95

Trp Ser Trp Glu Ser Glu Leu Pro Leu Glu Cys Ala Thr His Phe Val
100 105 110

Arg Ile Lys Ser Val Ile Asp Asp Ala Ser Phe Pro Glu Pro Asn Phe
115 120 125

Trp Ser Asn Trp Ser Ser Trp Glu Glu Val Ser Val Gln Asp Tyr Leu
130 135 140

Gly Arg Gly Thr Leu Phe Val Phe Pro Lys Asp Lys Leu Val Glu Glu
145 150 155 160

Gly Ser Asn Val Thr Ile Cys Tyr Val Ser Arg Asn Ile Gln Asn Asn
165 170 175

Val Ser Cys Tyr Leu Glu Gly Lys Gln Ile His Gly Glu Gln Leu Asp
180 185 190

Pro His Val Thr Ala Phe Asn Leu Asn Ser Val Pro Phe Ile Arg Asn
195 200 205

Arg Gly Thr Asn Ile Tyr Cys Glu Ala Ser Gln Gly Asn Val Ser Lys
210 215 220

Gly Ile Glu Gly Ile Val Leu Phe Val Ser Lys Val Leu Glu Glu Pro
225 230 235 240

Lys Asp Phe Ser Cys Glu Ser Gln Asp Phe Asn Thr Leu His Cys Thr
245 250 255

Trp Asp Pro Gly Thr Asp Thr Ala Leu Gly Trp Ser Lys Gln Pro Ser
260 265 270

```
Gln Ser Tyr Thr Leu Phe Glu Ser Phe Ser Gly Glu Lys Lys Leu Cys
        275                 280                 285

Thr His Lys Asn Trp Cys Asn Trp Gln Ile Thr Gln Asp Ser Gln Glu
    290                 295                 300

Met Tyr Asn Phe Thr Leu Ile Ala Glu Asn Tyr Leu Arg Lys Arg Ser
305                 310                 315                 320

Val Asn Ile Leu Phe Asn Leu Thr His Arg Val Tyr Leu Met Asn Pro
                325                 330                 335

Phe Ser Val Asn Phe Glu Asn Val Asn Ala Thr Asn Ala Ile Met Thr
            340                 345                 350

Trp Lys Val His Ser Met Arg Asn Asn Phe Thr Tyr Leu Cys Gln Ile
        355                 360                 365

Glu Leu His Gly Glu Gly Lys Met Met Gln Tyr Asp Val Ser Ile Asn
    370                 375                 380

Val Asn Gly Glu Tyr Phe Leu Ser Glu Leu Glu Pro Ala Thr Glu Tyr
385                 390                 395                 400

Met Ala Arg Val Arg Cys Ala Asp Ala Ser His Phe Trp Lys Trp Thr
                405                 410                 415

Glu Trp Ser Gly Gln Asn Phe Thr Thr Leu Glu Ala Ala Pro Ser Glu
            420                 425                 430

Ala Pro Asp Val Trp Arg Ser Val Asn Ser Glu Pro Gly Asn His Thr
        435                 440                 445

Val Thr Leu Phe Trp Lys Pro Leu Ser Lys Leu His Ala Asn Gly Lys
    450                 455                 460

Ile Leu Phe Tyr Asn Val Val Val Glu Asn Leu Asp Lys Pro Ser Arg
465                 470                 475                 480

Ser Glu Leu Arg Ser Ile Pro Ala Pro Ala Asn Ser Thr Lys Leu Ile
                485                 490                 495

Leu Asp Arg Cys Ser Tyr Gln Ile Cys Val Thr Ala Asn Asn Ser Val
            500                 505                 510

Gly Ala Ser Pro Ala Ser Ile Ile Val Ile Ser Ala Asp Pro Glu Asn
        515                 520                 525

Lys Glu Val Glu Glu Glu Arg Ile Ala Gly Thr Glu Gly Gly Phe Ser
    530                 535                 540

Leu Ser Trp Lys Pro Gln Pro Gly Asp Val Ile Gly Tyr Val Val Asp
545                 550                 555                 560

Trp Cys Asp His Pro Gln Asp Val Leu Gln Trp Lys Asn Val Gly Pro
                565                 570                 575

Asn Thr Thr Ser Thr Val Ile Ser Thr Asp Ala Phe Arg Pro Gly Val
            580                 585                 590

Arg Tyr Asp Phe Arg Ile Tyr Gly Leu Ser Thr Lys Arg Ile Ala Cys
        595                 600                 605
```

```
Leu Leu Glu Lys Lys Thr Gly Tyr Ser Gln Glu Leu Ala Pro Ser Asp
    610                 615                 620

Asn Pro His Val Leu Val Asp Met Leu Thr Ser His Ser Phe Thr Leu
625                 630                 635                 640

Ser Trp Lys Asp Tyr Ser Thr Glu Ser Gln Pro Gly Phe Ile Gln Gly
                645                 650                 655

Tyr His Val Tyr Leu Lys Ser Lys Ala Arg Gln Cys His Pro Arg Phe
            660                 665                 670

Gln Lys Ala Val Leu Ser Asp Gly Ser Glu Cys Cys Lys Tyr Lys Ile
        675                 680                 685

Asp Asn Pro Glu Glu Lys Ala Leu Ile Val Asp Asn Leu Lys Pro Glu
    690                 695                 700

Ser Phe Tyr Glu Phe Phe Val Thr Pro Phe Thr Ser Ala Gly Glu Gly
705                 710                 715                 720

Pro Asn Ala Thr Phe Thr Lys Val Thr Thr Pro Asp Glu His Ser Ser
                725                 730                 735

Met Leu Ile Arg Ile Leu Leu Pro Met Val Phe Cys Val Leu Leu Ile
            740                 745                 750

Met Ile Val Cys Tyr Leu Lys Ser Gln Trp Ile Lys Glu Thr Cys Tyr
        755                 760                 765

Pro Asp Ile Pro Asp Pro Tyr Lys Ser Ser Ile Leu Ser Leu Ile Lys
    770                 775                 780

Phe Lys Glu Asn Pro His Leu Thr Ile Met Asn Val Ser Asp Cys Ile
785                 790                 795                 800

Pro Asp Ala Ile Glu Val Val Ser Lys Pro Glu Gly Thr Lys Ile Gln
                805                 810                 815

Leu Leu Gly Thr Arg Lys Ser Leu Thr Glu Thr Glu Leu Thr Lys Pro
            820                 825                 830

Asn Tyr Leu Tyr Leu Leu Pro Thr Glu Lys Asn His Ser Gly Pro Gly
        835                 840                 845

Pro Cys Ile Cys Phe Glu Asn Phe Thr Tyr Asn Gln Ala Ala Ser Asp
    850                 855                 860

Ala Gly Ser Cys Gly His Val Pro Val Pro Pro Lys Ala Pro Pro Ser
865                 870                 875                 880

Met Leu Gly Leu Met Thr Ser Pro Glu Asn Val Leu Lys Ala Leu Glu
                885                 890                 895

Lys Asn Tyr Met Asn Ser Leu Gly Glu Val Pro Ala Gly Glu Thr Ser
            900                 905                 910

Leu Asn Tyr Val Ser Gln Leu Ala Ser Pro Met Ser Gly Asp Lys Asp
        915                 920                 925

Ser Leu Pro Thr Asn Pro Val Glu Pro Pro His Cys Ser Glu Tyr Lys
    930                 935                 940
```

Met Gln Met Ala Val Pro Leu Arg Leu Ala Leu Pro Pro Pro Thr Glu
945 950 955 960

Asn Ser Ser Leu Ser Ser Ile Thr Leu Leu Asp Pro Gly Glu His Tyr
965 970 975

Arg

<210> 71
<211> 1665
<212> DNA
<213> Macaca fascicularis

<400> 71
atgatgtgga cctgggcact gtggatgttc cctttactct gcaaattcgg cctggcagct 60

ctgccagcta agcctgagaa catttcctgt gtctactact ataggaaaaa tttaacctgc 120

acttggagtc caggaaagga aactagttat acccagtaca cagctaagag aacttacgct 180

tttggaaaaa aacatgataa ttgtacaacc agtagttcta caagtgaaaa tcgtgcttcg 240

tgctcttttt tccttccaag aataacgatc ccagataatt ataccattga ggtggaagct 300

gaaaatggag atggtgtaat taaatctgat atgacatgtt ggagattaga ggacatagcg 360

aaaactgaac cacctgagat tttcagtgtg aaaccagttt tgggcatcaa acgaatgatt 420

cggattgaat ggataaagcc tgagttggca cctgtttcat ctgatttaaa atatgcactt 480

cgattcagga cagtcaatag taccagctgg atggaagtca acttcgctaa gaaccgtaaa 540

gatacaaacc aaacctacaa ccttatgggg ctgcaggctt ttacagagta tgtcgtagct 600

ctgcgatgtg cggtcaagga gtcaaagttc tggagtgact ggagccaaga aaaaatggga 660

atgactgagg aagaagctcc atgtggcctg gaactgtgga gagtcctgaa accaactgag 720

gtggatggaa gaaggccagt gcggttgtta tggaagaagg caagaggagc cccagtccta 780

gagaaaacac ttggctacaa catatggtac tttccagaaa acaacactaa cctcacagag 840

acagtgaaca ccactaacca gcagcttgaa ctgcatctgg gaggcgagag ctattgggtg 900

tctatgattt cttataattc tcttgggaag tctccagtga ccaccctgag gattccagcc 960

attcaggaaa agtcatttcg gtgcattgag gtcatgcagg cctgccttgc tgaggaccag 1020

ctagtggtga agtggcaaag ctctgctcta gacgtgaaca cttggatgat tgaatggttt 1080

ccggacatgg actcagagca ccccactctt tcctgggaat ctgtgtctca ggccacgaac 1140

tggacaatcc agcaagataa attaaaacct ttctggtgct ataacatctc tgtgtatcca 1200

atgttgcacg acaaagttgg cgagccatat tccatccagg cttatgccaa agaaggcatt 1260

ccatcaaaag gtcctgagac caaggtggag aacattggcg tgaagacggt cacgatcaca 1320

tggaaagaga ttcccaagag tgagagaaag ggtatcatct gcaactacac catcttttac 1380

caagctgaag gtggaaaagg attctccaag acagtcaact ccagcatctt gcagtatggc 1440

ctggagtccc tgaaacgaaa gacctcttac actgttcggg tcatggccag caccagtgct 1500

gggggaatca acgggaccag cataaatttc aagacattgt cattcagtgt ttttgagatt 1560

atccttataa cttctctgat tggtggaggc cttcttattc tcattatcct gacggtggca 1620

tatggtctca aaaaacccaa caaattgact cacctgtgtt aatga 1665

<210> 72
<211> 553
<212> PRT
<213> Macaca fascicularis

<400> 72

Met Met Trp Thr Trp Ala Leu Trp Met Phe Pro Leu Leu Cys Lys Phe
1 5 10 15

Gly Leu Ala Ala Leu Pro Ala Lys Pro Glu Asn Ile Ser Cys Val Tyr
20 25 30

Tyr Tyr Arg Lys Asn Leu Thr Cys Thr Trp Ser Pro Gly Lys Glu Thr
35 40 45

Ser Tyr Thr Gln Tyr Thr Ala Lys Arg Thr Tyr Ala Phe Gly Lys Lys
50 55 60

His Asp Asn Cys Thr Thr Ser Ser Ser Thr Ser Glu Asn Arg Ala Ser
65 70 75 80

Cys Ser Phe Phe Leu Pro Arg Ile Thr Ile Pro Asp Asn Tyr Thr Ile
85 90 95

Glu Val Glu Ala Glu Asn Gly Asp Gly Val Ile Lys Ser Asp Met Thr
100 105 110

Cys Trp Arg Leu Glu Asp Ile Ala Lys Thr Glu Pro Pro Glu Ile Phe
115 120 125

Ser Val Lys Pro Val Leu Gly Ile Lys Arg Met Ile Arg Ile Glu Trp
130 135 140

Ile Lys Pro Glu Leu Ala Pro Val Ser Ser Asp Leu Lys Tyr Ala Leu
145 150 155 160

Arg Phe Arg Thr Val Asn Ser Thr Ser Trp Met Glu Val Asn Phe Ala
165 170 175

Lys Asn Arg Lys Asp Thr Asn Gln Thr Tyr Asn Leu Met Gly Leu Gln
180 185 190

Ala Phe Thr Glu Tyr Val Val Ala Leu Arg Cys Ala Val Lys Glu Ser
195 200 205

Lys Phe Trp Ser Asp Trp Ser Gln Glu Lys Met Gly Met Thr Glu Glu
210 215 220

Glu Ala Pro Cys Gly Leu Glu Leu Trp Arg Val Leu Lys Pro Thr Glu
225 230 235 240

Val Asp Gly Arg Arg Pro Val Arg Leu Leu Trp Lys Lys Ala Arg Gly
245 250 255

Ala Pro Val Leu Glu Lys Thr Leu Gly Tyr Asn Ile Trp Tyr Phe Pro
260 265 270

Glu Asn Asn Thr Asn Leu Thr Glu Thr Val Asn Thr Thr Asn Gln Gln
275 280 285

Leu Glu Leu His Leu Gly Gly Glu Ser Tyr Trp Val Ser Met Ile Ser
290 295 300

Tyr Asn Ser Leu Gly Lys Ser Pro Val Thr Thr Leu Arg Ile Pro Ala
305 310 315 320

Ile Gln Glu Lys Ser Phe Arg Cys Ile Glu Val Met Gln Ala Cys Leu
325 330 335

Ala Glu Asp Gln Leu Val Val Lys Trp Gln Ser Ser Ala Leu Asp Val
340 345 350

Asn Thr Trp Met Ile Glu Trp Phe Pro Asp Met Asp Ser Glu His Pro
355 360 365

Thr Leu Ser Trp Glu Ser Val Ser Gln Ala Thr Asn Trp Thr Ile Gln
370 375 380

Gln Asp Lys Leu Lys Pro Phe Trp Cys Tyr Asn Ile Ser Val Tyr Pro
385 390 395 400

Met Leu His Asp Lys Val Gly Glu Pro Tyr Ser Ile Gln Ala Tyr Ala
405 410 415

Lys Glu Gly Ile Pro Ser Lys Gly Pro Glu Thr Lys Val Glu Asn Ile
420 425 430

Gly Val Lys Thr Val Thr Ile Thr Trp Lys Glu Ile Pro Lys Ser Glu
435 440 445

Arg Lys Gly Ile Ile Cys Asn Tyr Thr Ile Phe Tyr Gln Ala Glu Gly
450 455 460

Gly Lys Gly Phe Ser Lys Thr Val Asn Ser Ser Ile Leu Gln Tyr Gly
465 470 475 480

Leu Glu Ser Leu Lys Arg Lys Thr Ser Tyr Thr Val Arg Val Met Ala
485 490 495

Ser Thr Ser Ala Gly Gly Ile Asn Gly Thr Ser Ile Asn Phe Lys Thr
500 505 510

Leu Ser Phe Ser Val Phe Glu Ile Ile Leu Ile Thr Ser Leu Ile Gly
515 520 525

Gly Gly Leu Leu Ile Leu Ile Ile Leu Thr Val Ala Tyr Gly Leu Lys
530 535 540

Lys Pro Asn Lys Leu Thr His Leu Cys
545 550

<210> 73
<211> 1665
<212> DNA
<213> Homo sapiens

<400> 73

atgatgtgga cctgggcact gtggatgctc ccctcactct gcaaattcag cctggcagct 60
ctgccagcta agcctgagaa catttcctgt gtctactact ataggaaaaa tttaacctgc 120
acttggagtc caggaaagga aaccagttat acccagtaca cagttaagag aacttacgct 180
tttggagaaa aacatgataa ttgtacaacc aatagttcta caagtgaaaa tcgtgcttcg 240
tgctcttttt tccttccaag aataacgatc ccagataatt ataccattga ggtggaagct 300
gaaaatggag atggtgtaat taaatctcat atgacatact ggagattaga gaacatagcg 360
aaaactgaac cacctaagat tttccgtgtg aaaccagttt tgggcatcaa acgaatgatt 420
caaattgaat ggataaagcc tgagttggcg cctgtttcat ctgatttaaa atacacactt 480
cgattcagga cagtcaacag taccagctgg atggaagtca acttcgctaa gaaccgtaag 540
gataaaaacc aaacgtacaa cctcacgggg ctgcagcctt ttacagaata tgtcatagct 600
ctgcgatgtg cggtcaagga gtcaaagttc tggagtgact ggagccaaga aaaaatggga 660
atgactgagg aagaagctcc atgtggcctg gaactgtgga gagtcctgaa accagctgag 720
gcggatggaa gaaggccagt gcggttgtta tggaagaagg caagaggagc cccagtccta 780
gagaaaacac ttggctacaa catatggtac tatccagaaa gcaacactaa cctcacagaa 840
acaatgaaca ctactaacca gcagcttgaa ctgcatctgg gaggcgagag cttttgggtg 900
tctatgattt cttataattc tcttgggaag tctccagtgg ccaccctgag gattccagct 960
attcaagaaa aatcgtttca gtgcattgag gtcatgcagg cctgcgttgc tgaggaccag 1020
ctagtggtga agtggcaaag ctctgctcta gacgtgaaca cttggatgat tgaatggttt 1080
ccggatgtgg actcagagcc caccaccctt tcctgggaat ctgtgtctca ggccacgaac 1140
tggacgatcc agcaagataa attaaaacct ttctggtgct ataacatctc tgtgtatcca 1200
atgttgcatg acaaagttgg cgagccatat tccatccagg cttatgccaa agaaggcgtt 1260
ccatcagaag gtcctgagac caaggtggag aacattggcg tgaagacggt cacgatcaca 1320
tggaaagaga ttcccaagag tgagagaaag ggtatcatct gcaactacac catcttttac 1380
caagctgaag gtggaaaagg attctccaag acagtcaatt ccagcatctt gcagtacggc 1440
ctggagtccc tgaaacgaaa gacctcttac attgttcagg tcatggccag caccagtgct 1500
gggggaacca acgggaccag cataaatttc aagacattgt cattcagtgt ctttgagatt 1560
atcctcataa cttctctgat tggtggaggc cttcttattc tcattatcct gacagtggca 1620
tatggtctca aaaaacccaa caaattgact catctgtgtt aatga 1665

<210> 74
<211> 553
<212> PRT
<213> Homo sapiens

<400> 74

```
Met Met Trp Thr Trp Ala Leu Trp Met Leu Pro Ser Leu Cys Lys Phe
1               5                   10                  15

Ser Leu Ala Ala Leu Pro Ala Lys Pro Glu Asn Ile Ser Cys Val Tyr
            20                  25                  30

Tyr Tyr Arg Lys Asn Leu Thr Cys Thr Trp Ser Pro Gly Lys Glu Thr
        35                  40                  45

Ser Tyr Thr Gln Tyr Thr Val Lys Arg Thr Tyr Ala Phe Gly Glu Lys
    50                  55                  60

His Asp Asn Cys Thr Thr Asn Ser Ser Thr Ser Glu Asn Arg Ala Ser
65                  70                  75                  80

Cys Ser Phe Phe Leu Pro Arg Ile Thr Ile Pro Asp Asn Tyr Thr Ile
                85                  90                  95

Glu Val Glu Ala Glu Asn Gly Asp Gly Val Ile Lys Ser His Met Thr
            100                 105                 110

Tyr Trp Arg Leu Glu Asn Ile Ala Lys Thr Glu Pro Pro Lys Ile Phe
        115                 120                 125

Arg Val Lys Pro Val Leu Gly Ile Lys Arg Met Ile Gln Ile Glu Trp
    130                 135                 140

Ile Lys Pro Glu Leu Ala Pro Val Ser Ser Asp Leu Lys Tyr Thr Leu
145                 150                 155                 160

Arg Phe Arg Thr Val Asn Ser Thr Ser Trp Met Glu Val Asn Phe Ala
                165                 170                 175

Lys Asn Arg Lys Asp Lys Asn Gln Thr Tyr Asn Leu Thr Gly Leu Gln
            180                 185                 190

Pro Phe Thr Glu Tyr Val Ile Ala Leu Arg Cys Ala Val Lys Glu Ser
        195                 200                 205

Lys Phe Trp Ser Asp Trp Ser Gln Glu Lys Met Gly Met Thr Glu Glu
    210                 215                 220

Glu Ala Pro Cys Gly Leu Glu Leu Trp Arg Val Leu Lys Pro Ala Glu
225                 230                 235                 240

Ala Asp Gly Arg Arg Pro Val Arg Leu Leu Trp Lys Lys Ala Arg Gly
                245                 250                 255

Ala Pro Val Leu Glu Lys Thr Leu Gly Tyr Asn Ile Trp Tyr Tyr Pro
            260                 265                 270

Glu Ser Asn Thr Asn Leu Thr Glu Thr Met Asn Thr Thr Asn Gln Gln
        275                 280                 285

Leu Glu Leu His Leu Gly Gly Glu Ser Phe Trp Val Ser Met Ile Ser
    290                 295                 300

Tyr Asn Ser Leu Gly Lys Ser Pro Val Ala Thr Leu Arg Ile Pro Ala
305                 310                 315                 320

Ile Gln Glu Lys Ser Phe Gln Cys Ile Glu Val Met Gln Ala Cys Val
                325                 330                 335
```

Ala Glu Asp Gln Leu Val Val Lys Trp Gln Ser Ser Ala Leu Asp Val
340 345 350

Asn Thr Trp Met Ile Glu Trp Phe Pro Asp Val Asp Ser Glu Pro Thr
355 360 365

Thr Leu Ser Trp Glu Ser Val Ser Gln Ala Thr Asn Trp Thr Ile Gln
370 375 380

Gln Asp Lys Leu Lys Pro Phe Trp Cys Tyr Asn Ile Ser Val Tyr Pro
385 390 395 400

Met Leu His Asp Lys Val Gly Glu Pro Tyr Ser Ile Gln Ala Tyr Ala
405 410 415

Lys Glu Gly Val Pro Ser Glu Gly Pro Glu Thr Lys Val Glu Asn Ile
420 425 430

Gly Val Lys Thr Val Thr Ile Thr Trp Lys Glu Ile Pro Lys Ser Glu
435 440 445

Arg Lys Gly Ile Ile Cys Asn Tyr Thr Ile Phe Tyr Gln Ala Glu Gly
450 455 460

Gly Lys Gly Phe Ser Lys Thr Val Asn Ser Ser Ile Leu Gln Tyr Gly
465 470 475 480

Leu Glu Ser Leu Lys Arg Lys Thr Ser Tyr Ile Val Gln Val Met Ala
485 490 495

Ser Thr Ser Ala Gly Gly Thr Asn Gly Thr Ser Ile Asn Phe Lys Thr
500 505 510

Leu Ser Phe Ser Val Phe Glu Ile Ile Leu Ile Thr Ser Leu Ile Gly
515 520 525

Gly Gly Leu Leu Ile Leu Ile Ile Leu Thr Val Ala Tyr Gly Leu Lys
530 535 540

Lys Pro Asn Lys Leu Thr His Leu Cys
545 550

<210> 75
<211> 2199
<212> DNA
<213> Homo sapiens

<400> 75
atgatgtgga cctgggcact gtggatgctc ccctcactct gcaaattcag cctggcagct 60

ctgccagcta agcctgagaa catttcctgt gtctactact ataggaaaaa tttaacctgc 120

acttggagtc caggaaagga aaccagttat acccagtaca cagttaagag aacttacgct 180

tttggagaaa aacatgataa ttgtacaacc aatagttcta caagtgaaaa tcgtgcttcg 240

tgctcttttt tccttccaag aataacgatc ccagataatt ataccattga ggtggaagct 300

gaaaatggag atggtgtaat taaatctcat atgacatact ggagattaga gaacatagcg 360

aaaactgaac cacctaagat tttccgtgtg aaaccagttt tgggcatcaa acgaatgatt 420

caaattgaat ggataaagcc tgagttggcg cctgtttcat ctgatttaaa atacacactt 480

cgattcagga cagtcaacag taccagctgg atggaagtca acttcgctaa gaaccgtaag 540

gataaaaacc aaacgtacaa cctcacgggg ctgcagcctt ttacagaata tgtcatagct 600

ctgcgatgtg cggtcaagga gtcaaagttc tggagtgact ggagccaaga aaaaatggga 660

atgactgagg aagaagctcc atgtggcctg gaactgtgga gagtcctgaa accagctgag 720

gcggatggaa gaaggccagt gcggttgtta tggaagaagg caagaggagc cccagtccta 780

gagaaaacac ttggctacaa catatggtac tatccagaaa gcaacactaa cctcacagaa 840

acaatgaaca ctactaacca gcagcttgaa ctgcatctgg gaggcgagag cttttgggtg 900

tctatgattt cttataattc tcttgggaag tctccagtgg ccaccctgag gattccagct 960

attcaagaaa aatcgtttca gtgcattgag gtcatgcagg cctgcgttgc tgaggaccag 1020

ctagtggtga agtggcaaag ctctgctcta gacgtgaaca cttggatgat tgaatggttt 1080

ccggatgtgg actcagagcc caccaccctt tcctgggaat ctgtgtctca ggccacgaac 1140

tggacgatcc agcaagataa attaaaacct ttctggtgct ataacatctc tgtgtatcca 1200

atgttgcatg acaaagttgg cgagccatat tccatccagg cttatgccaa agaaggcgtt 1260

ccatcagaag gtcctgagac caaggtggag aacattggcg tgaagacggt cacgatcaca 1320

tggaaagaga ttcccaagag tgagagaaag ggtatcatct gcaactacac catcttttac 1380

caagctgaag gtggaaaagg attctccaag acagtcaatt ccagcatctt gcagtacggc 1440

ctggagtccc tgaaacgaaa gacctcttac attgttcagg tcatggccag caccagtgct 1500

gggggaacca acgggaccag cataaatttc aagacattgt cattcagtgt ctttgagatt 1560

atcctcataa cttctctgat tggtggaggc cttcttattc tcattatcct gacagtggca 1620

tatggtctca aaaaacccaa caaattgact catctgtgtt ggcccaccgt tcccaaccct 1680

gctgaaagta gtatagccac atggcatgga gatgatttca aggataagct aaacctgaag 1740

gagtctgatg actctgtgaa cacagaagac aggatcttaa aaccatgttc cacccccagt 1800

gacaagttgg tgattgacaa gttggtggtg aactttggga atgttctgca agaaattttc 1860

acagatgaag ccagaacggg tcaggaaaac aatttaggag gggaaaagaa tgggtatgtg 1920

acctgcccct tcaggcctga ttgtcccctg ggaaaagtt ttgaggagct cccagtttca 1980

cctgagattc cgcccagaaa atcccaatac ctacgttcga ggatgccaga ggggacccgc 2040

ccagaagcca aagagcagct tctcttttct ggtcaaagtt tagtaccaga tcatctgtgt 2100

gaggaaggag ccccaaatcc atatttgaaa aattcagtga cagccaggga atttcttgtg 2160

tctgaaaaac ttccagagca caccaaggga gaagtctaa 2199

<210> 76
<211> 732
<212> PRT
<213> Homo sapiens

<400> 76

Met Met Trp Thr Trp Ala Leu Trp Met Leu Pro Ser Leu Cys Lys Phe
1 5 10 15

Ser Leu Ala Ala Leu Pro Ala Lys Pro Glu Asn Ile Ser Cys Val Tyr
20 25 30

Tyr Tyr Arg Lys Asn Leu Thr Cys Thr Trp Ser Pro Gly Lys Glu Thr
35 40 45

Ser Tyr Thr Gln Tyr Thr Val Lys Arg Thr Tyr Ala Phe Gly Glu Lys
50 55 60

His Asp Asn Cys Thr Thr Asn Ser Ser Thr Ser Glu Asn Arg Ala Ser
65 70 75 80

Cys Ser Phe Phe Leu Pro Arg Ile Thr Ile Pro Asp Asn Tyr Thr Ile
85 90 95

Glu Val Glu Ala Glu Asn Gly Asp Gly Val Ile Lys Ser His Met Thr
100 105 110

Tyr Trp Arg Leu Glu Asn Ile Ala Lys Thr Glu Pro Pro Lys Ile Phe
115 120 125

Arg Val Lys Pro Val Leu Gly Ile Lys Arg Met Ile Gln Ile Glu Trp
130 135 140

Ile Lys Pro Glu Leu Ala Pro Val Ser Ser Asp Leu Lys Tyr Thr Leu
145 150 155 160

Arg Phe Arg Thr Val Asn Ser Thr Ser Trp Met Glu Val Asn Phe Ala
165 170 175

Lys Asn Arg Lys Asp Lys Asn Gln Thr Tyr Asn Leu Thr Gly Leu Gln
180 185 190

Pro Phe Thr Glu Tyr Val Ile Ala Leu Arg Cys Ala Val Lys Glu Ser
195 200 205

Lys Phe Trp Ser Asp Trp Ser Gln Glu Lys Met Gly Met Thr Glu Glu
210 215 220

Glu Ala Pro Cys Gly Leu Glu Leu Trp Arg Val Leu Lys Pro Ala Glu
225 230 235 240

Ala Asp Gly Arg Arg Pro Val Arg Leu Leu Trp Lys Lys Ala Arg Gly
245 250 255

Ala Pro Val Leu Glu Lys Thr Leu Gly Tyr Asn Ile Trp Tyr Tyr Pro
260 265 270

Glu Ser Asn Thr Asn Leu Thr Glu Thr Met Asn Thr Thr Asn Gln Gln
275 280 285

Leu Glu Leu His Leu Gly Gly Glu Ser Phe Trp Val Ser Met Ile Ser
290 295 300

Tyr Asn Ser Leu Gly Lys Ser Pro Val Ala Thr Leu Arg Ile Pro Ala
305 310 315 320

```
Ile Gln Glu Lys Ser Phe Gln Cys Ile Glu Val Met Gln Ala Cys Val
                325                 330                 335

Ala Glu Asp Gln Leu Val Val Lys Trp Gln Ser Ser Ala Leu Asp Val
            340                 345                 350

Asn Thr Trp Met Ile Glu Trp Phe Pro Asp Val Asp Ser Glu Pro Thr
        355                 360                 365

Thr Leu Ser Trp Glu Ser Val Ser Gln Ala Thr Asn Trp Thr Ile Gln
    370                 375                 380

Gln Asp Lys Leu Lys Pro Phe Trp Cys Tyr Asn Ile Ser Val Tyr Pro
385                 390                 395                 400

Met Leu His Asp Lys Val Gly Glu Pro Tyr Ser Ile Gln Ala Tyr Ala
                405                 410                 415

Lys Glu Gly Val Pro Ser Glu Gly Pro Glu Thr Lys Val Glu Asn Ile
            420                 425                 430

Gly Val Lys Thr Val Thr Ile Thr Trp Lys Glu Ile Pro Lys Ser Glu
        435                 440                 445

Arg Lys Gly Ile Ile Cys Asn Tyr Thr Ile Phe Tyr Gln Ala Glu Gly
    450                 455                 460

Gly Lys Gly Phe Ser Lys Thr Val Asn Ser Ser Ile Leu Gln Tyr Gly
465                 470                 475                 480

Leu Glu Ser Leu Lys Arg Lys Thr Ser Tyr Ile Val Gln Val Met Ala
                485                 490                 495

Ser Thr Ser Ala Gly Gly Thr Asn Gly Thr Ser Ile Asn Phe Lys Thr
            500                 505                 510

Leu Ser Phe Ser Val Phe Glu Ile Ile Leu Ile Thr Ser Leu Ile Gly
        515                 520                 525

Gly Gly Leu Leu Ile Leu Ile Ile Leu Thr Val Ala Tyr Gly Leu Lys
    530                 535                 540

Lys Pro Asn Lys Leu Thr His Leu Cys Trp Pro Thr Val Pro Asn Pro
545                 550                 555                 560

Ala Glu Ser Ser Ile Ala Thr Trp His Gly Asp Asp Phe Lys Asp Lys
                565                 570                 575

Leu Asn Leu Lys Glu Ser Asp Asp Ser Val Asn Thr Glu Asp Arg Ile
            580                 585                 590

Leu Lys Pro Cys Ser Thr Pro Ser Asp Lys Leu Val Ile Asp Lys Leu
        595                 600                 605

Val Val Asn Phe Gly Asn Val Leu Gln Glu Ile Phe Thr Asp Glu Ala
    610                 615                 620

Arg Thr Gly Gln Glu Asn Asn Leu Gly Gly Glu Lys Asn Gly Tyr Val
625                 630                 635                 640

Thr Cys Pro Phe Arg Pro Asp Cys Pro Leu Gly Lys Ser Phe Glu Glu
                645                 650                 655
```

Leu Pro Val Ser Pro Glu Ile Pro Pro Arg Lys Ser Gln Tyr Leu Arg
660 665 670

Ser Arg Met Pro Glu Gly Thr Arg Pro Glu Ala Lys Glu Gln Leu Leu
675 680 685

Phe Ser Gly Gln Ser Leu Val Pro Asp His Leu Cys Glu Glu Gly Ala
690 695 700

Pro Asn Pro Tyr Leu Lys Asn Ser Val Thr Ala Arg Glu Phe Leu Val
705 710 715 720

Ser Glu Lys Leu Pro Glu His Thr Lys Gly Glu Val
725 730

<210> 77
<211> 1542
<212> DNA
<213> Homo sapiens

<400> 77
atgatgtgga cctgggcact gtggatgctc ccttcactct gcaaattcag cctggcagct 60

ctgccagcta agcctgagaa catttcctgt gtctactact ataggaaaaa tttaacctgc 120

acttggagtc caggaaagga aaccagttat acccagtaca cagttaagag aacttacgct 180

tttggagaaa aacatgataa ttgtacaacc aatagttcta caagtgaaaa tcgtgcttcg 240

tgctcttttt tccttccaag aataacgatc ccagataatt ataccattga ggtggaagct 300

gaaaatggag atggtgtaat taaatctcat atgacatact ggagattaga gaacatagcg 360

aaaactgaac cacctaagat tttccgtgtg aaaccagttt tgggcatcaa acgaatgatt 420

caaattgaat ggataaagcc tgagttggcg cctgtttcat ctgatttaaa atacacactt 480

cgattcagga cagtcaacag taccagctgg atggaagtca acttcgctaa gaaccgtaag 540

gataaaaacc aaacgtacaa cctcacgggg ctgcagcctt ttacagaata tgtcatagct 600

ctgcgatgtg cggtcaagga gtcaaagttc tggagtgact ggagccaaga aaaaatggga 660

atgactgagg aagaagctcc atgtggcctg gaactgtgga gagtcctgaa accagctgag 720

gcggatggaa gaaggccagt gcggttgtta tggaagaagg caagaggagc cccagtccta 780

gagaaaacac ttggctacaa catatggtac tatccagaaa gcaacactaa cctcacagaa 840

acaatgaaca ctactaacca gcagcttgaa ctgcatctgg gaggcgagag cttttgggtg 900

tctatgattt cttataattc tcttgggaag tctccagtgg ccaccctgag gattccagct 960

attcaagaaa aatcgtttca gtgcattgag gtcatgcagg cctgcgttgc tgaggaccag 1020

ctagtggtga agtggcaaag ctctgctcta gacgtgaaca cttggatgat tgaatggttt 1080

ccggatgtgg actcagagcc caccaccctt tcctgggaat ctgtgtctca ggccacgaac 1140

tggacgatcc agcaagataa attaaaacct ttctggtgct ataacatctc tgtgtatcca 1200

atgttgcatg acaaagttgg cgagccatat tccatccagg cttatgccaa agaaggcgtt 1260

ccatcagaag gtcctgagac caaggtggag aacattggcg tgaagacggt cacgatcaca 1320

tggaaagaga ttcccaagag tgagagaaag ggtatcatct gcaactacac catcttttac 1380

caagctgaag gtggaaaagg attctccaag acagtcaatt ccagcatctt gcagtacggc 1440

ctggagtccc tgaaacgaaa gacctcttac attgttcagg tcatggccag caccagtgct 1500

gggggaacca acgggaccag cataaatttc aagacattgt ca 1542

<210> 78
<211> 514
<212> PRT
<213> Homo sapiens

<400> 78

Met Met Trp Thr Trp Ala Leu Trp Met Leu Pro Ser Leu Cys Lys Phe
1 5 10 15

Ser Leu Ala Ala Leu Pro Ala Lys Pro Glu Asn Ile Ser Cys Val Tyr
20 25 30

Tyr Tyr Arg Lys Asn Leu Thr Cys Thr Trp Ser Pro Gly Lys Glu Thr
35 40 45

Ser Tyr Thr Gln Tyr Thr Val Lys Arg Thr Tyr Ala Phe Gly Glu Lys
50 55 60

His Asp Asn Cys Thr Thr Asn Ser Ser Thr Ser Glu Asn Arg Ala Ser
65 70 75 80

Cys Ser Phe Phe Leu Pro Arg Ile Thr Ile Pro Asp Asn Tyr Thr Ile
85 90 95

Glu Val Glu Ala Glu Asn Gly Asp Gly Val Ile Lys Ser His Met Thr
100 105 110

Tyr Trp Arg Leu Glu Asn Ile Ala Lys Thr Glu Pro Pro Lys Ile Phe
115 120 125

Arg Val Lys Pro Val Leu Gly Ile Lys Arg Met Ile Gln Ile Glu Trp
130 135 140

Ile Lys Pro Glu Leu Ala Pro Val Ser Ser Asp Leu Lys Tyr Thr Leu
145 150 155 160

Arg Phe Arg Thr Val Asn Ser Thr Ser Trp Met Glu Val Asn Phe Ala
165 170 175

Lys Asn Arg Lys Asp Lys Asn Gln Thr Tyr Asn Leu Thr Gly Leu Gln
180 185 190

Pro Phe Thr Glu Tyr Val Ile Ala Leu Arg Cys Ala Val Lys Glu Ser
195 200 205

Lys Phe Trp Ser Asp Trp Ser Gln Glu Lys Met Gly Met Thr Glu Glu
210 215 220

Glu Ala Pro Cys Gly Leu Glu Leu Trp Arg Val Leu Lys Pro Ala Glu
225 230 235 240

Ala Asp Gly Arg Arg Pro Val Arg Leu Leu Trp Lys Lys Ala Arg Gly

245 250 255

Ala Pro Val Leu Glu Lys Thr Leu Gly Tyr Asn Ile Trp Tyr Tyr Pro
260 265 270

Glu Ser Asn Thr Asn Leu Thr Glu Thr Met Asn Thr Thr Asn Gln Gln
275 280 285

Leu Glu Leu His Leu Gly Gly Glu Ser Phe Trp Val Ser Met Ile Ser
290 295 300

Tyr Asn Ser Leu Gly Lys Ser Pro Val Ala Thr Leu Arg Ile Pro Ala
305 310 315 320

Ile Gln Glu Lys Ser Phe Gln Cys Ile Glu Val Met Gln Ala Cys Val
325 330 335

Ala Glu Asp Gln Leu Val Val Lys Trp Gln Ser Ser Ala Leu Asp Val
340 345 350

Asn Thr Trp Met Ile Glu Trp Phe Pro Asp Val Asp Ser Glu Pro Thr
355 360 365

Thr Leu Ser Trp Glu Ser Val Ser Gln Ala Thr Asn Trp Thr Ile Gln
370 375 380

Gln Asp Lys Leu Lys Pro Phe Trp Cys Tyr Asn Ile Ser Val Tyr Pro
385 390 395 400

Met Leu His Asp Lys Val Gly Glu Pro Tyr Ser Ile Gln Ala Tyr Ala
405 410 415

Lys Glu Gly Val Pro Ser Glu Gly Pro Glu Thr Lys Val Glu Asn Ile
420 425 430

Gly Val Lys Thr Val Thr Ile Thr Trp Lys Glu Ile Pro Lys Ser Glu
435 440 445

Arg Lys Gly Ile Ile Cys Asn Tyr Thr Ile Phe Tyr Gln Ala Glu Gly
450 455 460

Gly Lys Gly Phe Ser Lys Thr Val Asn Ser Ser Ile Leu Gln Tyr Gly
465 470 475 480

Leu Glu Ser Leu Lys Arg Lys Thr Ser Tyr Ile Val Gln Val Met Ala
485 490 495

Ser Thr Ser Ala Gly Gly Thr Asn Gly Thr Ser Ile Asn Phe Lys Thr
500 505 510

Leu Ser

<210> 79
<211> 662
<212> PRT
<213> Homo sapiens

<400> 79

Met Lys Leu Ser Pro Gln Pro Ser Cys Val Asn Leu Gly Met Met Trp
1 5 10 15

```
Thr Trp Ala Leu Trp Met Leu Pro Ser Leu Cys Lys Phe Ser Leu Ala
            20                  25                  30

Ala Leu Pro Ala Lys Pro Glu Asn Ile Ser Cys Val Tyr Tyr Tyr Arg
        35                  40                  45

Lys Asn Leu Thr Cys Thr Trp Ser Pro Gly Lys Glu Thr Ser Tyr Thr
    50                  55                  60

Gln Tyr Thr Val Lys Arg Thr Tyr Ala Phe Gly Glu Lys His Asp Asn
65                  70                  75                  80

Cys Thr Thr Asn Ser Ser Thr Ser Glu Asn Arg Ala Ser Cys Ser Phe
                85                  90                  95

Phe Leu Pro Arg Ile Thr Ile Pro Asp Asn Tyr Thr Ile Glu Val Glu
            100                 105                 110

Ala Glu Asn Gly Asp Gly Val Ile Lys Ser His Met Thr Tyr Trp Arg
        115                 120                 125

Leu Glu Asn Ile Ala Lys Thr Glu Pro Pro Lys Ile Phe Arg Val Lys
    130                 135                 140

Pro Val Leu Gly Ile Lys Arg Met Ile Gln Ile Glu Trp Ile Lys Pro
145                 150                 155                 160

Glu Leu Ala Pro Val Ser Ser Asp Leu Lys Tyr Thr Leu Arg Phe Arg
                165                 170                 175

Thr Val Asn Ser Thr Ser Trp Met Glu Val Asn Phe Ala Lys Asn Arg
            180                 185                 190

Lys Asp Lys Asn Gln Thr Tyr Asn Leu Thr Gly Leu Gln Pro Phe Thr
        195                 200                 205

Glu Tyr Val Ile Ala Leu Arg Cys Ala Val Lys Glu Ser Lys Phe Trp
    210                 215                 220

Ser Asp Trp Ser Gln Glu Lys Met Gly Met Thr Glu Glu Glu Ala Pro
225                 230                 235                 240

Cys Gly Leu Glu Leu Trp Arg Val Leu Lys Pro Ala Glu Ala Asp Gly
                245                 250                 255

Arg Arg Pro Val Arg Leu Leu Trp Lys Lys Ala Arg Gly Ala Pro Val
            260                 265                 270

Leu Glu Lys Thr Leu Gly Tyr Asn Ile Trp Tyr Tyr Pro Glu Ser Asn
        275                 280                 285

Thr Asn Leu Thr Glu Thr Met Asn Thr Thr Asn Gln Gln Leu Glu Leu
    290                 295                 300

His Leu Gly Gly Glu Ser Phe Trp Val Ser Met Ile Ser Tyr Asn Ser
305                 310                 315                 320

Leu Gly Lys Ser Pro Val Ala Thr Leu Arg Ile Pro Ala Ile Gln Glu
                325                 330                 335

Lys Ser Phe Gln Cys Ile Glu Val Met Gln Ala Cys Val Ala Glu Asp
            340                 345                 350
```

```
Gln Leu Val Val Lys Trp Gln Ser Ser Ala Leu Asp Val Asn Thr Trp
        355                 360                 365

Met Ile Glu Trp Phe Pro Asp Val Asp Ser Glu Pro Thr Thr Leu Ser
    370                 375                 380

Trp Glu Ser Val Ser Gln Ala Thr Asn Trp Thr Ile Gln Gln Asp Lys
385                 390                 395                 400

Leu Lys Pro Phe Trp Cys Tyr Asn Ile Ser Val Tyr Pro Met Leu His
                405                 410                 415

Asp Lys Val Gly Glu Pro Tyr Ser Ile Gln Ala Tyr Ala Lys Glu Gly
            420                 425                 430

Val Pro Ser Glu Gly Pro Glu Thr Lys Val Glu Asn Ile Gly Val Lys
        435                 440                 445

Thr Val Thr Ile Thr Trp Lys Glu Ile Pro Lys Ser Glu Arg Lys Gly
    450                 455                 460

Ile Ile Cys Asn Tyr Thr Ile Phe Tyr Gln Ala Glu Gly Gly Lys Gly
465                 470                 475                 480

Phe Ser Lys Thr Val Asn Ser Ser Ile Leu Gln Tyr Gly Leu Glu Ser
                485                 490                 495

Leu Lys Arg Lys Thr Ser Tyr Ile Val Gln Val Met Ala Ser Thr Ser
            500                 505                 510

Ala Gly Gly Thr Asn Gly Thr Ser Ile Asn Phe Lys Thr Leu Ser Phe
        515                 520                 525

Ser Val Phe Glu Ile Ile Leu Ile Thr Ser Leu Ile Gly Gly Gly Leu
    530                 535                 540

Leu Ile Leu Ile Ile Leu Thr Val Ala Tyr Gly Leu Lys Lys Pro Asn
545                 550                 555                 560

Lys Leu Thr His Leu Cys Trp Pro Thr Val Pro Asn Pro Ala Glu Ser
                565                 570                 575

Ser Ile Ala Thr Trp His Gly Asp Asp Phe Lys Asp Lys Leu Asn Leu
            580                 585                 590

Lys Glu Ser Asp Asp Ser Val Asn Thr Glu Asp Arg Ile Leu Lys Pro
        595                 600                 605

Cys Ser Thr Pro Ser Asp Lys Leu Val Ile Asp Lys Leu Val Val Asn
    610                 615                 620

Phe Gly Asn Val Leu Gln Glu Ile Phe Thr Asp Glu Ala Arg Thr Gly
625                 630                 635                 640

Gln Glu Asn Asn Leu Gly Gly Glu Lys Asn Gly Thr Arg Ile Leu Ser
                645                 650                 655

Ser Cys Pro Thr Ser Ile
            660

<210>  80
<211>  732
<212>  PRT
```

<213> Homo sapiens

<220>
<221> SIGNAL
<222> (1)..(20)

<220>
<221> TRANSMEM
<222> (520)..(543)

<400> 80

```
Met Met Trp Thr Trp Ala Leu Trp Met Leu Pro Ser Leu Cys Lys Phe
1               5                   10                  15

Ser Leu Ala Ala Leu Pro Ala Lys Pro Glu Asn Ile Ser Cys Val Tyr
            20                  25                  30

Tyr Tyr Arg Lys Asn Leu Thr Cys Thr Trp Ser Pro Gly Lys Glu Thr
        35                  40                  45

Ser Tyr Thr Gln Tyr Thr Val Lys Arg Thr Tyr Ala Phe Gly Glu Lys
    50                  55                  60

His Asp Asn Cys Thr Thr Asn Ser Ser Thr Ser Glu Asn Arg Ala Ser
65                  70                  75                  80

Cys Ser Phe Phe Leu Pro Arg Ile Thr Ile Pro Asp Asn Tyr Thr Ile
                85                  90                  95

Glu Val Glu Ala Glu Asn Gly Asp Gly Val Ile Lys Ser His Met Thr
            100                 105                 110

Tyr Trp Arg Leu Glu Asn Ile Ala Lys Thr Glu Pro Pro Lys Ile Phe
        115                 120                 125

Arg Val Lys Pro Val Leu Gly Ile Lys Arg Met Ile Gln Ile Glu Trp
    130                 135                 140

Ile Lys Pro Glu Leu Ala Pro Val Ser Ser Asp Leu Lys Tyr Thr Leu
145                 150                 155                 160

Arg Phe Arg Thr Val Asn Ser Thr Ser Trp Met Glu Val Asn Phe Ala
                165                 170                 175

Lys Asn Arg Lys Asp Lys Asn Gln Thr Tyr Asn Leu Thr Gly Leu Gln
            180                 185                 190

Pro Phe Thr Glu Tyr Val Ile Ala Leu Arg Cys Ala Val Lys Glu Ser
        195                 200                 205

Lys Phe Trp Ser Asp Trp Ser Gln Glu Lys Met Gly Met Thr Glu Glu
    210                 215                 220

Glu Ala Pro Cys Gly Leu Glu Leu Trp Arg Val Leu Lys Pro Ala Glu
225                 230                 235                 240

Ala Asp Gly Arg Arg Pro Val Arg Leu Leu Trp Lys Lys Ala Arg Gly
                245                 250                 255

Ala Pro Val Leu Glu Lys Thr Leu Gly Tyr Asn Ile Trp Tyr Tyr Pro
            260                 265                 270
```

Glu Ser Asn Thr Asn Leu Thr Glu Thr Met Asn Thr Thr Asn Gln Gln
275 280 285

Leu Glu Leu His Leu Gly Gly Glu Ser Phe Trp Val Ser Met Ile Ser
290 295 300

Tyr Asn Ser Leu Gly Lys Ser Pro Val Ala Thr Leu Arg Ile Pro Ala
305 310 315 320

Ile Gln Glu Lys Ser Phe Gln Cys Ile Glu Val Met Gln Ala Cys Val
325 330 335

Ala Glu Asp Gln Leu Val Val Lys Trp Gln Ser Ser Ala Leu Asp Val
340 345 350

Asn Thr Trp Met Ile Glu Trp Phe Pro Asp Val Asp Ser Glu Pro Thr
355 360 365

Thr Leu Ser Trp Glu Ser Val Ser Gln Ala Thr Asn Trp Thr Ile Gln
370 375 380

Gln Asp Lys Leu Lys Pro Phe Trp Cys Tyr Asn Ile Ser Val Tyr Pro
385 390 395 400

Met Leu His Asp Lys Val Gly Glu Pro Tyr Ser Ile Gln Ala Tyr Ala
405 410 415

Lys Glu Gly Val Pro Ser Glu Gly Pro Glu Thr Lys Val Glu Asn Ile
420 425 430

Gly Val Lys Thr Val Thr Ile Thr Trp Lys Glu Ile Pro Lys Ser Glu
435 440 445

Arg Lys Gly Ile Ile Cys Asn Tyr Thr Ile Phe Tyr Gln Ala Glu Gly
450 455 460

Gly Lys Gly Phe Ser Lys Thr Val Asn Ser Ser Ile Leu Gln Tyr Gly
465 470 475 480

Leu Glu Ser Leu Lys Arg Lys Thr Ser Tyr Ile Val Gln Val Met Ala
485 490 495

Ser Thr Ser Ala Gly Gly Thr Asn Gly Thr Ser Ile Asn Phe Lys Thr
500 505 510

Leu Ser Phe Ser Val Phe Glu Ile Ile Leu Ile Thr Ser Leu Ile Gly
515 520 525

Gly Gly Leu Leu Ile Leu Ile Ile Leu Thr Val Ala Tyr Gly Leu Lys
530 535 540

Lys Pro Asn Lys Leu Thr His Leu Cys Trp Pro Thr Val Pro Asn Pro
545 550 555 560

Ala Glu Ser Ser Ile Ala Thr Trp His Gly Asp Asp Phe Lys Asp Lys
565 570 575

Leu Asn Leu Lys Glu Ser Asp Asp Ser Val Asn Thr Glu Asp Arg Ile
580 585 590

Leu Lys Pro Cys Ser Thr Pro Ser Asp Lys Leu Val Ile Asp Lys Leu
595 600 605

Val Val Asn Phe Gly Asn Val Leu Gln Glu Ile Phe Thr Asp Glu Ala
610 615 620

Arg Thr Gly Gln Glu Asn Asn Leu Gly Gly Glu Lys Asn Gly Tyr Val
625 630 635 640

Thr Cys Pro Phe Arg Pro Asp Cys Pro Leu Gly Lys Ser Phe Glu Glu
645 650 655

Leu Pro Val Ser Pro Glu Ile Pro Pro Arg Lys Ser Gln Tyr Leu Arg
660 665 670

Ser Arg Met Pro Glu Gly Thr Arg Pro Glu Ala Lys Glu Gln Leu Leu
675 680 685

Phe Ser Gly Gln Ser Leu Val Pro Asp His Leu Cys Glu Glu Gly Ala
690 695 700

Pro Asn Pro Tyr Leu Lys Asn Ser Val Thr Ala Arg Glu Phe Leu Val
705 710 715 720

Ser Glu Lys Leu Pro Glu His Thr Lys Gly Glu Val
725 730

<210> 81
<211> 716
<212> PRT
<213> Mus musculus

<400> 81

Met Trp Thr Leu Ala Leu Trp Ala Phe Ser Phe Leu Cys Lys Phe Ser
1 5 10 15

Leu Ala Val Leu Pro Thr Lys Pro Glu Asn Ile Ser Cys Val Phe Tyr
20 25 30

Phe Asp Arg Asn Leu Thr Cys Thr Trp Arg Pro Glu Lys Glu Thr Asn
35 40 45

Asp Thr Ser Tyr Ile Val Thr Leu Thr Tyr Ser Tyr Gly Lys Ser Asn
50 55 60

Tyr Ser Asp Asn Ala Thr Glu Ala Ser Tyr Ser Phe Pro Arg Ser Cys
65 70 75 80

Ala Met Pro Pro Asp Ile Cys Ser Val Glu Val Gln Ala Gln Asn Gly
85 90 95

Asp Gly Lys Val Lys Ser Asp Ile Thr Tyr Trp His Leu Ile Ser Ile
100 105 110

Ala Lys Thr Glu Pro Pro Ile Ile Leu Ser Val Asn Pro Ile Cys Asn
115 120 125

Arg Met Phe Gln Ile Gln Trp Lys Pro Arg Glu Lys Thr Arg Gly Phe
130 135 140

Pro Leu Val Cys Met Leu Arg Phe Arg Thr Val Asn Ser Ser Arg Trp
145 150 155 160

Thr Glu Val Asn Phe Glu Asn Cys Lys Gln Val Cys Asn Leu Thr Gly
165 170 175

Leu Gln Ala Phe Thr Glu Tyr Val Leu Ala Leu Arg Phe Arg Phe Asn
180 185 190

Asp Ser Arg Tyr Trp Ser Lys Trp Ser Lys Glu Glu Thr Arg Val Thr
195 200 205

Met Glu Glu Val Pro His Val Leu Asp Leu Trp Arg Ile Leu Glu Pro
210 215 220

Ala Asp Met Asn Gly Asp Arg Lys Val Arg Leu Leu Trp Lys Lys Ala
225 230 235 240

Arg Gly Ala Pro Val Leu Glu Lys Thr Phe Gly Tyr His Ile Gln Tyr
245 250 255

Phe Ala Glu Asn Ser Thr Asn Leu Thr Glu Ile Asn Asn Ile Thr Thr
260 265 270

Gln Gln Tyr Glu Leu Leu Leu Met Ser Gln Ala His Ser Val Ser Val
275 280 285

Thr Ser Phe Asn Ser Leu Gly Lys Ser Gln Glu Ala Ile Leu Arg Ile
290 295 300

Pro Asp Val His Glu Lys Thr Phe Gln Tyr Ile Lys Ser Met Lys Ala
305 310 315 320

Tyr Ile Ala Glu Pro Leu Leu Val Val Asn Trp Gln Ser Ser Ile Pro
325 330 335

Ala Val Asp Thr Trp Ile Val Glu Trp Leu Pro Glu Ala Ala Met Ser
340 345 350

Lys Phe Pro Ala Leu Ser Trp Glu Ser Val Ser Gln Val Thr Asn Trp
355 360 365

Thr Ile Glu Gln Asp Lys Leu Lys Pro Phe Thr Cys Tyr Asn Ile Ser
370 375 380

Val Tyr Pro Val Leu Gly His Arg Val Gly Glu Pro Tyr Ser Ile Gln
385 390 395 400

Ala Tyr Ala Lys Glu Gly Thr Pro Leu Lys Gly Pro Glu Thr Arg Val
405 410 415

Glu Asn Ile Gly Leu Arg Thr Ala Thr Ile Thr Trp Lys Glu Ile Pro
420 425 430

Lys Ser Ala Arg Asn Gly Phe Ile Asn Asn Tyr Thr Val Phe Tyr Gln
435 440 445

Ala Glu Gly Gly Lys Glu Leu Ser Lys Thr Val Asn Ser His Ala Leu
450 455 460

Gln Cys Asp Leu Glu Ser Leu Thr Arg Arg Thr Ser Tyr Thr Val Trp
465 470 475 480

Val Met Ala Ser Thr Arg Ala Gly Gly Thr Asn Gly Val Arg Ile Asn
485 490 495

Phe Lys Thr Leu Ser Ile Ser Val Phe Glu Ile Val Leu Leu Thr Ser
500 505 510

Leu Val Gly Gly Gly Leu Leu Leu Leu Ser Ile Lys Thr Val Thr Phe
        515                 520                 525

Gly Leu Arg Lys Pro Asn Arg Leu Thr Pro Leu Cys Cys Pro Asp Val
    530                 535                 540

Pro Asn Pro Ala Glu Ser Ser Leu Ala Thr Trp Leu Gly Asp Gly Phe
545                 550                 555                 560

Lys Lys Ser Asn Met Lys Glu Thr Gly Asn Ser Gly Asp Thr Glu Asp
                565                 570                 575

Val Val Leu Lys Pro Cys Pro Val Pro Ala Asp Leu Ile Asp Lys Leu
            580                 585                 590

Val Val Asn Phe Glu Asn Phe Leu Glu Val Val Leu Thr Glu Glu Ala
        595                 600                 605

Gly Lys Gly Gln Ala Ser Ile Leu Gly Gly Glu Ala Asn Glu Tyr Val
    610                 615                 620

Thr Ser Pro Ser Arg Pro Asp Gly Pro Pro Gly Lys Ser Phe Lys Glu
625                 630                 635                 640

Pro Ser Val Leu Thr Glu Val Ala Ser Glu Asp Ser His Ser Thr Cys
                645                 650                 655

Ser Arg Met Ala Asp Glu Ala Tyr Ser Glu Leu Ala Arg Gln Pro Ser
            660                 665                 670

Ser Ser Cys Gln Ser Pro Gly Leu Ser Pro Pro Arg Glu Asp Gln Ala
        675                 680                 685

Gln Asn Pro Tyr Leu Lys Asn Ser Val Thr Thr Arg Glu Phe Leu Val
    690                 695                 700

His Glu Asn Ile Pro Glu His Ser Lys Gly Glu Val
705                 710                 715

<210> 82
<211> 4
<212> PRT
<213> Artificial

<220>
<223> an artificially synthesized peptide sequence

<400> 82

Gly Gly Gly Ser
1

<210> 83
<211> 4
<212> PRT
<213> Artificial

<220>
<223> an artificially synthesized peptide sequence

<400> 83

Ser Gly Gly Gly
1

<210> 84
<211> 5
<212> PRT
<213> Artificial

<220>
<223> an artificially synthesized peptide sequence

<400> 84

Gly Gly Gly Gly Ser
1 5

<210> 85
<211> 5
<212> PRT
<213> Artificial

<220>
<223> an artificially synthesized peptide sequence

<400> 85

Ser Gly Gly Gly Gly
1 5

<210> 86
<211> 6
<212> PRT
<213> Artificial

<220>
<223> an artificially synthesized peptide sequence

<400> 86

Gly Gly Gly Gly Gly Ser
1 5

<210> 87
<211> 6
<212> PRT
<213> Artificial

<220>
<223> an artificially synthesized peptide sequence

<400> 87

Ser Gly Gly Gly Gly Gly
1 5

<210> 88
<211> 7
<212> PRT
<213> Artificial

<220>
<223> an artificially synthesized peptide sequence

<400> 88

Gly Gly Gly Gly Gly Gly Ser
1 5

<210> 89
<211> 7
<212> PRT
<213> Artificial

<220>
<223> an artificially synthesized peptide sequence

<400> 89

Ser Gly Gly Gly Gly Gly Gly
1 5

<210> 90
<211> 40
<212> DNA
<213> Artificial

<220>
<223> an artificially synthesized primer sequence

<400> 90
taatagcggc cgctcattat ttaccaggag agtgggagag 40

<210> 91
<211> 40
<212> DNA
<213> Artificial

<220>
<223> an artificially synthesized primer sequence

<400> 91
taatagcggc cgctcattaa cactcattcc tgttgaagct 40

<210> 92
<211> 33
<212> DNA
<213> Artificial

<220>
<223> an artificially synthesized primer sequence

<400> 92
gacgaattcc accatgggat ggagctggat ctt 33

<210> 93
<211> 33
<212> DNA
<213> Artificial

<220>
<223> an artificially synthesized primer sequence

<400> 93
gacgaattcc accatgagtg tgcccactca ggt 33

<210> 94

<211> 33
<212> DNA
<213> Artificial

<220>
<223> an artificially synthesized primer sequence

<400> 94
gacgaattcc accatggaat gtaactggat act 33

<210> 95
<211> 33
<212> DNA
<213> Artificial

<220>
<223> an artificially synthesized primer sequence

<400> 95
gacgaattcc accatggatt ttctggtgca gat 33

<210> 96
<211> 30
<212> PRT
<213> Homo sapiens

<400> 96

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr
20 25 30

<210> 97
<211> 14
<212> PRT
<213> Homo sapiens

<400> 97

Trp Val Arg Gln Ala Pro Gly Gln Gly Leu Glu Trp Met Gly
1 5 10

<210> 98
<211> 32
<212> PRT
<213> Homo sapiens

<400> 98

Arg Val Thr Ile Thr Ala Asp Glu Ser Thr Ser Thr Ala Tyr Met Glu
1 5 10 15

Leu Ser Ser Leu Arg Ser Glu Asp Thr Ala Val Tyr Tyr Cys Ala Arg
20 25 30

<210> 99
<211> 11
<212> PRT
<213> Homo sapiens

<400> 99

Trp Gly Gln Gly Thr Leu Val Thr Val Ser Ser
1               5                   10

<210>  100
<211>  23
<212>  PRT
<213>  Homo sapiens

<400>  100

Asp Ile Gln Met Thr Gln Ser Pro Ser Ser Leu Ser Ala Ser Val Gly
1               5                   10                  15

Asp Arg Val Thr Ile Thr Cys
            20

<210>  101
<211>  15
<212>  PRT
<213>  Homo sapiens

<400>  101

Trp Tyr Gln Gln Lys Pro Gly Lys Ala Pro Lys Leu Leu Ile Tyr
1               5                   10                  15

<210>  102
<211>  32
<212>  PRT
<213>  Homo sapiens

<400>  102

Gly Val Pro Ser Arg Phe Ser Gly Ser Gly Ser Gly Thr Asp Phe Thr
1               5                   10                  15

Leu Thr Ile Ser Ser Leu Gln Pro Glu Asp Phe Ala Thr Tyr Tyr Cys
            20                  25                  30

<210>  103
<211>  10
<212>  PRT
<213>  Homo sapiens

<400>  103

Phe Gly Gly Gly Thr Lys Val Glu Ile Lys
1               5                   10

<210>  104
<211>  119
<212>  PRT
<213>  Mus musculus

<400>  104

Asp Val Gln Leu Arg Glu Ser Gly Pro Gly Leu Val Lys Pro Ser Gln
1               5                   10                  15

Ser Leu Ser Leu Thr Cys Thr Val Thr Gly Tyr Ser Ile Thr Ser Asp
            20                  25                  30

```
Tyr Ala Trp Asn Trp Ile Arg Gln Phe Pro Gly Asn Lys Leu Glu Trp
        35                  40                  45

Met Gly Tyr Ile Ser Tyr Ser Gly Ser Thr Ser Tyr Asn Pro Ser Leu
    50                  55                  60

Lys Ser Arg Val Ser Ile Thr Arg Asp Thr Ser Lys Asn Gln Phe Phe
65                  70                  75                  80

Leu Gln Leu Asn Ser Val Thr Thr Glu Asp Thr Ala Thr Tyr Tyr Cys
                85                  90                  95

Ala Pro Met Ile Thr Thr Asp Trp Phe Phe Asp Val Trp Gly Ala Gly
            100                 105                 110

Thr Thr Val Thr Val Ser Ser
        115

<210>  105
<211>  6
<212>  PRT
<213>  Mus musculus

<400>  105

Ser Asp Tyr Ala Trp Asn
1               5

<210>  106
<211>  15
<212>  PRT
<213>  Mus musculus

<400>  106

Tyr Ile Ser Tyr Ser Gly Ser Thr Ser Tyr Asn Pro Ser Leu Lys
1               5                   10                  15

<210>  107
<211>  10
<212>  PRT
<213>  Mus musculus

<400>  107

Met Ile Thr Thr Asp Trp Phe Phe Asp Val
1               5                   10

<210>  108
<211>  107
<212>  PRT
<213>  Mus musculus

<400>  108

Asp Ile Val Met Thr Gln Ser Pro Ala Thr Leu Ser Val Thr Pro Gly
1               5                   10                  15

Asp Arg Val Ser Leu Ser Cys Arg Ala Ser His Asp Ile Ser Asp Phe
            20                  25                  30

Leu His Trp Tyr Gln Gln Lys Ser His Glu Ser Pro Arg Leu Leu Ile
        35                  40                  45
```

Lys Tyr Ala Ser Gln Ser Ile Ser Gly Ile Pro Ser Arg Phe Ser Gly
    50                  55                  60

Ser Gly Ser Gly Ser Asp Phe Thr Leu Ser Ile Asn Ser Val Glu Pro
65                  70                  75                  80

Glu Asp Val Gly Val Tyr Tyr Cys Gln Asn Gly His Ser Phe Pro Trp
                85                  90                  95

Thr Phe Gly Gly Gly Thr Lys Leu Glu Ile Lys
            100                 105

<210> 109
<211> 11
<212> PRT
<213> Mus musculus

<400> 109

Arg Ala Ser His Asp Ile Ser Asp Phe Leu His
1               5                   10

<210> 110
<211> 7
<212> PRT
<213> Mus musculus

<400> 110

Tyr Ala Ser Gln Ser Ile Ser
1               5

<210> 111
<211> 9
<212> PRT
<213> Mus musculus

<400> 111

Gln Asn Gly His Ser Phe Pro Trp Thr
1               5

<210> 112
<211> 121
<212> PRT
<213> Artificial

<220>
<223> An artificially synthesized peptide sequence

<400> 112

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ile Met Asn Trp Val Arg Gln Ala Pro Gly Gln Gly Leu Glu Trp Met
        35                  40                  45

Gly Leu Ile Asn Pro Tyr Asn Gly Gly Thr Ser Tyr Asn Gln Lys Phe

50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Met Asp Tyr Trp Gly
100 105 110

Gln Gly Thr Leu Val Thr Val Ser Ser
115 120

<210> 113
<211> 17
<212> PRT
<213> Artificial

<220>
<223> An artificially synthesized peptide sequence

<400> 113

Glu Ile Asn Pro Tyr Asn Gly Gly Thr Ser Tyr Asn Gln Lys Phe Lys
1 5 10 15

Gly

<210> 114
<211> 17
<212> PRT
<213> Artificial

<220>
<223> An artificially synthesized peptide sequence

<400> 114

Leu Ile Asp Pro Tyr Asn Gly Gly Thr Ser Tyr Asn Gln Lys Phe Lys
1 5 10 15

Gly

<210> 115
<211> 17
<212> PRT
<213> Artificial

<220>
<223> An artificially synthesized peptide sequence

<400> 115

Leu Ile Asn Pro Tyr Asn Gly Gly Thr Ser Tyr Asn Asp Lys Phe Lys
1 5 10 15

Gly

<210> 116

<211> 17
<212> PRT
<213> Artificial

<220>
<223> An artificially synthesized peptide sequence

<400> 116

Leu Ile Asn Pro Tyr Asn Gly Gly Thr Ser Tyr Asn Gln Gln Phe Lys
1               5                   10                  15

Gly

<210> 117
<211> 17
<212> PRT
<213> Artificial

<220>
<223> An artificially synthesized peptide sequence

<400> 117

Leu Ile Asn Pro Tyr Asn Gly Gly Thr Ser Tyr Asn Gln Lys Phe Gln
1               5                   10                  15

Gly

<210> 118
<211> 17
<212> PRT
<213> Artificial

<220>
<223> An artificially synthesized peptide sequence

<400> 118

Leu Ile Asn Pro Tyr Asn Gly Gly Thr Ser Tyr Asn Gln Lys Phe Lys
1               5                   10                  15

Asp

<210> 119
<211> 17
<212> PRT
<213> Artificial

<220>
<223> An artificially synthesized peptide sequence

<400> 119

Leu Ile Asn Pro Tyr Asn Gly Gly Thr Ser Tyr Asn Gln Lys Phe Gln
1               5                   10                  15

Asp

<210> 120
<211> 14
<212> PRT
<213> Artificial

<220>
<223> An artificially synthesized peptide sequence

<400> 120

Trp Val Gln Gln Ser Pro Gly Gln Gly Leu Glu Trp Met Gly
1 5 10

<210> 121
<211> 11
<212> PRT
<213> Artificial

<220>
<223> An artificially synthesized peptide sequence

<400> 121

Gln Thr Ser Glu Asn Ile Tyr Ser Phe Leu Ala
1 5 10

<210> 122
<211> 11
<212> PRT
<213> Artificial

<220>
<223> An artificially synthesized peptide sequence

<400> 122

Arg Thr Ser Glu Asp Ile Tyr Ser Phe Leu Ala
1 5 10

<210> 123
<211> 7
<212> PRT
<213> Artificial

<220>
<223> An artificially synthesized peptide sequence

<400> 123

Asp Ala Lys Thr Leu Ala Lys
1 5

<210> 124
<211> 7
<212> PRT
<213> Artificial

<220>
<223> An artificially synthesized peptide sequence

<400> 124

Asn Ala Gln Thr Leu Ala Lys

1 5

<210> 125
<211> 7
<212> PRT
<213> Artificial

<220>
<223> An artificially synthesized peptide sequence

<400> 125

Asn Ala Lys Thr Glu Ala Lys
1 5

<210> 126
<211> 7
<212> PRT
<213> Artificial

<220>
<223> An artificially synthesized peptide sequence

<400> 126

Asn Ala Lys Thr Leu Ala Gln
1 5

<210> 127
<211> 7
<212> PRT
<213> Artificial

<220>
<223> An artificially synthesized peptide sequence

<400> 127

Asn Ala Lys Thr Leu Ala Asp
1 5

<210> 128
<211> 324
<212> PRT
<213> Artificial

<220>
<223> An artificially synthesized peptide sequence

<400> 128

Ala Ser Thr Lys Gly Pro Ser Val Phe Pro Leu Ala Pro Ser Ser Lys
1 5 10 15

Ser Thr Ser Gly Gly Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Asn Phe Gly Thr Gln Thr
65 70 75 80

Tyr Thr Cys Asn Val Asp His Lys Pro Ser Asn Thr Lys Val Asp Lys
85 90 95

Thr Val Glu Arg Lys Ser Cys Val Glu Cys Pro Pro Cys Pro Ala Pro
100 105 110

Pro Val Ala Gly Pro Ser Val Phe Leu Phe Pro Pro Lys Pro Lys Asp
115 120 125

Thr Leu Met Ile Ser Arg Thr Pro Glu Val Thr Cys Val Val Val Asp
130 135 140

Val Ser Gln Glu Asp Pro Glu Val Gln Phe Asn Trp Tyr Val Asp Gly
145 150 155 160

Val Glu Val His Asn Ala Lys Thr Lys Pro Arg Glu Glu Gln Phe Asn
165 170 175

Ser Thr Phe Arg Val Val Ser Val Leu Thr Val Val His Gln Asp Trp
180 185 190

Leu Asn Gly Lys Glu Tyr Lys Cys Lys Val Ser Asn Lys Gly Leu Pro
195 200 205

Ala Pro Ile Glu Lys Thr Ile Ser Lys Thr Lys Gly Gln Pro Arg Glu
210 215 220

Pro Gln Val Tyr Thr Leu Pro Pro Ser Gln Glu Glu Met Thr Lys Asn
225 230 235 240

Gln Val Ser Leu Thr Cys Leu Val Lys Gly Phe Tyr Pro Ser Asp Ile
245 250 255

Ala Val Glu Trp Glu Ser Asn Gly Gln Pro Glu Asn Asn Tyr Lys Thr
260 265 270

Thr Pro Pro Met Leu Asp Ser Asp Gly Ser Phe Phe Leu Tyr Ser Lys
275 280 285

Leu Thr Val Asp Lys Ser Arg Trp Gln Glu Gly Asn Val Phe Ser Cys
290 295 300

Ser Val Met His Glu Ala Leu His Asn His Tyr Thr Gln Lys Ser Leu
305 310 315 320

Ser Leu Ser Pro

<210> 129
<211> 326
<212> PRT
<213> Artificial

<220>
<223> An artificially synthesized peptide sequence

<400> 129

Ala Ser Thr Lys Gly Pro Ser Val Phe Pro Leu Ala Pro Ser Ser Lys
1 5 10 15

```
Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Asn Phe Gly Thr Gln Thr
65                  70                  75                  80

Tyr Thr Cys Asn Val Asp His Lys Pro Ser Asn Thr Lys Val Asp Lys
                85                  90                  95

Thr Val Glu Arg Lys Ser Cys Val Glu Cys Pro Pro Cys Pro Ala Pro
            100                 105                 110

Pro Val Ala Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp
    130                 135                 140

Val Ser His Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ser Ser Ile Glu Lys Thr Ile Ser Lys Ala Lys Gly Gln Pro Arg Glu
    210                 215                 220

Pro Gln Val Tyr Thr Leu Pro Pro Ser Arg Glu Glu Met Thr Lys Asn
225                 230                 235                 240

Gln Val Ser Leu Thr Cys Leu Val Lys Gly Phe Tyr Pro Ser Asp Ile
                245                 250                 255

Ala Val Glu Trp Glu Ser Asn Gly Gln Pro Glu Asn Asn Tyr Lys Thr
            260                 265                 270

Thr Pro Pro Val Leu Asp Ser Asp Gly Ser Phe Phe Leu Tyr Ser Lys
        275                 280                 285

Leu Thr Val Asp Lys Ser Arg Trp Gln Gln Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Pro Gly Lys
                325

<210>  130
<211>  447
```

<212> PRT
<213> Artificial

<220>
<223> An artificially synthesized peptide sequence

<400> 130

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ile Met Asn Trp Val Arg Gln Ala Pro Gly Gln Gly Leu Glu Trp Met
        35                  40                  45

Gly Leu Ile Asn Pro Tyr Asn Gly Gly Thr Ser Tyr Asn Gln Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Gly Tyr Asp Asp Gly Pro Tyr Thr Met Asp Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Glu Ser Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Asn Phe Gly Thr Gln Thr Tyr Thr Cys Asn Val Asp His
        195                 200                 205

Lys Pro Ser Asn Thr Lys Val Asp Lys Thr Val Glu Arg Lys Ser Cys
    210                 215                 220

Val Glu Cys Pro Pro Cys Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Phe Arg Val Val Ser
```

290 295 300

Val Leu Thr Val Val His Gln Asp Trp Leu Asn Gly Lys Glu Tyr Lys
305 310 315 320

Cys Lys Val Ser Asn Lys Gly Leu Pro Ala Pro Ile Glu Lys Thr Ile
325 330 335

Ser Lys Thr Lys Gly Gln Pro Arg Glu Pro Gln Val Tyr Thr Leu Pro
340 345 350

Pro Ser Arg Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Met Leu Asp Ser
385 390 395 400

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Lys
435 440 445

<210> 131
<211> 32
<212> PRT
<213> Artificial

<220>
<223> An artificially synthesized peptide sequence

<400> 131

Arg Val Thr Ile Thr Ala Asp Lys Ser Thr Ser Thr Ala Tyr Met Glu
1 5 10 15

Leu Ser Ser Leu Arg Ser Glu Asp Thr Ala Val Tyr Tyr Cys Ala Arg
20 25 30

<210> 132
<211> 326
<212> PRT
<213> Artificial

<220>
<223> An artificially synthesized peptide sequence

<400> 132

Ala Ser Thr Lys Gly Pro Ser Val Phe Pro Leu Ala Pro Cys Ser Arg
1 5 10 15

Ser Thr Ser Glu Ser Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
    50                  55                  60

Leu Ser Ser Val Val Thr Val Pro Ser Ser Asn Phe Gly Thr Gln Thr
65                  70                  75                  80

Tyr Thr Cys Asn Val Asp His Lys Pro Ser Asn Thr Lys Val Asp Lys
                85                  90                  95

Thr Val Glu Arg Lys Cys Cys Val Glu Cys Pro Pro Cys Pro Ala Pro
            100                 105                 110

Pro Val Ala Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp
    130                 135                 140

Val Ser His Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ala Pro Ile Glu Lys Thr Ile Ser Lys Thr Lys Gly Gln Pro Arg Glu
    210                 215                 220

Pro Gln Val Tyr Thr Leu Pro Pro Ser Arg Glu Glu Met Thr Lys Asn
225                 230                 235                 240

Gln Val Ser Leu Thr Cys Leu Val Lys Gly Phe Tyr Pro Ser Asp Ile
                245                 250                 255

Ala Val Glu Trp Glu Ser Asn Gly Gln Pro Glu Asn Asn Tyr Lys Thr
            260                 265                 270

Thr Pro Pro Met Leu Asp Ser Asp Gly Ser Phe Phe Leu Tyr Ser Lys
        275                 280                 285

Leu Thr Val Asp Lys Ser Arg Trp Gln Gln Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Pro Gly Lys
                325

<210>  133
<211>  451
<212>  PRT
<213>  Artificial

<220>
<223>  An artificially synthesized peptide sequence

<400> 133

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ile Met Asn Trp Val Arg Gln Ala Pro Gly Gln Gly Leu Glu Trp Met
35 40 45

Gly Leu Ile Asn Pro Tyr Asn Gly Gly Thr Ser Tyr Asn Gln Lys Phe
50 55 60

Lys Gly Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Met Asp Tyr Trp Gly
100 105 110

Gln Gly Thr Leu Val Thr Val Ser Ser Ala Ser Thr Lys Gly Pro Ser
115 120 125

Val Phe Pro Leu Ala Pro Ser Ser Lys Ser Thr Ser Gly Gly Thr Ala
130 135 140

Ala Leu Gly Cys Leu Val Lys Asp Tyr Phe Pro Glu Pro Val Thr Val
145 150 155 160

Ser Trp Asn Ser Gly Ala Leu Thr Ser Gly Val His Thr Phe Pro Ala
165 170 175

Val Leu Gln Ser Ser Gly Leu Tyr Ser Leu Ser Ser Val Val Thr Val
180 185 190

Pro Ser Ser Ser Leu Gly Thr Gln Thr Tyr Ile Cys Asn Val Asn His
195 200 205

Lys Pro Ser Asn Thr Lys Val Asp Lys Lys Val Glu Pro Lys Ser Cys
210 215 220

Asp Lys Thr His Thr Cys Pro Pro Cys Pro Ala Pro Glu Leu Leu Gly
225 230 235 240

Gly Pro Ser Val Phe Leu Phe Pro Pro Lys Pro Lys Asp Thr Leu Met
245 250 255

Ile Ser Arg Thr Pro Glu Val Thr Cys Val Val Val Asp Val Ser His
260 265 270

Glu Asp Pro Glu Val Lys Phe Asn Trp Tyr Val Asp Gly Val Glu Val
275 280 285

His Asn Ala Lys Thr Lys Pro Arg Glu Glu Gln Tyr Asn Ser Thr Tyr
290 295 300

Arg Val Val Ser Val Leu Thr Val Leu His Gln Asp Trp Leu Asn Gly
305 310 315 320

Lys Glu Tyr Lys Cys Lys Val Ser Asn Lys Ala Leu Pro Ala Pro Ile

325 330 335

Glu Lys Thr Ile Ser Lys Ala Lys Gly Gln Pro Arg Glu Pro Gln Val
340 345 350

Tyr Thr Leu Pro Pro Ser Arg Asp Glu Leu Thr Lys Asn Gln Val Ser
355 360 365

Leu Thr Cys Leu Val Lys Gly Phe Tyr Pro Ser Asp Ile Ala Val Glu
370 375 380

Trp Glu Ser Asn Gly Gln Pro Glu Asn Asn Tyr Lys Thr Thr Pro Pro
385 390 395 400

Val Leu Asp Ser Asp Gly Ser Phe Phe Leu Tyr Ser Lys Leu Thr Val
405 410 415

Asp Lys Ser Arg Trp Gln Gln Gly Asn Val Phe Ser Cys Ser Val Met
420 425 430

His Glu Ala Leu His Asn His Tyr Thr Gln Lys Ser Leu Ser Leu Ser
435 440 445

Pro Gly Lys
450

<210> 134
<211> 447
<212> PRT
<213> Artificial

<220>
<223> An artificially synthesized peptide sequence

<400> 134

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ile Met Asn Trp Val Arg Gln Ala Pro Gly Gln Gly Leu Glu Trp Met
35 40 45

Gly Leu Ile Asn Pro Tyr Asn Gly Gly Thr Ser Tyr Asn Gln Lys Phe
50 55 60

Lys Gly Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Met Asp Tyr Trp Gly
100 105 110

Gln Gly Thr Leu Val Thr Val Ser Ser Ala Ser Thr Lys Gly Pro Ser
115 120 125

Val Phe Pro Leu Ala Pro Cys Ser Arg Ser Thr Ser Glu Ser Thr Ala
130 135 140

Ala Leu Gly Cys Leu Val Lys Asp Tyr Phe Pro Glu Pro Val Thr Val
145 150 155 160

Ser Trp Asn Ser Gly Ala Leu Thr Ser Gly Val His Thr Phe Pro Ala
165 170 175

Val Leu Gln Ser Ser Gly Leu Tyr Ser Leu Ser Ser Val Val Thr Val
180 185 190

Pro Ser Ser Asn Phe Gly Thr Gln Thr Tyr Thr Cys Asn Val Asp His
195 200 205

Lys Pro Ser Asn Thr Lys Val Asp Lys Thr Val Glu Arg Lys Cys Cys
210 215 220

Val Glu Cys Pro Pro Cys Pro Ala Pro Pro Val Ala Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser His Glu Asp Pro Glu
260 265 270

Val Gln Phe Asn Trp Tyr Val Asp Gly Val Glu Val His Asn Ala Lys
275 280 285

Thr Lys Pro Arg Glu Glu Gln Phe Asn Ser Thr Phe Arg Val Val Ser
290 295 300

Val Leu Thr Val Val His Gln Asp Trp Leu Asn Gly Lys Glu Tyr Lys
305 310 315 320

Cys Lys Val Ser Asn Lys Gly Leu Pro Ala Pro Ile Glu Lys Thr Ile
325 330 335

Ser Lys Thr Lys Gly Gln Pro Arg Glu Pro Gln Val Tyr Thr Leu Pro
340 345 350

Pro Ser Arg Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Met Leu Asp Ser
385 390 395 400

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Lys
435 440 445

<210> 135
<211> 447
<212> PRT
<213> Artificial

<220>

<223> An artificially synthesized peptide sequence

<400> 135

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ile Met Asn Trp Val Arg Gln Ala Pro Gly Gln Gly Leu Glu Trp Met
35 40 45

Gly Leu Ile Asn Pro Tyr Asn Gly Gly Thr Ser Tyr Asn Gln Lys Phe
50 55 60

Lys Gly Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Met Asp Tyr Trp Gly
100 105 110

Gln Gly Thr Leu Val Thr Val Ser Ser Ala Ser Thr Lys Gly Pro Ser
115 120 125

Val Phe Pro Leu Ala Pro Ser Ser Lys Ser Thr Ser Gly Gly Thr Ala
130 135 140

Ala Leu Gly Cys Leu Val Lys Asp Tyr Phe Pro Glu Pro Val Thr Val
145 150 155 160

Ser Trp Asn Ser Gly Ala Leu Thr Ser Gly Val His Thr Phe Pro Ala
165 170 175

Val Leu Gln Ser Ser Gly Leu Tyr Ser Leu Ser Ser Val Val Thr Val
180 185 190

Pro Ser Ser Asn Phe Gly Thr Gln Thr Tyr Thr Cys Asn Val Asp His
195 200 205

Lys Pro Ser Asn Thr Lys Val Asp Lys Thr Val Glu Arg Lys Ser Cys
210 215 220

Val Glu Cys Pro Pro Cys Pro Ala Pro Pro Val Ala Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser His Glu Asp Pro Glu
260 265 270

Val Gln Phe Asn Trp Tyr Val Asp Gly Val Glu Val His Asn Ala Lys
275 280 285

Thr Lys Pro Arg Glu Glu Gln Phe Asn Ser Thr Phe Arg Val Val Ser
290 295 300

Val Leu Thr Val Val His Gln Asp Trp Leu Asn Gly Lys Glu Tyr Lys
305 310 315 320

Cys Lys Val Ser Asn Lys Gly Leu Pro Ser Ser Ile Glu Lys Thr Ile
325 330 335

Ser Lys Ala Lys Gly Gln Pro Arg Glu Pro Gln Val Tyr Thr Leu Pro
340 345 350

Pro Ser Arg Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Val Leu Asp Ser
385 390 395 400

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Lys
435 440 445

<210> 136
<211> 445
<212> PRT
<213> Artificial

<220>
<223> An artificially synthesized peptide sequence

<400> 136

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ile Met Asn Trp Val Arg Gln Ala Pro Gly Gln Gly Leu Glu Trp Met
35 40 45

Gly Leu Ile Asn Pro Tyr Asn Gly Gly Thr Ser Tyr Asn Gln Lys Phe
50 55 60

Lys Gly Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Met Asp Tyr Trp Gly
100 105 110

Gln Gly Thr Leu Val Thr Val Ser Ser Ala Ser Thr Lys Gly Pro Ser
115 120 125

Val Phe Pro Leu Ala Pro Ser Ser Lys Ser Thr Ser Gly Gly Thr Ala
130 135 140

Ala Leu Gly Cys Leu Val Lys Asp Tyr Phe Pro Glu Pro Val Thr Val

145 150 155 160

Ser Trp Asn Ser Gly Ala Leu Thr Ser Gly Val His Thr Phe Pro Ala
165 170 175

Val Leu Gln Ser Ser Gly Leu Tyr Ser Leu Ser Ser Val Val Thr Val
180 185 190

Pro Ser Ser Asn Phe Gly Thr Gln Thr Tyr Thr Cys Asn Val Asp His
195 200 205

Lys Pro Ser Asn Thr Lys Val Asp Lys Thr Val Glu Arg Lys Ser Cys
210 215 220

Val Glu Cys Pro Pro Cys Pro Ala Pro Pro Val Ala Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser Gln Glu Asp Pro Glu
260 265 270

Val Gln Phe Asn Trp Tyr Val Asp Gly Val Glu Val His Asn Ala Lys
275 280 285

Thr Lys Pro Arg Glu Glu Gln Phe Asn Ser Thr Phe Arg Val Val Ser
290 295 300

Val Leu Thr Val Val His Gln Asp Trp Leu Asn Gly Lys Glu Tyr Lys
305 310 315 320

Cys Lys Val Ser Asn Lys Gly Leu Pro Ala Pro Ile Glu Lys Thr Ile
325 330 335

Ser Lys Thr Lys Gly Gln Pro Arg Glu Pro Gln Val Tyr Thr Leu Pro
340 345 350

Pro Ser Gln Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Met Leu Asp Ser
385 390 395 400

Asp Gly Ser Phe Phe Leu Tyr Ser Lys Leu Thr Val Asp Lys Ser Arg
405 410 415

Trp Gln Glu Gly Asn Val Phe Ser Cys Ser Val Met His Glu Ala Leu
420 425 430

His Asn His Tyr Thr Gln Lys Ser Leu Ser Leu Ser Pro
435 440 445

<210> 137
<211> 121
<212> PRT
<213> Artificial

<220>

<223> An artificially synthesized peptide sequence

<400> 137

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ile Met Asn Trp Val Gln Gln Ser Pro Gly Gln Gly Leu Glu Trp Met
        35                  40                  45

Gly Leu Ile Asn Pro Tyr Asn Gly Gly Thr Ser Tyr Asn Gln Lys Phe
    50                  55                  60

Lys Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Gly Tyr Asp Asp Gly Pro Tyr Thr Met Asp Tyr Trp Gly
            100                 105                 110

Gln Gly Thr Leu Val Thr Val Ser Ser
        115                 120

<210> 138
<211> 121
<212> PRT
<213> Artificial

<220>
<223> An artificially synthesized peptide sequence

<400> 138

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ile Met Asn Trp Val Arg Gln Ala Pro Gly Gln Gly Leu Glu Trp Met
        35                  40                  45

Gly Glu Ile Asn Pro Tyr Asn Gly Gly Thr Ser Tyr Asn Gln Lys Phe
    50                  55                  60

Lys Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

```
Ala Arg Asp Gly Tyr Asp Asp Gly Pro Tyr Thr Met Asp Tyr Trp Gly
            100                 105                 110

Gln Gly Thr Leu Val Thr Val Ser Ser
        115                 120
```

<210> 139
<211> 121
<212> PRT
<213> Artificial

<220>
<223> An artificially synthesized peptide sequence

<400> 139

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ile Met Asn Trp Val Arg Gln Ala Pro Gly Gln Gly Leu Glu Trp Met
        35                  40                  45

Gly Leu Ile Asp Pro Tyr Asn Gly Gly Thr Ser Tyr Asn Gln Lys Phe
    50                  55                  60

Lys Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Gly Tyr Asp Asp Gly Pro Tyr Thr Met Asp Tyr Trp Gly
            100                 105                 110

Gln Gly Thr Leu Val Thr Val Ser Ser
        115                 120
```

<210> 140
<211> 121
<212> PRT
<213> Artificial

<220>
<223> An artificially synthesized peptide sequence

<400> 140

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ile Met Asn Trp Val Arg Gln Ala Pro Gly Gln Gly Leu Glu Trp Met
        35                  40                  45

Gly Leu Ile Asn Pro Tyr Asn Gly Gly Thr Ser Tyr Asn Asp Lys Phe
    50                  55                  60

Lys Gly Arg Val Thr Ile Thr Ala Asp Glu Ser Thr Ser Thr Ala Tyr
```

65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Met Asp Tyr Trp Gly
100 105 110

Gln Gly Thr Leu Val Thr Val Ser Ser
115 120

<210> 141
<211> 121
<212> PRT
<213> Artificial

<220>
<223> An artificially synthesized peptide sequence

<400> 141

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ile Met Asn Trp Val Arg Gln Ala Pro Gly Gln Gly Leu Glu Trp Met
35 40 45

Gly Leu Ile Asn Pro Tyr Asn Gly Gly Thr Ser Tyr Asn Gln Gln Phe
50 55 60

Lys Gly Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Met Asp Tyr Trp Gly
100 105 110

Gln Gly Thr Leu Val Thr Val Ser Ser
115 120

<210> 142
<211> 121
<212> PRT
<213> Artificial

<220>
<223> An artificially synthesized peptide sequence

<400> 142

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ile Met Asn Trp Val Arg Gln Ala Pro Gly Gln Gly Leu Glu Trp Met
35 40 45

Gly Leu Ile Asn Pro Tyr Asn Gly Gly Thr Ser Tyr Asn Gln Lys Phe
    50                  55                  60

Gln Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Gly Tyr Asp Asp Gly Pro Tyr Thr Met Asp Tyr Trp Gly
            100                 105                 110

Gln Gly Thr Leu Val Thr Val Ser Ser
        115                 120

<210>  143
<211>  121
<212>  PRT
<213>  Artificial

<220>
<223>  An artificially synthesized peptide sequence

<400>  143

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ile Met Asn Trp Val Arg Gln Ala Pro Gly Gln Gly Leu Glu Trp Met
        35                  40                  45

Gly Leu Ile Asn Pro Tyr Asn Gly Gly Thr Ser Tyr Asn Gln Lys Phe
    50                  55                  60

Gln Asp Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Gly Tyr Asp Asp Gly Pro Tyr Thr Met Asp Tyr Trp Gly
            100                 105                 110

Gln Gly Thr Leu Val Thr Val Ser Ser
        115                 120

<210>  144
<211>  107
<212>  PRT
<213>  Artificial

<220>
<223>  An artificially synthesized peptide sequence

<400>  144

Asp Ile Gln Met Thr Gln Ser Pro Ser Ser Leu Ser Ala Ser Val Gly
1               5                   10                  15

```
Asp Arg Val Thr Ile Thr Cys Gln Thr Ser Glu Asn Ile Tyr Ser Phe
            20                  25                  30

Leu Ala Trp Tyr Gln Gln Lys Pro Gly Lys Ala Pro Lys Leu Leu Ile
        35                  40                  45

Tyr Asn Ala Lys Thr Leu Ala Lys Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln His His Tyr Glu Ser Pro Leu
                85                  90                  95

Thr Phe Gly Gly Gly Thr Lys Val Glu Ile Lys
            100                 105

<210>  145
<211>  107
<212>  PRT
<213>  Artificial

<220>
<223>  An artificially synthesized peptide sequence

<400>  145

Asp Ile Gln Met Thr Gln Ser Pro Ser Ser Leu Ser Ala Ser Val Gly
1               5                   10                  15

Asp Arg Val Thr Ile Thr Cys Arg Thr Ser Glu Asp Ile Tyr Ser Phe
            20                  25                  30

Leu Ala Trp Tyr Gln Gln Lys Pro Gly Lys Ala Pro Lys Leu Leu Ile
        35                  40                  45

Tyr Asn Ala Lys Thr Leu Ala Lys Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln His His Tyr Glu Ser Pro Leu
                85                  90                  95

Thr Phe Gly Gly Gly Thr Lys Val Glu Ile Lys
            100                 105

<210>  146
<211>  107
<212>  PRT
<213>  Artificial

<220>
<223>  An artificially synthesized peptide sequence

<400>  146

Asp Ile Gln Met Thr Gln Ser Pro Ser Ser Leu Ser Ala Ser Val Gly
1               5                   10                  15

Asp Arg Val Thr Ile Thr Cys Arg Thr Ser Glu Asn Ile Tyr Ser Phe
```

20 25 30

Leu Ala Trp Tyr Gln Gln Lys Pro Gly Lys Ala Pro Lys Leu Leu Ile
35 40 45

Tyr Asp Ala Lys Thr Leu Ala Lys Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln His His Tyr Glu Ser Pro Leu
85 90 95

Thr Phe Gly Gly Gly Thr Lys Val Glu Ile Lys
100 105

<210> 147
<211> 107
<212> PRT
<213> Artificial

<220>
<223> An artificially synthesized peptide sequence

<400> 147

Asp Ile Gln Met Thr Gln Ser Pro Ser Ser Leu Ser Ala Ser Val Gly
1 5 10 15

Asp Arg Val Thr Ile Thr Cys Arg Thr Ser Glu Asn Ile Tyr Ser Phe
20 25 30

Leu Ala Trp Tyr Gln Gln Lys Pro Gly Lys Ala Pro Lys Leu Leu Ile
35 40 45

Tyr Asn Ala Gln Thr Leu Ala Lys Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln His His Tyr Glu Ser Pro Leu
85 90 95

Thr Phe Gly Gly Gly Thr Lys Val Glu Ile Lys
100 105

<210> 148
<211> 107
<212> PRT
<213> Artificial

<220>
<223> An artificially synthesized peptide sequence

<400> 148

Asp Ile Gln Met Thr Gln Ser Pro Ser Ser Leu Ser Ala Ser Val Gly
1 5 10 15

Asp Arg Val Thr Ile Thr Cys Arg Thr Ser Glu Asn Ile Tyr Ser Phe
20 25 30

Leu Ala Trp Tyr Gln Gln Lys Pro Gly Lys Ala Pro Lys Leu Leu Ile
35 40 45

Tyr Asn Ala Lys Thr Glu Ala Lys Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln His His Tyr Glu Ser Pro Leu
85 90 95

Thr Phe Gly Gly Gly Thr Lys Val Glu Ile Lys
100 105

<210> 149
<211> 107
<212> PRT
<213> Artificial

<220>
<223> An artificially synthesized peptide sequence

<400> 149

Asp Ile Gln Met Thr Gln Ser Pro Ser Ser Leu Ser Ala Ser Val Gly
1 5 10 15

Asp Arg Val Thr Ile Thr Cys Arg Thr Ser Glu Asn Ile Tyr Ser Phe
20 25 30

Leu Ala Trp Tyr Gln Gln Lys Pro Gly Lys Ala Pro Lys Leu Leu Ile
35 40 45

Tyr Asn Ala Lys Thr Leu Ala Gln Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln His His Tyr Glu Ser Pro Leu
85 90 95

Thr Phe Gly Gly Gly Thr Lys Val Glu Ile Lys
100 105

<210> 150
<211> 107
<212> PRT
<213> Artificial

<220>
<223> An artificially synthesized peptide sequence

<400> 150

Asp Ile Gln Met Thr Gln Ser Pro Ser Ser Leu Ser Ala Ser Val Gly
1 5 10 15

Asp Arg Val Thr Ile Thr Cys Arg Thr Ser Glu Asn Ile Tyr Ser Phe
20 25 30

Leu Ala Trp Tyr Gln Gln Lys Pro Gly Lys Ala Pro Lys Leu Leu Ile
        35                  40                  45

Tyr Asn Ala Lys Thr Leu Ala Asp Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln His His Tyr Glu Ser Pro Leu
                85                  90                  95

Thr Phe Gly Gly Gly Thr Lys Val Glu Ile Lys
            100                 105

<210> 151
<211> 447
<212> PRT
<213> Artificial

<220>
<223> An artificially synthesized peptide sequence

<400> 151

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ile Met Asn Trp Val Arg Gln Ala Pro Gly Gln Gly Leu Glu Trp Met
        35                  40                  45

Gly Leu Ile Asn Pro Tyr Asn Gly Gly Thr Ser Tyr Asn Gln Gln Phe
    50                  55                  60

Gln Asp Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Gly Tyr Asp Asp Gly Pro Tyr Thr Met Asp Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Glu Ser Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Asn Phe Gly Thr Gln Thr Tyr Thr Cys Asn Val Asp His
        195                 200                 205

Lys Pro Ser Asn Thr Lys Val Asp Lys Thr Val Glu Arg Lys Ser Cys
    210                 215                 220

Val Glu Cys Pro Pro Cys Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Phe Arg Val Val Ser
    290                 295                 300

Val Leu Thr Val Val His Gln Asp Trp Leu Asn Gly Lys Glu Tyr Lys
305                 310                 315                 320

Cys Lys Val Ser Asn Lys Gly Leu Pro Ala Pro Ile Glu Lys Thr Ile
                325                 330                 335

Ser Lys Thr Lys Gly Gln Pro Arg Glu Pro Gln Val Tyr Thr Leu Pro
            340                 345                 350

Pro Ser Arg Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Met Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly Lys
        435                 440                 445

<210> 152
<211> 214
<212> PRT
<213> Artificial

<220>
<223> An artificially synthesized peptide sequence

<400> 152

Asp Ile Gln Met Thr Gln Ser Pro Ser Ser Leu Ser Ala Ser Val Gly
1               5                   10                  15

Asp Arg Val Thr Ile Thr Cys Gln Thr Ser Glu Asp Ile Tyr Ser Phe
            20                  25                  30

Leu Ala Trp Tyr Gln Gln Lys Pro Gly Lys Ala Pro Lys Leu Leu Ile

35 40 45

Tyr Asn Ala Gln Thr Glu Ala Gln Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln His His Tyr Glu Ser Pro Leu
85 90 95

Thr Phe Gly Gly Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 153
<211> 326
<212> PRT
<213> Artificial

<220>
<223> An artificially synthesized peptide sequence

<400> 153

Ala Ser Thr Lys Gly Pro Ser Val Phe Pro Leu Ala Pro Cys Ser Arg
1 5 10 15

Ser Thr Ser Glu Ser Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Asn Phe Gly Thr Gln Thr
65 70 75 80

Tyr Thr Cys Asn Val Asp His Lys Pro Ser Asn Thr Lys Val Asp Lys
85 90 95

```
Thr Val Glu Arg Lys Ser Cys Val Glu Cys Pro Pro Cys Pro Ala Pro
            100                 105                 110

Pro Val Ala Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp
    130                 135                 140

Val Ser His Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ala Pro Ile Glu Lys Thr Ile Ser Lys Thr Lys Gly Gln Pro Arg Glu
    210                 215                 220

Pro Gln Val Tyr Thr Leu Pro Pro Ser Arg Glu Glu Met Thr Lys Asn
225                 230                 235                 240

Gln Val Ser Leu Thr Cys Leu Val Lys Gly Phe Tyr Pro Ser Asp Ile
                245                 250                 255

Ala Val Glu Trp Glu Ser Asn Gly Gln Pro Glu Asn Asn Tyr Lys Thr
            260                 265                 270

Thr Pro Pro Met Leu Asp Ser Asp Gly Ser Phe Phe Leu Tyr Ser Lys
        275                 280                 285

Leu Thr Val Asp Lys Ser Arg Trp Gln Gln Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Pro Gly Lys
                325

<210>  154
<211>  326
<212>  PRT
<213>  Artificial

<220>
<223>  An artificially synthesized peptide sequence

<400>  154

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45
```

```
Gly Val His Thr Phe Pro Ala Val Leu Gln Ser Ser Gly Leu Tyr Ser
    50                  55                  60

Leu Ser Ser Val Val Thr Val Pro Ser Ser Asn Phe Gly Thr Gln Thr
65                  70                  75                  80

Tyr Thr Cys Asn Val Asp His Lys Pro Ser Asn Thr Lys Val Asp Lys
                85                  90                  95

Thr Val Glu Arg Lys Cys Ser Val Glu Cys Pro Pro Cys Pro Ala Pro
            100                 105                 110

Pro Val Ala Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp
    130                 135                 140

Val Ser His Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ala Pro Ile Glu Lys Thr Ile Ser Lys Thr Lys Gly Gln Pro Arg Glu
    210                 215                 220

Pro Gln Val Tyr Thr Leu Pro Pro Ser Arg Glu Glu Met Thr Lys Asn
225                 230                 235                 240

Gln Val Ser Leu Thr Cys Leu Val Lys Gly Phe Tyr Pro Ser Asp Ile
                245                 250                 255

Ala Val Glu Trp Glu Ser Asn Gly Gln Pro Glu Asn Asn Tyr Lys Thr
            260                 265                 270

Thr Pro Pro Met Leu Asp Ser Asp Gly Ser Phe Phe Leu Tyr Ser Lys
        275                 280                 285

Leu Thr Val Asp Lys Ser Arg Trp Gln Gln Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Pro Gly Lys
                325
```

<210> 155
<211> 119
<212> PRT
<213> Artificial

<220>
<223> An artificially synthesized peptide sequence

<400> 155

Gln Val Gln Leu Gln Glu Ser Gly Pro Gly Leu Val Arg Pro Ser Gln
1 5 10 15

Thr Leu Ser Leu Thr Cys Thr Val Ser Gly Tyr Ser Ile Thr Ser Asp
20 25 30

His Ala Trp Ser Trp Val Arg Gln Pro Pro Gly Arg Gly Leu Glu Trp
35 40 45

Ile Gly Tyr Ile Ser Tyr Ser Gly Ile Thr Thr Tyr Asn Pro Ser Leu
50 55 60

Lys Ser Arg Val Thr Met Leu Arg Asp Thr Ser Lys Asn Gln Phe Ser
65 70 75 80

Leu Arg Leu Ser Ser Val Thr Ala Ala Asp Thr Ala Val Tyr Tyr Cys
85 90 95

Ala Arg Ser Leu Ala Arg Thr Thr Ala Met Asp Tyr Trp Gly Gln Gly
100 105 110

Ser Leu Val Thr Val Ser Ser
115

<210> 156
<211> 107
<212> PRT
<213> Artificial

<220>
<223> An artificially synthesized peptide sequence

<400> 156

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Ile Ser Ser Tyr
20 25 30

Leu Asn Trp Tyr Gln Gln Lys Pro Gly Lys Ala Pro Lys Leu Leu Ile
35 40 45

Tyr Tyr Thr Ser Arg Leu His Ser Gly Val Pro Ser Arg Phe Ser Gly
50 55 60

Ser Gly Ser Gly Thr Asp Phe Thr Phe Thr Ile Ser Ser Leu Gln Pro
65 70 75 80

Glu Asp Ile Ala Thr Tyr Tyr Cys Gln Gln Gly Asn Thr Leu Pro Tyr
85 90 95

Thr Phe Gly Gln Gly Thr Lys Val Glu Ile Lys
100 105

<210> 157
<211> 445
<212> PRT
<213> Artificial

<220>

<223> An artificially synthesized peptide sequence

<400> 157

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Cys Ser Arg Ser Thr Ser Glu Ser Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Asn Phe Gly Thr Gln Thr Tyr Thr Cys Asn Val Asp His Lys Pro
        195                 200                 205

Ser Asn Thr Lys Val Asp Lys Thr Val Glu Arg Lys Ser Cys Val Glu
    210                 215                 220

Cys Pro Pro Cys Pro Ala Pro Pro Val Ala Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Phe Arg Val Val Ser Val Leu
    290                 295                 300

Thr Val Val His Gln Asp Trp Leu Asn Gly Lys Glu Tyr Lys Cys Lys
305                 310                 315                 320

Val Ser Asn Lys Gly Leu Pro Ala Pro Ile Glu Lys Thr Ile Ser Lys
325 330 335

Thr Lys Gly Gln Pro Arg Glu Pro Gln Val Tyr Thr Leu Pro Pro Ser
340 345 350

Arg Glu Glu Met Thr Lys Asn Gln Val Ser Leu Thr Cys Leu Val Lys
355 360 365

Gly Phe Tyr Pro Ser Asp Ile Ala Val Glu Trp Glu Ser Asn Gly Gln
370 375 380

Pro Glu Asn Asn Tyr Lys Thr Thr Pro Pro Met Leu Asp Ser Asp Gly
385 390 395 400

Ser Phe Phe Leu Tyr Ser Lys Leu Thr Val Asp Lys Ser Arg Trp Gln
405 410 415

Gln Gly Asn Val Phe Ser Cys Ser Val Met His Glu Ala Leu His Asn
420 425 430

His Tyr Thr Gln Lys Ser Leu Ser Leu Ser Pro Gly Lys
435 440 445

<210> 158
<211> 445
<212> PRT
<213> Artificial

<220>
<223> An artificially synthesized peptide sequence

<400> 158

Gln Val Gln Leu Gln Glu Ser Gly Pro Gly Leu Val Arg Pro Ser Gln
1 5 10 15

Thr Leu Ser Leu Thr Cys Thr Val Ser Gly Tyr Ser Ile Thr Ser Asp
20 25 30

His Ala Trp Ser Trp Val Arg Gln Pro Pro Gly Arg Gly Leu Glu Trp
35 40 45

Ile Gly Tyr Ile Ser Tyr Ser Gly Ile Thr Thr Tyr Asn Pro Ser Leu
50 55 60

Lys Ser Arg Val Thr Met Leu Arg Asp Thr Ser Lys Asn Gln Phe Ser
65 70 75 80

Leu Arg Leu Ser Ser Val Thr Ala Ala Asp Thr Ala Val Tyr Tyr Cys
85 90 95

Ala Arg Ser Leu Ala Arg Thr Thr Ala Met Asp Tyr Trp Gly Gln Gly
100 105 110

Ser Leu Val Thr Val Ser Ser Ala Ser Thr Lys Gly Pro Ser Val Phe
115 120 125

Pro Leu Ala Pro Cys Ser Arg Ser Thr Ser Glu Ser Thr Ala Ala Leu
130 135 140

Gly Cys Leu Val Lys Asp Tyr Phe Pro Glu Pro Val Thr Val Ser Trp

145 150 155 160

Asn Ser Gly Ala Leu Thr Ser Gly Val His Thr Phe Pro Ala Val Leu
165 170 175

Gln Ser Ser Gly Leu Tyr Ser Leu Ser Ser Val Val Thr Val Pro Ser
180 185 190

Ser Asn Phe Gly Thr Gln Thr Tyr Thr Cys Asn Val Asp His Lys Pro
195 200 205

Ser Asn Thr Lys Val Asp Lys Thr Val Glu Arg Lys Cys Ser Val Glu
210 215 220

Cys Pro Pro Cys Pro Ala Pro Pro Val Ala Gly Pro Ser Val Phe Leu
225 230 235 240

Phe Pro Pro Lys Pro Lys Asp Thr Leu Met Ile Ser Arg Thr Pro Glu
245 250 255

Val Thr Cys Val Val Val Asp Val Ser His Glu Asp Pro Glu Val Gln
260 265 270

Phe Asn Trp Tyr Val Asp Gly Val Glu Val His Asn Ala Lys Thr Lys
275 280 285

Pro Arg Glu Glu Gln Phe Asn Ser Thr Phe Arg Val Val Ser Val Leu
290 295 300

Thr Val Val His Gln Asp Trp Leu Asn Gly Lys Glu Tyr Lys Cys Lys
305 310 315 320

Val Ser Asn Lys Gly Leu Pro Ala Pro Ile Glu Lys Thr Ile Ser Lys
325 330 335

Thr Lys Gly Gln Pro Arg Glu Pro Gln Val Tyr Thr Leu Pro Pro Ser
340 345 350

Arg Glu Glu Met Thr Lys Asn Gln Val Ser Leu Thr Cys Leu Val Lys
355 360 365

Gly Phe Tyr Pro Ser Asp Ile Ala Val Glu Trp Glu Ser Asn Gly Gln
370 375 380

Pro Glu Asn Asn Tyr Lys Thr Thr Pro Pro Met Leu Asp Ser Asp Gly
385 390 395 400

Ser Phe Phe Leu Tyr Ser Lys Leu Thr Val Asp Lys Ser Arg Trp Gln
405 410 415

Gln Gly Asn Val Phe Ser Cys Ser Val Met His Glu Ala Leu His Asn
420 425 430

His Tyr Thr Gln Lys Ser Leu Ser Leu Ser Pro Gly Lys
435 440 445

<210> 159
<211> 449
<212> PRT
<213> Artificial

<220>
<223> An artificially synthesized peptide sequence

<400> 159

```
Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320
```

Tyr Lys Cys Lys Val Ser Asn Lys Ala Leu Pro Ala Pro Ile Glu Lys
325 330 335

Thr Ile Ser Lys Ala Lys Gly Gln Pro Arg Glu Pro Gln Val Tyr Thr
340 345 350

Leu Pro Pro Ser Arg Asp Glu Leu Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro Gly
435 440 445

Lys

<210> 160
<211> 445
<212> PRT
<213> Artificial

<220>
<223> An artificially synthesized peptide sequence

<400> 160

Gln Val Gln Leu Gln Glu Ser Gly Pro Gly Leu Val Arg Pro Ser Gln
1 5 10 15

Thr Leu Ser Leu Thr Cys Thr Val Ser Gly Tyr Ser Ile Thr Ser Asp
20 25 30

His Ala Trp Ser Trp Val Arg Gln Pro Pro Gly Arg Gly Leu Glu Trp
35 40 45

Ile Gly Tyr Ile Ser Tyr Ser Gly Ile Thr Thr Tyr Asn Pro Ser Leu
50 55 60

Lys Ser Arg Val Thr Met Leu Arg Asp Thr Ser Lys Asn Gln Phe Ser
65 70 75 80

Leu Arg Leu Ser Ser Val Thr Ala Ala Asp Thr Ala Val Tyr Tyr Cys
85 90 95

Ala Arg Ser Leu Ala Arg Thr Thr Ala Met Asp Tyr Trp Gly Gln Gly
100 105 110

Ser Leu Val Thr Val Ser Ser Ala Ser Thr Lys Gly Pro Ser Val Phe
115 120 125

Pro Leu Ala Pro Cys Ser Arg Ser Thr Ser Glu Ser Thr Ala Ala Leu
130 135 140

Gly Cys Leu Val Lys Asp Tyr Phe Pro Glu Pro Val Thr Val Ser Trp
145 150 155 160

Asn Ser Gly Ala Leu Thr Ser Gly Val His Thr Phe Pro Ala Val Leu
165 170 175

Gln Ser Ser Gly Leu Tyr Ser Leu Ser Ser Val Val Thr Val Pro Ser
180 185 190

Ser Asn Phe Gly Thr Gln Thr Tyr Thr Cys Asn Val Asp His Lys Pro
195 200 205

Ser Asn Thr Lys Val Asp Lys Thr Val Glu Arg Lys Cys Cys Val Glu
210 215 220

Cys Pro Pro Cys Pro Ala Pro Pro Val Ala Gly Pro Ser Val Phe Leu
225 230 235 240

Phe Pro Pro Lys Pro Lys Asp Thr Leu Met Ile Ser Arg Thr Pro Glu
245 250 255

Val Thr Cys Val Val Val Asp Val Ser His Glu Asp Pro Glu Val Gln
260 265 270

Phe Asn Trp Tyr Val Asp Gly Val Glu Val His Asn Ala Lys Thr Lys
275 280 285

Pro Arg Glu Glu Gln Phe Asn Ser Thr Phe Arg Val Val Ser Val Leu
290 295 300

Thr Val Val His Gln Asp Trp Leu Asn Gly Lys Glu Tyr Lys Cys Lys
305 310 315 320

Val Ser Asn Lys Gly Leu Pro Ala Pro Ile Glu Lys Thr Ile Ser Lys
325 330 335

Thr Lys Gly Gln Pro Arg Glu Pro Gln Val Tyr Thr Leu Pro Pro Ser
340 345 350

Arg Glu Glu Met Thr Lys Asn Gln Val Ser Leu Thr Cys Leu Val Lys
355 360 365

Gly Phe Tyr Pro Ser Asp Ile Ala Val Glu Trp Glu Ser Asn Gly Gln
370 375 380

Pro Glu Asn Asn Tyr Lys Thr Thr Pro Pro Met Leu Asp Ser Asp Gly
385 390 395 400

Ser Phe Phe Leu Tyr Ser Lys Leu Thr Val Asp Lys Ser Arg Trp Gln
405 410 415

Gln Gly Asn Val Phe Ser Cys Ser Val Met His Glu Ala Leu His Asn
420 425 430

His Tyr Thr Gln Lys Ser Leu Ser Leu Ser Pro Gly Lys
435 440 445

<210> 161
<211> 445
<212> PRT
<213> Artificial

<220>
<223> An artificially synthesized peptide sequence

<400> 161

Gln Val Gln Leu Gln Glu Ser Gly Pro Gly Leu Val Arg Pro Ser Gln
1 5 10 15

Thr Leu Ser Leu Thr Cys Thr Val Ser Gly Tyr Ser Ile Thr Ser Asp
20 25 30

His Ala Trp Ser Trp Val Arg Gln Pro Pro Gly Arg Gly Leu Glu Trp
35 40 45

Ile Gly Tyr Ile Ser Tyr Ser Gly Ile Thr Thr Tyr Asn Pro Ser Leu
50 55 60

Lys Ser Arg Val Thr Met Leu Arg Asp Thr Ser Lys Asn Gln Phe Ser
65 70 75 80

Leu Arg Leu Ser Ser Val Thr Ala Ala Asp Thr Ala Val Tyr Tyr Cys
85 90 95

Ala Arg Ser Leu Ala Arg Thr Thr Ala Met Asp Tyr Trp Gly Gln Gly
100 105 110

Ser Leu Val Thr Val Ser Ser Ala Ser Thr Lys Gly Pro Ser Val Phe
115 120 125

Pro Leu Ala Pro Ser Ser Lys Ser Thr Ser Glu Ser Thr Ala Ala Leu
130 135 140

Gly Cys Leu Val Lys Asp Tyr Phe Pro Glu Pro Val Thr Val Ser Trp
145 150 155 160

Asn Ser Gly Ala Leu Thr Ser Gly Val His Thr Phe Pro Ala Val Leu
165 170 175

Gln Ser Ser Gly Leu Tyr Ser Leu Ser Ser Val Val Thr Val Pro Ser
180 185 190

Ser Asn Phe Gly Thr Gln Thr Tyr Thr Cys Asn Val Asp His Lys Pro
195 200 205

Ser Asn Thr Lys Val Asp Lys Thr Val Glu Arg Lys Ser Cys Val Glu
210 215 220

Cys Pro Pro Cys Pro Ala Pro Pro Val Ala Gly Pro Ser Val Phe Leu
225 230 235 240

Phe Pro Pro Lys Pro Lys Asp Thr Leu Met Ile Ser Arg Thr Pro Glu
245 250 255

Val Thr Cys Val Val Val Asp Val Ser His Glu Asp Pro Glu Val Gln
260 265 270

Phe Asn Trp Tyr Val Asp Gly Val Glu Val His Asn Ala Lys Thr Lys
275 280 285

Pro Arg Glu Glu Gln Phe Asn Ser Thr Phe Arg Val Val Ser Val Leu
290 295 300

Thr Val Val His Gln Asp Trp Leu Asn Gly Lys Glu Tyr Lys Cys Lys

305 310 315 320

Val Ser Asn Lys Gly Leu Pro Ala Pro Ile Glu Lys Thr Ile Ser Lys
325 330 335

Thr Lys Gly Gln Pro Arg Glu Pro Gln Val Tyr Thr Leu Pro Pro Ser
340 345 350

Arg Glu Glu Met Thr Lys Asn Gln Val Ser Leu Thr Cys Leu Val Lys
355 360 365

Gly Phe Tyr Pro Ser Asp Ile Ala Val Glu Trp Glu Ser Asn Gly Gln
370 375 380

Pro Glu Asn Asn Tyr Lys Thr Thr Pro Pro Met Leu Asp Ser Asp Gly
385 390 395 400

Ser Phe Phe Leu Tyr Ser Lys Leu Thr Val Asp Lys Ser Arg Trp Gln
405 410 415

Gln Gly Asn Val Phe Ser Cys Ser Val Met His Glu Ala Leu His Asn
420 425 430

His Tyr Thr Gln Lys Ser Leu Ser Leu Ser Pro Gly Lys
435 440 445

<210> 162
<211> 214
<212> PRT
<213> Artificial

<220>
<223> An artificially synthesized peptide sequence

<400> 162

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Ile Ser Ser Tyr
20 25 30

Leu Asn Trp Tyr Gln Gln Lys Pro Gly Lys Ala Pro Lys Leu Leu Ile
35 40 45

Tyr Tyr Thr Ser Arg Leu His Ser Gly Val Pro Ser Arg Phe Ser Gly
50 55 60

Ser Gly Ser Gly Thr Asp Phe Thr Phe Thr Ile Ser Ser Leu Gln Pro
65 70 75 80

Glu Asp Ile Ala Thr Tyr Tyr Cys Gln Gln Gly Asn Thr Leu Pro Tyr
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 163
<211> 445
<212> PRT
<213> Artificial

<220>
<223> An artificially synthesized peptide sequence

<400> 163

Gln Val Gln Leu Gln Glu Ser Gly Pro Gly Leu Val Arg Pro Ser Gln
1 5 10 15

Thr Leu Ser Leu Thr Cys Thr Val Ser Gly Tyr Ser Ile Thr Ser Asp
20 25 30

His Ala Trp Ser Trp Val Arg Gln Pro Pro Gly Arg Gly Leu Glu Trp
35 40 45

Ile Gly Tyr Ile Ser Tyr Ser Gly Ile Thr Thr Tyr Asn Pro Ser Leu
50 55 60

Lys Ser Arg Val Thr Met Leu Arg Asp Thr Ser Lys Asn Gln Phe Ser
65 70 75 80

Leu Arg Leu Ser Ser Val Thr Ala Ala Asp Thr Ala Val Tyr Tyr Cys
85 90 95

Ala Arg Ser Leu Ala Arg Thr Thr Ala Met Asp Tyr Trp Gly Gln Gly
100 105 110

Ser Leu Val Thr Val Ser Ser Ala Ser Thr Lys Gly Pro Ser Val Phe
115 120 125

Pro Leu Ala Pro Ser Ser Lys Ser Thr Ser Gly Gly Thr Ala Ala Leu
130 135 140

Gly Cys Leu Val Lys Asp Tyr Phe Pro Glu Pro Val Thr Val Ser Trp
145 150 155 160

Asn Ser Gly Ala Leu Thr Ser Gly Val His Thr Phe Pro Ala Val Leu
165 170 175

Gln Ser Ser Gly Leu Tyr Ser Leu Ser Ser Val Val Thr Val Pro Ser
180 185 190

Ser Asn Phe Gly Thr Gln Thr Tyr Thr Cys Asn Val Asp His Lys Pro
195 200 205

Ser Asn Thr Lys Val Asp Lys Thr Val Glu Arg Lys Ser Cys Val Glu
    210                 215                 220

Cys Pro Pro Cys Pro Ala Pro Pro Val Ala Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Phe Arg Val Val Ser Val Leu
    290                 295                 300

Thr Val Val His Gln Asp Trp Leu Asn Gly Lys Glu Tyr Lys Cys Lys
305                 310                 315                 320

Val Ser Asn Lys Gly Leu Pro Ser Ser Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Arg Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Gln Gly Asn Val Phe Ser Cys Ser Val Met His Glu Ala Leu His Asn
            420                 425                 430

His Tyr Thr Gln Lys Ser Leu Ser Leu Ser Pro Gly Lys
        435                 440                 445

<210> 164
<211> 443
<212> PRT
<213> Artificial

<220>
<223> An artificially synthesized peptide sequence

<400> 164

Gln Val Gln Leu Gln Glu Ser Gly Pro Gly Leu Val Arg Pro Ser Gln
1               5                   10                  15

Thr Leu Ser Leu Thr Cys Thr Val Ser Gly Tyr Ser Ile Thr Ser Asp
            20                  25                  30

His Ala Trp Ser Trp Val Arg Gln Pro Pro Gly Arg Gly Leu Glu Trp

```
        35                  40                  45

Ile Gly Tyr Ile Ser Tyr Ser Gly Ile Thr Thr Tyr Asn Pro Ser Leu
    50                  55                  60

Lys Ser Arg Val Thr Met Leu Arg Asp Thr Ser Lys Asn Gln Phe Ser
65                  70                  75                  80

Leu Arg Leu Ser Ser Val Thr Ala Ala Asp Thr Ala Val Tyr Tyr Cys
                85                  90                  95

Ala Arg Ser Leu Ala Arg Thr Thr Ala Met Asp Tyr Trp Gly Gln Gly
            100                 105                 110

Ser Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Asn Phe Gly Thr Gln Thr Tyr Thr Cys Asn Val Asp His Lys Pro
        195                 200                 205

Ser Asn Thr Lys Val Asp Lys Thr Val Glu Arg Lys Ser Cys Val Glu
    210                 215                 220

Cys Pro Pro Cys Pro Ala Pro Pro Val Ala Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser Gln Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Phe Arg Val Val Ser Val Leu
    290                 295                 300

Thr Val Val His Gln Asp Trp Leu Asn Gly Lys Glu Tyr Lys Cys Lys
305                 310                 315                 320

Val Ser Asn Lys Gly Leu Pro Ala Pro Ile Glu Lys Thr Ile Ser Lys
                325                 330                 335

Thr Lys Gly Gln Pro Arg Glu Pro Gln Val Tyr Thr Leu Pro Pro Ser
            340                 345                 350

Gln Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
```

370 375 380

Pro Glu Asn Asn Tyr Lys Thr Thr Pro Pro Met Leu Asp Ser Asp Gly
385 390 395 400

Ser Phe Phe Leu Tyr Ser Lys Leu Thr Val Asp Lys Ser Arg Trp Gln
405 410 415

Glu Gly Asn Val Phe Ser Cys Ser Val Met His Glu Ala Leu His Asn
420 425 430

His Tyr Thr Gln Lys Ser Leu Ser Leu Ser Pro
435 440

<210> 165
<211> 267
<212> PRT
<213> Artificial

<220>
<223> An artificially synthesized peptide sequence

<400> 165

Ala Glu Ser His Leu Ser Leu Leu Tyr His Leu Thr Ala Val Ser Ser
1 5 10 15

Pro Ala Pro Gly Thr Pro Ala Phe Trp Val Ser Gly Trp Leu Gly Pro
20 25 30

Gln Gln Tyr Leu Ser Tyr Asn Ser Leu Arg Gly Glu Ala Glu Pro Cys
35 40 45

Gly Ala Trp Val Trp Glu Asn Gln Val Ser Trp Tyr Trp Glu Lys Glu
50 55 60

Thr Thr Asp Leu Arg Ile Lys Glu Lys Leu Phe Leu Glu Ala Phe Lys
65 70 75 80

Ala Leu Gly Gly Lys Gly Pro Tyr Thr Leu Gln Gly Leu Leu Gly Cys
85 90 95

Glu Leu Gly Pro Asp Asn Thr Ser Val Pro Thr Ala Lys Phe Ala Leu
100 105 110

Asn Gly Glu Glu Phe Met Asn Phe Asp Leu Lys Gln Gly Thr Trp Gly
115 120 125

Gly Asp Trp Pro Glu Ala Leu Ala Ile Ser Gln Arg Trp Gln Gln Gln
130 135 140

Asp Lys Ala Ala Asn Lys Glu Leu Thr Phe Leu Leu Phe Ser Cys Pro
145 150 155 160

His Arg Leu Arg Glu His Leu Glu Arg Gly Arg Gly Asn Leu Glu Trp
165 170 175

Lys Glu Pro Pro Ser Met Arg Leu Lys Ala Arg Pro Ser Ser Pro Gly
180 185 190

Phe Ser Val Leu Thr Cys Ser Ala Phe Ser Phe Tyr Pro Pro Glu Leu
195 200 205

Gln Leu Arg Phe Leu Arg Asn Gly Leu Ala Ala Gly Thr Gly Gln Gly
    210                 215                 220

Asp Phe Gly Pro Asn Ser Asp Gly Ser Phe His Ala Ser Ser Ser Leu
225                 230                 235                 240

Thr Val Lys Ser Gly Asp Glu His His Tyr Cys Cys Ile Val Gln His
                245                 250                 255

Ala Gly Leu Ala Gln Pro Leu Arg Val Glu Leu
            260                 265

<210> 166
<211> 99
<212> PRT
<213> Artificial

<220>
<223> An artificially synthesized peptide sequence

<400> 166

Ile Gln Arg Thr Pro Lys Ile Gln Val Tyr Ser Arg His Pro Ala Glu
1               5                   10                  15

Asn Gly Lys Ser Asn Phe Leu Asn Cys Tyr Val Ser Gly Phe His Pro
            20                  25                  30

Ser Asp Ile Glu Val Asp Leu Leu Lys Asn Gly Glu Arg Ile Glu Lys
        35                  40                  45

Val Glu His Ser Asp Leu Ser Phe Ser Lys Asp Trp Ser Phe Tyr Leu
    50                  55                  60

Leu Tyr Tyr Thr Glu Phe Thr Pro Thr Glu Lys Asp Glu Tyr Ala Cys
65                  70                  75                  80

Arg Val Asn His Val Thr Leu Ser Gln Pro Lys Ile Val Lys Trp Asp
                85                  90                  95

Arg Asp Met

<210> 167
<211> 121
<212> PRT
<213> Artificial

<220>
<223> An artificially synthesized peptide sequence

<400> 167

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ile Met Asn Trp Val Arg Gln Ala Pro Gly Gln Gly Leu Glu Trp Met
        35                  40                  45

Gly Leu Ile Asn Pro Tyr Asn Gly Gly Thr Ser Tyr Asn Gln Gln Phe

50 55 60

Gln Asp Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Met Asp Tyr Trp Gly
100 105 110

Gln Gly Thr Leu Val Thr Val Ser Ser
115 120

<210> 168
<211> 107
<212> PRT
<213> Artificial

<220>
<223> An artificially synthesized peptide sequence

<400> 168

Asp Ile Gln Met Thr Gln Ser Pro Ser Ser Leu Ser Ala Ser Val Gly
1 5 10 15

Asp Arg Val Thr Ile Thr Cys Gln Thr Ser Glu Asp Ile Tyr Ser Phe
20 25 30

Leu Ala Trp Tyr Gln Gln Lys Pro Gly Lys Ala Pro Lys Leu Leu Ile
35 40 45

Tyr Asn Ala Gln Thr Glu Ala Gln Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln His His Tyr Glu Ser Pro Leu
85 90 95

Thr Phe Gly Gly Gly Thr Lys Val Glu Ile Lys
100 105

<210> 169
<211> 11
<212> PRT
<213> Artificial

<220>
<223> An artificially synthesized peptide sequence

<400> 169

Gln Thr Ser Glu Asp Ile Tyr Ser Phe Leu Ala
1 5 10

<210> 170
<211> 7

<212> PRT
<213> Artificial

<220>
<223> An artificially synthesized peptide sequence

<400> 170

Asn Ala Gln Thr Glu Ala Gln
1 5

<210> 171
<211> 17
<212> PRT
<213> Artificial

<220>
<223> An artificially synthesized peptide sequence

<400> 171

Leu Ile Asn Pro Tyr Asn Gly Gly Thr Ser Tyr Asn Gln Gln Phe Gln
1 5 10 15

Asp

<210> 172
<211> 17
<212> PRT
<213> Artificial

<220>
<223> An artificially synthesized peptide sequence

<400> 172

Leu Ile Asn Pro Tyr Asn Gly Gly Thr Ser Tyr Asn Asp Gln Phe Gln
1 5 10 15

Asp

<210> 173
<211> 5
<212> PRT
<213> Artificial

<220>
<223> An artificially synthesized peptide sequence

<400> 173

Gly Tyr Val Met Asn
1 5

<210> 174
<211> 5
<212> PRT
<213> Artificial

<220>
<223> An artificially synthesized peptide sequence

<400> 174

Gly Tyr Ile Ile Asn
1 5

<210> 175
<211> 5
<212> PRT
<213> Artificial

<220>
<223> An artificially synthesized peptide sequence

<400> 175

Gly Tyr Ile Leu Asn
1 5

<210> 176
<211> 5
<212> PRT
<213> Artificial

<220>
<223> An artificially synthesized peptide sequence

<400> 176

Gly Tyr Ala Met Asn
1 5

<210> 177
<211> 17
<212> PRT
<213> Artificial

<220>
<223> An artificially synthesized peptide sequence

<400> 177

Leu Ile Asn Pro Tyr Asn Gly Gly Thr Asp Tyr Asn Gln Lys Phe Lys
1 5 10 15

Gly

<210> 178
<211> 17
<212> PRT
<213> Artificial

<220>
<223> An artificially synthesized peptide sequence

<400> 178

Leu Ile Asn Pro Tyr Asn Gly Gly Thr Ser Tyr Asn Pro Lys Phe Lys
1 5 10 15

Gly

<210> 179
<211> 12
<212> PRT
<213> Artificial

<220>
<223> An artificially synthesized peptide sequence

<400> 179

Asp Gly Leu Asp Asp Gly Pro Tyr Thr Met Asp Tyr
1 5 10

<210> 180
<211> 12
<212> PRT
<213> Artificial

<220>
<223> An artificially synthesized peptide sequence

<400> 180

Asp Gly Tyr Asp Asp Gly Pro Tyr Thr Leu Asp Tyr
1 5 10

<210> 181
<211> 12
<212> PRT
<213> Artificial

<220>
<223> An artificially synthesized peptide sequence

<400> 181

Asp Gly Tyr Asp Asp Gly Pro Tyr Thr Met Glu Tyr
1 5 10

<210> 182
<211> 12
<212> PRT
<213> Artificial

<220>
<223> An artificially synthesized peptide sequence

<400> 182

Asp Gly Tyr Asp Asp Gly Pro Tyr Thr Met Asp Thr

1 5 10

<210> 183
<211> 12
<212> PRT
<213> Artificial

<220>
<223> An artificially synthesized peptide sequence

<400> 183

Asp Gly Tyr Asp Asp Gly Pro Tyr Thr Met Asp Ser
1 5 10

<210> 184
<211> 12
<212> PRT
<213> Artificial

<220>
<223> An artificially synthesized peptide sequence

<400> 184

Asp Gly Tyr Asp Asp Gly Pro Tyr Thr Leu Glu Thr
1 5 10

<210> 185
<211> 12
<212> PRT
<213> Artificial

<220>
<223> An artificially synthesized peptide sequence

<400> 185

Asp Gly Tyr Asp Asp Gly Pro Tyr Thr Met Glu Thr
1 5 10

<210> 186
<211> 12
<212> PRT
<213> Artificial

<220>
<223> An artificially synthesized peptide sequence

<400> 186

Asp Gly Leu Asp Asp Gly Pro Tyr Thr Met Glu Thr
1 5 10

<210> 187
<211> 12
<212> PRT
<213> Artificial

<220>
<223> An artificially synthesized peptide sequence

<400> 187

Asp Gly Leu Asp Asp Gly Pro Tyr Thr Met Glu Ser
1 5 10

<210> 188
<211> 11
<212> PRT
<213> Artificial

<220>
<223> An artificially synthesized peptide sequence

<400> 188

Arg Thr Ser Glu Asn Ile Tyr Arg Phe Leu Ala
1 5 10

<210> 189
<211> 11
<212> PRT
<213> Artificial

<220>
<223> An artificially synthesized peptide sequence

<400> 189

Arg Thr Ser Glu Asn Ile Tyr Ser Phe Val Ala
1 5 10

<210> 190
<211> 11
<212> PRT
<213> Artificial

<220>
<223> An artificially synthesized peptide sequence

<400> 190

Arg Thr Ser Glu Asn Ile Tyr Arg Phe Val Ala
1 5 10

<210> 191
<211> 11
<212> PRT
<213> Artificial

<220>
<223> An artificially synthesized peptide sequence

<400> 191

Arg Ala Ser Glu Asn Ile Tyr Ser Phe Leu Ala

1 5 10

<210> 192
<211> 11
<212> PRT
<213> Artificial

<220>
<223> An artificially synthesized peptide sequence

<400> 192

Arg Ser Ser Glu Asn Ile Tyr Ser Phe Leu Ala
1 5 10

<210> 193
<211> 9
<212> PRT
<213> Artificial

<220>
<223> An artificially synthesized peptide sequence

<400> 193

Gln His His Tyr Asp Ser Pro Leu Thr
1 5

<210> 194
<211> 9
<212> PRT
<213> Artificial

<220>
<223> An artificially synthesized peptide sequence

<400> 194

Gln His His Tyr Glu Asp Pro Leu Thr
1 5

<210> 195
<211> 9
<212> PRT
<213> Artificial

<220>
<223> An artificially synthesized peptide sequence

<400> 195

Gln His His Tyr Glu Ser Pro Leu Phe
1 5

<210> 196
<211> 5
<212> PRT
<213> Artificial

<220>
<223>  An artificially synthesized peptide sequence

<400>  196

Gly Tyr Val Leu Asn
1               5

<210>  197
<211>  17
<212>  PRT
<213>  Artificial

<220>
<223>  An artificially synthesized peptide sequence

<400>  197

Leu Ile Asn Pro Tyr Asn Gly Gly Thr Asp Tyr Asn Pro Gln Phe Gln
1               5                   10                  15

Asp

<210>  198
<211>  17
<212>  PRT
<213>  Artificial

<220>
<223>  An artificially synthesized peptide sequence

<400>  198

Leu Ile Asn Pro Tyr Asn Gly Gly Thr Asp Tyr Asn Pro Gln Phe Gln
1               5                   10                  15

Gly

<210>  199
<211>  17
<212>  PRT
<213>  Artificial

<220>
<223>  An artificially synthesized peptide sequence

<400>  199

Leu Ile Asn Pro Tyr Asn Gly Gly Thr Ser Tyr Asn Gln Gln Phe Gln
1               5                   10                  15

Asp

<210> 200
<211> 11
<212> PRT
<213> Artificial

<220>
<223> An artificially synthesized peptide sequence

<400> 200

Gln Thr Ser Glu Asp Ile Tyr Arg Phe Val Ala
1               5                   10

<210> 201
<211> 11
<212> PRT
<213> Artificial

<220>
<223> An artificially synthesized peptide sequence

<400> 201

Gln Thr Ser Glu Asp Ile Tyr Ser Phe Val Ala
1               5                   10

<210> 202
<211> 11
<212> PRT
<213> Artificial

<220>
<223> An artificially synthesized peptide sequence

<400> 202

Gln Ala Ser Glu Asp Ile Tyr Ser Phe Val Ala
1               5                   10

<210> 203
<211> 11
<212> PRT
<213> Artificial

<220>
<223> An artificially synthesized peptide sequence

<400> 203

Gln Ala Ser Glu Asp Ile Tyr Ser Phe Leu Ala
1               5                   10

<210> 204
<211> 121
<212> PRT
<213> Artificial

<220>

<223> An artificially synthesized peptide sequence

<400> 204

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Val Leu Asn Trp Val Arg Gln Ala Pro Gly Gln Gly Leu Glu Trp Met
35 40 45

Gly Leu Ile Asn Pro Tyr Asn Gly Gly Thr Asp Tyr Asn Pro Gln Phe
50 55 60

Gln Asp Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Met Asp Tyr Trp Gly
100 105 110

Gln Gly Thr Leu Val Thr Val Ser Ser
115 120

<210> 205
<211> 121
<212> PRT
<213> Artificial

<220>
<223> An artificially synthesized peptide sequence

<400> 205

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ala Met Asn Trp Val Arg Gln Ala Pro Gly Gln Gly Leu Glu Trp Met
35 40 45

Gly Leu Ile Asn Pro Tyr Asn Gly Gly Thr Asp Tyr Asn Pro Gln Phe
50 55 60

Gln Asp Arg Val Thr Ile Thr Ala Asp Glu Ser Thr Ser Thr Ala Tyr

65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Met Asp Tyr Trp Gly
100 105 110

Gln Gly Thr Leu Val Thr Val Ser Ser
115 120

<210> 206
<211> 121
<212> PRT
<213> Artificial

<220>
<223> An artificially synthesized peptide sequence

<400> 206

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Val Leu Asn Trp Val Arg Gln Ala Pro Gly Gln Gly Leu Glu Trp Met
35 40 45

Gly Leu Ile Asn Pro Tyr Asn Gly Gly Thr Asp Tyr Asn Pro Gln Phe
50 55 60

Gln Asp Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Leu Glu Thr Trp Gly
100 105 110

Gln Gly Thr Leu Val Thr Val Ser Ser
115 120

<210> 207
<211> 121
<212> PRT
<213> Artificial

<220>

<223> An artificially synthesized peptide sequence

<400> 207

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ile Met Asn Trp Val Arg Gln Ala Pro Gly Gln Gly Leu Glu Trp Met
        35                  40                  45

Gly Leu Ile Asn Pro Tyr Asn Gly Gly Thr Asp Tyr Asn Pro Gln Phe
    50                  55                  60

Gln Asp Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Gly Tyr Asp Asp Gly Pro Tyr Thr Leu Glu Thr Trp Gly
            100                 105                 110

Gln Gly Thr Leu Val Thr Val Ser Ser
        115                 120

<210> 208
<211> 121
<212> PRT
<213> Artificial

<220>
<223> An artificially synthesized peptide sequence

<400> 208

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ala Met Asn Trp Val Arg Gln Ala Pro Gly Gln Gly Leu Glu Trp Met
        35                  40                  45

Gly Leu Ile Asn Pro Tyr Asn Gly Gly Thr Asp Tyr Asn Pro Gln Phe
    50                  55                  60

Gln Asp Arg Val Thr Ile Thr Ala Asp Glu Ser Thr Ser Thr Ala Tyr

65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Leu Glu Thr Trp Gly
100 105 110

Gln Gly Thr Leu Val Thr Val Ser Ser
115 120

<210> 209
<211> 121
<212> PRT
<213> Artificial

<220>
<223> An artificially synthesized peptide sequence

<400> 209

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Val Leu Asn Trp Val Arg Gln Ala Pro Gly Gln Gly Leu Glu Trp Met
35 40 45

Gly Leu Ile Asn Pro Tyr Asn Gly Gly Thr Asp Tyr Asn Pro Gln Phe
50 55 60

Gln Asp Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Leu Glu Thr Trp Gly
100 105 110

Gln Gly Thr Leu Val Thr Val Ser Ser
115 120

<210> 210
<211> 121
<212> PRT
<213> Artificial

<220>

<223>  An artificially synthesized peptide sequence

<400>  210

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ile Met Asn Trp Val Arg Gln Ala Pro Gly Gln Gly Leu Glu Trp Met
        35                  40                  45

Gly Leu Ile Asn Pro Tyr Asn Gly Gly Thr Asp Tyr Asn Pro Gln Phe
    50                  55                  60

Gln Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Gly Tyr Asp Asp Gly Pro Tyr Thr Leu Glu Thr Trp Gly
            100                 105                 110

Gln Gly Thr Leu Val Thr Val Ser Ser
        115                 120

<210>  211
<211>  121
<212>  PRT
<213>  Artificial

<220>
<223>  An artificially synthesized peptide sequence

<400>  211

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ala Met Asn Trp Val Arg Gln Ala Pro Gly Gln Gly Leu Glu Trp Met
        35                  40                  45

Gly Leu Ile Asn Pro Tyr Asn Gly Gly Thr Asp Tyr Asn Pro Gln Phe
    50                  55                  60

Gln Asp Arg Val Thr Ile Thr Ala Asp Lys Ser Thr Ser Thr Ala Tyr

65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Leu Glu Thr Trp Gly
100 105 110

Gln Gly Thr Leu Val Thr Val Ser Ser
115 120

<210> 212
<211> 121
<212> PRT
<213> Artificial

<220>
<223> An artificially synthesized peptide sequence

<400> 212

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ile Met Asn Trp Val Arg Gln Ala Pro Gly Gln Gly Leu Glu Trp Met
35 40 45

Gly Leu Ile Asn Pro Tyr Asn Gly Gly Thr Asp Tyr Asn Pro Gln Phe
50 55 60

Gln Gly Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Leu Glu Thr Trp Gly
100 105 110

Gln Gly Thr Leu Val Thr Val Ser Ser
115 120

<210> 213
<211> 121
<212> PRT
<213> Artificial

<220>

<223> An artificially synthesized peptide sequence

<400> 213

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ala Met Asn Trp Val Arg Gln Ala Pro Gly Gln Gly Leu Glu Trp Met
35 40 45

Gly Leu Ile Asn Pro Tyr Asn Gly Gly Thr Asp Tyr Asn Pro Gln Phe
50 55 60

Gln Gly Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Leu Glu Thr Trp Gly
100 105 110

Gln Gly Thr Leu Val Thr Val Ser Ser
115 120

<210> 214
<211> 121
<212> PRT
<213> Artificial

<220>
<223> An artificially synthesized peptide sequence

<400> 214

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ile Met Asn Trp Val Arg Gln Ala Pro Gly Gln Gly Leu Glu Trp Met
35 40 45

Gly Leu Ile Asn Pro Tyr Asn Gly Gly Thr Asp Tyr Asn Pro Gln Phe
50 55 60

Gln Gly Arg Val Thr Ile Thr Ala Asp Lys Ser Thr Ser Thr Ala Tyr

65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Leu Glu Thr Trp Gly
100 105 110

Gln Gly Thr Leu Val Thr Val Ser Ser
115 120

<210> 215
<211> 121
<212> PRT
<213> Artificial

<220>
<223> An artificially synthesized peptide sequence

<400> 215

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ala Met Asn Trp Val Arg Gln Ala Pro Gly Gln Gly Leu Glu Trp Met
35 40 45

Gly Leu Ile Asn Pro Tyr Asn Gly Gly Thr Asp Tyr Asn Pro Gln Phe
50 55 60

Gln Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Leu Glu Thr Trp Gly
100 105 110

Gln Gly Thr Leu Val Thr Val Ser Ser
115 120

<210> 216
<211> 121
<212> PRT
<213> Artificial

<220>

<223> An artificially synthesized peptide sequence

<400> 216

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ile Met Asn Trp Val Arg Gln Ala Pro Gly Gln Gly Leu Glu Trp Met
35 40 45

Gly Leu Ile Asn Pro Tyr Asn Gly Gly Thr Ser Tyr Asn Gln Gln Phe
50 55 60

Gln Asp Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Leu Glu Thr Trp Gly
100 105 110

Gln Gly Thr Leu Val Thr Val Ser Ser
115 120

<210> 217
<211> 121
<212> PRT
<213> Artificial

<220>
<223> An artificially synthesized peptide sequence

<400> 217

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ile Met Asn Trp Val Arg Gln Ala Pro Gly Gln Gly Leu Glu Trp Met
35 40 45

Gly Leu Ile Asn Pro Tyr Asn Gly Gly Thr Ser Tyr Asn Gln Gln Phe
50 55 60

Gln Asp Arg Val Thr Ile Thr Ala Asp Lys Ser Thr Ser Thr Ala Tyr

65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Leu Glu Thr Trp Gly
100 105 110

Gln Gly Thr Leu Val Thr Val Ser Ser
115 120

<210> 218
<211> 107
<212> PRT
<213> Artificial

<220>
<223> An artificially synthesized peptide sequence

<400> 218

Asp Ile Gln Met Thr Gln Ser Pro Ser Ser Leu Ser Ala Ser Val Gly
1 5 10 15

Asp Arg Val Thr Ile Thr Cys Gln Thr Ser Glu Asp Ile Tyr Arg Phe
20 25 30

Val Ala Trp Tyr Gln Gln Lys Pro Gly Lys Ala Pro Lys Leu Leu Ile
35 40 45

Tyr Asn Ala Gln Thr Glu Ala Gln Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln His His Tyr Asp Ser Pro Leu
85 90 95

Thr Phe Gly Gly Gly Thr Lys Val Glu Ile Lys
100 105

<210> 219
<211> 107
<212> PRT
<213> Artificial

<220>
<223> An artificially synthesized peptide sequence

<400> 219

```
Asp Ile Gln Met Thr Gln Ser Pro Ser Ser Leu Ser Ala Ser Val Gly
1               5                   10                  15

Asp Arg Val Thr Ile Thr Cys Gln Thr Ser Glu Asp Ile Tyr Ser Phe
            20                  25                  30

Val Ala Trp Tyr Gln Gln Lys Pro Gly Lys Ala Pro Lys Leu Leu Ile
        35                  40                  45

Tyr Asn Ala Gln Thr Glu Ala Gln Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln His His Tyr Asp Ser Pro Leu
                85                  90                  95

Thr Phe Gly Gly Gly Thr Lys Val Glu Ile Lys
            100                 105


<210>  220
<211>  107
<212>  PRT
<213>  Artificial

<220>
<223>  An artificially synthesized peptide sequence

<400>  220

Asp Ile Gln Met Thr Gln Ser Pro Ser Ser Leu Ser Ala Ser Val Gly
1               5                   10                  15

Asp Arg Val Thr Ile Thr Cys Gln Ala Ser Glu Asp Ile Tyr Ser Phe
            20                  25                  30

Val Ala Trp Tyr Gln Gln Lys Pro Gly Lys Ala Pro Lys Leu Leu Ile
        35                  40                  45

Tyr Asn Ala Gln Thr Glu Ala Gln Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln His His Tyr Asp Ser Pro Leu
                85                  90                  95

Thr Phe Gly Gly Gly Thr Lys Val Glu Ile Lys
```

100 105

<210> 221
<211> 107
<212> PRT
<213> Artificial

<220>
<223> An artificially synthesized peptide sequence

<400> 221

Asp Ile Gln Met Thr Gln Ser Pro Ser Ser Leu Ser Ala Ser Val Gly
1 5 10 15

Asp Arg Val Thr Ile Thr Cys Gln Ala Ser Glu Asp Ile Tyr Ser Phe
20 25 30

Leu Ala Trp Tyr Gln Gln Lys Pro Gly Lys Ala Pro Lys Leu Leu Ile
35 40 45

Tyr Asn Ala Gln Thr Glu Ala Gln Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln His His Tyr Asp Ser Pro Leu
85 90 95

Thr Phe Gly Gly Gly Thr Lys Val Glu Ile Lys
100 105

<210> 222
<211> 445
<212> PRT
<213> Artificial

<220>
<223> An artificially synthesized peptide sequence

<400> 222

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Val Leu Asn Trp Val Arg Gln Ala Pro Gly Gln Gly Leu Glu Trp Met
35 40 45

Gly Leu Ile Asn Pro Tyr Asn Gly Gly Thr Asp Tyr Asn Pro Gln Phe
    50                  55                  60

Gln Asp Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Gly Tyr Asp Asp Gly Pro Tyr Thr Met Asp Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Asn Phe Gly Thr Gln Thr Tyr Thr Cys Asn Val Asp His
        195                 200                 205

Lys Pro Ser Asn Thr Lys Val Asp Lys Thr Val Glu Arg Lys Ser Cys
    210                 215                 220

Val Glu Cys Pro Pro Cys Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Phe Arg Val Val Ser
    290                 295                 300

Val Leu Thr Val Val His Gln Asp Trp Leu Asn Gly Lys Glu Tyr Lys
305                 310                 315                 320

Cys Lys Val Ser Asn Lys Gly Leu Pro Ala Pro Ile Glu Lys Thr Ile
                325                 330                 335

Ser Lys Thr Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Met Leu Asp Ser
385                 390                 395                 400

Asp Gly Ser Phe Phe Leu Tyr Ser Lys Leu Thr Val Asp Lys Ser Arg
                405                 410                 415

Trp Gln Glu Gly Asn Val Phe Ser Cys Ser Val Met His Glu Ala Leu
            420                 425                 430

His Asn His Tyr Thr Gln Lys Ser Leu Ser Leu Ser Pro
        435                 440                 445

<210>  223
<211>  445
<212>  PRT
<213>  Artificial

<220>
<223>  An artificially synthesized peptide sequence

<400>  223

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ala Met Asn Trp Val Arg Gln Ala Pro Gly Gln Gly Leu Glu Trp Met
        35                  40                  45

Gly Leu Ile Asn Pro Tyr Asn Gly Gly Thr Asp Tyr Asn Pro Gln Phe
    50                  55                  60

Gln Asp Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Gly Tyr Asp Asp Gly Pro Tyr Thr Met Asp Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Asn Phe Gly Thr Gln Thr Tyr Thr Cys Asn Val Asp His
        195                 200                 205

Lys Pro Ser Asn Thr Lys Val Asp Lys Thr Val Glu Arg Lys Ser Cys
    210                 215                 220

Val Glu Cys Pro Pro Cys Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Phe Arg Val Val Ser
    290                 295                 300

Val Leu Thr Val Val His Gln Asp Trp Leu Asn Gly Lys Glu Tyr Lys
305                 310                 315                 320

Cys Lys Val Ser Asn Lys Gly Leu Pro Ala Pro Ile Glu Lys Thr Ile
325 330 335

Ser Lys Thr Lys Gly Gln Pro Arg Glu Pro Gln Val Tyr Thr Leu Pro
340 345 350

Pro Ser Gln Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Met Leu Asp Ser
385 390 395 400

Asp Gly Ser Phe Phe Leu Tyr Ser Lys Leu Thr Val Asp Lys Ser Arg
405 410 415

Trp Gln Glu Gly Asn Val Phe Ser Cys Ser Val Met His Glu Ala Leu
420 425 430

His Asn His Tyr Thr Gln Lys Ser Leu Ser Leu Ser Pro
435 440 445

<210> 224
<211> 445
<212> PRT
<213> Artificial

<220>
<223> An artificially synthesized peptide sequence

<400> 224

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Val Leu Asn Trp Val Arg Gln Ala Pro Gly Gln Gly Leu Glu Trp Met
35 40 45

Gly Leu Ile Asn Pro Tyr Asn Gly Gly Thr Asp Tyr Asn Pro Gln Phe
50 55 60

Gln Asp Arg Val Thr Ile Thr Ala Asp Glu Ser Thr Ser Thr Ala Tyr
65 70 75 80

```
Met Glu Leu Ser Ser Leu Arg Ser Glu Asp Thr Ala Val Tyr Tyr Cys
                85                  90                  95

Ala Arg Asp Gly Tyr Asp Asp Gly Pro Tyr Thr Leu Glu Thr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Asn Phe Gly Thr Gln Thr Tyr Thr Cys Asn Val Asp His
        195                 200                 205

Lys Pro Ser Asn Thr Lys Val Asp Lys Thr Val Glu Arg Lys Ser Cys
    210                 215                 220

Val Glu Cys Pro Pro Cys Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Phe Arg Val Val Ser
    290                 295                 300

Val Leu Thr Val Val His Gln Asp Trp Leu Asn Gly Lys Glu Tyr Lys
305                 310                 315                 320

Cys Lys Val Ser Asn Lys Gly Leu Pro Ala Pro Ile Glu Lys Thr Ile
                325                 330                 335
```

Ser Lys Thr Lys Gly Gln Pro Arg Glu Pro Gln Val Tyr Thr Leu Pro
340 345 350

Pro Ser Gln Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Met Leu Asp Ser
385 390 395 400

Asp Gly Ser Phe Phe Leu Tyr Ser Lys Leu Thr Val Asp Lys Ser Arg
405 410 415

Trp Gln Glu Gly Asn Val Phe Ser Cys Ser Val Met His Glu Ala Leu
420 425 430

His Asn His Tyr Thr Gln Lys Ser Leu Ser Leu Ser Pro
435 440 445

<210> 225
<211> 445
<212> PRT
<213> Artificial

<220>
<223> An artificially synthesized peptide sequence

<400> 225

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ile Met Asn Trp Val Arg Gln Ala Pro Gly Gln Gly Leu Glu Trp Met
35 40 45

Gly Leu Ile Asn Pro Tyr Asn Gly Gly Thr Asp Tyr Asn Pro Gln Phe
50 55 60

Gln Asp Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

```
Ala Arg Asp Gly Tyr Asp Asp Gly Pro Tyr Thr Leu Glu Thr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Asn Phe Gly Thr Gln Thr Tyr Thr Cys Asn Val Asp His
        195                 200                 205

Lys Pro Ser Asn Thr Lys Val Asp Lys Thr Val Glu Arg Lys Ser Cys
    210                 215                 220

Val Glu Cys Pro Pro Cys Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Phe Arg Val Val Ser
    290                 295                 300

Val Leu Thr Val Val His Gln Asp Trp Leu Asn Gly Lys Glu Tyr Lys
305                 310                 315                 320

Cys Lys Val Ser Asn Lys Gly Leu Pro Ala Pro Ile Glu Lys Thr Ile
                325                 330                 335

Ser Lys Thr Lys Gly Gln Pro Arg Glu Pro Gln Val Tyr Thr Leu Pro
            340                 345                 350
```

Pro Ser Gln Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Met Leu Asp Ser
385 390 395 400

Asp Gly Ser Phe Phe Leu Tyr Ser Lys Leu Thr Val Asp Lys Ser Arg
405 410 415

Trp Gln Glu Gly Asn Val Phe Ser Cys Ser Val Met His Glu Ala Leu
420 425 430

His Asn His Tyr Thr Gln Lys Ser Leu Ser Leu Ser Pro
435 440 445

<210> 226
<211> 445
<212> PRT
<213> Artificial

<220>
<223> An artificially synthesized peptide sequence

<400> 226

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ala Met Asn Trp Val Arg Gln Ala Pro Gly Gln Gly Leu Glu Trp Met
35 40 45

Gly Leu Ile Asn Pro Tyr Asn Gly Gly Thr Asp Tyr Asn Pro Gln Phe
50 55 60

Gln Asp Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Leu Glu Thr Trp Gly
100 105 110

```
Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Asn Phe Gly Thr Gln Thr Tyr Thr Cys Asn Val Asp His
        195                 200                 205

Lys Pro Ser Asn Thr Lys Val Asp Lys Thr Val Glu Arg Lys Ser Cys
    210                 215                 220

Val Glu Cys Pro Pro Cys Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Phe Arg Val Val Ser
    290                 295                 300

Val Leu Thr Val Val His Gln Asp Trp Leu Asn Gly Lys Glu Tyr Lys
305                 310                 315                 320

Cys Lys Val Ser Asn Lys Gly Leu Pro Ala Pro Ile Glu Lys Thr Ile
                325                 330                 335

Ser Lys Thr Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
        355                 360                 365
```

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Met Leu Asp Ser
385                 390                 395                 400

Asp Gly Ser Phe Phe Leu Tyr Ser Lys Leu Thr Val Asp Lys Ser Arg
                405                 410                 415

Trp Gln Glu Gly Asn Val Phe Ser Cys Ser Val Met His Glu Ala Leu
            420                 425                 430

His Asn His Tyr Thr Gln Lys Ser Leu Ser Leu Ser Pro
        435                 440                 445

<210>  227
<211>  445
<212>  PRT
<213>  Artificial

<220>
<223>  An artificially synthesized peptide sequence

<400>  227

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Val Leu Asn Trp Val Arg Gln Ala Pro Gly Gln Gly Leu Glu Trp Met
        35                  40                  45

Gly Leu Ile Asn Pro Tyr Asn Gly Gly Thr Asp Tyr Asn Pro Gln Phe
    50                  55                  60

Gln Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Gly Tyr Asp Asp Gly Pro Tyr Thr Leu Glu Thr Trp Gly
            100                 105                 110

Gln Gly Thr Leu Val Thr Val Ser Ser Ala Ser Thr Lys Gly Pro Ser
        115                 120                 125

```
Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Asn Phe Gly Thr Gln Thr Tyr Thr Cys Asn Val Asp His
        195                 200                 205

Lys Pro Ser Asn Thr Lys Val Asp Lys Thr Val Glu Arg Lys Ser Cys
    210                 215                 220

Val Glu Cys Pro Pro Cys Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Phe Arg Val Val Ser
    290                 295                 300

Val Leu Thr Val Val His Gln Asp Trp Leu Asn Gly Lys Glu Tyr Lys
305                 310                 315                 320

Cys Lys Val Ser Asn Lys Gly Leu Pro Ala Pro Ile Glu Lys Thr Ile
                325                 330                 335

Ser Lys Thr Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380
```

```
Gly Gln Pro Glu Asn Asn Tyr Lys Thr Thr Pro Pro Met Leu Asp Ser
385                 390                 395                 400

Asp Gly Ser Phe Phe Leu Tyr Ser Lys Leu Thr Val Asp Lys Ser Arg
                405                 410                 415

Trp Gln Glu Gly Asn Val Phe Ser Cys Ser Val Met His Glu Ala Leu
            420                 425                 430

His Asn His Tyr Thr Gln Lys Ser Leu Ser Leu Ser Pro
        435                 440                 445


<210>  228
<211>  445
<212>  PRT
<213>  Artificial

<220>
<223>  An artificially synthesized peptide sequence

<400>  228

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ile Met Asn Trp Val Arg Gln Ala Pro Gly Gln Gly Leu Glu Trp Met
        35                  40                  45

Gly Leu Ile Asn Pro Tyr Asn Gly Gly Thr Asp Tyr Asn Pro Gln Phe
    50                  55                  60

Gln Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Gly Tyr Asp Asp Gly Pro Tyr Thr Leu Glu Thr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140
```

```
Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Asn Phe Gly Thr Gln Thr Tyr Thr Cys Asn Val Asp His
        195                 200                 205

Lys Pro Ser Asn Thr Lys Val Asp Lys Thr Val Glu Arg Lys Ser Cys
    210                 215                 220

Val Glu Cys Pro Pro Cys Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Phe Arg Val Val Ser
    290                 295                 300

Val Leu Thr Val Val His Gln Asp Trp Leu Asn Gly Lys Glu Tyr Lys
305                 310                 315                 320

Cys Lys Val Ser Asn Lys Gly Leu Pro Ala Pro Ile Glu Lys Thr Ile
                325                 330                 335

Ser Lys Thr Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Met Leu Asp Ser
385                 390                 395                 400
```

Asp Gly Ser Phe Phe Leu Tyr Ser Lys Leu Thr Val Asp Lys Ser Arg
405 410 415

Trp Gln Glu Gly Asn Val Phe Ser Cys Ser Val Met His Glu Ala Leu
420 425 430

His Asn His Tyr Thr Gln Lys Ser Leu Ser Leu Ser Pro
435 440 445

<210> 229
<211> 445
<212> PRT
<213> Artificial

<220>
<223> An artificially synthesized peptide sequence

<400> 229

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ala Met Asn Trp Val Arg Gln Ala Pro Gly Gln Gly Leu Glu Trp Met
35 40 45

Gly Leu Ile Asn Pro Tyr Asn Gly Gly Thr Asp Tyr Asn Pro Gln Phe
50 55 60

Gln Asp Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Leu Glu Thr Trp Gly
100 105 110

Gln Gly Thr Leu Val Thr Val Ser Ser Ala Ser Thr Lys Gly Pro Ser
115 120 125

Val Phe Pro Leu Ala Pro Ser Ser Lys Ser Thr Ser Gly Gly Thr Ala
130 135 140

Ala Leu Gly Cys Leu Val Lys Asp Tyr Phe Pro Glu Pro Val Thr Val
145 150 155 160

```
Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Asn Phe Gly Thr Gln Thr Tyr Thr Cys Asn Val Asp His
        195                 200                 205

Lys Pro Ser Asn Thr Lys Val Asp Lys Thr Val Glu Arg Lys Ser Cys
    210                 215                 220

Val Glu Cys Pro Pro Cys Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Phe Arg Val Val Ser
    290                 295                 300

Val Leu Thr Val Val His Gln Asp Trp Leu Asn Gly Lys Glu Tyr Lys
305                 310                 315                 320

Cys Lys Val Ser Asn Lys Gly Leu Pro Ala Pro Ile Glu Lys Thr Ile
                325                 330                 335

Ser Lys Thr Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Met Leu Asp Ser
385                 390                 395                 400

Asp Gly Ser Phe Phe Leu Tyr Ser Lys Leu Thr Val Asp Lys Ser Arg
                405                 410                 415
```

Trp Gln Glu Gly Asn Val Phe Ser Cys Ser Val Met His Glu Ala Leu
420 425 430

His Asn His Tyr Thr Gln Lys Ser Leu Ser Leu Ser Pro
435 440 445

<210> 230
<211> 445
<212> PRT
<213> Artificial

<220>
<223> An artificially synthesized peptide sequence

<400> 230

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ile Met Asn Trp Val Arg Gln Ala Pro Gly Gln Gly Leu Glu Trp Met
35 40 45

Gly Leu Ile Asn Pro Tyr Asn Gly Gly Thr Asp Tyr Asn Pro Gln Phe
50 55 60

Gln Gly Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Leu Glu Thr Trp Gly
100 105 110

Gln Gly Thr Leu Val Thr Val Ser Ser Ala Ser Thr Lys Gly Pro Ser
115 120 125

Val Phe Pro Leu Ala Pro Ser Ser Lys Ser Thr Ser Gly Gly Thr Ala
130 135 140

Ala Leu Gly Cys Leu Val Lys Asp Tyr Phe Pro Glu Pro Val Thr Val
145 150 155 160

Ser Trp Asn Ser Gly Ala Leu Thr Ser Gly Val His Thr Phe Pro Ala
165 170 175

Val Leu Gln Ser Ser Gly Leu Tyr Ser Leu Ser Ser Val Val Thr Val
180 185 190

Pro Ser Ser Asn Phe Gly Thr Gln Thr Tyr Thr Cys Asn Val Asp His
195 200 205

Lys Pro Ser Asn Thr Lys Val Asp Lys Thr Val Glu Arg Lys Ser Cys
210 215 220

Val Glu Cys Pro Pro Cys Pro Ala Pro Pro Val Ala Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser Gln Glu Asp Pro Glu
260 265 270

Val Gln Phe Asn Trp Tyr Val Asp Gly Val Glu Val His Asn Ala Lys
275 280 285

Thr Lys Pro Arg Glu Glu Gln Phe Asn Ser Thr Phe Arg Val Val Ser
290 295 300

Val Leu Thr Val Val His Gln Asp Trp Leu Asn Gly Lys Glu Tyr Lys
305 310 315 320

Cys Lys Val Ser Asn Lys Gly Leu Pro Ala Pro Ile Glu Lys Thr Ile
325 330 335

Ser Lys Thr Lys Gly Gln Pro Arg Glu Pro Gln Val Tyr Thr Leu Pro
340 345 350

Pro Ser Gln Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Met Leu Asp Ser
385 390 395 400

Asp Gly Ser Phe Phe Leu Tyr Ser Lys Leu Thr Val Asp Lys Ser Arg
405 410 415

Trp Gln Glu Gly Asn Val Phe Ser Cys Ser Val Met His Glu Ala Leu
420 425 430

His Asn His Tyr Thr Gln Lys Ser Leu Ser Leu Ser Pro
435 440 445

<210> 231
<211> 445
<212> PRT
<213> Artificial

<220>
<223> An artificially synthesized peptide sequence

<400> 231

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ala Met Asn Trp Val Arg Gln Ala Pro Gly Gln Gly Leu Glu Trp Met
35 40 45

Gly Leu Ile Asn Pro Tyr Asn Gly Gly Thr Asp Tyr Asn Pro Gln Phe
50 55 60

Gln Gly Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Leu Glu Thr Trp Gly
100 105 110

Gln Gly Thr Leu Val Thr Val Ser Ser Ala Ser Thr Lys Gly Pro Ser
115 120 125

Val Phe Pro Leu Ala Pro Ser Ser Lys Ser Thr Ser Gly Gly Thr Ala
130 135 140

Ala Leu Gly Cys Leu Val Lys Asp Tyr Phe Pro Glu Pro Val Thr Val
145 150 155 160

Ser Trp Asn Ser Gly Ala Leu Thr Ser Gly Val His Thr Phe Pro Ala
165 170 175

Val Leu Gln Ser Ser Gly Leu Tyr Ser Leu Ser Ser Val Val Thr Val
180 185 190

```
Pro Ser Ser Asn Phe Gly Thr Gln Thr Tyr Thr Cys Asn Val Asp His
        195                 200                 205

Lys Pro Ser Asn Thr Lys Val Asp Lys Thr Val Glu Arg Lys Ser Cys
    210                 215                 220

Val Glu Cys Pro Pro Cys Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Phe Arg Val Val Ser
    290                 295                 300

Val Leu Thr Val Val His Gln Asp Trp Leu Asn Gly Lys Glu Tyr Lys
305                 310                 315                 320

Cys Lys Val Ser Asn Lys Gly Leu Pro Ala Pro Ile Glu Lys Thr Ile
                325                 330                 335

Ser Lys Thr Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Met Leu Asp Ser
385                 390                 395                 400

Asp Gly Ser Phe Phe Leu Tyr Ser Lys Leu Thr Val Asp Lys Ser Arg
                405                 410                 415

Trp Gln Glu Gly Asn Val Phe Ser Cys Ser Val Met His Glu Ala Leu
            420                 425                 430

His Asn His Tyr Thr Gln Lys Ser Leu Ser Leu Ser Pro
        435                 440                 445
```

<210> 232
<211> 445
<212> PRT
<213> Artificial

<220>
<223> An artificially synthesized peptide sequence

<400> 232

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ile Met Asn Trp Val Arg Gln Ala Pro Gly Gln Gly Leu Glu Trp Met
        35                  40                  45

Gly Leu Ile Asn Pro Tyr Asn Gly Gly Thr Asp Tyr Asn Pro Gln Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Gly Tyr Asp Asp Gly Pro Tyr Thr Leu Glu Thr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Asn Phe Gly Thr Gln Thr Tyr Thr Cys Asn Val Asp His
        195                 200                 205
```

```
Lys Pro Ser Asn Thr Lys Val Asp Lys Thr Val Glu Arg Lys Ser Cys
    210                 215                 220

Val Glu Cys Pro Pro Cys Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Phe Arg Val Val Ser
    290                 295                 300

Val Leu Thr Val Val His Gln Asp Trp Leu Asn Gly Lys Glu Tyr Lys
305                 310                 315                 320

Cys Lys Val Ser Asn Lys Gly Leu Pro Ala Pro Ile Glu Lys Thr Ile
                325                 330                 335

Ser Lys Thr Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Met Leu Asp Ser
385                 390                 395                 400

Asp Gly Ser Phe Phe Leu Tyr Ser Lys Leu Thr Val Asp Lys Ser Arg
                405                 410                 415

Trp Gln Glu Gly Asn Val Phe Ser Cys Ser Val Met His Glu Ala Leu
            420                 425                 430

His Asn His Tyr Thr Gln Lys Ser Leu Ser Leu Ser Pro
        435                 440                 445

<210>  233
<211>  445
<212>  PRT
```

<213>  Artificial

<220>
<223>  An artificially synthesized peptide sequence

<400>  233

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ala Met Asn Trp Val Arg Gln Ala Pro Gly Gln Gly Leu Glu Trp Met
        35                  40                  45

Gly Leu Ile Asn Pro Tyr Asn Gly Gly Thr Asp Tyr Asn Pro Gln Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Gly Tyr Asp Asp Gly Pro Tyr Thr Leu Glu Thr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Asn Phe Gly Thr Gln Thr Tyr Thr Cys Asn Val Asp His
        195                 200                 205

Lys Pro Ser Asn Thr Lys Val Asp Lys Thr Val Glu Arg Lys Ser Cys
    210                 215                 220
```

Val Glu Cys Pro Pro Cys Pro Ala Pro Pro Val Ala Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser Gln Glu Asp Pro Glu
260 265 270

Val Gln Phe Asn Trp Tyr Val Asp Gly Val Glu Val His Asn Ala Lys
275 280 285

Thr Lys Pro Arg Glu Glu Gln Phe Asn Ser Thr Phe Arg Val Val Ser
290 295 300

Val Leu Thr Val Val His Gln Asp Trp Leu Asn Gly Lys Glu Tyr Lys
305 310 315 320

Cys Lys Val Ser Asn Lys Gly Leu Pro Ala Pro Ile Glu Lys Thr Ile
325 330 335

Ser Lys Thr Lys Gly Gln Pro Arg Glu Pro Gln Val Tyr Thr Leu Pro
340 345 350

Pro Ser Gln Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Met Leu Asp Ser
385 390 395 400

Asp Gly Ser Phe Phe Leu Tyr Ser Lys Leu Thr Val Asp Lys Ser Arg
405 410 415

Trp Gln Glu Gly Asn Val Phe Ser Cys Ser Val Met His Glu Ala Leu
420 425 430

His Asn His Tyr Thr Gln Lys Ser Leu Ser Leu Ser Pro
435 440 445

<210> 234
<211> 445
<212> PRT
<213> Artificial

<220>
<223> An artificially synthesized peptide sequence

<400> 234

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ile Met Asn Trp Val Arg Gln Ala Pro Gly Gln Gly Leu Glu Trp Met
        35                  40                  45

Gly Leu Ile Asn Pro Tyr Asn Gly Gly Thr Ser Tyr Asn Gln Gln Phe
    50                  55                  60

Gln Asp Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Gly Tyr Asp Asp Gly Pro Tyr Thr Leu Glu Thr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Asn Phe Gly Thr Gln Thr Tyr Thr Cys Asn Val Asp His
        195                 200                 205

Lys Pro Ser Asn Thr Lys Val Asp Lys Thr Val Glu Arg Lys Ser Cys
    210                 215                 220

Val Glu Cys Pro Pro Cys Pro Ala Pro Pro Val Ala Gly Pro Ser Val
225                 230                 235                 240
```

Phe Leu Phe Pro Pro Lys Pro Lys Asp Thr Leu Met Ile Ser Arg Thr
245 250 255

Pro Glu Val Thr Cys Val Val Val Asp Val Ser Gln Glu Asp Pro Glu
260 265 270

Val Gln Phe Asn Trp Tyr Val Asp Gly Val Glu Val His Asn Ala Lys
275 280 285

Thr Lys Pro Arg Glu Glu Gln Phe Asn Ser Thr Phe Arg Val Val Ser
290 295 300

Val Leu Thr Val Val His Gln Asp Trp Leu Asn Gly Lys Glu Tyr Lys
305 310 315 320

Cys Lys Val Ser Asn Lys Gly Leu Pro Ala Pro Ile Glu Lys Thr Ile
325 330 335

Ser Lys Thr Lys Gly Gln Pro Arg Glu Pro Gln Val Tyr Thr Leu Pro
340 345 350

Pro Ser Gln Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Met Leu Asp Ser
385 390 395 400

Asp Gly Ser Phe Phe Leu Tyr Ser Lys Leu Thr Val Asp Lys Ser Arg
405 410 415

Trp Gln Glu Gly Asn Val Phe Ser Cys Ser Val Met His Glu Ala Leu
420 425 430

His Asn His Tyr Thr Gln Lys Ser Leu Ser Leu Ser Pro
435 440 445

<210> 235
<211> 445
<212> PRT
<213> Artificial

<220>
<223> An artificially synthesized peptide sequence

<400> 235

Gln Val Gln Leu Val Gln Ser Gly Ala Glu Val Lys Lys Pro Gly Ala

```
1               5                   10                  15

Ser Val Lys Val Ser Cys Lys Ala Ser Gly Tyr Thr Phe Thr Gly Tyr
            20                  25                  30

Ile Met Asn Trp Val Arg Gln Ala Pro Gly Gln Gly Leu Glu Trp Met
        35                  40                  45

Gly Leu Ile Asn Pro Tyr Asn Gly Gly Thr Ser Tyr Asn Gln Gln Phe
    50                  55                  60

Gln Asp Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Gly Tyr Asp Asp Gly Pro Tyr Thr Leu Glu Thr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Asn Phe Gly Thr Gln Thr Tyr Thr Cys Asn Val Asp His
        195                 200                 205

Lys Pro Ser Asn Thr Lys Val Asp Lys Thr Val Glu Arg Lys Ser Cys
    210                 215                 220

Val Glu Cys Pro Pro Cys Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255
```

```
Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Phe Arg Val Val Ser
    290                 295                 300

Val Leu Thr Val Val His Gln Asp Trp Leu Asn Gly Lys Glu Tyr Lys
305                 310                 315                 320

Cys Lys Val Ser Asn Lys Gly Leu Pro Ala Pro Ile Glu Lys Thr Ile
                325                 330                 335

Ser Lys Thr Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Met Leu Asp Ser
385                 390                 395                 400

Asp Gly Ser Phe Phe Leu Tyr Ser Lys Leu Thr Val Asp Lys Ser Arg
                405                 410                 415

Trp Gln Glu Gly Asn Val Phe Ser Cys Ser Val Met His Glu Ala Leu
            420                 425                 430

His Asn His Tyr Thr Gln Lys Ser Leu Ser Leu Ser Pro
        435                 440                 445


<210>  236
<211>  214
<212>  PRT
<213>  Artificial

<220>
<223>  An artificially synthesized peptide sequence

<400>  236

Asp Ile Gln Met Thr Gln Ser Pro Ser Ser Leu Ser Ala Ser Val Gly
1               5                   10                  15

Asp Arg Val Thr Ile Thr Cys Gln Thr Ser Glu Asp Ile Tyr Arg Phe
```

```
            20                  25                  30

Val Ala Trp Tyr Gln Gln Lys Pro Gly Lys Ala Pro Lys Leu Leu Ile
        35                  40                  45

Tyr Asn Ala Gln Thr Glu Ala Gln Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln His His Tyr Asp Ser Pro Leu
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210


<210>  237
<211>  214
<212>  PRT
<213>  Artificial

<220>
<223>  An artificially synthesized peptide sequence

<400>  237

Asp Ile Gln Met Thr Gln Ser Pro Ser Ser Leu Ser Ala Ser Val Gly
1               5                   10                  15
```

Asp Arg Val Thr Ile Thr Cys Gln Thr Ser Glu Asp Ile Tyr Ser Phe
            20                  25                  30

Val Ala Trp Tyr Gln Gln Lys Pro Gly Lys Ala Pro Lys Leu Leu Ile
        35                  40                  45

Tyr Asn Ala Gln Thr Glu Ala Gln Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln His His Tyr Asp Ser Pro Leu
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 238
<211> 214
<212> PRT
<213> Artificial

<220>
<223> An artificially synthesized peptide sequence

<400> 238

Asp Ile Gln Met Thr Gln Ser Pro Ser Ser Leu Ser Ala Ser Val Gly
1 5 10 15

Asp Arg Val Thr Ile Thr Cys Gln Ala Ser Glu Asp Ile Tyr Ser Phe
20 25 30

Val Ala Trp Tyr Gln Gln Lys Pro Gly Lys Ala Pro Lys Leu Leu Ile
35 40 45

Tyr Asn Ala Gln Thr Glu Ala Gln Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln His His Tyr Asp Ser Pro Leu
85 90 95

Thr Phe Gly Gly Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 239
<211> 214
<212> PRT
<213> Artificial

<220>

<223> An artificially synthesized peptide sequence

<400> 239

Asp Ile Gln Met Thr Gln Ser Pro Ser Ser Leu Ser Ala Ser Val Gly
1 5 10 15

Asp Arg Val Thr Ile Thr Cys Gln Ala Ser Glu Asp Ile Tyr Ser Phe
20 25 30

Leu Ala Trp Tyr Gln Gln Lys Pro Gly Lys Ala Pro Lys Leu Leu Ile
35 40 45

Tyr Asn Ala Gln Thr Glu Ala Gln Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln His His Tyr Asp Ser Pro Leu
85 90 95

Thr Phe Gly Gly Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 240
<211> 445
<212> PRT

<213>  Artificial

<220>
<223>  An artificially synthesized peptide sequence

<400>  240

```
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ile Met Asn Trp Val Arg Gln Ala Pro Gly Gln Gly Leu Glu Trp Met
        35                  40                  45

Gly Leu Ile Asn Pro Tyr Asn Gly Gly Thr Ser Tyr Asn Gln Gln Phe
    50                  55                  60

Gln Asp Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Gly Tyr Asp Asp Gly Pro Tyr Thr Met Asp Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Asn Phe Gly Thr Gln Thr Tyr Thr Cys Asn Val Asp His
        195                 200                 205

Lys Pro Ser Asn Thr Lys Val Asp Lys Thr Val Glu Arg Lys Ser Cys
    210                 215                 220
```

Val Glu Cys Pro Pro Cys Pro Ala Pro Pro Val Ala Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser Gln Glu Asp Pro Glu
260 265 270

Val Gln Phe Asn Trp Tyr Val Asp Gly Val Glu Val His Asn Ala Lys
275 280 285

Thr Lys Pro Arg Glu Glu Gln Phe Asn Ser Thr Phe Arg Val Val Ser
290 295 300

Val Leu Thr Val Val His Gln Asp Trp Leu Asn Gly Lys Glu Tyr Lys
305 310 315 320

Cys Lys Val Ser Asn Lys Gly Leu Pro Ala Pro Ile Glu Lys Thr Ile
325 330 335

Ser Lys Thr Lys Gly Gln Pro Arg Glu Pro Gln Val Tyr Thr Leu Pro
340 345 350

Pro Ser Gln Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Met Leu Asp Ser
385 390 395 400

Asp Gly Ser Phe Phe Leu Tyr Ser Lys Leu Thr Val Asp Lys Ser Arg
405 410 415

Trp Gln Glu Gly Asn Val Phe Ser Cys Ser Val Met His Glu Ala Leu
420 425 430

His Asn His Tyr Thr Gln Lys Ser Leu Ser Leu Ser Pro
435 440 445

<210> 241
<211> 447
<212> PRT
<213> Artificial

<220>
<223> An artificially synthesized peptide sequence

<400>  241

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ile Met Asn Trp Val Arg Gln Ala Pro Gly Gln Gly Leu Glu Trp Met
        35                  40                  45

Gly Leu Ile Asn Pro Tyr Asn Gly Gly Thr Ser Tyr Asn Gln Gln Phe
    50                  55                  60

Gln Asp Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Gly Tyr Asp Asp Gly Pro Tyr Thr Met Asp Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Cys Ser Arg Ser Thr Ser Glu Ser Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Asn Phe Gly Thr Gln Thr Tyr Thr Cys Asn Val Asp His
        195                 200                 205

Lys Pro Ser Asn Thr Lys Val Asp Lys Thr Val Glu Arg Lys Cys Cys
    210                 215                 220

Val Glu Cys Pro Pro Cys Pro Ala Pro Pro Val Ala Gly Pro Ser Val
225                 230                 235                 240

Phe Leu Phe Pro Pro Lys Pro Lys Asp Thr Leu Met Ile Ser Arg Thr
245 250 255

Pro Glu Val Thr Cys Val Val Val Asp Val Ser His Glu Asp Pro Glu
260 265 270

Val Gln Phe Asn Trp Tyr Val Asp Gly Val Glu Val His Asn Ala Lys
275 280 285

Thr Lys Pro Arg Glu Glu Gln Phe Asn Ser Thr Phe Arg Val Val Ser
290 295 300

Val Leu Thr Val Val His Gln Asp Trp Leu Asn Gly Lys Glu Tyr Lys
305 310 315 320

Cys Lys Val Ser Asn Lys Gly Leu Pro Ala Pro Ile Glu Lys Thr Ile
325 330 335

Ser Lys Thr Lys Gly Gln Pro Arg Glu Pro Gln Val Tyr Thr Leu Pro
340 345 350

Pro Ser Arg Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Met Leu Asp Ser
385 390 395 400

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Lys
435 440 445

<210> 242
<211> 447
<212> PRT
<213> Artificial

<220>
<223> An artificially synthesized peptide sequence

<400> 242

Gln Val Gln Leu Val Gln Ser Gly Ala Glu Val Lys Lys Pro Gly Ala

```
1               5                   10                  15

Ser Val Lys Val Ser Cys Lys Ala Ser Gly Tyr Thr Phe Thr Gly Tyr
            20                  25                  30

Ile Met Asn Trp Val Arg Gln Ala Pro Gly Gln Gly Leu Glu Trp Met
        35                  40                  45

Gly Leu Ile Asn Pro Tyr Asn Gly Gly Thr Ser Tyr Asn Gln Lys Phe
    50                  55                  60

Lys Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Gly Tyr Asp Asp Gly Pro Tyr Thr Leu Glu Thr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Glu Ser Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Asn Phe Gly Thr Gln Thr Tyr Thr Cys Asn Val Asp His
        195                 200                 205

Lys Pro Ser Asn Thr Lys Val Asp Lys Thr Val Glu Arg Lys Ser Cys
    210                 215                 220

Val Glu Cys Pro Pro Cys Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255
```

Pro Glu Val Thr Cys Val Val Val Asp Val Ser His Glu Asp Pro Glu
260 265 270

Val Gln Phe Asn Trp Tyr Val Asp Gly Val Glu Val His Asn Ala Lys
275 280 285

Thr Lys Pro Arg Glu Glu Gln Phe Asn Ser Thr Phe Arg Val Val Ser
290 295 300

Val Leu Thr Val Val His Gln Asp Trp Leu Asn Gly Lys Glu Tyr Lys
305 310 315 320

Cys Lys Val Ser Asn Lys Gly Leu Pro Ala Pro Ile Glu Lys Thr Ile
325 330 335

Ser Lys Thr Lys Gly Gln Pro Arg Glu Pro Gln Val Tyr Thr Leu Pro
340 345 350

Pro Ser Arg Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Met Leu Asp Ser
385 390 395 400

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Lys
435 440 445

<210> 243
<211> 447
<212> PRT
<213> Artificial

<220>
<223> An artificially synthesized peptide sequence

<400> 243

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr

20 25 30

Ile Met Asn Trp Val Arg Gln Ala Pro Gly Gln Gly Leu Glu Trp Met
35 40 45

Gly Leu Ile Asn Pro Tyr Asn Gly Gly Thr Ser Tyr Asn Gln Lys Phe
50 55 60

Lys Gly Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Met Glu Thr Trp Gly
100 105 110

Gln Gly Thr Leu Val Thr Val Ser Ser Ala Ser Thr Lys Gly Pro Ser
115 120 125

Val Phe Pro Leu Ala Pro Ser Ser Lys Ser Thr Ser Glu Ser Thr Ala
130 135 140

Ala Leu Gly Cys Leu Val Lys Asp Tyr Phe Pro Glu Pro Val Thr Val
145 150 155 160

Ser Trp Asn Ser Gly Ala Leu Thr Ser Gly Val His Thr Phe Pro Ala
165 170 175

Val Leu Gln Ser Ser Gly Leu Tyr Ser Leu Ser Ser Val Val Thr Val
180 185 190

Pro Ser Ser Asn Phe Gly Thr Gln Thr Tyr Thr Cys Asn Val Asp His
195 200 205

Lys Pro Ser Asn Thr Lys Val Asp Lys Thr Val Glu Arg Lys Ser Cys
210 215 220

Val Glu Cys Pro Pro Cys Pro Ala Pro Pro Val Ala Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser His Glu Asp Pro Glu
260 265 270

Val Gln Phe Asn Trp Tyr Val Asp Gly Val Glu Val His Asn Ala Lys
275 280 285

Thr Lys Pro Arg Glu Glu Gln Phe Asn Ser Thr Phe Arg Val Val Ser
290 295 300

Val Leu Thr Val Val His Gln Asp Trp Leu Asn Gly Lys Glu Tyr Lys
305 310 315 320

Cys Lys Val Ser Asn Lys Gly Leu Pro Ala Pro Ile Glu Lys Thr Ile
325 330 335

Ser Lys Thr Lys Gly Gln Pro Arg Glu Pro Gln Val Tyr Thr Leu Pro
340 345 350

Pro Ser Arg Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Met Leu Asp Ser
385 390 395 400

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Lys
435 440 445

<210> 244
<211> 447
<212> PRT
<213> Artificial

<220>
<223> An artificially synthesized peptide sequence

<400> 244

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ile Met Asn Trp Val Arg Gln Ala Pro Gly Gln Gly Leu Glu Trp Met

35 40 45

Gly Leu Ile Asn Pro Tyr Asn Gly Gly Thr Ser Tyr Asn Gln Lys Phe
50 55 60

Lys Gly Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Leu Asp Asp Gly Pro Tyr Thr Met Glu Thr Trp Gly
100 105 110

Gln Gly Thr Leu Val Thr Val Ser Ser Ala Ser Thr Lys Gly Pro Ser
115 120 125

Val Phe Pro Leu Ala Pro Ser Ser Lys Ser Thr Ser Glu Ser Thr Ala
130 135 140

Ala Leu Gly Cys Leu Val Lys Asp Tyr Phe Pro Glu Pro Val Thr Val
145 150 155 160

Ser Trp Asn Ser Gly Ala Leu Thr Ser Gly Val His Thr Phe Pro Ala
165 170 175

Val Leu Gln Ser Ser Gly Leu Tyr Ser Leu Ser Ser Val Val Thr Val
180 185 190

Pro Ser Ser Asn Phe Gly Thr Gln Thr Tyr Thr Cys Asn Val Asp His
195 200 205

Lys Pro Ser Asn Thr Lys Val Asp Lys Thr Val Glu Arg Lys Ser Cys
210 215 220

Val Glu Cys Pro Pro Cys Pro Ala Pro Pro Val Ala Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser His Glu Asp Pro Glu
260 265 270

Val Gln Phe Asn Trp Tyr Val Asp Gly Val Glu Val His Asn Ala Lys
275 280 285

Thr Lys Pro Arg Glu Glu Gln Phe Asn Ser Thr Phe Arg Val Val Ser
    290                 295                 300

Val Leu Thr Val Val His Gln Asp Trp Leu Asn Gly Lys Glu Tyr Lys
305                 310                 315                 320

Cys Lys Val Ser Asn Lys Gly Leu Pro Ala Pro Ile Glu Lys Thr Ile
                325                 330                 335

Ser Lys Thr Lys Gly Gln Pro Arg Glu Pro Gln Val Tyr Thr Leu Pro
            340                 345                 350

Pro Ser Arg Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Met Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly Lys
        435                 440                 445

<210>  245
<211>  447
<212>  PRT
<213>  Artificial

<220>
<223>  An artificially synthesized peptide sequence

<400>  245

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ile Met Asn Trp Val Arg Gln Ala Pro Gly Gln Gly Leu Glu Trp Met
        35                  40                  45

Gly Leu Ile Asn Pro Tyr Asn Gly Gly Thr Ser Tyr Asn Gln Lys Phe

50 55 60

Lys Gly Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Leu Asp Asp Gly Pro Tyr Thr Met Glu Ser Trp Gly
100 105 110

Gln Gly Thr Leu Val Thr Val Ser Ser Ala Ser Thr Lys Gly Pro Ser
115 120 125

Val Phe Pro Leu Ala Pro Ser Ser Lys Ser Thr Ser Glu Ser Thr Ala
130 135 140

Ala Leu Gly Cys Leu Val Lys Asp Tyr Phe Pro Glu Pro Val Thr Val
145 150 155 160

Ser Trp Asn Ser Gly Ala Leu Thr Ser Gly Val His Thr Phe Pro Ala
165 170 175

Val Leu Gln Ser Ser Gly Leu Tyr Ser Leu Ser Ser Val Val Thr Val
180 185 190

Pro Ser Ser Asn Phe Gly Thr Gln Thr Tyr Thr Cys Asn Val Asp His
195 200 205

Lys Pro Ser Asn Thr Lys Val Asp Lys Thr Val Glu Arg Lys Ser Cys
210 215 220

Val Glu Cys Pro Pro Cys Pro Ala Pro Pro Val Ala Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser His Glu Asp Pro Glu
260 265 270

Val Gln Phe Asn Trp Tyr Val Asp Gly Val Glu Val His Asn Ala Lys
275 280 285

Thr Lys Pro Arg Glu Glu Gln Phe Asn Ser Thr Phe Arg Val Val Ser
290 295 300

Val Leu Thr Val Val His Gln Asp Trp Leu Asn Gly Lys Glu Tyr Lys
305 310 315 320

Cys Lys Val Ser Asn Lys Gly Leu Pro Ala Pro Ile Glu Lys Thr Ile
325 330 335

Ser Lys Thr Lys Gly Gln Pro Arg Glu Pro Gln Val Tyr Thr Leu Pro
340 345 350

Pro Ser Arg Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Met Leu Asp Ser
385 390 395 400

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Lys
435 440 445

<210> 246
<211> 447
<212> PRT
<213> Artificial

<220>
<223> An artificially synthesized peptide sequence

<400> 246

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Val Met Asn Trp Val Arg Gln Ala Pro Gly Gln Gly Leu Glu Trp Met
35 40 45

Gly Leu Ile Asn Pro Tyr Asn Gly Gly Thr Ser Tyr Asn Gln Lys Phe
50 55 60

Lys Gly Arg Val Thr Ile Thr Ala Asp Glu Ser Thr Ser Thr Ala Tyr

```
65                  70                  75                  80

Met Glu Leu Ser Ser Leu Arg Ser Glu Asp Thr Ala Val Tyr Tyr Cys
                85                  90                  95

Ala Arg Asp Gly Tyr Asp Asp Gly Pro Tyr Thr Met Asp Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Glu Ser Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Asn Phe Gly Thr Gln Thr Tyr Thr Cys Asn Val Asp His
        195                 200                 205

Lys Pro Ser Asn Thr Lys Val Asp Lys Thr Val Glu Arg Lys Ser Cys
    210                 215                 220

Val Glu Cys Pro Pro Cys Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Phe Arg Val Val Ser
    290                 295                 300

Val Leu Thr Val Val His Gln Asp Trp Leu Asn Gly Lys Glu Tyr Lys
305                 310                 315                 320
```

Cys Lys Val Ser Asn Lys Gly Leu Pro Ala Pro Ile Glu Lys Thr Ile
325 330 335

Ser Lys Thr Lys Gly Gln Pro Arg Glu Pro Gln Val Tyr Thr Leu Pro
340 345 350

Pro Ser Arg Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Met Leu Asp Ser
385 390 395 400

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Lys
435 440 445

<210> 247
<211> 447
<212> PRT
<213> Artificial

<220>
<223> An artificially synthesized peptide sequence

<400> 247

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ile Ile Asn Trp Val Arg Gln Ala Pro Gly Gln Gly Leu Glu Trp Met
35 40 45

Gly Leu Ile Asn Pro Tyr Asn Gly Gly Thr Ser Tyr Asn Gln Lys Phe
50 55 60

Lys Gly Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys

```
                85                  90                  95

Ala Arg Asp Gly Tyr Asp Asp Gly Pro Tyr Thr Met Asp Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Glu Ser Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Asn Phe Gly Thr Gln Thr Tyr Thr Cys Asn Val Asp His
        195                 200                 205

Lys Pro Ser Asn Thr Lys Val Asp Lys Thr Val Glu Arg Lys Ser Cys
    210                 215                 220

Val Glu Cys Pro Pro Cys Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Phe Arg Val Val Ser
    290                 295                 300

Val Leu Thr Val Val His Gln Asp Trp Leu Asn Gly Lys Glu Tyr Lys
305                 310                 315                 320

Cys Lys Val Ser Asn Lys Gly Leu Pro Ala Pro Ile Glu Lys Thr Ile
                325                 330                 335
```

Ser Lys Thr Lys Gly Gln Pro Arg Glu Pro Gln Val Tyr Thr Leu Pro
340 345 350

Pro Ser Arg Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Met Leu Asp Ser
385 390 395 400

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Lys
435 440 445

<210> 248
<211> 447
<212> PRT
<213> Artificial

<220>
<223> An artificially synthesized peptide sequence

<400> 248

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ile Leu Asn Trp Val Arg Gln Ala Pro Gly Gln Gly Leu Glu Trp Met
35 40 45

Gly Leu Ile Asn Pro Tyr Asn Gly Gly Thr Ser Tyr Asn Gln Lys Phe
50 55 60

Lys Gly Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Met Asp Tyr Trp Gly

100 105 110

Gln Gly Thr Leu Val Thr Val Ser Ser Ala Ser Thr Lys Gly Pro Ser
115 120 125

Val Phe Pro Leu Ala Pro Ser Ser Lys Ser Thr Ser Glu Ser Thr Ala
130 135 140

Ala Leu Gly Cys Leu Val Lys Asp Tyr Phe Pro Glu Pro Val Thr Val
145 150 155 160

Ser Trp Asn Ser Gly Ala Leu Thr Ser Gly Val His Thr Phe Pro Ala
165 170 175

Val Leu Gln Ser Ser Gly Leu Tyr Ser Leu Ser Ser Val Val Thr Val
180 185 190

Pro Ser Ser Asn Phe Gly Thr Gln Thr Tyr Thr Cys Asn Val Asp His
195 200 205

Lys Pro Ser Asn Thr Lys Val Asp Lys Thr Val Glu Arg Lys Ser Cys
210 215 220

Val Glu Cys Pro Pro Cys Pro Ala Pro Pro Val Ala Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser His Glu Asp Pro Glu
260 265 270

Val Gln Phe Asn Trp Tyr Val Asp Gly Val Glu Val His Asn Ala Lys
275 280 285

Thr Lys Pro Arg Glu Glu Gln Phe Asn Ser Thr Phe Arg Val Val Ser
290 295 300

Val Leu Thr Val Val His Gln Asp Trp Leu Asn Gly Lys Glu Tyr Lys
305 310 315 320

Cys Lys Val Ser Asn Lys Gly Leu Pro Ala Pro Ile Glu Lys Thr Ile
325 330 335

Ser Lys Thr Lys Gly Gln Pro Arg Glu Pro Gln Val Tyr Thr Leu Pro
340 345 350

Pro Ser Arg Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Met Leu Asp Ser
385 390 395 400

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Lys
435 440 445

<210> 249
<211> 447
<212> PRT
<213> Artificial

<220>
<223> An artificially synthesized peptide sequence

<400> 249

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ile Met Asn Trp Val Arg Gln Ala Pro Gly Gln Gly Leu Glu Trp Met
35 40 45

Gly Leu Ile Asn Pro Tyr Asn Gly Gly Thr Asp Tyr Asn Gln Lys Phe
50 55 60

Lys Gly Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Met Asp Tyr Trp Gly
100 105 110

Gln Gly Thr Leu Val Thr Val Ser Ser Ala Ser Thr Lys Gly Pro Ser

115 120 125

Val Phe Pro Leu Ala Pro Ser Ser Lys Ser Thr Ser Glu Ser Thr Ala
130 135 140

Ala Leu Gly Cys Leu Val Lys Asp Tyr Phe Pro Glu Pro Val Thr Val
145 150 155 160

Ser Trp Asn Ser Gly Ala Leu Thr Ser Gly Val His Thr Phe Pro Ala
165 170 175

Val Leu Gln Ser Ser Gly Leu Tyr Ser Leu Ser Ser Val Val Thr Val
180 185 190

Pro Ser Ser Asn Phe Gly Thr Gln Thr Tyr Thr Cys Asn Val Asp His
195 200 205

Lys Pro Ser Asn Thr Lys Val Asp Lys Thr Val Glu Arg Lys Ser Cys
210 215 220

Val Glu Cys Pro Pro Cys Pro Ala Pro Pro Val Ala Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser His Glu Asp Pro Glu
260 265 270

Val Gln Phe Asn Trp Tyr Val Asp Gly Val Glu Val His Asn Ala Lys
275 280 285

Thr Lys Pro Arg Glu Glu Gln Phe Asn Ser Thr Phe Arg Val Val Ser
290 295 300

Val Leu Thr Val Val His Gln Asp Trp Leu Asn Gly Lys Glu Tyr Lys
305 310 315 320

Cys Lys Val Ser Asn Lys Gly Leu Pro Ala Pro Ile Glu Lys Thr Ile
325 330 335

Ser Lys Thr Lys Gly Gln Pro Arg Glu Pro Gln Val Tyr Thr Leu Pro
340 345 350

Pro Ser Arg Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Met Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly Lys
        435                 440                 445

<210> 250
<211> 447
<212> PRT
<213> Artificial

<220>
<223> An artificially synthesized peptide sequence

<400> 250

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr
            20                  25                  30

Ile Met Asn Trp Val Arg Gln Ala Pro Gly Gln Gly Leu Glu Trp Met
        35                  40                  45

Gly Leu Ile Asn Pro Tyr Asn Gly Gly Thr Ser Tyr Asn Pro Lys Phe
    50                  55                  60

Lys Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asp Gly Tyr Asp Asp Gly Pro Tyr Thr Met Asp Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Glu Ser Thr Ala

130 135 140

Ala Leu Gly Cys Leu Val Lys Asp Tyr Phe Pro Glu Pro Val Thr Val
145 150 155 160

Ser Trp Asn Ser Gly Ala Leu Thr Ser Gly Val His Thr Phe Pro Ala
165 170 175

Val Leu Gln Ser Ser Gly Leu Tyr Ser Leu Ser Ser Val Val Thr Val
180 185 190

Pro Ser Ser Asn Phe Gly Thr Gln Thr Tyr Thr Cys Asn Val Asp His
195 200 205

Lys Pro Ser Asn Thr Lys Val Asp Lys Thr Val Glu Arg Lys Ser Cys
210 215 220

Val Glu Cys Pro Pro Cys Pro Ala Pro Pro Val Ala Gly Pro Ser Val
225 230 235 240

Phe Leu Phe Pro Pro Lys Pro Lys Asp Thr Leu Met Ile Ser Arg Thr
245 250 255

Pro Glu Val Thr Cys Val Val Val Asp Val Ser His Glu Asp Pro Glu
260 265 270

Val Gln Phe Asn Trp Tyr Val Asp Gly Val Glu Val His Asn Ala Lys
275 280 285

Thr Lys Pro Arg Glu Glu Gln Phe Asn Ser Thr Phe Arg Val Val Ser
290 295 300

Val Leu Thr Val Val His Gln Asp Trp Leu Asn Gly Lys Glu Tyr Lys
305 310 315 320

Cys Lys Val Ser Asn Lys Gly Leu Pro Ala Pro Ile Glu Lys Thr Ile
325 330 335

Ser Lys Thr Lys Gly Gln Pro Arg Glu Pro Gln Val Tyr Thr Leu Pro
340 345 350

Pro Ser Arg Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Met Leu Asp Ser
385 390 395 400

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Lys
435 440 445

<210> 251
<211> 214
<212> PRT
<213> Artificial

<220>
<223> An artificially synthesized peptide sequence

<400> 251

Asp Ile Gln Met Thr Gln Ser Pro Ser Ser Leu Ser Ala Ser Val Gly
1 5 10 15

Asp Arg Val Thr Ile Thr Cys Arg Thr Ser Glu Asn Ile Tyr Arg Phe
20 25 30

Leu Ala Trp Tyr Gln Gln Lys Pro Gly Lys Ala Pro Lys Leu Leu Ile
35 40 45

Tyr Asn Ala Lys Thr Leu Ala Lys Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln His His Tyr Glu Ser Pro Leu
85 90 95

Thr Phe Gly Gly Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln

145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 252
<211> 214
<212> PRT
<213> Artificial

<220>
<223> An artificially synthesized peptide sequence

<400> 252

Asp Ile Gln Met Thr Gln Ser Pro Ser Ser Leu Ser Ala Ser Val Gly
1 5 10 15

Asp Arg Val Thr Ile Thr Cys Arg Thr Ser Glu Asn Ile Tyr Arg Phe
20 25 30

Val Ala Trp Tyr Gln Gln Lys Pro Gly Lys Ala Pro Lys Leu Leu Ile
35 40 45

Tyr Asn Ala Lys Thr Leu Ala Lys Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln His His Tyr Glu Ser Pro Leu
85 90 95

Thr Phe Gly Gly Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 253
<211> 214
<212> PRT
<213> Artificial

<220>
<223> An artificially synthesized peptide sequence

<400> 253

Asp Ile Gln Met Thr Gln Ser Pro Ser Ser Leu Ser Ala Ser Val Gly
1 5 10 15

Asp Arg Val Thr Ile Thr Cys Arg Thr Ser Glu Asn Ile Tyr Ser Phe
20 25 30

Leu Ala Trp Tyr Gln Gln Lys Pro Gly Lys Ala Pro Lys Leu Leu Ile
35 40 45

Tyr Asn Ala Lys Thr Leu Ala Lys Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln His His Tyr Asp Ser Pro Leu
85 90 95

Thr Phe Gly Gly Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 254
<211> 214
<212> PRT
<213> Artificial

<220>
<223> An artificially synthesized peptide sequence

<400> 254

Asp Ile Gln Met Thr Gln Ser Pro Ser Ser Leu Ser Ala Ser Val Gly
1 5 10 15

Asp Arg Val Thr Ile Thr Cys Arg Thr Ser Glu Asn Ile Tyr Ser Phe
20 25 30

Leu Ala Trp Tyr Gln Gln Lys Pro Gly Lys Ala Pro Lys Leu Leu Ile
35 40 45

Tyr Asn Ala Lys Thr Leu Ala Lys Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln His His Tyr Glu Asp Pro Leu
85 90 95

Thr Phe Gly Gly Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 255
<211> 447
<212> PRT
<213> Artificial

<220>
<223> An artificially synthesized peptide sequence

<400> 255

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Val Ile Asn Trp Val Arg Gln Ala Pro Gly Gln Gly Leu Glu Trp Met
35 40 45

Gly Leu Ile Asn Pro Tyr Asn Gly Gly Thr Asp Tyr Asn Gln Lys Phe
50 55 60

Lys Gly Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Met Asp Tyr Trp Gly

```
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Glu Ser Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Asn Phe Gly Thr Gln Thr Tyr Thr Cys Asn Val Asp His
        195                 200                 205

Lys Pro Ser Asn Thr Lys Val Asp Lys Thr Val Glu Arg Lys Ser Cys
    210                 215                 220

Val Glu Cys Pro Pro Cys Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Phe Arg Val Val Ser
    290                 295                 300

Val Leu Thr Val Val His Gln Asp Trp Leu Asn Gly Lys Glu Tyr Lys
305                 310                 315                 320

Cys Lys Val Ser Asn Lys Gly Leu Pro Ala Pro Ile Glu Lys Thr Ile
                325                 330                 335

Ser Lys Thr Lys Gly Gln Pro Arg Glu Pro Gln Val Tyr Thr Leu Pro
            340                 345                 350
```

Pro Ser Arg Glu Glu Met Thr Lys Asn Gln Val Ser Leu Thr Cys Leu
355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Met Leu Asp Ser
385 390 395 400

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Lys
435 440 445

<210> 256
<211> 214
<212> PRT
<213> Artificial

<220>
<223> An artificially synthesized peptide sequence

<400> 256

Asp Ile Gln Met Thr Gln Ser Pro Ser Ser Leu Ser Ala Ser Val Gly
1 5 10 15

Asp Arg Val Thr Ile Thr Cys Gln Thr Ser Glu Asn Ile Tyr Arg Phe
20 25 30

Val Ala Trp Tyr Gln Gln Lys Pro Gly Lys Ala Pro Lys Leu Leu Ile
35 40 45

Tyr Asn Ala Lys Thr Leu Ala Lys Gly Val Pro Ser Arg Phe Ser Gly
50 55 60

Ser Gly Ser Gly Thr Asp Phe Thr Leu Thr Ile Ser Ser Leu Gln Pro
65 70 75 80

Glu Asp Phe Ala Thr Tyr Tyr Cys Gln His His Tyr Asp Ser Pro Leu
85 90 95

Thr Phe Gly Gly Gly Thr Lys Val Glu Ile Lys Arg Thr Val Ala Ala
100 105 110

Pro Ser Val Phe Ile Phe Pro Pro Ser Asp Glu Gln Leu Lys Ser Gly

115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 257
<211> 445
<212> PRT
<213> Artificial

<220>
<223> An artificially synthesized peptide sequence

<400> 257

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Ile Met Asn Trp Val Arg Gln Ala Pro Gly Gln Gly Leu Glu Trp Met
35 40 45

Gly Leu Ile Asn Pro Tyr Asn Gly Gly Thr Ser Tyr Asn Gln Lys Phe
50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Asp Gly Tyr Asp Asp Gly Pro Tyr Thr Met Asp Tyr Trp Gly
100 105 110

```
Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Asn Phe Gly Thr Gln Thr Tyr Thr Cys Asn Val Asp His
        195                 200                 205

Lys Pro Ser Asn Thr Lys Val Asp Lys Thr Val Glu Arg Lys Ser Cys
    210                 215                 220

Val Glu Cys Pro Pro Cys Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Phe Arg Val Val Ser
    290                 295                 300

Val Leu Thr Val Val His Gln Asp Trp Leu Asn Gly Lys Glu Tyr Lys
305                 310                 315                 320

Cys Lys Val Ser Asn Lys Gly Leu Pro Ala Pro Ile Glu Lys Thr Ile
                325                 330                 335

Ser Lys Thr Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
```

355 360 365

Val Lys Gly Phe Tyr Pro Ser Asp Ile Ala Val Glu Trp Glu Ser Asn
370 375 380

Gly Gln Pro Glu Asn Asn Tyr Lys Thr Thr Pro Pro Met Leu Asp Ser
385 390 395 400

Asp Gly Ser Phe Phe Leu Tyr Ser Lys Leu Thr Val Asp Lys Ser Arg
405 410 415

Trp Gln Glu Gly Asn Val Phe Ser Cys Ser Val Met His Glu Ala Leu
420 425 430

His Asn His Tyr Thr Gln Lys Ser Leu Ser Leu Ser Pro
435 440 445

<210> 258
<211> 530
<212> PRT
<213> Homo sapiens

<400> 258

Met Met Trp Thr Trp Ala Leu Trp Met Leu Pro Ser Leu Cys Lys Phe
1 5 10 15

Ser Leu Ala Ala Leu Pro Ala Lys Pro Glu Asn Ile Ser Cys Val Tyr
20 25 30

Tyr Tyr Arg Lys Asn Leu Thr Cys Thr Trp Ser Pro Gly Lys Glu Thr
35 40 45

Ser Tyr Thr Gln Tyr Thr Val Lys Arg Thr Tyr Ala Phe Gly Glu Lys
50 55 60

His Asp Asn Cys Thr Thr Asn Ser Ser Thr Ser Glu Asn Arg Ala Ser
65 70 75 80

Cys Ser Phe Phe Leu Pro Arg Ile Thr Ile Pro Asp Asn Tyr Thr Ile
85 90 95

Glu Val Glu Ala Glu Asn Gly Asp Gly Val Ile Lys Ser His Met Thr
100 105 110

Tyr Trp Arg Leu Glu Asn Ile Ala Lys Thr Glu Pro Pro Lys Ile Phe
115 120 125

Arg Val Lys Pro Val Leu Gly Ile Lys Arg Met Ile Gln Ile Glu Trp

130 135 140

Ile Lys Pro Glu Leu Ala Pro Val Ser Ser Asp Leu Lys Tyr Thr Leu
145 150 155 160

Arg Phe Arg Thr Val Asn Ser Thr Ser Trp Met Glu Val Asn Phe Ala
165 170 175

Lys Asn Arg Lys Asp Lys Asn Gln Thr Tyr Asn Leu Thr Gly Leu Gln
180 185 190

Pro Phe Thr Glu Tyr Val Ile Ala Leu Arg Cys Ala Val Lys Glu Ser
195 200 205

Lys Phe Trp Ser Asp Trp Ser Gln Glu Lys Met Gly Met Thr Glu Glu
210 215 220

Glu Ala Pro Cys Gly Leu Glu Leu Trp Arg Val Leu Lys Pro Ala Glu
225 230 235 240

Ala Asp Gly Arg Arg Pro Val Arg Leu Leu Trp Lys Lys Ala Arg Gly
245 250 255

Ala Pro Val Leu Glu Lys Thr Leu Gly Tyr Asn Ile Trp Tyr Tyr Pro
260 265 270

Glu Ser Asn Thr Asn Leu Thr Glu Thr Met Asn Thr Thr Asn Gln Gln
275 280 285

Leu Glu Leu His Leu Gly Gly Glu Ser Phe Trp Val Ser Met Ile Ser
290 295 300

Tyr Asn Ser Leu Gly Lys Ser Pro Val Ala Thr Leu Arg Ile Pro Ala
305 310 315 320

Ile Gln Glu Lys Ser Phe Gln Cys Ile Glu Val Met Gln Ala Cys Val
325 330 335

Ala Glu Asp Gln Leu Val Val Lys Trp Gln Ser Ser Ala Leu Asp Val
340 345 350

Asn Thr Trp Met Ile Glu Trp Phe Pro Asp Val Asp Ser Glu Pro Thr
355 360 365

Thr Leu Ser Trp Glu Ser Val Ser Gln Ala Thr Asn Trp Thr Ile Gln
370 375 380

Gln Asp Lys Leu Lys Pro Phe Trp Cys Tyr Asn Ile Ser Val Tyr Pro
385 390 395 400

Met Leu His Asp Lys Val Gly Glu Pro Tyr Ser Ile Gln Ala Tyr Ala
405 410 415

Lys Glu Gly Val Pro Ser Glu Gly Pro Glu Thr Lys Val Glu Asn Ile
420 425 430

Gly Val Lys Thr Val Thr Ile Thr Trp Lys Glu Ile Pro Lys Ser Glu
435 440 445

Arg Lys Gly Ile Ile Cys Asn Tyr Thr Ile Phe Tyr Gln Ala Glu Gly
450 455 460

Gly Lys Gly Phe Ser Lys Thr Val Asn Ser Ser Ile Leu Gln Tyr Gly
465 470 475 480

Leu Glu Ser Leu Lys Arg Lys Thr Ser Tyr Ile Val Gln Val Met Ala
485 490 495

Ser Thr Ser Ala Gly Gly Thr Asn Gly Thr Ser Ile Asn Phe Lys Thr
500 505 510

Leu Ser His His His His His His Glu Gln Lys Leu Ile Ser Glu Glu
515 520 525

Asp Leu
530

<210> 259
<211> 517
<212> PRT
<213> Mus musculus

<400> 259

Met Trp Thr Leu Ala Leu Trp Ala Phe Ser Phe Leu Cys Lys Phe Ser
1 5 10 15

Leu Ala Val Leu Pro Thr Lys Pro Glu Asn Ile Ser Cys Val Phe Tyr
20 25 30

Phe Asp Arg Asn Leu Thr Cys Thr Trp Arg Pro Glu Lys Glu Thr Asn
35 40 45

Asp Thr Ser Tyr Ile Val Thr Leu Thr Tyr Ser Tyr Gly Lys Ser Asn
50 55 60

```
Tyr Ser Asp Asn Ala Thr Glu Ala Ser Tyr Ser Phe Pro Arg Ser Cys
65                  70                  75                  80

Ala Met Pro Pro Asp Ile Cys Ser Val Glu Val Gln Ala Gln Asn Gly
                85                  90                  95

Asp Gly Lys Val Lys Ser Asp Ile Thr Tyr Trp His Leu Ile Ser Ile
            100                 105                 110

Ala Lys Thr Glu Pro Pro Ile Ile Leu Ser Val Asn Pro Ile Cys Asn
        115                 120                 125

Arg Met Phe Gln Ile Gln Trp Lys Pro Arg Glu Lys Thr Arg Gly Phe
    130                 135                 140

Pro Leu Val Cys Met Leu Arg Phe Arg Thr Val Asn Ser Ser Arg Trp
145                 150                 155                 160

Thr Glu Val Asn Phe Glu Asn Cys Lys Gln Val Cys Asn Leu Thr Gly
                165                 170                 175

Leu Gln Ala Phe Thr Glu Tyr Val Leu Ala Leu Arg Phe Arg Phe Asn
            180                 185                 190

Asp Ser Arg Tyr Trp Ser Lys Trp Ser Lys Glu Glu Thr Arg Val Thr
        195                 200                 205

Met Glu Glu Val Pro His Val Leu Asp Leu Trp Arg Ile Leu Glu Pro
    210                 215                 220

Ala Asp Met Asn Gly Asp Arg Lys Val Arg Leu Leu Trp Lys Lys Ala
225                 230                 235                 240

Arg Gly Ala Pro Val Leu Glu Lys Thr Phe Gly Tyr His Ile Gln Tyr
                245                 250                 255

Phe Ala Glu Asn Ser Thr Asn Leu Thr Glu Ile Asn Asn Ile Thr Thr
            260                 265                 270

Gln Gln Tyr Glu Leu Leu Leu Met Ser Gln Ala His Ser Val Ser Val
        275                 280                 285

Thr Ser Phe Asn Ser Leu Gly Lys Ser Gln Glu Thr Ile Leu Arg Ile
    290                 295                 300

Pro Asp Val His Glu Lys Thr Phe Gln Tyr Ile Lys Ser Met Gln Ala
305                 310                 315                 320
```

Tyr Ile Ala Glu Pro Leu Leu Val Val Asn Trp Gln Ser Ser Ile Pro
325 330 335

Ala Val Asp Thr Trp Ile Val Glu Trp Leu Pro Glu Ala Ala Met Ser
340 345 350

Lys Phe Pro Ala Leu Ser Trp Glu Ser Val Ser Gln Val Thr Asn Trp
355 360 365

Thr Ile Glu Gln Asp Lys Leu Lys Pro Phe Thr Cys Tyr Asn Ile Ser
370 375 380

Val Tyr Pro Val Leu Gly His Arg Val Gly Glu Pro Tyr Ser Ile Gln
385 390 395 400

Ala Tyr Ala Lys Glu Gly Thr Pro Leu Lys Gly Pro Glu Thr Arg Val
405 410 415

Glu Asn Ile Gly Leu Arg Thr Ala Thr Ile Thr Trp Lys Glu Ile Pro
420 425 430

Lys Ser Ala Arg Asn Gly Phe Ile Asn Asn Tyr Thr Val Phe Tyr Gln
435 440 445

Ala Glu Gly Gly Lys Glu Leu Ser Lys Thr Val Asn Ser His Ala Leu
450 455 460

Gln Cys Asp Leu Glu Ser Leu Thr Arg Arg Thr Ser Tyr Thr Val Trp
465 470 475 480

Val Met Ala Ser Thr Arg Ala Gly Gly Thr Asn Gly Val Arg Ile Asn
485 490 495

Phe Lys Thr Leu Ser His His His His His His Glu Gln Lys Leu Ile
500 505 510

Ser Glu Glu Asp Leu
515

<210> 260
<211> 531
<212> PRT
<213> Artificial

<220>
<223> An artificially synthesized peptide sequence

<400> 260

```
Met Met Trp Thr Trp Ala Leu Trp Met Leu Pro Ser Leu Cys Lys Phe
1               5                   10                  15

Ser Leu Ala Ala Leu Pro Ala Lys Pro Glu Asn Ile Ser Cys Val Tyr
            20                  25                  30

Tyr Tyr Arg Lys Asn Leu Thr Cys Thr Trp Ser Pro Gly Lys Glu Thr
        35                  40                  45

Ser Tyr Thr Gln Tyr Thr Val Lys Arg Thr Tyr Ala Phe Gly Glu Lys
    50                  55                  60

His Asp Asn Cys Thr Thr Asn Ser Ser Thr Ser Glu Asn Arg Ala Ser
65                  70                  75                  80

Cys Ser Phe Phe Leu Pro Arg Ile Thr Ile Pro Asp Asn Tyr Thr Ile
                85                  90                  95

Glu Val Glu Ala Glu Asn Gly Asp Gly Val Ile Lys Ser His Met Thr
            100                 105                 110

Tyr Trp Arg Leu Glu Asn Ile Ala Lys Thr Glu Pro Pro Lys Ile Phe
        115                 120                 125

Arg Val Lys Pro Val Leu Gly Ile Lys Arg Met Ile Gln Ile Glu Trp
    130                 135                 140

Ile Lys Pro Glu Leu Ala Pro Val Ser Ser Asp Leu Lys Tyr Thr Leu
145                 150                 155                 160

Arg Phe Arg Thr Val Asn Ser Thr Ser Trp Met Glu Val Asn Phe Ala
                165                 170                 175

Lys Asn Arg Lys Asp Lys Asn Gln Thr Tyr Asn Leu Thr Gly Leu Gln
            180                 185                 190

Pro Phe Thr Glu Tyr Val Ile Ala Leu Arg Cys Ala Val Lys Glu Ser
        195                 200                 205

Lys Phe Trp Ser Asp Trp Ser Gln Glu Lys Met Gly Met Thr Glu Glu
    210                 215                 220

Glu Ala Pro His Val Leu Asp Leu Trp Arg Ile Leu Glu Pro Ala Asp
225                 230                 235                 240

Met Asn Gly Asp Arg Lys Val Arg Leu Leu Trp Lys Lys Ala Arg Gly
                245                 250                 255
```

Ala Pro Val Leu Glu Lys Thr Phe Gly Tyr His Ile Gln Tyr Phe Ala
260 265 270

Glu Asn Ser Thr Asn Leu Thr Glu Ile Asn Asn Ile Thr Thr Gln Gln
275 280 285

Tyr Glu Leu Leu Leu Met Ser Gln Ala His Ser Val Ser Val Thr Ser
290 295 300

Phe Asn Ser Leu Gly Lys Ser Gln Glu Thr Ile Leu Arg Ile Pro Asp
305 310 315 320

Val His Glu Lys Thr Phe Gln Tyr Ile Lys Ser Met Gln Ala Tyr Ile
325 330 335

Ala Glu Pro Leu Leu Val Val Asn Trp Gln Ser Ser Ile Pro Ala Val
340 345 350

Asp Thr Trp Ile Val Glu Trp Leu Pro Glu Ala Ala Met Ser Lys Phe
355 360 365

Pro Ala Leu Ser Trp Glu Ser Val Ser Gln Val Thr Asn Trp Thr Ile
370 375 380

Glu Gln Asp Lys Leu Lys Pro Phe Thr Cys Tyr Asn Ile Ser Val Tyr
385 390 395 400

Pro Val Leu Gly His Arg Val Gly Glu Pro Tyr Ser Ile Gln Ala Tyr
405 410 415

Ala Lys Glu Gly Thr Pro Leu Lys Gly Pro Glu Thr Arg Val Glu Asn
420 425 430

Ile Gly Leu Arg Thr Ala Thr Ile Thr Trp Lys Glu Ile Pro Lys Ser
435 440 445

Ala Arg Asn Gly Phe Ile Asn Asn Tyr Thr Val Phe Tyr Gln Ala Glu
450 455 460

Gly Gly Lys Glu Leu Ser Lys Thr Val Asn Ser His Ala Leu Gln Cys
465 470 475 480

Asp Leu Glu Ser Leu Thr Arg Arg Thr Ser Tyr Thr Val Trp Val Met
485 490 495

Ala Ser Thr Arg Ala Gly Gly Thr Asn Gly Val Arg Ile Asn Phe Lys
500 505 510

Thr Leu Ser His His His His His His Glu Gln Lys Leu Ile Ser Glu
515 520 525

Glu Asp Leu
530

<210> 261
<211> 516
<212> PRT
<213> Artificial

<220>
<223> An artificially synthesized peptide sequence

<400> 261

Met Trp Thr Leu Ala Leu Trp Ala Phe Ser Phe Leu Cys Lys Phe Ser
1 5 10 15

Leu Ala Val Leu Pro Thr Lys Pro Glu Asn Ile Ser Cys Val Phe Tyr
20 25 30

Phe Asp Arg Asn Leu Thr Cys Thr Trp Arg Pro Glu Lys Glu Thr Asn
35 40 45

Asp Thr Ser Tyr Ile Val Thr Leu Thr Tyr Ser Tyr Gly Lys Ser Asn
50 55 60

Tyr Ser Asp Asn Ala Thr Glu Ala Ser Tyr Ser Phe Pro Arg Ser Cys
65 70 75 80

Ala Met Pro Pro Asp Ile Cys Ser Val Glu Val Gln Ala Gln Asn Gly
85 90 95

Asp Gly Lys Val Lys Ser Asp Ile Thr Tyr Trp His Leu Ile Ser Ile
100 105 110

Ala Lys Thr Glu Pro Pro Ile Ile Leu Ser Val Asn Pro Ile Cys Asn
115 120 125

Arg Met Phe Gln Ile Gln Trp Lys Pro Arg Glu Lys Thr Arg Gly Phe
130 135 140

Pro Leu Val Cys Met Leu Arg Phe Arg Thr Val Asn Ser Ser Arg Trp
145 150 155 160

Thr Glu Val Asn Phe Glu Asn Cys Lys Gln Val Cys Asn Leu Thr Gly
165 170 175

```
Leu Gln Ala Phe Thr Glu Tyr Val Leu Ala Leu Arg Phe Arg Phe Asn
            180                 185                 190

Asp Ser Arg Tyr Trp Ser Lys Trp Ser Lys Glu Glu Thr Arg Val Thr
        195                 200                 205

Met Glu Glu Val Pro Cys Gly Leu Glu Leu Trp Arg Val Leu Lys Pro
    210                 215                 220

Ala Glu Ala Asp Gly Arg Arg Pro Val Arg Leu Leu Trp Lys Lys Ala
225                 230                 235                 240

Arg Gly Ala Pro Val Leu Glu Lys Thr Leu Gly Tyr Asn Ile Trp Tyr
                245                 250                 255

Tyr Pro Glu Ser Asn Thr Asn Leu Thr Glu Thr Met Asn Thr Thr Asn
            260                 265                 270

Gln Gln Leu Glu Leu His Leu Gly Gly Glu Ser Phe Trp Val Ser Met
        275                 280                 285

Ile Ser Tyr Asn Ser Leu Gly Lys Ser Pro Val Ala Thr Leu Arg Ile
    290                 295                 300

Pro Ala Ile Gln Glu Lys Ser Phe Gln Cys Ile Glu Val Met Gln Ala
305                 310                 315                 320

Cys Val Ala Glu Asp Gln Leu Val Val Lys Trp Gln Ser Ser Ala Leu
                325                 330                 335

Asp Val Asn Thr Trp Met Ile Glu Trp Phe Pro Asp Val Asp Ser Glu
            340                 345                 350

Pro Thr Thr Leu Ser Trp Glu Ser Val Ser Gln Ala Thr Asn Trp Thr
        355                 360                 365

Ile Gln Gln Asp Lys Leu Lys Pro Phe Trp Cys Tyr Asn Ile Ser Val
    370                 375                 380

Tyr Pro Met Leu His Asp Lys Val Gly Glu Pro Tyr Ser Ile Gln Ala
385                 390                 395                 400

Tyr Ala Lys Glu Gly Val Pro Ser Glu Gly Pro Glu Thr Lys Val Glu
                405                 410                 415

Asn Ile Gly Val Lys Thr Val Thr Ile Thr Trp Lys Glu Ile Pro Lys
            420                 425                 430
```

Ser Glu Arg Lys Gly Ile Ile Cys Asn Tyr Thr Ile Phe Tyr Gln Ala
        435                 440                 445

Glu Gly Gly Lys Gly Phe Ser Lys Thr Val Asn Ser Ser Ile Leu Gln
    450                 455                 460

Tyr Gly Leu Glu Ser Leu Lys Arg Lys Thr Ser Tyr Ile Val Gln Val
465                 470                 475                 480

Met Ala Ser Thr Ser Ala Gly Gly Thr Asn Gly Thr Ser Ile Asn Phe
                485                 490                 495

Lys Thr Leu Ser His His His His His His Glu Gln Lys Leu Ile Ser
            500                 505                 510

Glu Glu Asp Leu
        515

<210> 262
<211> 524
<212> PRT
<213> Artificial

<220>
<223> An artificially synthesized peptide sequence

<400> 262

Met Met Trp Thr Trp Ala Leu Trp Met Leu Pro Ser Leu Cys Lys Phe
1               5                   10                  15

Ser Leu Ala Ala Leu Pro Ala Lys Pro Glu Asn Ile Ser Cys Val Tyr
            20                  25                  30

Tyr Tyr Arg Lys Asn Leu Thr Cys Thr Trp Ser Pro Gly Lys Glu Thr
        35                  40                  45

Ser Tyr Thr Gln Tyr Thr Val Lys Arg Thr Tyr Ala Phe Gly Glu Lys
    50                  55                  60

His Asp Asn Cys Thr Thr Asn Ser Ser Thr Ser Glu Asn Arg Ala Ser
65                  70                  75                  80

Cys Ser Phe Phe Leu Pro Arg Ile Thr Ile Pro Asp Asn Tyr Thr Ile
                85                  90                  95

Glu Val Glu Ala Glu Asn Gly Asp Gly Val Ile Lys Ser His Met Thr
            100                 105                 110

Tyr Trp Arg Leu Glu Asn Ile Ala Lys Thr Glu Pro Pro Ile Ile Leu
115 120 125

Ser Val Asn Pro Ile Cys Asn Arg Met Phe Gln Ile Gln Trp Lys Pro
130 135 140

Arg Glu Lys Thr Arg Gly Phe Pro Leu Val Cys Met Leu Arg Phe Arg
145 150 155 160

Thr Val Asn Ser Ser Arg Trp Thr Glu Val Asn Phe Glu Asn Cys Lys
165 170 175

Gln Val Cys Asn Leu Thr Gly Leu Gln Ala Phe Thr Glu Tyr Val Leu
180 185 190

Ala Leu Arg Phe Arg Phe Asn Asp Ser Arg Tyr Trp Ser Lys Trp Ser
195 200 205

Lys Glu Glu Thr Arg Val Thr Met Glu Glu Val Pro His Val Leu Asp
210 215 220

Leu Trp Arg Ile Leu Glu Pro Ala Asp Met Asn Gly Asp Arg Lys Val
225 230 235 240

Arg Leu Leu Trp Lys Lys Ala Arg Gly Ala Pro Val Leu Glu Lys Thr
245 250 255

Phe Gly Tyr His Ile Gln Tyr Phe Ala Glu Asn Ser Thr Asn Leu Thr
260 265 270

Glu Ile Asn Asn Ile Thr Thr Gln Gln Tyr Glu Leu Leu Leu Met Ser
275 280 285

Gln Ala His Ser Val Ser Val Thr Ser Phe Asn Ser Leu Gly Lys Ser
290 295 300

Gln Glu Thr Ile Leu Arg Ile Pro Asp Val His Glu Lys Thr Phe Gln
305 310 315 320

Tyr Ile Lys Ser Met Gln Ala Tyr Ile Ala Glu Pro Leu Leu Val Val
325 330 335

Asn Trp Gln Ser Ser Ile Pro Ala Val Asp Thr Trp Ile Val Glu Trp
340 345 350

Leu Pro Glu Ala Ala Met Ser Lys Phe Pro Ala Leu Ser Trp Glu Ser
355 360 365

Val Ser Gln Val Thr Asn Trp Thr Ile Glu Gln Asp Lys Leu Lys Pro
    370                 375                 380

Phe Thr Cys Tyr Asn Ile Ser Val Tyr Pro Val Leu Gly His Arg Val
385                 390                 395                 400

Gly Glu Pro Tyr Ser Ile Gln Ala Tyr Ala Lys Glu Gly Thr Pro Leu
                405                 410                 415

Lys Gly Pro Glu Thr Arg Val Glu Asn Ile Gly Leu Arg Thr Ala Thr
            420                 425                 430

Ile Thr Trp Lys Glu Ile Pro Lys Ser Ala Arg Asn Gly Phe Ile Asn
        435                 440                 445

Asn Tyr Thr Val Phe Tyr Gln Ala Glu Gly Gly Lys Glu Leu Ser Lys
    450                 455                 460

Thr Val Asn Ser His Ala Leu Gln Cys Asp Leu Glu Ser Leu Thr Arg
465                 470                 475                 480

Arg Thr Ser Tyr Thr Val Trp Val Met Ala Ser Thr Arg Ala Gly Gly
                485                 490                 495

Thr Asn Gly Val Arg Ile Asn Phe Lys Thr Leu Ser His His His His
            500                 505                 510

His His Glu Gln Lys Leu Ile Ser Glu Glu Asp Leu
        515                 520

<210> 263
<211> 524
<212> PRT
<213> Artificial

<220>
<223> An artificially synthesized peptide sequence

<400> 263

Met Trp Thr Leu Ala Leu Trp Ala Phe Ser Phe Leu Cys Lys Phe Ser
1               5                   10                  15

Leu Ala Val Leu Pro Thr Lys Pro Glu Asn Ile Ser Cys Val Phe Tyr
            20                  25                  30

Phe Asp Arg Asn Leu Thr Cys Thr Trp Arg Pro Glu Lys Glu Thr Asn
        35                  40                  45

Asp Thr Ser Tyr Ile Val Thr Leu Thr Tyr Ser Tyr Gly Lys Ser Asn
    50                  55                  60

Tyr Ser Asp Asn Ala Thr Glu Ala Ser Tyr Ser Phe Pro Arg Ser Cys
65                  70                  75                  80

Ala Met Pro Pro Asp Ile Cys Ser Val Glu Val Gln Ala Gln Asn Gly
                85                  90                  95

Asp Gly Lys Val Lys Ser Asp Ile Thr Tyr Trp His Leu Ile Ser Ile
            100                 105                 110

Ala Lys Thr Glu Pro Pro Lys Ile Phe Arg Val Lys Pro Val Leu Gly
        115                 120                 125

Ile Lys Arg Met Ile Gln Ile Glu Trp Ile Lys Pro Glu Leu Ala Pro
    130                 135                 140

Val Ser Ser Asp Leu Lys Tyr Thr Leu Arg Phe Arg Thr Val Asn Ser
145                 150                 155                 160

Thr Ser Trp Met Glu Val Asn Phe Ala Lys Asn Arg Lys Asp Lys Asn
                165                 170                 175

Gln Thr Tyr Asn Leu Thr Gly Leu Gln Pro Phe Thr Glu Tyr Val Ile
            180                 185                 190

Ala Leu Arg Cys Ala Val Lys Glu Ser Lys Phe Trp Ser Asp Trp Ser
        195                 200                 205

Gln Glu Lys Met Gly Met Thr Glu Glu Glu Ala Pro His Val Leu Asp
    210                 215                 220

Leu Trp Arg Ile Leu Glu Pro Ala Asp Met Asn Gly Asp Arg Lys Val
225                 230                 235                 240

Arg Leu Leu Trp Lys Lys Ala Arg Gly Ala Pro Val Leu Glu Lys Thr
                245                 250                 255

Phe Gly Tyr His Ile Gln Tyr Phe Ala Glu Asn Ser Thr Asn Leu Thr
            260                 265                 270

Glu Ile Asn Asn Ile Thr Thr Gln Gln Tyr Glu Leu Leu Leu Met Ser
        275                 280                 285

Gln Ala His Ser Val Ser Val Thr Ser Phe Asn Ser Leu Gly Lys Ser
    290                 295                 300

Gln Glu Thr Ile Leu Arg Ile Pro Asp Val His Glu Lys Thr Phe Gln
305 310 315 320

Tyr Ile Lys Ser Met Gln Ala Tyr Ile Ala Glu Pro Leu Leu Val Val
325 330 335

Asn Trp Gln Ser Ser Ile Pro Ala Val Asp Thr Trp Ile Val Glu Trp
340 345 350

Leu Pro Glu Ala Ala Met Ser Lys Phe Pro Ala Leu Ser Trp Glu Ser
355 360 365

Val Ser Gln Val Thr Asn Trp Thr Ile Glu Gln Asp Lys Leu Lys Pro
370 375 380

Phe Thr Cys Tyr Asn Ile Ser Val Tyr Pro Val Leu Gly His Arg Val
385 390 395 400

Gly Glu Pro Tyr Ser Ile Gln Ala Tyr Ala Lys Glu Gly Thr Pro Leu
405 410 415

Lys Gly Pro Glu Thr Arg Val Glu Asn Ile Gly Leu Arg Thr Ala Thr
420 425 430

Ile Thr Trp Lys Glu Ile Pro Lys Ser Ala Arg Asn Gly Phe Ile Asn
435 440 445

Asn Tyr Thr Val Phe Tyr Gln Ala Glu Gly Gly Lys Glu Leu Ser Lys
450 455 460

Thr Val Asn Ser His Ala Leu Gln Cys Asp Leu Glu Ser Leu Thr Arg
465 470 475 480

Arg Thr Ser Tyr Thr Val Trp Val Met Ala Ser Thr Arg Ala Gly Gly
485 490 495

Thr Asn Gly Val Arg Ile Asn Phe Lys Thr Leu Ser His His His His
500 505 510

His His Glu Gln Lys Leu Ile Ser Glu Glu Asp Leu
515 520

<210> 264
<211> 523
<212> PRT
<213> Artificial

<220>
<223> An artificially synthesized peptide sequence

<400> 264

Met Trp Thr Leu Ala Leu Trp Ala Phe Ser Phe Leu Cys Lys Phe Ser
1 5 10 15

Leu Ala Val Leu Pro Thr Lys Pro Glu Asn Ile Ser Cys Val Phe Tyr
20 25 30

Phe Asp Arg Asn Leu Thr Cys Thr Trp Arg Pro Glu Lys Glu Thr Asn
35 40 45

Asp Thr Ser Tyr Ile Val Thr Leu Thr Tyr Ser Tyr Gly Lys Ser Asn
50 55 60

Tyr Ser Asp Asn Ala Thr Glu Ala Ser Tyr Ser Phe Pro Arg Ser Cys
65 70 75 80

Ala Met Pro Pro Asp Ile Cys Ser Val Glu Val Gln Ala Gln Asn Gly
85 90 95

Asp Gly Lys Val Lys Ser Asp Ile Thr Tyr Trp His Leu Ile Ser Ile
100 105 110

Ala Lys Thr Glu Pro Pro Lys Ile Phe Arg Val Lys Pro Val Leu Gly
115 120 125

Ile Lys Arg Met Ile Gln Ile Glu Trp Ile Lys Pro Glu Leu Ala Pro
130 135 140

Val Ser Ser Asp Leu Lys Tyr Thr Leu Arg Phe Arg Thr Val Asn Ser
145 150 155 160

Thr Ser Trp Met Glu Val Asn Phe Ala Lys Asn Arg Lys Asp Lys Asn
165 170 175

Gln Thr Tyr Asn Leu Thr Gly Leu Gln Pro Phe Thr Glu Tyr Val Ile
180 185 190

Ala Leu Arg Cys Ala Val Lys Glu Ser Lys Phe Trp Ser Asp Trp Ser
195 200 205

Gln Glu Lys Met Gly Met Thr Glu Glu Glu Ala Pro Cys Gly Leu Glu
210 215 220

Leu Trp Arg Val Leu Lys Pro Ala Glu Ala Asp Gly Arg Arg Pro Val
225 230 235 240

```
Arg Leu Leu Trp Lys Lys Ala Arg Gly Ala Pro Val Leu Glu Lys Thr
                245                 250                 255

Leu Gly Tyr Asn Ile Trp Tyr Tyr Pro Glu Ser Asn Thr Asn Leu Thr
            260                 265                 270

Glu Thr Met Asn Thr Thr Asn Gln Gln Leu Glu Leu His Leu Gly Gly
        275                 280                 285

Glu Ser Phe Trp Val Ser Met Ile Ser Tyr Asn Ser Leu Gly Lys Ser
    290                 295                 300

Pro Val Ala Thr Leu Arg Ile Pro Ala Ile Gln Glu Lys Ser Phe Gln
305                 310                 315                 320

Cys Ile Glu Val Met Gln Ala Cys Val Ala Glu Asp Gln Leu Val Val
                325                 330                 335

Lys Trp Gln Ser Ser Ala Leu Asp Val Asn Thr Trp Met Ile Glu Trp
            340                 345                 350

Phe Pro Asp Val Asp Ser Glu Pro Thr Thr Leu Ser Trp Glu Ser Val
        355                 360                 365

Ser Gln Ala Thr Asn Trp Thr Ile Gln Gln Asp Lys Leu Lys Pro Phe
    370                 375                 380

Trp Cys Tyr Asn Ile Ser Val Tyr Pro Met Leu His Asp Lys Val Gly
385                 390                 395                 400

Glu Pro Tyr Ser Ile Gln Ala Tyr Ala Lys Glu Gly Val Pro Ser Glu
                405                 410                 415

Gly Pro Glu Thr Lys Val Glu Asn Ile Gly Val Lys Thr Val Thr Ile
            420                 425                 430

Thr Trp Lys Glu Ile Pro Lys Ser Glu Arg Lys Gly Ile Ile Cys Asn
        435                 440                 445

Tyr Thr Ile Phe Tyr Gln Ala Glu Gly Gly Lys Gly Phe Ser Lys Thr
    450                 455                 460

Val Asn Ser Ser Ile Leu Gln Tyr Gly Leu Glu Ser Leu Lys Arg Lys
465                 470                 475                 480

Thr Ser Tyr Ile Val Gln Val Met Ala Ser Thr Ser Ala Gly Gly Thr
```

485 490 495

Asn Gly Thr Ser Ile Asn Phe Lys Thr Leu Ser His His His His His
500 505 510

His Glu Gln Lys Leu Ile Ser Glu Glu Asp Leu
515 520

<210> 265
<211> 12
<212> PRT
<213> Artificial

<220>
<223> An artificially synthesized peptide sequence

<400> 265

Asp Gly Tyr Asp Asp Gly Pro Tyr Thr Met Asp Tyr
1 5 10

<210> 266
<211> 12
<212> PRT
<213> Artificial

<220>
<223> An artificially synthesized peptide sequence

<400> 266

Asp Gly Tyr Asp Asp Gly Pro Tyr Thr Leu Glu Thr
1 5 10

<210> 267
<211> 12
<212> PRT
<213> Artificial

<220>
<223> An artificially synthesized peptide sequence

<400> 267

Asp Gly Tyr Asp Asp Gly Pro Tyr Thr Met Glu Thr
1 5 10

<210> 268
<211> 12
<212> PRT
<213> Artificial

<220>
<223> An artificially synthesized peptide sequence

<400> 268

Asp Gly Leu Asp Asp Gly Pro Tyr Thr Met Glu Thr
1 5 10

<210> 269
<211> 12
<212> PRT
<213> Artificial

<220>
<223> An artificially synthesized peptide sequence

<400> 269

Asp Gly Leu Asp Asp Gly Pro Tyr Thr Met Glu Ser
1 5 10

<210> 270
<211> 5
<212> PRT
<213> Artificial

<220>
<223> An artificially synthesized peptide sequence

<400> 270

Gly Tyr Ile Met Asn
1 5

<210> 271
<211> 17
<212> PRT
<213> Artificial

<220>
<223> An artificially synthesized peptide sequence

<400> 271

Leu Ile Asn Pro Tyr Asn Gly Gly Thr Ser Tyr Asn Gln Lys Phe Lys
1 5 10 15

Gly

<210> 272
<211> 5
<212> PRT
<213> Artificial

<220>
<223> An artificially synthesized peptide sequence

<400> 272

Gly Tyr Val Met Asn
1 5

<210> 273
<211> 5
<212> PRT
<213> Artificial

<220>
<223> An artificially synthesized peptide sequence

<400> 273

Gly Tyr Ile Ile Asn
1 5

<210> 274
<211> 5
<212> PRT
<213> Artificial

<220>
<223> An artificially synthesized peptide sequence

<400> 274

Gly Tyr Ile Leu Asn
1 5

<210> 275
<211> 17
<212> PRT
<213> Artificial

<220>
<223> An artificially synthesized peptide sequence

<400> 275

Leu Ile Asn Pro Tyr Asn Gly Gly Thr Asp Tyr Asn Gln Lys Phe Lys
1 5 10 15

Gly

<210> 276
<211> 17
<212> PRT
<213> Artificial

<220>
<223> An artificially synthesized peptide sequence

<400> 276

Leu Ile Asn Pro Tyr Asn Gly Gly Thr Ser Tyr Asn Pro Lys Phe Lys
1 5 10 15

Gly

<210> 277
<211> 11
<212> PRT
<213> Artificial

<220>
<223> An artificially synthesized peptide sequence

<400> 277

Arg Thr Ser Glu Asn Ile Tyr Ser Phe Leu Ala
1               5                   10

<210> 278
<211> 9
<212> PRT
<213> Artificial

<220>
<223> An artificially synthesized peptide sequence

<400> 278

Gln His His Tyr Glu Ser Pro Leu Thr
1               5

<210> 279
<211> 11
<212> PRT
<213> Artificial

<220>
<223> An artificially synthesized peptide sequence

<400> 279

Arg Thr Ser Glu Asn Ile Tyr Arg Phe Leu Ala
1               5                   10

<210> 280
<211> 11
<212> PRT
<213> Artificial

<220>
<223> An artificially synthesized peptide sequence

<400> 280

Arg Thr Ser Glu Asn Ile Tyr Arg Phe Val Ala
1               5                   10

<210> 281
<211> 9
<212> PRT
<213> Artificial

<220>
<223> An artificially synthesized peptide sequence

<400> 281

Gln His His Tyr Asp Ser Pro Leu Thr
1               5

<210> 282
<211> 9
<212> PRT
<213> Artificial

<220>
<223> An artificially synthesized peptide sequence

<400> 282

Gln His His Tyr Glu Asp Pro Leu Thr
1               5

<210> 283
<211> 9
<212> PRT
<213> Artificial

<220>
<223> An artificially synthesized peptide sequence

<400> 283

Gln His His Thr Glu Ser Pro Leu Phe
1               5

<210> 284
<211> 5
<212> PRT
<213> Artificial

<220>
<223> An artificially synthesized peptide sequence

<400> 284

Gly Tyr Ala Met Asn
1               5

<210> 285
<211> 11
<212> PRT
<213> Artificial

<220>
<223> An artificially synthesized peptide sequence

<400> 285

Arg Ala Ser Glu Asn Ile Tyr Ser Phe Leu Ala
1               5                   10

<210> 286
<211> 11
<212> PRT
<213> Artificial

<220>
<223> An artificially synthesized peptide sequence

<400> 286

```
Arg Ser Ser Glu Asn Ile Tyr Ser Phe Leu Ala
1               5                   10
```

<210> 287
<211> 16
<212> PRT
<213> Artificial

<220>
<223> An artificially synthesized sequence

<400> 287

```
His His His His His His Glu Gln Lys Leu Ile Ser Glu Glu Asp Leu
1               5                   10                  15
```

## Description

Technical Field

The present invention relates to anti-NR10 antibodies, and pharmaceutical compositions comprising an anti-NR10 antibody.

Background Art

Many cytokines are known as humoral factors involved in the growth and differentiation of various types of cells, or in the activation of differentiated mature cell functions. Cytokine-stimulated cells produce different types of cytokines, thereby forming networks of multiple cytokines in the body. Biological homeostasis is maintained by a delicate balance of the mutual regulation between cytokines in these networks. Many inflammatory diseases are thought to result from a failure of such cytokine networks. Thus, monoclonal antibody-based anti-cytokine therapy is drawing much attention. For example, anti-TNF antibodies and anti-IL-6 receptor antibodies have been demonstrated to be highly effective clinically. On the other hand, there are many examples of failure where no therapeutic effects were produced when a single cytokine, such as IL-4, was blocked alone, due to the activation of compensatory pathways in actual pathological conditions.

The present inventors succeeded in isolating a novel cytokine receptor NR10 that was highly homologous to gp130, a receptor for IL-6 signal transduction (Patent Document 1). NR10 forms a heterodimer with oncostatin M receptor (OSMR) and functions as an IL-31 receptor (Non-patent Document 1). Regarding IL-31, it has been reported that transgenic mice overexpressing IL-31 spontaneously develop pruritic dermatitis (Patent Document 2).

Antibodies that bind to NR10 and inhibit the binding between NR10 and IL-31 may be effective in treating inflammatory diseases. For clinical use, anti-NR10 antibodies are required to have low immunogenicity. Furthermore, in order to achieve high therapeutic effects, antibodies with strong NR10-binding or neutralizing activity are desired.

Prior art documents of the present invention are described below.

Patent Document 1: WO00/75314
Patent Document 2: WO03/060090
Non-patent Document 1: IL-31 is associated with cutaneous lymphocyte antigen-positive skin homing T cells in patients with atopic dermatitis., J Allergy Clin Immunol. 2006 Feb; 117(2): 418-25.

Disclosure of the Invention

[Problems to be Solved by the Invention]

The present invention was achieved in view of the circumstances described above. An objective of the present invention is to provide anti-NR10 antibodies, and pharmaceutical compositions comprising an anti-NR10 antibody.

[Means for Solving the Problems]

The present inventors conducted dedicated studies to achieve the objective described above. The present inventors succeeded in obtaining anti-NR10 antibodies having an effective neutralizing activity against NR10. Furthermore, the present inventors succeeded in humanizing the antibodies while maintaining their activity. The present inventors also successfully produced antibodies with improved pharmacokinetics, enhanced antigen-binding activity, improved stability, and/or reduced risk of immunogenicity. These antibodies are useful as therapeutic agents for inflammatory diseases.

The present invention relates to anti-NR10 antibodies, and pharmaceutical compositions comprising an anti-NR10 antibody. More specifically, the present invention includes:

[1] an antibody that recognizes domain 1 of NR10;
[2] the antibody of [1], which has a neutralizing activity;
[3] the antibody of [1] or [2], which is a humanized antibody;
[4] an anti-NR10 antibody which is any one of:

(1) an antibody comprising a heavy chain variable region which comprises CDR1 comprising the amino acid sequence of SEQ ID NO: 1, CDR2 comprising the amino acid sequence of SEQ ID NO: 2, and CDR3 comprising the amino acid sequence of SEQ ID NO: 3;

(2) an antibody comprising the heavy chain variable region of SEQ ID NO: 4;
(3) an antibody comprising a light chain variable region which comprises CDR1 comprising the amino acid sequence of SEQ ID NO: 5, CDR2 comprising the amino acid sequence of SEQ ID NO: 6, and CDR3 comprising the amino acid sequence of SEQ ID NO: 7;
(4) an antibody comprising the light chain variable region of SEQ ID NO: 8;
(5) an antibody comprising the heavy chain variable region of (1) and the light chain variable region of (3);
(6) an antibody comprising the heavy chain variable region of (2) and the light chain variable region of (4);
(7) an antibody in which one or more amino acids are substituted, deleted, added, and/or inserted in the antibody of any one of (1) to (6), which has an activity equivalent to that of the antibody of any one of (1) to (6); and
(8) an antibody which binds to the same epitope as an epitope bound by the antibody of any one of (1) to (7);

[5] an anti-NR10 antibody which is any one of:

(1) an antibody comprising a heavy chain variable region which comprises CDR1 comprising the amino acid sequence of SEQ ID NO: 9, CDR2 comprising the amino acid sequence of SEQ ID NO: 10, and CDR3 comprising the amino acid sequence of SEQ ID NO: 11;
(2) an antibody comprising the heavy chain variable region of SEQ ID NO: 12;
(3) an antibody comprising a light chain variable region which comprises CDR1 comprising the amino acid sequence of SEQ ID NO: 13, CDR2 comprising the amino acid sequence of SEQ ID NO: 14, and CDR3 comprising the amino acid sequence of SEQ ID NO: 15;
(4) an antibody comprising the light chain variable region of SEQ ID NO: 16;
(5) an antibody comprising the heavy chain variable region of (1) and the light chain variable region of (3);
(6) an antibody comprising the heavy chain variable region of (2) and the light chain variable region of (4);
(7) an antibody in which one or more amino acids are substituted, deleted, added, and/or inserted in the antibody of any one of (1) to (6), which has an activity equivalent to that of the antibody of any one of (1) to (6); and
(8) an antibody which binds to the same epitope as an epitope bound by the antibody of any one of (1) to (7);

[6] an anti-NR10 antibody which is any one of:

(1) an antibody comprising a heavy chain variable region which comprises CDR1 comprising the amino acid sequence of SEQ ID NO: 17, CDR2 comprising the amino acid sequence of SEQ ID NO: 18, and CDR3 comprising the amino acid sequence of SEQ ID NO: 19;
(2) an antibody comprising the heavy chain variable region of SEQ ID NO: 20;
(3) an antibody comprising a light chain variable region which comprises CDR1 comprising the amino acid sequence of SEQ ID NO: 21, CDR2 comprising the amino acid sequence of SEQ ID NO: 22, and CDR3 comprising the amino acid sequence of SEQ ID NO: 23;
(4) an antibody comprising the light chain variable region of SEQ ID NO: 24;
(5) an antibody comprising the heavy chain variable region of (1) and the light chain variable region of (3);
(6) an antibody comprising the heavy chain variable region of (2) and the light chain variable region of (4);
(7) an antibody in which one or more amino acids are substituted, deleted, added, and/or inserted in the antibody of any one of (1) to (6), which has an activity equivalent to that of the antibody of any one of (1) to (6); and
(8) an antibody which binds to the same epitope as an epitope bound by the antibody of any one of (1) to (7);

[7] an anti-NR10 antibody which is any one of:

(1) an antibody comprising a heavy chain variable region which comprises CDR1 comprising the amino acid sequence of SEQ ID NO: 25, CDR2 comprising the amino acid sequence of SEQ ID NO: 26, and CDR3 comprising the amino acid sequence of SEQ ID NO: 27;
(2) an antibody comprising the heavy chain variable region of SEQ ID NO: 28;
(3) an antibody comprising a light chain variable region which comprises CDR1 comprising the amino acid sequence of SEQ ID NO: 29, CDR2 comprising the amino acid sequence of SEQ ID NO: 30, and CDR3 comprising the amino acid sequence of SEQ ID NO: 31;
(4) an antibody comprising the light chain variable region of SEQ ID NO: 32;
(5) an antibody comprising the heavy chain variable region of (1) and the light chain variable region of (3);
(6) an antibody comprising the heavy chain variable region of (2) and the light chain variable region of (4);
(7) an antibody in which one or more amino acids are substituted, deleted, added, and/or inserted in the antibody of any one of (1) to (6), which has an activity equivalent to that of the antibody of any one of (1) to (6); and
(8) an antibody which binds to the same epitope as an epitope bound by the antibody of any one of (1) to (7);

[8] an antibody or antibody variable region which is any one of:

(1) a heavy chain variable region comprising CDR1 of SEQ ID NO: 196, CDR2 of SEQ ID NO: 197, and CDR3 of SEQ ID NO: 11 (H17);
(2) a heavy chain variable region comprising CDR1 of SEQ ID NO: 176, CDR2 of SEQ ID NO: 197, and CDR3 of SEQ ID NO: 11 (H19);
(3) a heavy chain variable region comprising CDR1 of SEQ ID NO: 196, CDR2 of SEQ ID NO: 197, and CDR3 of SEQ ID NO: 184 (H28, H42);
(4) a heavy chain variable region comprising CDR1 of SEQ ID NO: 9, CDR2 of SEQ ID NO: 197, and CDR3 of SEQ ID NO: 184 (H30, H44);
(5) a heavy chain variable region comprising CDR1 of SEQ ID NO: 176, CDR2 of SEQ ID NO: 197, CDR3 of SEQ ID NO: 184 (H34, H46);
(6) a heavy chain variable region comprising CDR1 of SEQ ID NO: 9, CDR2 of SEQ ID NO: 198, and CDR3 of SEQ ID NO: 184 (H57, H78);
(7) a heavy chain variable region comprising CDR1 of SEQ ID NO: 176, CDR2 of SEQ ID NO: 198, and CDR3 of SEQ ID NO: 184 (H71, H92);
(8) a heavy chain variable region comprising CDR1 of SEQ ID NO: 9, CDR2 of SEQ ID NO: 199, and CDR3 of SEQ ID NO: 184 (H97, H98);
(9) a light chain variable region comprising CDR1 of SEQ ID NO: 200, CDR2 of SEQ ID NO: 170, and CDR3 of SEQ ID NO: 193 (L11);
(10) a light chain variable region comprising CDR1 of SEQ ID NO: 201, CDR2 of SEQ ID NO: 170, and CDR3 of SEQ ID NO: 193 (L12);
(11) a light chain variable region comprising CDR1 of SEQ ID NO: 202, CDR2 of SEQ ID NO: 170, and CDR3 of SEQ ID NO: 193 (L17);
(12) a light chain variable region comprising CDR1 of SEQ ID NO: 203, CDR2 of SEQ ID NO: 170, and CDR3 of SEQ ID NO: 193 (L50);
(13) an antibody comprising the heavy chain variable region of (3) and the light chain variable region of (11);
(14) an antibody comprising the heavy chain variable region of (4) and the light chain variable region of (11);
(15) an antibody comprising the heavy chain variable region of (5) and the light chain variable region of (11);
(16) an antibody comprising the heavy chain variable region of (6) and the light chain variable region of (11);
(17) an antibody comprising the heavy chain variable region of (7) and the light chain variable region of (11);
(18) an antibody comprising the heavy chain variable region of (8) and the light chain variable region of (12);
(19) an antibody in which one or more amino acids are substituted, deleted, added, and/or inserted in the antibody of any one of (13) to (18), which has an activity equivalent to that of the antibody of any one of (13) to (18); and
(20) an antibody which binds to the same epitope as an epitope bound by the antibody of any one of (13) to (18);

[9] an antibody or antibody variable region which is any one of:

(1) a heavy chain variable region comprising the amino acid sequence of SEQ ID NO: 204 (H17);
(2) a heavy chain variable region comprising the amino acid sequence of SEQ ID NO: 205 (H19);
(3) a heavy chain variable region comprising the amino acid sequence of SEQ ID NO: 206 (H28);
(4) a heavy chain variable region comprising the amino acid sequence of SEQ ID NO: 207 (H30);
(5) a heavy chain variable region comprising the amino acid sequence of SEQ ID NO: 208 (H34),
(6) a heavy chain variable region comprising the amino acid sequence of SEQ ID NO: 209 (H42);
(7) a heavy chain variable region comprising the amino acid sequence of SEQ ID NO: 210 (H44);
(8) a heavy chain variable region comprising the amino acid sequence of SEQ ID NO: 211 (H46);
(9) a heavy chain variable region comprising the amino acid sequence of SEQ ID NO: 212 (H57);
(10) a heavy chain variable region comprising the amino acid sequence of SEQ ID NO: 213 (H71);
(11) a heavy chain variable region comprising the amino acid sequence of SEQ ID NO: 214 (H78);
(12) a heavy chain variable region comprising the amino acid sequence of SEQ ID NO: 215 (H92);
(13) a heavy chain variable region comprising the amino acid sequence of SEQ ID NO: 216 (H97);
(14) a heavy chain variable region comprising the amino acid sequence of SEQ ID NO: 217 (H98);
(15) a light chain variable region comprising the amino acid sequence of SEQ ID NO: 218 (L11);
(16) a light chain variable region comprising the amino acid sequence of SEQ ID NO: 219 (L12);
(17) a light chain variable region comprising the amino acid sequence of SEQ ID NO: 220 (L17);
(18) a light chain variable region comprising the amino acid sequence of SEQ ID NO: 221 (L50);
(19) an antibody comprising the heavy chain variable region of (3) and the light chain variable region of (17) (H28L17);
(20) an antibody comprising the heavy chain variable region of (4) and the light chain variable region of (17) (H30L17);
(21) an antibody comprising the heavy chain variable region of (5) and the light chain variable region of (17) (H34L17);
(22) an antibody comprising the heavy chain variable region of (6) and the light chain variable region of (17) (H42L17);
(23) an antibody comprising the heavy chain variable region of (7) and the light chain variable region of (17) (H44L17);
(24) an antibody comprising the heavy chain variable region of (8) and the light chain variable region of (17) (H46L17);
(25) an antibody comprising the heavy chain variable region of (9) and the light chain variable region of (17) (H57L17);
(26) an antibody comprising the heavy chain variable region of (10) and the light chain variable region of (17) (H71L17);
(27) an antibody comprising the heavy chain variable region of (11) and the light chain variable region of (17) (H78L17);
(28) an antibody comprising the heavy chain variable region of (12) and the light chain variable region of (17) (H92L17);
(29) an antibody comprising the heavy chain variable region of (13) and the light chain variable region of (18) (H97L50);
(30) an antibody comprising the heavy chain variable region of (14) and the light chain variable region of (18) (H98L50),
(31) an antibody in which one or more amino acids are substituted, deleted, added, and/or inserted in the antibody of any one of (19) to (30), which has an activity equivalent to that of the antibody of any one of (19) to (30); and
(32) an antibody which binds to the same epitope as an epitope bound by the antibody of any one of (19) to (30);

[10] the anti-NR10 antibody of any one of [4] to [9], which is a humanized antibody;
[11] an antibody, antibody heavy chain, or antibody light chain, which is any one of:

(1) a heavy chain comprising the amino acid sequence of SEQ ID NO: 222 (H17);
(2) a heavy chain comprising the amino acid sequence of SEQ ID NO: 223 (H19);
(3) a heavy chain comprising the amino acid sequence of SEQ ID NO: 224 (H28);
(4) a heavy chain comprising the amino acid sequence of SEQ ID NO: 225 (H30);
(5) a heavy chain comprising the amino acid sequence of SEQ ID NO: 226 (H34);
(6) a heavy chain comprising the amino acid sequence of SEQ ID NO: 227 (H42);
(7) a heavy chain comprising the amino acid sequence of SEQ ID NO: 228 (H44);
(8) a heavy chain comprising the amino acid sequence of SEQ ID NO: 229 (H46);
(9) a heavy chain comprising the amino acid sequence of SEQ ID NO: 230 (H57);
(10) a heavy chain comprising the amino acid sequence of SEQ ID NO: 231 (H71);
(11) a heavy chain comprising the amino acid sequence of SEQ ID NO: 232 (H78);
(12) a heavy chain comprising the amino acid sequence of SEQ ID NO: 233 (H92);
(13) a heavy chain comprising the amino acid sequence of SEQ ID NO: 234 (H97);
(14) a heavy chain comprising the amino acid sequence of SEQ ID NO: 235 (H98);
(15) a light chain comprising the amino acid sequence of SEQ ID NO: 236 (L11);
(16) a light chain comprising the amino acid sequence of SEQ ID NO: 237 (L12);
(17) a light chain comprising the amino acid sequence of SEQ ID NO: 238 (L17);
(18) a light chain comprising the amino acid sequence of SEQ ID NO: 239 (L50);
(19) an antibody comprising the heavy chain of (3) and the light chain of (17) (H28L17);
(20) an antibody comprising the heavy chain of (4) and the light chain of (17) (H30L17);
(21) an antibody comprising the heavy chain of (5) and the light chain of (17) (H34L17);
(22) an antibody comprising the heavy chain of (6) and the light chain of (17) (H42L17);
(23) an antibody comprising the heavy chain of (7) and the light chain of (17) (H44L17);
(24) an antibody comprising the heavy chain of (8) and the light chain of (17) (H46L17);
(25) an antibody comprising the heavy chain of (9) and the light chain of (17) (H57L17);
(26) an antibody comprising the heavy chain of (10) and the light chain of (17) (H71L17);
(27) an antibody comprising the heavy chain of (11) and the light chain of (17) (H78L17);

(28) an antibody comprising the heavy chain of (12) and the light chain of (17) (H92L17);
(29) an antibody comprising the heavy chain of (13) and the light chain of (18) (H97L50);
(30) an antibody comprising the heavy chain of (14) and the light chain of (18) (H98L50);
(31) an antibody in which one or more amino acids are substituted, deleted, added, and/or inserted in the antibody of any one of (19) to (30), which has an activity equivalent to that of the antibody of any one of (19) to (30); and
(32) an antibody which binds to the same epitope as an epitope bound by the antibody of any one of (19) to (30);

[12] a pharmaceutical composition comprising the antibody of any one of [1] to [11];
[13] the pharmaceutical composition of [12], which is an agent for treating an inflammatory disease;
[14] a method for treating or preventing an inflammatory disease, which comprises the step of administering the antibody of any one of [1] to [11]; and
[15] use of the antibody of any one of [1] to [11] in the preparation of a therapeutic agent for an inflammatory disease.

<u>Brief Description of the Drawings</u>

Fig. 1 shows the amino acid sequences of the heavy chain variable regions of mouse antibodies NS18, NS22, NS23, and NS33.
Fig. 2 shows the amino acid sequences of the light chain variable regions of mouse antibodies NS18, NS22, NS23, and NS33.
Fig. 3 is a graph showing the inhibition of hNR10/hOSMR/BaF3 cell growth by hybridoma culture supernatants.
Fig. 4 is a graph showing the inhibition of cynNR10/cynOSMR/BaF3 cell growth by hybridoma culture supernatants.
Fig. 5 is a graph showing the assessment of the activity of chimeric NS22 (BaF).
Fig. 6 is a graph showing the assessment of the activity of chimeric NS22 (DU-145).
Fig. 7 is a graph showing the assessment of the competition of chimeric NS22 with IL-31.
Fig. 8 is a graph showing the NR10 competitive binding activity of anti-NR10 antibodies.
Fig. 9 is a set of graphs showing the assessment of the competition of humanized NS22 (H0L0) with IL-31.
Fig. 10 shows the effect of the constant region of humanized anti-NR10 antibody H0L0 on the heterogeneity assessed by cation exchange chromatography.
Fig. 11 is a set of graphs showing the assessment of the competition of mutants of the humanized anti-NR10 antibody of which the isoelectric point of the variable regions is lowered without significant loss of the binding to NR10, with IL-31.
Fig. 12 shows the effect of the constant region of anti-IL-6 receptor antibody on the heterogeneity assessed by cation exchange chromatography.
Fig. 13 shows the effect of the constant region of anti-IL-6 receptor antibody on the denaturation peak assessed by DSC.
Fig. 14 shows the effect of the novel constant region M14 on the heterogeneity in an anti-IL-6 receptor antibody, assessed by cation exchange chromatography.
Fig. 15 shows the effect of the novel constant region M58 on the heterogeneity in an anti-IL-6 receptor antibody, assessed by cation exchange chromatography.
Fig. 16 shows the effect of the novel constant region M58 on the denaturation peak in an anti-IL-6 receptor antibody, assessed by DSC.
Fig. 17 shows the result of assaying the retention of huPM1-IgG1 and huPM1-M58 in the plasma of human FcRn transgenic mice.
Fig. 18 shows the biological activity of each antibody assessed using BaF/NR10.
Fig. 19 shows the analysis of thermally-accelerated (dotted line) and non-accelerated (solid line) samples of each modified antibody by cation exchange chromatography to compare the generation of degradation products between before and after thermal acceleration. Arrow indicates the peak position of basic component which was altered.
Fig. 20 is a set of graphs showing the assessment (BaF) of the activity of each variant.
Fig. 21 is a graph showing the assessment (BaF) of the activity of Ha401La402 and H0L0.
Fig. 22 is a graph showing the assessment (BaF) of the activity of H17L11 and H0L0.
Fig. 23 is a graph showing the assessment (BaF) of the activity of H19L12 and H0L0.
Fig. 24 is a graph showing the biological activity of H0L12 and H0L17 assessed using BaF/NR10.
Fig. 25-1 is a set of graphs showing the assessment (BaF) of the activity of each variant.
Fig. 25-2 is a continuation of Fig. 25-1.
Fig. 26 is a schematic diagram for human/mouse wild-type and chimeric NR10-ECD.

Fig. 27 is a set of photographs showing the detection of the binding domain by Western blotting. A is a photograph showing the result of detection using a humanized anti-human NR10 antibody; B is a photograph showing the result of detection using a mouse anti-human NR10 antibody; and C is a photograph showing the result of detection using an anti-Myc antibody. With the anti-human NR10 antibody a binding antigen was detected only in hhh, hhm, and hmm, but not in mmm, mmh, and mhm.

Fig. 28-1 shows the amino acid sequence of each variant of H0 (SEQ ID NO: 50).

Fig. 28-2 is a continuation of Fig. 28-1.

Fig. 28-3 is a continuation of Fig. 28-2.

Fig. 29-1 shows the amino acid sequence of each variant of L0 (SEQ ID NO: 52).

Fig. 29-2 is a continuation of Fig. 29-1.

Mode for Carrying Out the Invention

NR10

NR10 is a protein that forms a heterodimer with oncostatin M receptor (OSMR) and functions as an IL-31 receptor. NR10 is also known as glm-r (J Biol Chem 277, 16831-6, 2002), GPL (J Biol Chem 278,49850-9,2003), IL31RA (Nat Immunol 5, 752-60,2004), and such. Thus, NR10 in the present invention also includes proteins called by such names.

In the present invention, NR10 (also referred to as IL31RA, GPL, or glm-r) is not particularly limited in terms of its origin, and includes those derived from humans, mice, monkeys, and other mammals. NR10 derived from humans, mice, and monkeys is preferred, and human-derived NR10 is particularly preferred.

There are multiple known splicing variants of human-derived NR10 (WO 00/075314). Of the above-described splicing variants, NR10.1 consists of 662 amino acids and contains a transmembrane domain. NR10.2 is a soluble receptor-like protein consisting of 252 amino acids without the transmembrane domain. Meanwhile, known NR10 splicing variants that function as transmembrane receptor proteins include NR10.3 and IL-31RAv3. The human NR10 of the present invention is not particularly limited, as long as it forms a heterodimer with oncostatin M receptor (OSMR) and functions as an IL-31 receptor. Preferred NR10 includes NR10.3 (also referred to as ILRAv4 (Nat Immunol 5, 752-60, 2004)) and IL-31RAv3. NR 10.3 (IL31RAv4) consists of 662 amino acids (WO 00/075314; Nat Immunol 5, 752-60,2004) and IL31RAv3 consists of 732 amino acids (GenBank Accession No: NM_139017). The amino acid sequence of IL31RAv4 is shown in SEQ ID NO: 79, and the amino acid sequence of IL31RAv3 is shown in SEQ ID NO: 80. Meanwhile, mouse-derived NR10 includes proteins comprising the amino acid sequence of SEQ ID NO: 81. In addition, cynomolgus monkey-derived NR10 includes proteins comprising the amino acid sequence of SEQ ID NO: 66.

Antibodies (sequences)

Preferred embodiments of the anti-NR10 antibody of the present invention include the anti-NR10 antibodies of any one of (1) to (8) in (A) to (D) below.

(A)NS18

(1) antibodies having a heavy chain variable region that comprises CDR1 having the amino acid sequence of SEQ ID NO: 1 (HCDR1), CDR2 having the amino acid sequence of SEQ ID NO: 2 (HCDR2), and CDR3 having the amino acid sequence of SEQ ID NO: 3 (HCDR3);

(2) antibodies having the heavy chain variable region of SEQ ID NO: 4 (VH);

(3) antibodies having a light chain variable region that comprises CDR1 having the amino acid sequence of SEQ ID NO: 5 (LCDR1), CDR2 having the amino acid sequence of SEQ ID NO: 6 (LCDR2), and CDR3 having the amino acid sequence of SEQ ID NO: 7 (LCDR3);

(4) antibodies having the light chain variable region of SEQ ID NO: 8 (VL);

(5) antibodies having the heavy chain variable region of (1) and the light chain variable region of (3);

(6) antibodies having the heavy chain variable region of (2) and the light chain variable region of (4);

(7) antibodies in which one or more amino acids are substituted, deleted, added, and/or inserted in the antibodies of any one of (1) to (6), which have an activity equivalent to that of the antibodies of any one of (1) to (6); and

(8) antibodies that bind to the same epitope as an epitope bound by the antibodies of any one of (1) to (7).

(B) NS22

(1) antibodies having a heavy chain variable region that comprises CDR1 having the amino acid sequence of SEQ ID NO: 9 (HCDR1), CDR2 having the amino acid sequence of SEQ ID NO: 10 (HCDR2), and CDR3 having the amino acid sequence of SEQ ID NO: 11 (HCDR3);
(2) antibodies having the heavy chain variable region of SEQ ID NO: 12 (VH);
(3) antibodies having a light chain variable region that comprises CDR1 having the amino acid sequence of SEQ ID NO: 13 (LCDR1), CDR2 having the amino acid sequence of SEQ ID NO: 14 (LCDR2), and CDR3 having the amino acid sequence of SEQ ID NO: 15 (LCDR3);
(4) antibodies having the light chain variable region of SEQ ID NO: 16 (VL);
(5) antibodies having the heavy chain variable region of (1) and the light chain variable region of (3);
(6) antibodies having the heavy chain variable region of (2) and the light chain variable region of (4);
(7) antibodies in which one or more amino acids are substituted, deleted, added, and/or inserted in the antibodies of any one of (1) to (6), which have an activity equivalent to that of the antibodies of any one of (1) to (6); and
(8) antibodies that bind to the same epitope as an epitope bound by the antibodies of any one of (1) to (7).

Specific examples of the above-described substitution, deletion, addition, and/or insertion of one or more amino acids are not particularly limited and include, for example, the following modifications.
Substitution of Ile at position 3 in the heavy chain CDR1 of SEQ ID NO: 9 with another amino acid. The amino acid after substitution is not particularly limited but preferred examples thereof include Val.
Substitution of Met at position 4 in the heavy chain CDR1 of SEQ ID NO: 9 with another amino acid. The amino acid after substitution is not particularly limited but preferred examples thereof include Ile.
Substitution of Met at position 4 in the heavy chain CDR1 of SEQ ID NO: 9 with another amino acid. The amino acid after substitution is not particularly limited but preferred examples thereof include Leu.
Substitution of Ile at position 3 in the heavy chain CDR1 of SEQ ID NO: 9 with another amino acid. The amino acid after substitution is not particularly limited but preferred examples thereof include Ala.
Substitution of Leu at position 1 in the heavy chain CDR2 of SEQ ID NO: 10 with another amino acid. The amino acid after substitution is not particularly limited but preferred examples thereof include Glu.
Substitution of Asn at position 3 in the heavy chain CDR2 of SEQ ID NO: 10 with another amino acid. The amino acid after substitution is not particularly limited but preferred examples thereof include Asp.
Substitution of Gln at position 13 in the heavy chain CDR2 of SEQ ID NO: 10 with another amino acid. The amino acid after substitution is not particularly limited but preferred examples thereof include Asp.
Substitution of Lys at position 14 in the heavy chain CDR2 of SEQ ID NO: 10 with another amino acid. The amino acid after substitution is not particularly limited but preferred examples thereof include Gln.
Substitution of Lys at position 16 in the heavy chain CDR2 of SEQ ID NO: 10 with another amino acid. The amino acid after substitution is not particularly limited but preferred examples thereof include Gln.
Substitution of Gly at position 17 in the heavy chain CDR2 of SEQ ID NO: 10 with another amino acid. The amino acid after substitution is not particularly limited but preferred examples thereof include Asp.
Substitution of Lys and Gly at positions 16 and 17, respectively, in the heavy chain CDR2 of SEQ ID NO: 10 with another amino acid. The amino acid after substitution is not particularly limited but preferred examples include substitution of Lys at position 16 with Gln, and Gly at position 17 with Asp.
Substitution of Lys, Lys, and Gly at positions 14, 16, and 17, respectively, in the heavy chain CDR2 of SEQ ID NO: 10 with another amino acid. The amino acid after substitution is not particularly limited but preferred examples include substitution of Lys at position 14 with Gln, Lys at position 16 with Gln, and Gly at position 17 with Asp.
Substitution of Gln, Lys, Lys, and Gly at positions 13, 14, 16, and 17, respectively, in the heavy chain CDR2 of SEQ ID NO: 10 with another amino acid. The amino acid after substitution is not particularly limited but preferred examples include substitution of Gln at position 13 with Asp, Lys at position 14 with Gln, Lys at position 16 with Gln, and Gly at position 17 with Asp.
Substitution of Ser at position 10 in the heavy chain CDR2 of SEQ ID NO: 10 with another amino acid. The amino acid after substitution is not particularly limited but preferred examples thereof include Asp.
Substitution of Gln at position 13 in the heavy chain CDR2 of SEQ ID NO: 10 with another amino acid. The amino acid after substitution is not particularly limited but preferred examples thereof include Pro.
Substitution of Tyr at position 3 in the heavy chain CDR3 of SEQ ID NO: 11 with another amino acid. The amino acid after substitution is not particularly limited but preferred examples thereof include Leu.
Substitution of Met at position 10 in the heavy chain CDR3 of SEQ ID NO: 11 with another amino acid. The amino acid after substitution is not particularly limited but preferred examples thereof include Leu.

Substitution of Asp at position 11 in the heavy chain CDR3 of SEQ ID NO: 11 with another amino acid. The amino acid after substitution is not particularly limited but preferred examples thereof include Glu.

Substitution of Tyr at position 12 in the heavy chain CDR3 of SEQ ID NO: 11 with another amino acid. The amino acid after substitution is not particularly limited but preferred examples thereof include Thr and Ser.

Substitution of Met, Asp, and Tyr at positions 10, 11, and 12, respectively, in the heavy chain CDR3 of SEQ ID NO: 11 with another amino acid. The amino acid after substitution is not particularly limited but preferred examples include substitution of Met at position 10 with Leu, Asp at position 11 with Glu, and Tyr at position 12 with Thr.

Substitution of Asp and Tyr at positions 11 and 12, respectively, in the heavy chain CDR3 of SEQ ID NO: 11 with another amino acid. The amino acid after substitution is not particularly limited but preferred examples include substitution of Asp at position 11 with Glu, and Tyr at position 12 with Thr.

Substitution of Tyr, Asp, and Tyr at positions 3, 11, and 12, respectively, in the heavy chain CDR3 of SEQ ID NO: 11 with another amino acid. The amino acid after substitution is not particularly limited but preferred examples include substitution of Tyr at position 3 with Leu, Asp at position 11 with Glu, and Tyr at position 12 with Thr or Ser.

Substitution of Arg at position 1 in the light chain CDR1 of SEQ ID NO: 13 with another amino acid. The amino acid after substitution is not particularly limited but preferred examples thereof include Gln.

Substitution of Asn at position 5 in the light chain CDR1 of SEQ ID NO: 13 with another amino acid. The amino acid after substitution is not particularly limited but preferred examples thereof include Asp.

Substitution of Arg and Asn at positions 1 and 5, respectively, in the light chain CDR1 of SEQ ID NO: 13 with another amino acid. The amino acid after substitution is not particularly limited but preferred examples include substitution of Arg at position 1 with Gln, and Asn at position 5 with Asp.

Substitution of Ser at position 8 in the light chain CDR1 of SEQ ID NO: 13 with another amino acid. The amino acid after substitution is not particularly limited but preferred examples thereof include Arg.

Substitution of Leu at position 10 in the light chain CDR1 of SEQ ID NO: 13 with another amino acid. The amino acid after substitution is not particularly limited but preferred examples thereof include Val.

Substitution of Ser and Leu at positions 8 and 10, respectively, in the light chain CDR1 of SEQ ID NO: 13 with another amino acid. The amino acid after substitution is not particularly limited but preferred examples include substitution of Ser at position 8 with Arg, and Leu at position 10 with Val.

Substitution of Thr at position 2 in the light chain CDR1 of SEQ ID NO: 13 with another amino acid. The amino acid after substitution is not particularly limited but preferred examples thereof include Ala and Ser.

Substitution of Asn at position 1 in the light chain CDR2 of SEQ ID NO: 14 with another amino acid. The amino acid after substitution is not particularly limited but preferred examples thereof include Asp.

Substitution of Lys at position 3 in the light chain CDR2 of SEQ ID NO: 14 with another amino acid. The amino acid after substitution is not particularly limited but preferred examples thereof include Gln.

Substitution of Leu at position 5 in the light chain CDR2 of SEQ ID NO: 14 with another amino acid. The amino acid after substitution is not particularly limited but preferred examples thereof include Glu.

Substitution of Lys at position 7 in the light chain CDR2 of SEQ ID NO: 14 with another amino acid. The amino acid after substitution is not particularly limited but preferred examples thereof include Gln and Asp.

Substitution of Lys, Leu, and Lys at positions 3, 5, and 7, respectively, in the light chain CDR2 of SEQ ID NO: 14 with another amino acid. The amino acid after substitution is not particularly limited but preferred examples include substitution of Lys at position 3 with Gln, Leu at position 5 with Glu, and Lys at position 7 with Gln.

Substitution of Glu at position 5 in the light chain CDR3 of SEQ ID NO: 15 with another amino acid. The amino acid after substitution is not particularly limited but preferred examples thereof include Asp.

Substitution of Ser at position 6 in the light chain CDR3 of SEQ ID NO: 15 with another amino acid. The amino acid after substitution is not particularly limited but preferred examples thereof include Asp.

Substitution of Thr at position 9 in the light chain CDR3 of SEQ ID NO: 15 with another amino acid. The amino acid after substitution is not particularly limited but preferred examples thereof include Phe.

Each of the above-described substitutions may be made alone, or multiple substitutions may be made in combination. Furthermore, the above substitutions may be combined with other substitutions. These substitutions can improve the antibody pharmacokinetics (retention in plasma), enhance the antigen-binding activity, improve the stability, and/or reduce the risk of immunogenicity.

In the present invention, specific examples of the variable regions having a combination of the above-described substitutions include, for example, heavy chain variable regions having the amino acid sequence of SEQ ID NO: 167 and light chain variable regions having the amino acid sequence of SEQ ID NO: 168. Moreover, examples of the antibodies having a combination of the above-described substitutions include, for example, antibodies that comprise a heavy chain variable region having the amino acid sequence of SEQ ID NO: 167 and a light chain variable region having the amino acid sequence of SEQ ID NO: 168.

Moreover, specific examples of the heavy chain or light chain variable regions having a combination of the above-described substitutions include, for example, the following variable regions:

(a) heavy chain variable regions that comprise CDR1 of SEQ ID NO: 196, CDR2 of SEQ ID NO: 197, and CDR3 of SEQ ID NO: 11 (H17);
(b) heavy chain variable regions that comprise CDR1 of SEQ ID NO: 176, CDR2 of SEQ ID NO: 197, and CDR3 of SEQ ID NO: 11 (H19);
(c) heavy chain variable regions that comprise CDR1 of SEQ ID NO: 196, CDR2 of SEQ ID NO: 197, and CDR3 of SEQ ID NO: 184 (H28, H42);
(d) heavy chain variable regions that comprises CDR1 of SEQ ID NO: 9, CDR2 of SEQ ID NO: 197, and CDR3 of SEQ ID NO: 184 (H30, H44);
(e) heavy chain variable regions that comprise CDR1 of SEQ ID NO: 176, CDR2 of SEQ ID NO: 197, and CDR3 of SEQ ID NO: 184 (H34, H46);
(f) heavy chain variable regions that comprise CDR1 of SEQ ID NO: 9, CDR2 of SEQ ID NO: 198, and CDR3 of SEQ ID NO: 184 (H57, H78);
(g) heavy chain variable regions that comprise CDR1 of SEQ ID NO: 176, CDR2 of SEQ ID NO: 198, and CDR3 of SEQ ID NO: 184 (H71, H92);
(h) heavy chain variable regions that comprise CDR1 of SEQ ID NO: 9, CDR2 of SEQ ID NO: 199, and CDR3 of SEQ ID NO: 184 (H97, H98);
(i) light chain variable regions that comprise CDR1 of SEQ ID NO: 200, CDR2 of SEQ ID NO: 170, and CDR3 of SEQ ID NO: 193 (L11);
(j) light chain variable regions that comprise CDR1 of SEQ ID NO: 201, CDR2 of SEQ ID NO: 170, and CDR3 of SEQ ID NO: 193 (L12);
(k) light chain variable regions that comprise CDR1 of SEQ ID NO: 202, CDR2 of SEQ ID NO: 170, and CDR3 of SEQ ID NO: 193 (L17); and
(l) light chain variable regions that comprise CDR1 of SEQ ID NO: 203, CDR2 of SEQ ID NO: 170, and CDR3 of SEQ ID NO: 193 (L50).

Furthermore, specific examples of the antibodies having a combination of the above-described substitutions include, for example:

(i) antibodies that comprise the heavy chain variable region of (c) and the light chain variable region of (k);
(ii) antibodies that comprise the heavy chain variable region of (d) and the light chain variable region of (k);
(iii) antibodies that comprise the heavy chain variable region of (e) and the light chain variable region of (k);
(iv) antibodies that comprise the heavy chain variable region of (f) and the light chain variable region of (k);
(v) antibodies that comprise the heavy chain variable region of (g) and the light chain variable region of (k); and
(vi) antibodies that comprise the heavy chain variable region of (h) and the light chain variable region of (1).

(C) NS23

(1) antibodies having a heavy chain variable region that comprises CDR1 having the amino acid sequence of SEQ ID NO: 17 (HCDR1), CDR2 having the amino acid sequence of SEQ ID NO: 18 (HCDR2), and CDR3 having the amino acid sequence of SEQ ID NO: 19 (HCDR3);
(2) antibodies having the heavy chain variable region of SEQ ID NO: 20 (VH);
(3) antibodies having a light chain variable region that comprises CDR1 having the amino acid sequence of SEQ ID NO: 21 (LCDR1), CDR2 having the amino acid sequence of SEQ ID NO: 22 (LCDR2), and CDR3 having the amino acid sequence of SEQ ID NO: 23 (LCDR3);
(4) antibodies having the light chain variable region of SEQ ID NO: 24 (VL);
(5) antibodies having the heavy chain variable region of (1) and the light chain variable region of (3);
(6) antibodies having the heavy chain variable region of (2) and the light chain variable region of (4);
(7) antibodies in which one or more amino acids are substituted, deleted, added, and/or inserted in the antibodies of any one of (1) to (6), which have an activity equivalent to that of the antibodies of any one of (1) to (6); and
(8) antibodies that bind to the same epitope as an epitope bound by the antibodies of any one of (1) to (7).

(D) NS33

(1) antibodies having a heavy chain variable region that comprise CDR1 having the amino acid sequence of SEQ ID NO: 25 (HCDR1), CDR2 having the amino acid sequence of SEQ ID NO: 26 (HCDR2), and CDR3 having the amino acid sequence of SEQ ID NO: 27 (HCDR3);
(2) antibodies having the heavy chain variable region of SEQ ID NO: 28 (VH);
(3) antibodies having a light chain variable region that comprise CDR1 having the amino acid sequence of SEQ ID NO: 29 (LCDR1), CDR2 having the amino acid sequence of SEQ ID NO: 30 (LCDR2), and CDR3 having the amino acid sequence of SEQ ID NO: 31 (LCDR3);
(4) antibodies having the light chain variable region of SEQ ID NO: 32 (VL);
(5) antibodies having the heavy chain variable region of (1) and the light chain variable region of (3);
(6) antibodies having the heavy chain variable region of (2) and the light chain variable region of (4);
(7) antibodies in which one or more amino acids are substituted, deleted, added, and/or inserted in the antibodies of any one of (1) to (6), which have an activity equivalent to that of the antibodies of any one of (1) to (6); and
(8) antibodies that bind to the same epitope as an epitope bound by the antibodies of any one of (1) to (7).

Any framework regions (FR) may be used for the above-described antibodies of (1) or (3); however, FRs derived from human are preferably used. Furthermore, any constant regions may be used for the above-described antibodies of (1) to (8); however, constant regions derived from human are preferably used. For the antibodies of the present invention, the amino acid sequence of the original FR or constant region may be used without modification, or after being modified to a different amino acid sequence by substitution, deletion, addition, and/or insertion of one or more amino acids.
The amino acid sequence of the heavy chain of the above-described NS18 is shown in SEQ ID NO: 34 and the nucleotide sequence encoding this amino acid sequence is shown in SEQ ID NO: 33. Meanwhile, the amino acid sequence of the light chain is shown in SEQ ID NO: 36 and the nucleotide sequence encoding this amino acid sequence is shown in SEQ ID NO: 35.
The amino acid sequence of the heavy chain of NS22 is shown in SEQ ID NO: 38 and the nucleotide sequence encoding this amino acid sequence is shown in SEQ ID NO: 37. Meanwhile, the amino acid sequence of the light chain is shown in SEQ ID NO: 40 and the nucleotide sequence encoding this amino acid sequence is shown in SEQ ID NO: 39.
The amino acid sequence of the heavy chain of NS23 is shown in SEQ ID NO: 42 and the nucleotide sequence encoding this amino acid sequence is shown in SEQ ID NO: 41. Meanwhile, the amino acid sequence of the light chain is shown in SEQ ID NO: 44 and the nucleotide sequence encoding this amino acid sequence is shown in SEQ ID NO: 43.
The amino acid sequence of the heavy chain of NS33 is shown in SEQ ID NO: 46 and the nucleotide sequence encoding this amino acid sequence is shown in SEQ ID NO: 45. Meanwhile, the amino acid sequence of the light chain is shown in SEQ ID NO: 48 and the nucleotide sequence encoding this amino acid sequence is shown in SEQ ID NO: 47.
In the present invention, the "activity equivalent to that of the antibody of any one of (1) to (6)" means that the activity of binding and/or neutralizing NR10 (for example, human NR10) is equivalent. In the present invention, the term "equivalent" means that the activity is not necessarily the same but may be enhanced or reduced as long as the activity is retained. Antibodies with a reduced activity include, for example, antibodies having an activity that is 30% or more, preferably 50% or more, and more preferably 80% or more of that of the original antibody.
The antibodies of any one of (1) to (6) mentioned above may have a substitution, deletion, addition, and/or insertion of one or more amino acids in the amino acid sequence of the variable regions (CDR sequences and/or FR sequences), as long as the NR10-binding and/or neutralizing activity is retained. Methods well known to those skilled in the art to prepare the amino acid sequence of an antibody that has a substitution, deletion, addition, and/or insertion of one or more amino acids in the amino acid sequence and retains NR10-binding and/or neutralizing activity, include methods for introducing mutations into proteins. For example, those skilled in the art can prepare mutants functionally equivalent to the antibody having NR10-binding and/or neutralizing activity by introducing appropriate mutations into the amino acid sequence of the antibody having NR10-binding and/or neutralizing activity using site-directed mutagenesis (Hashimoto-Gotoh, T, Mizuno, T, Ogasahara, Y, and Nakagawa, M. (1995) An oligodeoxyribonucleotide-directed dual amber method for site-directed mutagenesis. Gene 152,271-275, Zoller, MJ, and Smith, M.(1983) Oligonucleotide-directed mutagenesis of DNA fragments cloned into M13 vectors. Methods Enzymol. 100, 468-500, Kramer, W, Drutsa, V, Jansen, HW, Kramer, B, Pflugfelder, M, and Fritz, HJ (1984) The gapped duplex DNA approach to oligonucleotide-directed mutation construction. Nucleic Acids Res. 12, 9441-9456, Kramer W, and Fritz HJ (1987) Oligonucleotide-directed construction of mutations via gapped duplex DNA Methods. Enzymol. 154, 350-367, Kunkel, TA (1985) Rapid and efficient site-specific mutagenesis without phenotypic selection. Proc Natl Acad Sci USA. 82, 488-492) or the like. Thus, antibodies that contain one or more amino acid mutations in the variable regions and have NR10-binding and/or neutralizing activity are also included in the antibody of the present invention.
When an amino acid residue is altered, the amino acid is preferably mutated for a different amino acid(s) that conserves the properties of the amino acid side-chain. Examples of amino acid side chain properties are: hydrophobic amino acids (A, I, L, M, F, P, W, Y, and V), hydrophilic amino acids (R, D, N, C, E, Q, G, H, K, S, and T), amino acids containing aliphatic side chains (G, A, V, L, I, and P), amino acids containing hydroxyl group-containing side chains (S, T, and Y), amino acids containing sulfur-containing side chains (C and M), amino acids containing carboxylic acid- and amide-containing side chains (D, N, E, and Q), amino acids containing basic side chains (R, K, and H), and amino acids containing aromatic side chains (H, F, Y, and W) (amino acids are represented by one-letter codes in parentheses). Amino acid substitutions within each group are called conservative substitutions. It is already known that a polypeptide containing a modified amino acid sequence in which one or more amino acid residues in a given amino acid sequence are deleted, added, and/or substituted with other amino acids can retain the original biological activity (Mark, D. F. et al., Proc. Natl. Acad. Sci. USA; (1984) 81:5662-6; Zoller, M. J. and Smith, M., Nucleic Acids Res. (1982) 10:6487-500; Wang, A. et al., Science (1984) 224:1431-3; Dalbadie-McFarland, G. et al., Proc. Natl. Acad. Sci. USA (1982) 79:6409-13). Such mutants have an amino acid identity of at least 70%, more preferably at least 75%, even more preferably at least 80%, still more preferably at least 85%, yet more preferably at least 90%, and most preferably at least 95%, with the variable regions (for example, CDR sequences, FR sequences, or whole variable regions) of the present invention. Herein, sequence identity is defined as the percentage of residues identical to those in the original amino acid sequence of the heavy chain variable region or light chain variable region, determined after the sequences are aligned and gaps are appropriately introduced to maximize the sequence identity as necessary. The identity of amino acid sequences can be determined by the method described below.

Alternatively, the amino acid sequences of variable regions that have a substitution, deletion, addition, and/or insertion of one or more amino acids in the amino acid sequence of the variable regions (CDR sequences and/or FR sequences) and retain NR10-binding and/or neutralizing activity can be obtained from nucleic acids that hybridize under stringent conditions to nucleic acid composed of the nucleotide sequence encoding the amino acid sequence of the variable regions. Stringent hybridization conditions to isolate a nucleic acid that hybridizes under stringent conditions to a nucleic acid that includes the nucleotide sequence encoding the amino acid sequence of the variable regions include, for example, the conditions of 6M urea, 0.4% SDS, 0.5x SSC, and 37°C, or hybridization conditions with stringencies equivalent thereto. With more stringent conditions, for example, the conditions of 6M urea, 0.4% SDS, 0.1x SSC, and 42°C, isolation of nucleic acids with a much higher homology can be expected. The sequences of the isolated nucleic acids can be determined by the known methods described below. The overall nucleotide sequence homology of the isolated nucleic acid is at least 50% or higher sequence identity, preferably 70% or higher, more preferably 90% or higher (for example, 95%, 96%, 97%, 98%, 99%, or higher).

Nucleic acids that hybridize under stringent conditions to a nucleic acid composed of the nucleotide sequence encoding the amino acid sequence of the variable regions can also be isolated using, instead of the above-described methods using hybridization techniques, gene amplification methods such as polymerase chain reaction (PCR) using primers synthesized based on the information of nucleotide sequence encoding the amino acid sequence of the variable regions.

Specifically, the identity of one nucleotide sequence or amino acid sequence to another can be determined using the algorithm BLAST, by Karlin and Altschul (Proc. Natl. Acad. Sci. USA (1993) 90, 5873-7). Programs such as BLASTN and BLASTX were developed based on this algorithm (Altschul et al., J. Mol. Biol. (1990) 215, 403-10). To analyze nucleotide sequences according to BLASTN based on BLAST, the parameters are set, for example, as score= 100 and wordlength= 12. On the other hand, parameters used for the analysis of amino acid sequences by BLASTX based on BLAST include, for example, score= 50 and wordlength= 3. Default parameters for each program are used when using the BLAST and Gapped BLAST programs. Specific techniques for such analyses are known in the art (see the website of the National Center for Biotechnology Information (NCBI), Basic Local Alignment Search Tool (BLAST); http://www.ncbi.nlm.nih.gov).

The present invention also provides antibodies that bind to the same epitope as an epitope bound by the antibodies of any one of (1) to (7).

Whether an antibody recognizes the same epitope as that recognized by another antibody can be confirmed by the competition between the two antibodies against the epitope. Competition between the antibodies can be evaluated by competitive binding assays using means such as ELISA, fluorescence energy transfer method (FRET), and fluorometric microvolume assay technology (FMAT(R)). The amount of antibodies bound to an antigen indirectly correlate with the binding ability of candidate competitor antibodies (test antibodies) that competitively bind to the same epitope. In other words, as the amount of or the affinity of test antibodies against the same epitope increases, the amount of antibodies bound to the antigen decreases, and the amount of test antibodies bound to the antigen increases. Specifically, appropriately labeled antibodies and antibodies to be evaluated are simultaneously added to the antigens, and the thus bound antibodies are detected using the label. The amount of antibodies bound to the antigen can be easily determined by labeling the antibodies beforehand. This label is not particularly limited, and the labeling method is selected according to the assay technique used. The labeling method includes fluorescent labeling, radiolabeling, enzymatic labeling, and such.

For example, fluorescently labeled antibodies and unlabeled antibodies or test antibodies are simultaneously added to animal cells expressing NR10, and the labeled antibodies are detected by fluorometric microvolume assay technology.

Herein, the "antibody that recognizes the same epitope" refers to an antibody that can reduce the binding of the labeled antibody by at least 50% at a concentration that is usually 100 times higher, preferably 80 times higher, more preferably 50 times higher, even more preferably 30 times higher, and still more preferably 10 times higher than a concentration at which the non-labeled antibody reduces the binding of the labeled antibody by 50% ($IC_{50}$).

Antibodies that bind to the epitope to which the antibodies set forth in any one of (1) to (7) above bind are useful because they have a particularly high neutralizing activity.

The antibodies set forth in any one of (1) to (8) above are preferably humanized antibodies, but are not particularly limited thereto.

Furthermore, the present invention provides genes encoding the anti-NR10 antibodies of any one of (1) to (8) of (A) to (D) above. The genes of the present invention may be any form of genes, for example, DNAs or RNAs.

Antibodies (humanized)

Preferred embodiments of the antibodies of the present invention include humanized antibodies that bind to NR10. The humanized antibodies can be prepared by methods known to those skilled in the art.

The variable region of antibody is typically composed of three complementarity-determining regions (CDRs) sandwiched by four frames (FRs). The CDRs substantially determine the binding specificity of antibody. The amino acid sequences of CDRs are highly diverse. In contrast, the amino acid sequences of FRs often exhibit high homology between antibodies having different binding specificities. It is therefore said in general that the binding specificity of an antibody can be transplanted to a different antibody by grafting the CDRs.

Humanized antibodies are also referred to as reshaped human antibodies, and they are prepared by transferring the CDRs of an antibody derived from a non-human mammal such as a mouse, to the CDRs of a human antibody. General genetic recombination techniques for their preparation are also known (see European Patent Application Publication No. 125023 and WO 96/02576).

Specifically, for example, when the CDRs are derived from a mouse antibody, a DNA sequence designed such that the CDRs of the mouse antibody are linked with framework regions (FRs) of human antibody is synthesized by PCR using, as primers, several oligonucleotides that have portions overlapping the ends of both CDRs and FRs (see the method described in WO 98/13388). The resulting DNA is then ligated to a DNA encoding a human antibody constant region, inserted into an expression vector, and introduced into a host to produce the antibody (see European Patent Application Publication No. EP 239400 and International Patent Application Publication No. WO 96/02576).

Human antibody framework regions to be linked with CDRs are selected so that the CDRs form a favorable antigen-binding site. If needed, amino acid substitution, deletion, addition, and/or insertion may be introduced into the framework regions of antibody variable region so that the CDRs of the reshaped human antibody form a proper antigen-binding site. For example, mutations can be introduced into the amino acid sequence of FR by applying the PCR method which is used to graft mouse CDRs to human FRs. Specifically, mutations can be introduced into a portion of the nucleotide sequences of primers that anneal to the FRs. The mutations are introduced into FRs synthesized by such primers. The antigen-binding activity of mutant antibodies having amino acid substitutions can be determined and assessed by the method described above, and thereby mutant FR sequences having desired properties can be selected (Sato, K. et al., Cancer Res. (1993) 53, 851-856).

Constant (C) regions from human antibodies are used for those of humanized antibodies. For example, $C\gamma1$, Cy2, $C\gamma3$, $C\gamma4$, $C\mu$, $C\delta$, $C\alpha1$, $C\alpha2$, and $C\varepsilon$ are used for H chains; and $C\kappa$ and $C\lambda$ are used for L chains. The amino acid sequence of $C\kappa$ is shown in SEQ ID NO: 58, and the nucleotide sequence encoding this amino acid sequence is shown in SEQ ID NO: 57. The amino acid sequence of $C\gamma1$ is shown in SEQ ID NO: 60, and the nucleotide sequence encoding this amino acid sequence is shown in SEQ ID NO: 59. The amino acid sequence of $C\gamma2$ is shown in SEQ ID NO: 62, and the nucleotide sequence encoding this amino acid sequence is shown in SEQ ID NO: 61. The amino acid sequence of $C\gamma4$ is shown in SEQ ID NO: 64, and the nucleotide sequence encoding this amino acid sequence is shown in SEQ ID NO: 63. Furthermore, human antibody C regions may be modified to improve the stability of antibody or antibody production. Modified human antibody C regions include, for example, the C regions described herein below. Human antibodies used for humanization may be of any isotype such as IgG, IgM, IgA, IgE, or IgD; however, IgG is preferably used in the present invention. IgG that can be used includes IgG1, IgG2, IgG3, IgG4, and the like.

Moreover, after a humanized antibody is prepared, amino acids in the variable region (for example, CDR and FR) and constant region of the humanized antibody may be deleted, added, inserted, and/or substituted with other amino acids. The antibodies of the present invention also include such humanized antibodies with amino acid substitutions and the like.

The origin of CDRs of a humanized antibody is not particularly limited, and may be any animal. For example, it is possible to use the sequences of mouse antibodies, rat antibodies, rabbit antibodies, camel antibodies, and the like. CDR sequences of mouse antibodies are preferred.

In general, it is difficult to humanize antibodies while retaining the binding and neutralizing activities of the original antibodies. The present invention, however, succeeded in obtaining humanized antibodies having the binding and/or neutralizing activities equivalent to those of the original mouse antibodies. Humanized antibodies are useful when administered to humans for the therapeutic purposes, because they exhibit reduced immunogenicity in the human body.

Preferred examples of the humanized anti-NR10 antibodies of the present invention include, for example:

(a) humanized antibodies that comprise a heavy chain variable region having the amino acid sequence of SEQ ID NO: 50 (H0-VH);
(b) humanized antibodies that comprise a heavy chain variable region having the amino acid sequence of SEQ ID NO: 112 (H1-VH);
(c) humanized antibodies that comprise a light chain variable region having the amino acid sequence of SEQ ID NO: 52 (L0-VL);
(d) humanized antibodies that comprise a heavy chain variable region having the amino acid sequence of SEQ ID NO: 50 (H0-VH) and a light chain variable region having the amino acid sequence of SEQ ID NO: 52 (L0-VL); and
(e) humanized antibodies that comprise a heavy chain variable region having the amino acid sequence of SEQ ID NO: 112 and a light chain variable region having the amino acid sequence of SEQ ID NO: 52.

The heavy chain variable region having the amino acid sequence of SEQ ID NO: 50 (H0-VH), heavy chain variable region having the amino acid sequence of SEQ ID NO: 112, and light chain variable region having the amino acid sequence of SEQ ID NO: 52 (L0-VL) may have a substitution, deletion, addition, and/or insertion of one or more amino acids. The substitution, deletion, addition, and/or insertion of amino acids may be made in either or both of the CDRs and FRs.

Thus, other preferred embodiments of the humanized anti-NR10 antibody of the present invention include, for example:

(f) antibodies that comprise a heavy chain variable region having an amino acid sequence in which one or more amino acids are substituted, deleted, added, and/or inserted in the amino acid sequence of SEQ ID NO: 50 (H0-VH);
(g) antibodies that comprise a heavy chain variable region having an amino acid sequence in which one or more amino acids are substituted, deleted, added, and/or inserted in the amino acid sequence of SEQ ID NO: 112 (H1-VH);
(h) antibodies that comprise a light chain variable region having an amino acid sequence in which one or more amino acids are substituted, deleted, added, and/or inserted in the amino acid sequence of SEQ ID NO: 52 (L0-VL);
(i) antibodies that comprise a heavy chain variable region having an amino acid sequence in which one or more amino acids are substituted, deleted, added, and/or inserted in the amino acid sequence of SEQ ID NO: 50 (H0-VH), and a light chain variable region having an amino acid sequence in which one or more amino acids are substituted, deleted, added, and/or inserted in the amino acid sequence of SEQ ID NO: 52 (L0-VL);
(j) antibodies that comprise a heavy chain variable region having an amino acid sequence in which one or more amino acids are substituted, deleted, added, and/or inserted in the amino acid sequence of SEQ ID NO: 112 (H1-VH), and a light chain variable region having an amino acid sequence in which one or more amino acids are substituted, deleted, added, and/or inserted in the amino acid sequence of SEQ ID NO: 52 (L0-VL);

Without particular limitation, the antibodies of any one of (f) to (j) preferably have an activity similar to that of the antibodies of any one of (a) to (e).

The substitution, deletion, addition, and/or insertion of amino acids are not particularly limited, but specific examples include, for example, the above-described amino acid substitutions.

More specifically, for example, the following amino acid substitutions may be included:

Substitution of Ile at position 3 of CDR1 (SEQ ID NO: 9) in the heavy chain variable region of SEQ ID NO: 50 or 112 with Val (SEQ ID NO: 173). Thus, the present invention provides heavy chain variable regions in which CDR1 having the amino acid sequence of SEQ ID NO: 9 is substituted with CDR1 having the amino acid sequence of SEQ ID NO: 173 in a heavy chain variable region having the amino acid sequence of SEQ ID NO: 50 or 112.

Substitution of Met at position 4 of CDR1 (SEQ ID NO: 9) in the heavy chain variable region of SEQ ID NO: 50 or 112 with Ile (SEQ ID NO: 174). Thus, the present invention provides heavy chain variable regions in which CDR1 having the amino acid sequence of SEQ ID NO: 9 is substituted with CDR1 having the amino acid sequence of SEQ ID NO: 174 in a heavy chain variable region having the amino acid sequence of SEQ ID NO: 50 or 112.

Substitution of Met at position 4 of CDR1 (SEQ ID NO: 9) in the heavy chain variable region of SEQ ID NO: 50 or 112 with Leu (SEQ ID NO: 175). Thus, the present invention provides heavy chain variable regions in which CDR1 having the amino acid sequence of SEQ ID NO: 9 is substituted with CDR1 having the amino acid sequence of SEQ ID NO: 175 in a heavy chain variable region having the amino acid sequence of SEQ ID NO: 50 or 112.

Substitution of Ile at position 3 of CDR1 (SEQ ID NO: 9) in the heavy chain variable region of SEQ ID NO: 50 or 112 with Ala (SEQ ID NO: 176). Thus, the present invention provides heavy chain variable regions in which CDR1 having the amino acid sequence of SEQ ID NO: 9 is substituted with CDR1 having the amino acid sequence of SEQ ID NO: 176 in a heavy chain variable region having the amino acid sequence of SEQ ID NO: 50 or 112.

Substitution of Leu at position 1 of CDR2 (SEQ ID NO: 10) in the heavy chain variable region of SEQ ID NO: 50 or 112 with Glu (SEQ ID NO: 113). Thus, the present invention provides heavy chain variable regions in which CDR2 having the amino acid sequence of SEQ ID NO: 10 is substituted with CDR2 having the amino acid sequence of SEQ ID NO: 113 in a heavy chain variable region having the amino acid sequence of SEQ ID NO: 50 or 112.

Substitution of Asn at position 3 of CDR2 (SEQ ID NO: 10) in the heavy chain variable region of SEQ ID NO: 50 or 112 with Asp (SEQ ID NO: 114). Thus, the present invention provides heavy chain variable regions in which CDR2 having the amino acid sequence of SEQ ID NO: 10 is substituted with CDR2 having the amino acid sequence of SEQ ID NO: 114 in a heavy chain variable region having the amino acid sequence of SEQ ID NO: 50 or 112.

Substitution of Gln at position 13 of CDR2 (SEQ ID NO: 10) in the heavy chain variable region of SEQ ID NO: 50 or 112 with Asp (SEQ ID NO: 115). Thus, the present invention provides heavy chain variable regions in which CDR2 having the amino acid sequence of SEQ ID NO: 10 is substituted with CDR2 having the amino acid sequence of SEQ ID NO: 115 in a heavy chain variable region having the amino acid sequence of SEQ ID NO: 50 or 112.

Substitution of Lys at position 14 of CDR2 (SEQ ID NO: 10) in the heavy chain variable region of SEQ ID NO: 50 or 112 with Gln (SEQ ID NO: 116). Thus, the present invention provides heavy chain variable regions in which CDR2 having the amino acid sequence of SEQ ID NO: 10 is substituted with CDR2 having the amino acid sequence of SEQ ID NO: 116 in a heavy chain variable region having the amino acid sequence of SEQ ID NO: 50 or 112.

Substitution of Lys at position 16 of CDR2 (SEQ ID NO: 10) in the heavy chain variable region of SEQ ID NO: 50 or 112 with Gln (SEQ ID NO: 117). Thus, the present invention provides heavy chain variable regions in which CDR2 having the amino acid sequence of SEQ ID NO: 10 is substituted with CDR2 having the amino acid sequence of SEQ ID NO: 117 in a heavy chain variable region having the amino acid sequence of SEQ ID NO: 50 or 112.

Substitution of Gly at position 17 of CDR2 (SEQ ID NO: 10) in the heavy chain variable region of SEQ ID NO: 50 or 112 with Asp (SEQ ID NO: 118). Thus, the present invention provides heavy chain variable regions in which CDR2 having the amino acid sequence of SEQ ID NO: 10 is substituted with CDR2 having the amino acid sequence of SEQ ID NO: 118 in a heavy chain variable region having the amino acid sequence of SEQ ID NO: 50 or 112.

Substitution of Lys at position 16 and Gly at position 17 of CDR2 (SEQ ID NO: 10) in the heavy chain variable region of SEQ ID NO: 50 or 112 with Gln and Asp, respectively (SEQ ID NO: 119). Thus, the present invention provides heavy chain variable regions in which CDR2 having the amino acid sequence of SEQ ID NO: 10 is substituted with CDR2 having the amino acid sequence of SEQ ID NO: 119 in a heavy chain variable region having the amino acid sequence of SEQ ID NO: 50 or 112.

Substitution of Lys at position 14, Lys at position 16, and Gly at position 17 of CDR2 (SEQ ID NO: 10) in the heavy chain variable region of SEQ ID NO: 50 or 112 with Gln, Gln, and Asp, respectively (SEQ ID NO: 167). Thus, the present invention provides heavy chain variable regions in which CDR2 having the amino acid sequence of SEQ ID NO: 10 is substituted with CDR2 having the amino acid sequence of SEQ ID NO: 171 in a heavy chain variable region having the amino acid sequence of SEQ ID NO: 50 or 112.

Substitution of Gln at position 13, Lys at position 14, Lys at position 16, and Gly at position 17 of CDR2 (SEQ ID NO: 10) in the heavy chain variable region of SEQ ID NO: 50 or 112 with Asp, Gln, Gln, and Asp, respectively (SEQ ID NO: 172). Thus, the present invention provides heavy chain variable regions in which CDR2 having the amino acid sequence of SEQ ID NO: 10 is substituted with CDR2 having the amino acid sequence of SEQ ID NO: 172 in a heavy chain variable region having the amino acid sequence of SEQ ID NO: 50 or 112.

Substitution of Ser at position 10 of CDR2 (SEQ ID NO: 10) in the heavy chain variable region of SEQ ID NO: 50 or 112 with Asp (SEQ ID NO: 177). Thus, the present invention provides heavy chain variable regions in which CDR2 having the amino acid sequence of SEQ ID NO: 10 is substituted with CDR2 having the amino acid sequence of SEQ ID NO: 177 in a heavy chain variable region having the amino acid sequence of SEQ ID NO: 50 or 112.

Substitution of Gln at position 13 of CDR2 (SEQ ID NO: 10) in the heavy chain variable region of SEQ ID NO: 50 or 112 with Pro (SEQ ID NO: 178). Thus, the present invention provides heavy chain variable regions in which CDR2 having the amino acid sequence of SEQ ID NO: 10 is substituted with CDR2 having the amino acid sequence of SEQ ID NO: 178 in a heavy chain variable region having the amino acid sequence of SEQ ID NO: 50 or 112.

Substitution of Tyr at position 3 of CDR3 (SEQ ID NO: 11) in the heavy chain variable region of SEQ ID NO: 50 or 112 with Leu (SEQ ID NO: 179). Thus, the present invention provides heavy chain variable regions in which CDR3 having the amino acid sequence of SEQ ID NO: 11 is substituted with CDR3 having the amino acid sequence of SEQ ID NO: 179 in a heavy chain variable region having the amino acid sequence of SEQ ID NO: 50 or 112.

Substitution of Met at position 10 of CDR3 (SEQ ID NO: 11) in the heavy chain variable region of SEQ ID NO: 50 or 112 with Leu (SEQ ID NO: 180). Thus, the present invention provides heavy chain variable regions in which CDR3 having the amino acid sequence of SEQ ID NO: 11 is substituted with CDR3 having the amino acid sequence of SEQ ID NO: 180 in a heavy chain variable region having the amino acid sequence of SEQ ID NO: 50 or 112.

Substitution of Asp at position 11 of CDR3 (SEQ ID NO: 11) in the heavy chain variable region of SEQ ID NO: 50 or 112 with Glu (SEQ ID NO: 181). Thus, the present invention provides heavy chain variable regions in which CDR3 having the amino acid sequence of SEQ ID NO: 11 is substituted with CDR3 having the amino acid sequence of SEQ ID NO: 181 in a heavy chain variable region having the amino acid sequence of SEQ ID NO: 50 or 112.

Substitution of Tyr at position 12 of CDR3 (SEQ ID NO: 11) in the heavy chain variable region of SEQ ID NO: 50 or 112 with Thr (SEQ ID NO: 182). Thus, the present invention provides heavy chain variable regions in which CDR3 having the amino acid sequence of SEQ ID NO: 11 is substituted with CDR3 having the amino acid sequence of SEQ ID NO: 182 in a heavy chain variable region having the amino acid sequence of SEQ ID NO: 50 or 112.

Substitution of Tyr at position 12 of CDR3 (SEQ ID NO: 11) in the heavy chain variable region of SEQ ID NO: 50 or 112 with Ser (SEQ ID NO: 183). Thus, the present invention provides heavy chain variable regions in which CDR3 having the amino acid sequence of SEQ ID NO: 11 is substituted with CDR3 having the amino acid sequence of SEQ ID NO: 183 in a heavy chain variable region having the amino acid sequence of SEQ ID NO: 50 or 112.

Substitution of Met at position 10, Asp at position 11, and Tyr at position 12 of CDR3 (SEQ ID NO: 11) in the heavy chain variable region of SEQ ID NO: 50 or 112 with Leu, Glu, Thr, respectively (SEQ ID NO: 184). Thus, the present invention provides heavy chain variable regions in which CDR3 having the amino acid sequence of SEQ ID NO: 11 is substituted with CDR3 having the amino acid sequence of SEQ ID NO: 184 in a heavy chain variable region having the amino acid sequence of SEQ ID NO: 50 or 112.

Substitution of Asp at position 11 and Tyr at position 12 of CDR3 (SEQ ID NO: 11) in the heavy chain variable region of SEQ ID NO: 50 or 112 with Glu and Thr, respectively (SEQ ID NO: 185). Thus, the present invention provides heavy chain variable regions in which CDR3 having the amino acid sequence of SEQ ID NO: 11 is substituted with CDR3 having the amino acid sequence of SEQ ID NO: 185 in a heavy chain variable region having the amino acid sequence of SEQ ID NO: 50 or 112.

Substitution of Tyr at position 3, Asp at position 11, and Tyr at position 12 of CDR3 (SEQ ID NO: 11) in the heavy chain variable region of SEQ ID NO: 50 or 112 with Leu, Glu, and Thr, respectively (SEQ ID NO: 186). Thus, the present invention provides heavy chain variable regions in which CDR3 having the amino acid sequence of SEQ ID NO: 11 is substituted with CDR3 having the amino acid sequence of SEQ ID NO: 186 in a heavy chain variable region having the amino acid sequence of SEQ ID NO: 50 or 112.

Substitution of Tyr at position 3, Asp at position 11, and Tyr at position 12 of CDR3 (SEQ ID NO: 11) in the heavy chain variable region of SEQ ID NO: 50 or 112 with Leu, Glu, and Ser, respectively (SEQ ID NO: 187). Thus, the present invention provides heavy chain variable regions in which CDR3 having the amino acid sequence of SEQ ID NO: 11 is substituted with CDR3 having the amino acid sequence of SEQ ID NO: 187 in a heavy chain variable region having the amino acid sequence of SEQ ID NO: 50 or 112.

Substitution of Arg at position 1 of CDR1 (SEQ ID NO: 13) in the light chain variable region of SEQ ID NO: 52 with Gln (SEQ ID NO: 121). Thus, the present invention provides light chain variable regions in which CDR1 having the amino acid sequence of SEQ ID NO: 13 is substituted with CDR1 having the amino acid sequence of SEQ ID NO: 121 in a light chain variable region having the amino acid sequence of SEQ ID NO: 52.

Substitution of Asn at position 5 of CDR1 (SEQ ID NO: 13) in the light chain variable region of SEQ ID NO: 52 with Asp (SEQ ID NO: 122). Thus, the present invention provides light chain variable regions in which CDR1 having the amino acid sequence of SEQ ID NO: 13 is substituted with CDR1 having the amino acid sequence of SEQ ID NO: 122 in a light chain variable region having the amino acid sequence of SEQ ID NO: 52.

Substitution of Ser at position 8 of CDR1 (SEQ ID NO: 13) in the light chain variable region of SEQ ID NO: 52 with Arg (SEQ ID NO: 188). Thus, the present invention provides light chain variable regions in which CDR1 having the amino acid sequence of SEQ ID NO: 13 is substituted with CDR1 having the amino acid sequence of SEQ ID NO: 188 in a light chain variable region having the amino acid sequence of SEQ ID NO: 52.

Substitution of Leu at position 10 of CDR1 (SEQ ID NO: 13) of the light chain variable region of SEQ ID NO: 52 with Val (SEQ ID NO: 189). Thus, the present invention provides light chain variable regions in which CDR1 having the amino acid sequence of SEQ ID NO: 13 is substituted with CDR1 having the amino acid sequence of SEQ ID NO: 189 in a light chain variable region having the amino acid sequence of SEQ ID NO: 52.

Substitution of Ser at position 8 and Leu at position 10 of CDR1 (SEQ ID NO: 13) of the light chain variable region of SEQ ID NO: 52 with Arg and Val, respectively (SEQ ID NO: 190). Thus, the present invention provides light chain variable regions in which CDR1 having the amino acid sequence of SEQ ID NO: 13 is substituted with CDR1 having the amino acid sequence of SEQ ID NO: 190 in a light chain variable region having the amino acid sequence of SEQ ID NO: 52.

Substitution of Thr at position 2 of CDR1 (SEQ ID NO: 13) in the light chain variable region of SEQ ID NO: 52 with Ala (SEQ ID NO: 191). Thus, the present invention provides light chain variable regions in which CDR1 having the amino acid sequence of SEQ ID NO: 13 is substituted with CDR1 having the amino acid sequence of SEQ ID NO: 191 in a light chain variable region having the amino acid sequence of SEQ ID NO: 52.

Substitution of Thr at position 2 of CDR1 (SEQ ID NO: 13) in the light chain variable region of SEQ ID NO: 52 with Ser (SEQ ID NO: 192). Thus, the present invention provides light chain variable regions in which CDR1 having the amino acid sequence of SEQ ID NO: 13 is substituted with CDR1 having the amino acid sequence of SEQ ID NO: 192 in a light chain variable region having the amino acid sequence of SEQ ID NO: 52.

Substitution of Asn at position 1 of CDR2 (SEQ ID NO: 14) in the light chain variable region of SEQ ID NO: 52 with Asp (SEQ ID NO: 123). Thus, the present invention provides light chain variable regions in which CDR2 having the amino acid sequence of SEQ ID NO: 14 is substituted with CDR2 having the amino acid sequence of SEQ ID NO: 123 in a light chain variable region having the amino acid sequence of SEQ ID NO: 52.

Substitution of Lys at position 3 of CDR2 (SEQ ID NO: 14) in the light chain variable region of SEQ ID NO: 52 with Gln (SEQ ID NO: 124). Thus, the present invention provides light chain variable regions in which CDR2 having the amino acid sequence of SEQ ID NO: 14 is substituted with CDR2 having the amino acid sequence of SEQ ID NO: 124 in a light chain variable region having the amino acid sequence of SEQ ID NO: 52.

Substitution of Leu at position 5 of CDR2 (SEQ ID NO: 14) in the light chain variable region of SEQ ID NO: 52 with Glu (SEQ ID NO: 125). Thus, the present invention provides light chain variable regions in which CDR2 having the amino acid sequence of SEQ ID NO: 14 is substituted with CDR2 having the amino acid sequence of SEQ ID NO: 125 in a light chain variable region having the amino acid sequence of SEQ ID NO: 52.

Substitution of Lys at position 7 of CDR2 (SEQ ID NO: 14) in the light chain variable region of SEQ ID NO: 52 with Gln (SEQ ID NO: 126). Thus, the present invention provides light chain variable regions in which CDR2 having the amino acid sequence of SEQ ID NO: 14 is substituted with CDR2 having the amino acid sequence of SEQ ID NO: 126 in a light chain variable region having the amino acid sequence of SEQ ID NO: 52.

Substitution of Lys at position 7 of CDR2 (SEQ ID NO: 14) in the light chain variable region of SEQ ID NO: 52 with Asp (SEQ ID NO: 127). Thus, the present invention provides light chain variable regions in which CDR2 having the amino acid sequence of SEQ ID NO: 14 is substituted with CDR2 having the amino acid sequence of SEQ ID NO: 127 in a light chain variable region having the amino acid sequence of SEQ ID NO: 52.

Substitution of Arg at position 1 and Asn at position 5 of CDR1 (SEQ ID NO: 13) in the light chain variable region of SEQ ID NO: 52 with Gln and Asp, respectively (SEQ ID NO: 169). Thus, the present invention provides light chain variable regions in which CDR1 having the amino acid sequence of SEQ ID NO: 13 is substituted with CDR1 having the amino acid sequence of SEQ ID NO: 169 in a light chain variable region having the amino acid sequence of SEQ ID NO: 52.

Substitution of Lys at position 3, Leu at position 5, and Lys at position 7 of CDR2 (SEQ ID NO: 14) in the light chain variable region of SEQ ID NO: 52 with Gln, Glu, and Gln, respectively (SEQ ID NO: 170). Thus, the present invention provides light chain variable regions in which CDR2 having the amino acid sequence of SEQ ID NO: 14 is substituted with CDR2 having the amino acid sequence of SEQ ID NO: 170 in a light chain variable region having the amino acid sequence of SEQ ID NO: 52.

Substitution of Glu at position 5 of CDR3 (SEQ ID NO: 15) in the light chain variable region of SEQ ID NO: 52 with Asp (SEQ ID NO: 193). Thus, the present invention provides light chain variable regions in which CDR3 having the amino acid sequence of SEQ ID NO: 15 is substituted with CDR3 having the amino acid sequence of SEQ ID NO: 193 in a light chain variable region having the amino acid sequence of SEQ ID NO: 52.

Substitution of Ser at position 6 of CDR3 (SEQ ID NO: 15) in the light chain variable region of SEQ ID NO: 52 with Asp (SEQ ID NO: 194). Thus, the present invention provides light chain variable regions in which CDR3 having the amino acid sequence of SEQ ID NO: 15 is substituted with CDR3 having the amino acid sequence of SEQ ID NO: 194 in a light chain variable region having the amino acid sequence of SEQ ID NO: 52.

Substitution of Thr at position 9 of CDR3 (SEQ ID NO: 15) in the light chain variable region of SEQ ID NO: 52 with Phe (SEQ ID NO: 195). Thus, the present invention provides light chain variable regions in which CDR3 having the amino acid sequence of SEQ ID NO: 15 is substituted with CDR3 having the amino acid sequence of SEQ ID NO: 195 in a light chain variable region having the amino acid sequence of SEQ ID NO: 52.

In addition, the substitutions other than those described above include a substitution of Arg at position 3 of heavy chain FR2 having the amino acid sequence of SEQ ID NO: 97 with another amino acid. The amino acid after substitution is not particularly limited; but preferred examples thereof include Gln. When Arg at position 3 in SEQ ID NO: 97 has been replaced with Gln, Ala at position 5 may be substituted with Ser to produce a human FR2 sequence. The amino acid sequence in which Arg and Ala at positions 3 and 5 in the amino acid sequence of SEQ ID NO: 97 have been replaced with Gln and Ser, respectively, is shown in SEQ ID NO: 120. Thus, the present invention provides heavy chain variable regions in which FR2 having the amino acid sequence of SEQ ID NO: 97 is substituted with FR2 having the amino acid sequence of SEQ ID NO: 120 in a heavy chain variable region having the amino acid sequence of SEQ ID NO: 50 or 112.

Each of the above-described amino acid substitutions may be used alone or in combination with other amino acid substitutions described above. They also may be combined with amino acid substitutions other than those described above.

Specific examples of the antibodies in which the above-described substitutions have been carried out include, for example, antibodies that comprise a heavy chain variable region having the amino acid sequence of SEQ ID NO: 167, antibodies that comprise a light chain variable region having the amino acid sequence of SEQ ID NO: 168, and antibodies that comprise a heavy chain variable region having the amino acid sequence of SEQ ID NO: 167 and a light chain variable region having the amino acid sequence of SEQ ID NO: 168.

Furthermore, specific examples of the heavy chain variable regions in which the above-described substitutions have been carried out include, for example, the following heavy chain variable regions:

(1) heavy chain variable regions having the amino acid sequence of SEQ ID NO: 204 (H17);
(2) heavy chain variable regions having the amino acid sequence of SEQ ID NO: 205 (H19);
(3) heavy chain variable regions having the amino acid sequence of SEQ ID NO: 206 (H28);
(4) heavy chain variable regions having the amino acid sequence of SEQ ID NO: 207 (H30);
(5) heavy chain variable regions having the amino acid sequence of SEQ ID NO: 208 (H34);
(6) heavy chain variable regions having the amino acid sequence of SEQ ID NO: 209 (H42);
(7) heavy chain variable regions having the amino acid sequence of SEQ ID NO: 210 (H44);
(8) heavy chain variable regions having the amino acid sequence of SEQ ID NO: 211 (H46);
(9) heavy chain variable regions having the amino acid sequence of SEQ ID NO: 212 (H57);
(10) heavy chain variable regions having the amino acid sequence of SEQ ID NO: 213 (H71);
(11) heavy chain variable regions having the amino acid sequence of SEQ ID NO: 214 (H78);
(12) heavy chain variable regions having the amino acid sequence of SEQ ID NO: 215 (H92);
(13) heavy chain variable regions having the amino acid sequence of SEQ ID NO: 216 (H97); and
(14) heavy chain variable regions having the amino acid sequence of SEQ ID NO: 217 (H98).

Meanwhile, specific examples of the light chain variable regions in which the above-described substitutions carried out include, for example, the following light chain variable regions:

(15) light chain variable regions having the amino acid sequence of SEQ ID NO: 218 (L11);
(16) light chain variable regions having the amino acid sequence of SEQ ID NO: 219 (L12);
(17) light chain variable regions having the amino acid sequence of SEQ ID NO: 220 (L17); and
(18) light chain variable regions having the amino acid sequence of SEQ ID NO: 221 (L50).

Furthermore, specific examples of the antibodies comprising the above-described heavy chain and light chain variable regions include, for example, the following antibodies:

(19) antibodies that comprise the heavy chain variable region of (3) and the light chain variable region of (17) (H28L17);
(20) antibodies that comprise the heavy chain variable region of (4) and the light chain variable region of (17) (H30L17);
(21) antibodies that comprise the heavy chain variable region of (5) and the light chain variable region of (17) (H34L17);
(22) antibodies that comprise the heavy chain variable region of (6) and the light chain variable region of (17) (H42L17);
(23) antibodies that comprise the heavy chain variable region of (7) and the light chain variable region of (17) (H44L17);
(24) antibodies that comprise the heavy chain variable region of (8) and the light chain variable region of (17) (H46L17);
(25) antibodies that comprise the heavy chain variable region of (9) and the light chain variable region of (17) (H57L17);
(26) antibodies that comprise the heavy chain variable region of (10) and the light chain variable region of (17) (H71L17);
(27) antibodies that comprise the heavy chain variable region of (11) and the light chain variable region of (17) (H78L17);
(28) antibodies that comprise the heavy chain variable region of (12) and the light chain variable region of (17) (H92L17);
(29) antibodies that comprise the heavy chain variable region of (13) and the light chain variable region of (18) (H97L50); and
(30) antibodies that comprise the heavy chain variable region of (14) and the light chain variable region of (18) (H98L50).

The constant region used for the humanized antibodies of the present invention may be any constant region derived from a human antibody. Preferred examples of such constant regions derived from human antibodies include, for example, constant regions derived from IgG1 or IgG2. Moreover, constant regions in which one or more amino acids are substituted, deleted, added, and/or inserted in the constant region derived from a human antibody may also be used.

The constant regions in which one or more amino acids are substituted, deleted, added, and/or inserted in the constant region derived from a human antibody are not particularly limited, and include, for example, the following constant regions:

constant regions having the amino acid sequence of SEQ ID NO: 128 (M58);
constant regions having the amino acid sequence of SEQ ID NO: 129 (M14); and
constant regions having the amino acid sequence of SEQ ID NO: 62 (SKSC).

Specific examples of the heavy chains or antibodies having the above-described constant regions include, for example:

(1) heavy chains that comprise a variable region having the amino acid sequence of SEQ ID NO: 167 and a constant region having the amino acid sequence of SEQ ID NO: 128;
(2) heavy chains in which CDR2 having the amino acid sequence of SEQ ID NO: 171 in the heavy chains of (1) is substituted with CDR2 having the amino acid sequence of SEQ ID NO: 172;
(3) antibodies that comprise the heavy chain of (1) and a light chain having the amino acid sequence of SEQ ID NO: 152; and
(4) antibodies that comprise the heavy chain of (2) and a light chain having the amino acid sequence of SEQ ID NO: 152.

More specific examples of the humanized anti-NR10 antibodies of the present invention include, for example, the following antibodies:

(k) antibodies that comprise a heavy chain having the amino acid sequence of SEQ ID NO: 54 (H0-VH + constant region);
(l) antibodies that comprise a heavy chain having the amino acid sequence of SEQ ID NO: 130 (H1-VH + constant region);
(m) antibodies that comprise a light chain having the amino acid sequence of SEQ ID NO: 56 (L0-VL + constant region);
(n) antibodies that comprise a heavy chain having the amino acid sequence of SEQ ID NO: 54 (H0-VH + constant region) and a light chain having the amino acid sequence of SEQ ID NO: 56 (L0-VL + constant region); and
(o) antibodies that comprise a heavy chain having the amino acid sequence of SEQ ID NO: 130 (H1-VH + constant region) and a light chain having the amino acid sequence of SEQ ID NO: 56 (L0-VL + constant region).

The heavy chain having the amino acid sequence of SEQ ID NO: 54 (H0-VH + constant region) and the light chain having the amino acid sequence of SEQ ID NO: 56 (L0-VL + constant region) may have a substitution, deletion, addition, and/or insertion of one or more amino acids. The substitution, deletion, addition, and/or insertion of amino acids may be carried out in either or both of the variable and constant regions.
Thus, the present invention provides:

(p) antibodies that comprise a heavy chain having an amino acid sequence in which one or more amino acids are substituted, deleted, added, and/or inserted in the amino acid sequence of SEQ ID NO: 54 (H0-VH + constant region);
(q) antibodies that comprise a heavy chain having an amino acid sequence in which one or more amino acids are substituted, deleted, added, and/or inserted in the amino acid sequence of SEQ ID NO: 130 (H1-VH + constant region);
(r) antibodies that comprise a light chain having an amino acid sequence in which one or more amino acids are substituted, deleted, added, and/or inserted in the amino acid sequence of SEQ ID NO: 56 (L0-VL + constant region);
(s) antibodies that comprise a heavy chain having an amino acid sequence in which one or more amino acids are substituted, deleted, added, and/or inserted in the amino acid sequence of SEQ ID NO: 54 (H0-VH + constant region) and a light chain having an amino acid sequence in which one or more amino acids are substituted, deleted, added, and/or inserted in the amino acid sequence of SEQ ID NO: 56 (L0-VL + constant region); and
(t) antibodies that comprise a heavy chain having an amino acid sequence in which one or more amino acids are substituted, deleted, added, and/or inserted in the amino acid sequence of SEQ ID NO: 130 (H1-VH + constant region) and a light chain having an amino acid sequence in which one or more amino acids are substituted, deleted, added, and/or inserted in the amino acid sequence of SEQ ID NO: 56 (L0-VL + constant region).

Without particular limitation, the antibodies of any one of (p) to (t) preferably have an activity similar to that of the antibodies of any one of (k) to (o).
The substitution, deletion, addition, and/or insertion of amino acids are not particularly limited, but specific examples thereof include, for example, the above-described amino acid substitutions.
Furthermore, the nucleotide sequence encoding the amino acid sequence of the above-described humanized heavy chain variable region (SEQ ID NO: 50) is shown in SEQ ID NO: 49. The nucleotide sequence encoding the amino acid sequence of the humanized light chain variable region (SEQ ID NO: 52) is shown in SEQ ID NO: 51. The nucleotide sequence encoding the amino acid sequence of the humanized heavy chain (SEQ ID NO: 54) is shown in SEQ ID NO: 53. The nucleotide sequence encoding the amino acid sequence of the humanized light chain (SEQ ID NO: 56) is shown in SEQ ID NO: 55.

Moreover, the present invention provides antibodies that recognize the same epitope as recognized by the antibodies of any one of (a) to (t) above. The binding to the same epitope is as already described above.

Furthermore, the present invention provides the following antibodies:

(u) antibodies that comprise a heavy chain having the amino acid sequence of SEQ ID NO: 151;
(v) antibodies that comprise a light chain comprising the amino acid sequence of SEQ ID NO: 152; and
(w) antibodies that comprise the heavy chain of (u) and the light chain of (v).

Moreover, the present invention provides the following heavy and light chains and antibodies:

(1) heavy chains having the amino acid sequence of SEQ ID NO: 222 (H17);
(2) heavy chains having the amino acid sequence of SEQ ID NO: 223 (H19);
(3) heavy chains having the amino acid sequence of SEQ ID NO: 224 (H28);
(4) heavy chains having the amino acid sequence of SEQ ID NO: 225 (H30);
(5) heavy chains having the amino acid sequence of SEQ ID NO: 226 (H34);
(6) heavy chains having the amino acid sequence of SEQ ID NO: 227 (H42);
(7) heavy chains having the amino acid sequence of SEQ ID NO: 228 (H44);
(8) heavy chains having the amino acid sequence of SEQ ID NO: 229 (H46);
(9) heavy chains having the amino acid sequence of SEQ ID NO: 230 (H57);
(10) heavy chains having the amino acid sequence of SEQ ID NO: 231 (H71);
(11) heavy chains having the amino acid sequence of SEQ ID NO: 232 (H78);
(12) heavy chains having the amino acid sequence of SEQ ID NO: 233 (H92);
(13) heavy chains having the amino acid sequence of SEQ ID NO: 234 (H97);
(14) heavy chains having the amino acid sequence of SEQ ID NO: 235 (H98);
(15) light chains having the amino acid sequence of SEQ ID NO: 236 (L11)
(16) light chains having the amino acid sequence of SEQ ID NO: 237 (L12);
(17) light chains having the amino acid sequence of SEQ ID NO: 238 (L17);
(18) light chains having the amino acid sequence of SEQ ID NO: 239 (L50);
(19) antibodies that comprise the heavy chain of (3) and the light chain of (17) (H28L17);
(20) antibodies that comprise the heavy chain of (4) and the light chain of (17) (H30L17);
(21) antibodies that comprise the heavy chain of (5) and the light chain of (17) (H34L17);
(22) antibodies that comprise the heavy chain of (6) and the light chain of (17) (H42L17);
(23) antibodies that comprise the heavy chain of (7) and the light chain of (17) (H44L17);
(24) antibodies that comprise the heavy chain of (8) and the light chain of (17) (H46L17);
(25) antibodies that comprise the heavy chain of (9) and the light chain of (17) (H57L17);
(26) antibodies that comprise the heavy chain of (10) and the light chain of (17) (H71L17);
(27) antibodies that comprise the heavy chain of (11) and the light chain of (17) (H78L17);
(28) antibodies that comprise the heavy chain of (12) and the light chain of (17) (H92L17);
(29) antibodies that comprise the heavy chain of (13) and the light chain of (18) (H97L50);
(30) antibodies that comprise the heavy chain of (14) and the light chain of (18) (H98L50);
(31) heavy chains having an amino acid sequence in which one or more amino acids are substituted, deleted, added and/or inserted in the heavy chains of any one of (1) to (14);
(32) light chains having an amino acid sequence in which one or more amino acids are substituted, deleted, added and/or inserted in the light chains of any one of (15) to (18);
(33) antibodies having an amino acid sequence in which one or more amino acids are substituted, deleted, added and/or inserted in the antibodies of any one of (19) to (30); and
(34) antibodies that recognize the same epitope as recognized by the antibodies of any one of (19) to (33).

The substitution, deletion, addition, and/or insertion of amino acids are as described above. Antibodies that recognize the same epitope as recognized by an antibody are also described above.

The present invention also provides genes encoding the variable regions, heavy chains, light chains, or antibodies of the present invention.

The present invention also provides vectors carrying the above-described genes.

The present invention also provides host cells transformed with the above-described vectors.

The present invention also relates to methods for producing variable regions, heavy chains, light chains, or antibodies of the present invention, which comprise the step of culturing the above-described host cells.
The vectors, host cells, and culture of host cells are described herein below.

Antibodies that recognize domains

Preferred embodiments of the anti-NR10 antibody of the present invention include antibodies that recognize domain 1 or domain 2. In the present invention, domain 1 refers to the region of amino acids at positions 21 to 120 (LPAKP to LENIA) in the amino acid sequence of human NR10 of SEQ ID NO: 76, where the amino acid numbering is based on the sequence including the signal peptide. In addition, in the present invention, domain 2 refers to the region of amino acids at positions 121 to 227 (KTEPP to EEEAP) in the amino acid sequence of human NR10 of SEQ ID NO: 76, where the amino acid numbering is based on the sequence including the signal peptide.
Such antibodies are not particularly limited; however, in general, they have a neutralizing activity, and preferably are humanized antibodies.
Examples of the preferred antibodies in the present invention include antibodies that recognize domain 1. The antibodies that recognize domain 1 have a strong neutralizing activity, and thus are particularly useful as pharmaceuticals.

Antibodies (neutralizing activity)

The present invention also provides anti-NR10 antibodies having a neutralizing activity.
In the present invention, the neutralizing activity against NR10 refers to an activity of inhibiting the binding between NR10 and its ligand IL-31, and preferably an activity of suppressing a biological activity based on NR10.
Antibodies having a NR10-neutralizing activity can be selected, for example, by adding candidate antibodies to an IL-31-dependent cell line and observing their growth-suppressing effect on the cell line. In this method, antibodies that suppress the growth of the IL-31-dependent cell line are determined as antibodies having a neutralizing activity against NR10.

Antibodies (general)

The antibodies of the present invention are not limited in terms of their origin, and may be derived from any animals such as humans, mice, and rats. Moreover, the antibodies may be recombinant antibodies such as chimeric antibodies and humanized antibodies. As described above, the preferred antibodies of the present invention include humanized antibodies.
The chimeric antibodies contain, for example, the heavy and light chain constant regions of a human antibody, and the heavy and light chain variable regions of an antibody of a non-human mammal, such as mouse. The chimeric antibodies can be produced by known methods. For example, the antibodies can be produced by cloning an antibody gene from hybridomas, inserting it into an appropriate vector, and introducing the construct into hosts (see, for example, Carl, A. K. Borrebaeck, James, W. Larrick, THERAPEUTIC MONOCLONAL ANTIBODIES, Published in the United Kingdom by MACMILLAN PUBLISHERS LTD, 1990). Specifically, cDNAs of the antibody variable regions (V regions) are synthesized from mRNA of hybridomas using reverse transcriptase. Once DNAs encoding the V regions of an antibody of interest are obtained, these are linked with DNAs encoding the constant regions (C regions) of a desired human antibody. The resulting constructs are inserted into expression vectors. Alternatively, the DNAs encoding the antibody V regions may be inserted into expression vectors comprising DNAs encoding the C regions of a human antibody. The DNAs are inserted into expression vectors so that they are expressed under the regulation of the expression regulatory regions, for example, enhancers and promoters. In the next step, host cells can be transformed with the expression vectors to allow expression of chimeric antibodies.
Methods for obtaining human antibodies are also known. For example, desired human antibodies with antigen-binding activity can be obtained by (1) sensitizing human lymphocytes with antigens of interest or cells expressing antigens of interest *in vitro;* and (2) fusing the sensitized lymphocytes with human myeloma cells such as U266 (see Japanese Patent Application Kokoku Publication No. (JP-B) H01-59878 (examined, approved Japanese patent application published for opposition)). Alternatively, the desired human antibody can also be obtained by immunizing a transgenic animal having an entire repertoire of human antibody genes with a desired antigen (see International Patent Application Publication Nos. WO 93/12227, WO 92/03918, WO 94/02602, WO 94/25585, WO 96/34096, and WO 96/33735).
Furthermore, techniques to obtain human antibodies by panning with a human antibody phage library are known. For example, the variable region of a human antibody is expressed as a single chain antibody (scFv) on the surface of a phage, using a phage display method, and phages that bind to the antigen can be selected. By analyzing the genes of selected phages, the DNA sequences encoding the variable regions of human antibodies that bind to the antigen can be determined. If the DNA sequences of scFvs that bind to the antigen are identified, appropriate expression vectors comprising these sequences can be constructed to obtain human antibodies. Such methods are well known. Reference can be made to WO 92/01047, WO 92/20791, WO 93/06213, WO 93/11236, WO 93/19172, WO 95/01438, WO 95/15388, and such.

The antibodies of the present invention include not only divalent antibodies as represented by IgG, but also monovalent antibodies, multivalent antibodies as represented by IgM, and bispecific antibodies capable of binding to different antigens, as long as they have a NR10-binding activity and/or neutralizing activity. The multivalent antibodies of the present invention include multivalent antibodies in which the antigen-binding sites are all identical, and multivalent antibodies in which all or some of the antigen-binding sites are different. The antibodies of the present invention are not limited to full-length antibody molecules, but may also be low-molecular-weight antibodies or modified products thereof, as long as they bind to NR10 protein.

Alternatively, the antibodies of the present invention may be low-molecular-weight antibodies. Such low-molecular-weight antibodies are antibodies including antibody fragments lacking some portions of a whole antibody (for example, whole IgG), and are not particularly limited as long as they retain NR10-binding and/or neutralizing activity. In the present invention, the low-molecular-weight antibodies are not particularly limited, as long as they contain a portion of whole antibodies. The low-molecular-weight antibodies preferably contain a heavy chain variable region (VH) or light chain variable region (VL). Particularly preferred low-molecular-weight antibodies contain both VH and VL. In addition, preferred examples of the low-molecular-weight antibodies of the present invention include low-molecular-weight antibodies containing CDRs of an antibody. The CDRs contained in the low-molecular-weight antibodies may include some or all of the six CDRs of an antibody.

The low-molecular-weight antibodies of the present invention preferably have a smaller molecular weight than whole antibodies. However, the low-molecular-weight antibodies may form multimers, for example, dimers, trimers, or tetramers, and thus their molecular weights can be greater than those of whole antibodies.

Specific examples of the antibody fragments include, for example, Fab, Fab', F(ab')2, and Fv. Meanwhile, specific examples of the low-molecular-weight antibodies include, for example, Fab, Fab', F(ab')2, Fv, scFv (single chain Fv), diabodies, and sc(Fv)2 (single chain (Fv)2). Multimers (for example, dimers, trimers, tetramers, and polymers) of these antibodies are also included in the low-molecular-weight antibodies of the present invention.

Antibody fragments can be obtained, for example, by treating antibodies with enzymes to produce antibody fragments. Enzymes known to generate antibody fragments include, for example, papain, pepsin, and plasmin. Alternatively, a gene encoding such an antibody fragment can be constructed, introduced into an expression vector, and expressed in appropriate host cells (see, for example, Co, M.S. et al., J. Immunol. (1994)152,2968-2976; Better, M. & Horwitz, A. H. Methods in Enzymology (1989)178, 476-496; Plueckthun, A. & Skerra, A. Methods in Enzymology (1989)178, 476-496; Lamoyi, E., Methods in Enzymology (1989)121, 652-663; Rousseaux, J. et al., Methods in Enzymology(1989)121, 663-669; Bird, R. E. et al., TIBTECH (1991)9, 132-137).

Digestive enzymes cleave a specific site of an antibody fragment, yielding antibody fragments of specific structures shown below. Genetic engineering techniques can be applied to such enzymatically-obtained antibody fragments to delete an arbitrary portion of the antibody.

Antibody fragments obtained by using the above-described digestive enzymes are as follows:

Papain digestion: F(ab)2 or Fab
Pepsin digestion: F(ab')2 or Fab'
Plasmin digestion: Facb

The low-molecular-weight antibodies of the present invention include antibody fragments lacking an arbitrary region, as long as they have a NR10-binding activity and/or neutralizing activity.

"Diabody" refers to a bivalent antibody fragment constructed by gene fusion (Holliger P et al., Proc. Natl. Acad. Sci. USA 90: 6444-6448 (1993); EP 404,097; WO 93/11161, etc). Diabodies are dimers composed of two polypeptide chains. In each of the polypeptide chains forming a dimer, a VL and a VH are usually linked by a linker in the same chain. In general, the linker in a diabody is short enough such that the VL and VH cannot bind to each other. Specifically, the number of amino acid residues constituting the linker is, for example, about five residues. Thus, the VL and VH encoded on the same polypeptide cannot form a single-chain variable region fragment, and will form a dimer with another single-chain variable region fragment. As a result, the diabody has two antigen binding sites.

ScFv antibodies are single-chain polypeptides produced by linking a heavy chain variable region ([VH]) and a light chain variable region ([VL]) via a linker or such (Huston, J. S. et al., Proc. Natl. Acad. Sci. U.S.A. (1988) 85, 5879-5883; Pluckthun "The Pharmacology of Monoclonal Antibodies" Vol. 113, eds., Resenburg and Moore, Springer Verlag, New York, pp. 269-315, (1994)). The H-chain V region and L-chain V region of scFv may be derived from any antibody described herein. The peptide linker for linking the V regions is not particularly limited. For example, an arbitrary single-chain peptide containing about three to 25 residues can be used as the linker. Specifically, it is possible to use the peptide linkers or such described below.

The V regions of both chains can be linked, for example, by PCR as described above. First, among the following DNAs, a DNA encoding a complete or desired partial amino acid sequence is used as a template to link the V regions by PCR:

DNA sequence encoding an H chain or H-chain V region of an antibody, and
DNA sequence encoding an L chain or L-chain V region of an antibody.

DNAs encoding the V regions of H chain and L chain are amplified by PCR using a pair of primers having sequences corresponding to those at both ends of the DNA to be amplified. Then, a DNA encoding the peptide linker portion is prepared. The peptide linker-encoding DNA can also be synthesized by PCR. Here, nucleotide sequences that can be ligated to the amplification products of V regions synthesized separately are added to the 5' end of the primers to be used. Then, PCR is carried out using each DNA of the [H chain V region DNA] - [peptide linker DNA] - [L chain V region DNA], and assembly PCR primers.

The assembly PCR primers are composed of a combination of a primer that anneals to the 5' end of the [H chain V region DNA] and a primer that anneals to the 3' end of the [L chain V region DNA]. In other words, the assembly PCR primers are a set of primers that can be used to amplify DNA encoding the full-length sequence of scFv to be synthesized. Meanwhile, nucleotide sequences that can be ligated to the V-region DNAs have been added to the [peptide linker DNA]. Thus, these DNAs are linked together, and then the whole scFv is ultimately generated as an amplification product by the assembly PCR primers. Once the scFv-encoding DNAs are generated, expression vectors carrying these DNAs and recombinant cells transformed with these expression vectors can be obtained by conventional methods. Furthermore, the scFv can be obtained by culturing the resulting recombinant cells to express the scFv-encoding DNAs.

The order of the heavy chain and light chain variable regions to be linked together is not particularly limited, and they may be arranged in any order. Examples of the arrangement are listed below.

[VH] linker [VL]
[VL] linker [VH]

sc(Fv)2 is a single-chain low-molecular-weight antibody produced by linking two VHs and two VLs using linkers and such (Hudson et al., J Immunol. Methods 1999; 231: 177-189). For example, sc(Fv)2 can be produced by linking scFvs via a linker.

Antibodies in which two VHs and two VLs are arranged in the order of VH-VL-VH-VL ([VH] linker [VL] linker [VH] linker [VL]) from the N terminus of the single-chain polypeptide are preferred. However, the order of the two VHs and two VLs is not limited to the above arrangement, and they may be arranged in any order. Examples of the arrangement are listed below:

[VL] linker [VH] linker [VH] linker [VL]
[VH] linker [VL] linker [VL] linker [VH]
[VH] linker [VH] linker [VL] linker [VL]
[VL] linker [VL] linker [VH] linker [VH]
[VL] linker [VH] linker [VL] linker [VH]

The amino acid sequence of the heavy chain variable region or light chain variable region in a low-molecular-weight antibody may contain a substitution, deletion, addition, and/or insertion. Furthermore, the heavy chain variable region and light chain variable region may also lack some portions or be added with other polypeptides, as long as they have antigen binding ability when linked together. Alternatively, the variable regions may be chimerized or humanized.

In the present invention, linkers which bind the variable regions of the antibody include arbitrary peptide linkers that can be introduced using genetic engineering, or synthetic linkers such as those disclosed in Protein Engineering, 9(3), 299-305, 1996.

The preferred linkers in the present invention are peptide linkers. The lengths of the peptide linkers are not particularly limited and those skilled in the art can appropriately select the lengths depending on the purpose. Typical lengths are one to 100 amino acids, preferably 3 to 50 amino acids, more preferably 5 to 30 amino acids, and particularly preferably 12 to 18 amino acids (for example, 15 amino acids).

Amino acid sequences of such peptide linkers include, for example:

Ser;
Gly-Ser;
Gly-Gly-Ser;

Ser-Gly-Gly;
Gly-Gly-Gly-Ser (SEQ ID NO: 82);
Ser-Gly-Gly-Gly (SEQ ID NO: 83);
Gly-Gly-Gly-Gly-Ser (SEQ ID NO: 84);
Ser-Gly-Gly-Gly-Gly (SEQ ID NO: 85);
Gly-Gly-Gly-Gly-Gly-Ser (SEQ ID NO: 86);
Ser-Gly-Gly-Gly-Gly-Gly (SEQ ID NO: 87);
Gly-Gly-Gly-Gly-Gly-Gly-Ser (SEQ ID NO: 88);
Ser-Gly-Gly-Gly-Gly-Gly-Gly (SEQ ID NO: 89);
(Gly-Gly-Gly-Gly-Ser (SEQ ID NO: 84))n; and
(Ser-Gly-Gly-Gly-Gly (SEQ ID NO: 85))n,

where n is an integer of 1 or larger.

The amino acid sequence of peptide linker can be appropriately selected by those skilled in the art depending on the purpose. For example, the above-mentioned "n", which determines the length of the peptide linker, is usually 1 to 5, preferably 1 to 3, and more preferably 1 or 2.

Synthetic linkers (chemical crosslinking agents) include crosslinking agents that are routinely used to crosslink peptides, for example, N-hydroxy succinimide (NHS), disuccinimidyl suberate (DSS), bis(sulfosuccinimidyl) suberate ($BS^3$), dithiobis(succinimidyl propionate) (DSP), dithiobis(sulfosuccinimidyl propionate) (DTSSP), ethylene glycol bis(succinimidyl succinate) (EGS), ethylene glycol bis(sulfosuccinimidyl succinate) (sulfo-EGS), disuccinimidyl tartrate (DST), disulfosuccinimidyl tartrate (sulfo-DST), bis[2-(succinimidoxycarbonyloxy)ethyl] sulfone (BSOCOES), and bis[2-(sulfosuccinimidoxycarbonyloxy)ethyl] sulfone (sulfo-BSOCOES). These crosslinking agents are commercially available.

When four antibody variable regions are linked, three linkers are usually required. Such multiple linkers may be the same or different.

The antibodies of the present invention include antibodies in which one or more amino acid residues have been added to the amino acid sequence of an antibody of the present invention. Further, fusion proteins which result from a fusion between one of the above antibodies and a second peptide or protein is included in the present invention. The fusion proteins can be prepared by ligating a polynucleotide encoding an antibody of the present invention and a polynucleotide encoding a second peptide or polypeptide in frame, inserting this into an expression vector, and expressing the fusion construct in a host. Some techniques known to those skilled in the art are available for this purpose. The partner peptide or polypeptide to be fused with an antibody of the present invention may be a known peptide, for example, FLAG (Hopp, T. P. et al., BioTechnology 6, 1204-1210 (1988)), 6x His consisting of six His (histidine) residues, 10x His, influenza hemagglutinin (HA), human c-myc fragment, VSV-GP fragment, p18HIV fragment, T7-tag, HSV-tag, E-tag, SV40 T antigen fragment, lck tag, α-tubulin fragment, B-tag, Protein C fragment. Other partner polypeptides to be fused with the antibodies of the present invention include, for example, GST (glutathione-S-transferase), HA (influenza hemagglutinin), immunoglobulin constant region, β-galactosidase, and MBP (maltose-binding protein). A polynucleotide encoding one of these commercially available peptides or polypeptides can be fused with a polynucleotide encoding an antibody of the present invention. The fusion polypeptide can be prepared by expressing the fusion construct.

Furthermore, the antibodies of the present invention may be conjugated antibodies which are linked to any of various molecules including polymeric substances such as polyethylene glycol (PEG) and hyaluronic acid, radioactive substances, fluorescent substances, luminescent substances, enzymes, and toxins. Such conjugated antibodies can be obtained by chemically modifying the obtained antibodies. Methods for modifying antibodies have been established in this field (for example, US 5057313 and US 5156840). The "antibodies" of the present invention also include such conjugated antibodies.

Furthermore, the antibodies used in the present invention may be bispecific antibodies. The bispecific antibody refers to an antibody that has variable regions recognizing different epitopes in the same antibody molecule. In the present invention, the bispecific antibodies may recognize different epitopes on an NR10 molecule, or recognize NR10 with one antigen-binding site and a different substance with the other antigen-binding site.

Methods for producing bispecific antibodies are known. Bispecific antibodies can be prepared, for example, by linking two antibodies that recognize different antigens. Antibodies to be linked together may be half molecules each of which contains an H chain and an L chain, or quarter molecules that consist of only one H chain. Alternatively, hybridomas producing different monoclonal antibodies can be fused to produce a bispecific antibody-producing fused cell. Furthermore, bispecific antibodies can be produced by genetic engineering techniques.

The antibodies of the present invention may differ in amino acid sequence, molecular weight, isoelectric point, presence/absence of sugar chains, and conformation depending on the cell or host producing the antibody or the purification method as described below. However, a resulting antibody is included in the present invention, as long as it is functionally equivalent to an antibody of the present invention. For example, when an antibody of the present invention is expressed in prokaryotic cells, for example *E. coli,* a methionine residue is added to the N terminus of the original antibody amino acid sequence. Such antibodies are included in the present invention.

Antibody production

The antibodies of the present invention may be polyclonal or monoclonal antibodies. Such monoclonal antibodies having NR10-binding and/or neutralizing activity can be obtained, for example, by the following procedure: anti-NR10 monoclonal antibodies are prepared by using as an antigen NR10 or a fragment thereof that is derived from a mammal such as human or mouse by known methods, and then antibodies having NR10-binding and/or neutralizing activity are selected from the thus obtained anti-NR10 monoclonal antibodies. Specifically, a desired antigen or cells expressing the desired antigen are used as a sensitizing antigen for immunization according to conventional immunization methods. Anti-NR10 monoclonal antibodies can be prepared by fusing the obtained immune cells with known parental cells using conventional cell fusion methods, and screening them for monoclonal antibody-producing cells (hybridomas) by conventional screening methods. Animals to be immunized include, for example, mammals such as mice, rats, rabbits, sheep, monkeys, goats, donkeys, cows, horses, and pigs. The antigen can be prepared using the known NR10 gene sequence according to known methods, for example, by methods using baculovirus (for example, WO 98/46777).

Hybridomas can be prepared, for example, according to the method of Milstein *et al.* (Kohler, G. and Milstein, C., Methods Enzymol. (1981) 73: 3-46) or such. When the immunogenicity of an antigen is low, immunization may be performed after linking the antigen with a macromolecule having immunogenicity, such as albumin.

Embodiments of the antibodies of the present invention that have a binding and/or neutralizing activity against NR10 include monoclonal antibodies that have a binding and/or neutralizing activity against human NR10. Antigens used to prepare monoclonal antibodies that have a binding and/or neutralizing activity against human NR10 are not particularly limited, as long as they enable preparation of antibodies that have a binding and/or neutralizing activity against human NR10. For example, it is known that there are a number of variants of human NR10, and any variant may be used as an immunogen as long as it enables preparation of antibodies that have a binding and/or neutralizing activity against human NR10. Alternatively, under the same condition, a peptide fragment of NR10 or a protein in which artificial mutations have been introduced into the natural NR10 sequence may be used as an immunogen. Human NR10.3 is one of preferred immunogens in preparing antibodies that have an activity of binding and/or neutralizing NR10 in the present invention.

Furthermore, the binding and/or neutralizing activity of antibody against NR10 can be measured, for example, by observing the effect of suppressing the growth of the IL-31-dependent cell line as described in the Examples.

Meanwhile, monoclonal antibodies can also be obtained by DNA immunization. DNA immunization is a method in which a vector DNA constructed such that the gene encoding an antigen protein can be expressed in an animal to be immunized is administered to the animal, and the immunogen is expressed within the body of the animal to provide immunostimulation. As compared to common immunization methods based on the administration of protein antigens, the DNA immunization is expected to be advantageous in that:

- it enables immunostimulation while retaining the structure of a membrane protein; and
- the immunogen does not need to be purified.

On the other hand, it is difficult to combine DNA immunization with an immunostimulating means such as an adjuvant.

In order to obtain a monoclonal antibody by DNA immunization, first, DNA encoding NR10 is administered to an animal to be immunized. The DNA encoding NR10 can be synthesized by known methods such as PCR. The resulting DNA is inserted into an appropriate expression vector, and administered to the animal to be immunized. Expression vectors that can be used include commercially available expression vectors such as pcDNA3.1. The vector can be administered to the living body by conventional methods. For example, DNA immunization can be carried out by introducing gold particles coated with the expression vector into cells by gene gun. Booster using NR10-expressing cells after DNA immunization is a preferred method to yield a monoclonal antibody.

Once the mammal is immunized as described above and the serum level of a desired antibody is confirmed to be increased, immune cells are collected from the mammal and subjected to cell fusion. Preferred immune cells are spleen cells in particular.

Mammalian myeloma cells are used for fusion with the above immune cells. It is preferred that myeloma cells have appropriate selection markers for screening. The selection marker refers to a phenotype that allows (or does not allow) survival under particular culture conditions. Known selection markers include hypoxanthine-guanine phosphoribosyltransferase deficiency (hereinafter abbreviated as "HGPRT deficiency") and thymidine kinase deficiency (hereinafter abbreviated as "TK deficiency"). HGPRT- or TK-deficient cells exhibit hypoxanthine-aminopterin-thymidine sensitivity (hereinafter abbreviated as "HAT sensitivity"). In HAT selection medium, HAT-sensitive cells cannot synthesize DNA and thus will die. However, when fused with normal cells, they can continue to synthesize DNA via the salvage pathway of the normal cells and thus can grow even in HAT selection medium.

HGPRT- or TK-deficient cells can be selected using a medium containing 6-thioguanine, 8-azaguanine (hereinafter abbreviated as "8AG"), or 5'-bromodeoxyuridine. While normal cells are killed due to incorporation of these pyrimidine analogs into DNA, cells lacking these enzymes can survive in the selection medium because they cannot incorporate these pyrimidine analogs. Another selection marker called G418 resistance confers resistance to 2-deoxystreptamine antibiotics (gentamicin analogs) due to the neomycin resistance gene. Various myeloma cells suitable for cell fusion are known.

Cell fusion between immune cells and myeloma cells can be essentially carried out according to known methods, for example, the method by Kohler and Milstein (Kohler. G. and Milstein, C., Methods Enzymol. (1981) 73, 3-46).

More specifically, cell fusion can be carried out, for example, in a common culture medium in the presence of a cell fusion-promoting agent. The fusion-promoting agent includes, for example, polyethylene glycol (PEG) and Sendai virus (HVJ). If required, an auxiliary agent such as dimethyl sulfoxide may also be added to improve fusion efficiency.

The immune cells and myeloma cells may be used at an arbitrarily determined ratio. For example, the ratio of immune cells to myeloma cells is preferably from 1 to 10. Culture media to be used for cell fusion include, for example, media that are suitable for the cell growth of myeloma cell line, such as RPMI 1640 and MEM, and other common culture media used for this type of cell culture. In addition, the culture media may also be supplemented with serum supplement such as fetal calf serum (FCS).

Predetermined amounts of immune cells and myeloma cells are mixed well in the culture medium, and then mixed with a PEG solution pre-heated to 37°C to produce fused cells (hybridomas). In the cell fusion method, for example, PEG with mean molecular weight of about 1,000-6,000 can be added to the cells typically at a concentration of 30% to 60% (w/v). Then, successive addition of the appropriate culture medium listed above and removal of supernatant by centrifugation are repeated to eliminate the cell fusion agent and such, which are unfavorable to the growth of hybridomas.

The resulting hybridomas can be screened using a selection medium according to the selection marker possessed by myeloma cells used in the cell fusion. For example, HGPRT- or TK-deficient cells can be screened by culturing them in a HAT medium (a medium containing hypoxanthine, aminopterin, and thymidine). Specifically, when HAT-sensitive myeloma cells are used in cell fusion, cells successfully fused with normal cells can be selectively grown in the HAT medium. The cell culture using the above HAT medium is continued for a sufficient period of time to allow all cells except the desired hybridomas (non-fused cells) to die. Specifically, in general, the desired hybridomas can be selected by culturing the cells for several days to several weeks. Then, screening and single cloning of hybridomas that produce an antibody of interest can be carried out by performing ordinary limiting dilution methods. Alternatively, antibodies that recognize NR10 can be prepared by the method described in WO 03/104453.

Screening and single cloning of an antibody of interest can be suitably carried out by known screening methods based on antigen-antibody reaction. For example, an antigen is bound to a carrier such as beads made of polystyrene or such and commercially available 96-well microtiter plates, and then reacted with the culture supernatant of hybridoma. Next, the carrier is washed and then reacted with an enzyme-labeled secondary antibody or such. When the culture supernatant contains an antibody of interest reactive to the sensitizing antigen, the secondary antibody binds to the carrier via this antibody. Finally, the secondary antibody bound to the carrier is detected to determine whether the culture supernatant contains the antibody of interest. Hybridomas producing a desired antibody capable of binding to the antigen can be cloned by the limiting dilution method or such. Not only the antigen used for immunization but also an NR10 protein substantially equivalent thereto can be preferably used as an antigen for this purpose. For example, a cell line expressing NR10, the extracellular domain of NR10, or an oligopeptide composed of a partial amino acid sequence constituting the domain may be used as the antigen.

In addition to the above-described method for preparing hybridomas through immunization of a nonhuman animal with an antigen, antibodies of interest can also be obtained by sensitizing human lymphocytes with an antigen. Specifically, first, human lymphocytes are sensitized with an NR10 protein *in vitro.* Then, the sensitized lymphocytes are fused with an appropriate fusion partner. For example, human-derived myeloma cells with the ability to divide permanently can be used as the fusion partner (see Japanese Patent Application Kokoku Publication No. (JP-B) H1-59878 (examined, approved Japanese patent application published for opposition). Antibodies obtained by this method are human antibodies having an activity of binding to the NR10 protein.

The nucleotide sequence encoding an anti-NR10 antibody obtained by the above-described method or such, and its amino acid sequence can be obtained by methods known to those skilled in the art. Amino acids contained in the amino acid sequences described in the present invention may undergo post-translational modification (for example, modification of N-terminal glutamine into pyroglutamic acid by pyroglutamylation is well-known to persons skilled in the art). Naturally, such sequences with post-translationally modified amino acids are also included in the amino acid sequences described in the present invention.

Based on the obtained sequence of the anti-NR10 antibody, the anti-NR10 antibody can be prepared, for example, by genetic recombination techniques known to those skilled in the art. Specifically, a polynucleotide encoding an antibody can be constructed based on the sequence of the NR10-recognizing antibody, inserted into an expression vector, and then expressed in appropriate host cells (see for example, Co, M. S. et al., J. Immunol. (1994) 152,2968-2976; Better, M. and Horwitz, A. H., Methods Enzymol. (1989) 178,476-496; Pluckthun, A. and Skerra, A., Methods Enzymol. (1989) 178, 497-515; Lamoyi, E., Methods Enzymol. (1986) 121, 652-663; Rousseaux, J. et al., Methods Enzymol. (1986) 121, 663-669; Bird, R. E. and Walker, B. W., Trends Biotechnol. (1991) 9,132-137).

The vectors include M13 vectors, pUC vectors, pBR322, pBluescript, and pCR-Script. Alternatively, when aiming to subclone and excise cDNA, the vectors include, for example, pGEM-T, pDIRECT, and pT7, in addition to the vectors described above. Expression vectors are particularly useful when using vectors for producing the antibodies of the present invention. For example, when aiming for expression in *E. coli* such as JM109, DH5α, HB101, and XL1-Blue, the expression vectors not only have the above-described characteristics that allow vector amplification in *E. coli,* but must also carry a promoter that allows efficient expression in *E. coli,* for example, lacZ promoter (Ward et al., Nature (1989) 341, 544-546; FASEB J. (1992) 6, 2422-2427), araB promoter (Better et al., Science (1988) 240, 1041-1043), T7 promoter or such. Such vectors include pGEX-5X-1 (Pharmacia), "QIAexpress system" (Qiagen), pEGFP, or pET (in this case, the host is preferably BL21 that expresses T7 RNA polymerase) in addition to the vectors described above.

The vectors may contain signal sequences for antibody secretion. As a signal sequence for antibody secretion, a pelB signal sequence (Lei, S. P. et al J. Bacteriol. (1987) 169, 4379) may be used when a protein is secreted into the *E. coli* periplasm. The vector can be introduced into host cells by calcium chloride or electroporation methods, for example.

In addition to vectors for *E. coli,* the vectors for producing the antibodies of the present invention include mammalian expression vectors (for example, pcDNA3 (Invitrogen), pEF-BOS (Nucleic Acids. Res. 1990, 18(17), p5322), pEF, and pCDM8), insect cell-derived expression vectors (for example, the "Bac-to-BAC baculovirus expression system" (Gibco-BRL) and pBacPAK8), plant-derived expression vectors (for example, pMH1 and pMH2), animal virus-derived expression vectors (for example, pHSV, pMV, and pAdexLcw), retroviral expression vectors (for example, pZIPneo), yeast expression vectors (for example, "Pichia Expression Kit" (Invitrogen), pNV.11, and SP-Q01), and *Bacillus subtilis* expression vectors (for example, pPL608 and pKTH50), for example.

When aiming for expression in animal cells such as CHO, COS, and NIH3T3 cells, the vectors must have a promoter essential for expression in cells, for example, SV40 promoter (Mulligan et al., Nature (1979) 277, 108), MMLV-LTR promoter, EF1α promoter (Mizushima et al., Nucleic Acids Res. (1990) 18, 5322), and CMV promoter, and more preferably they have a gene for selecting transformed cells (for example, a drug resistance gene that allows evaluation using an agent (neomycin, G418, or such). Vectors with such characteristics include pMAM, pDR2, pBK-RSV, pBK-CMV, pOPRSV, and pOP13, for example.

In addition, the following method can be used for stable gene expression and gene amplification in cells: CHO cells deficient in a nucleic acid synthesis pathway are introduced with a vector (for example, pSV2-dhfr (Molecular Cloning 2nd edition, Cold Spring Harbor Laboratory Press, 1989)) that carries a DHFR gene which compensates for the deficiency, and the vector is amplified using methotrexate (MTX). Alternatively, the following method can be used for transient gene expression: COS cells with a gene expressing SV40 T antigen on their chromosome are transformed with a vector (pcD and such) with an SV40 replication origin. Replication origins derived from polyoma virus, adenovirus, bovine papilloma virus (BPV), and such can also be used. To amplify gene copy number in host cells, the expression vectors may further carry selection markers such as aminoglycoside transferase (APH) gene, thymidine kinase (TK) gene, *E. coli* xanthine-guanine phosphoribosyltransferase (Ecogpt) gene, and dihydxofolate reductase (dhfr) gene.

Thus, the present invention provides methods for producing the polypeptides of the present invention or polypeptides encoded by genes encoding the polypeptides of the present invention, which comprise the step of culturing host cells containing a vector into which a polynucleotide encoding the polypeptide of the present invention has been introduced.

More specifically, the present invention provides methods for producing the polypeptides of the present invention, comprising the steps of:

(a) culturing a host cell containing a vector into which a gene encoding the polypeptide of the present invention has been introduced; and
(b) obtaining the polypeptide encoded by the gene.

The antibodies of the present invention obtained by the methods described above can be isolated from inside host cells or from outside the cells (the medium, or such), and purified to homogeneity. The antibodies can be isolated and purified by methods routinely used for isolating and purifying antibodies, and the type of method is not limited. For example, the antibodies can be isolated and purified by appropriately selecting and combining column chromatography, filtration, ultrafiltration, salting out, solvent precipitation, solvent extraction, distillation, immunoprecipitation, SDS-polyacrylamide gel electrophoresis, isoelectrofocusing, dialysis, recrystallization, and such.

The chromatographies include, for example, affinity chromatography, ion exchange chromatography, hydrophobic chromatography, gel filtration, reverse phase chromatography, and adsorption chromatography (Strategies for Protein Purification and Characterization: A Laboratory Course Manual. Ed Daniel R. Marshak *et al.,* Cold Spring Harbor Laboratory Press, 1996). The chromatographic methods described above can be conducted using liquid chromatography, for example, HPLC and FPLC. Columns that can be used for affinity chromatography include protein A columns and protein G columns. Columns using protein A include, for example, Hyper D, POROS, and Sepharose FF (GE Amersham Biosciences). The present invention includes antibodies that are highly purified using these purification methods.

The NR10-binding activity of the obtained antibodies can be determined by methods known to those skilled in the art. Methods for determining the antigen-binding activity of an antibody include, for example, ELISA (enzyme-linked immunosorbent assay), EIA (enzyme immunoassay), RIA (radioimmunoassay), and fluorescent antibody method. For example, when enzyme immunoassay is used, antibody-containing samples, such as purified antibodies and culture supernatants of antibody-producing cells, are added to antigen-coated plates. A secondary antibody labeled with an enzyme, such as alkaline phosphatase, is added and the plates are incubated. After washing, an enzyme substrate, such as p-nitrophenyl phosphate, is added, and the absorbance is measured to evaluate the antigen-binding activity.

## Pharmaceutical compositions

The present invention also provides pharmaceutical compositions comprising the antibody mentioned above as an active ingredient. Moreover, the present invention provides therapeutic agents for inflammatory diseases which comprise the antibody mentioned above as an active ingredient.

In the present invention, inflammatory disease refers to diseases with pathological features involved in cytological and histological reactions that occur in affected blood vessels and adjacent tissues in response to an injury or abnormal stimulation caused by physical, chemical, or biological agents (Stedman's Medical Dictionary, 5th Ed., MEDICAL VIEW CO., 2005). Generally, inflammatory diseases include, dermatitis (atopic dermatitis, chronic dermatitis, and such), inflammatory bowel diseases (colitis and such), asthma, arthritis (rheumatoid arthritis, osteoarthritis, and such), bronchitis, Th2 autoimmune diseases, systemic lupus erythematosus, myasthenia gravis, chronic GVHD, Crohn's disease, spondylitis deformans, lumbar pain, gout, inflammation after surgery or injury, swelling, neuralgia, laryngopharyngitis, cystitis, hepatitis (non-alcoholic steatohepatitis, alcoholic hepatitis, and such), hepatitis B, hepatitis C, arteriosclerosis, and pruritus.

Preferred examples of inflammatory diseases that are subjects of the present invention include atopic dermatitis, chronic dermatitis, rheumatism, osteoarthritis, chronic asthma, and pruritus.

The phrase "comprise(s) an anti-NR10 antibody as an active ingredient" means comprising an anti-NR10 antibody as at least one of the active ingredients, and does not limit the proportion of the antibody. In addition, the therapeutic agents for inflammatory diseases in the present invention may also comprise, in combination with the anti-NR10 antibody mentioned above, other ingredients that enhance the treatment of inflammatory diseases.

The therapeutic agents of the present invention may also be used for preventive purposes.

The anti-NR10 antibody of the present invention may be prepared as formulations according to standard methods (see, for example, Remington's Pharmaceutical Science, latest edition, Mark Publishing Company, Easton, USA). Further, they may contain pharmaceutically acceptable carriers and/or additives if necessary. For example, they may contain surfactants (for example, PEG and Tween), excipients, antioxidants (for example, ascorbic acid), coloring agents, flavoring agents, preservatives, stabilizers, buffering agents (for example, phosphoric acid, citric acid, and other organic acids), chelating agents (for example, EDTA), suspending agents, isotonizing agents, binders, disintegrators, lubricants, fluidity promoters, and corrigents. However, without limitation to these, the agents for preventing or treating inflammatory diseases of the present invention may contain other commonly used carriers. Such carriers specifically include light anhydrous silicic acid, lactose, crystalline cellulose, mannitol, starch, carmelose calcium, carmelose sodium, hydroxypropylcellulose, hydroxypropylmethylcellulose, polyvinylacetaldiethylaminoacetate, polyvinylpyrrolidone, gelatin, medium chain fatty acid triglyceride, polyoxyethylene hydrogenated castor oil 60, sucrose, carboxymethylcellulose, corn starch, and inorganic salt. The agents may also contain other low-molecular-weight polypeptides, proteins such as serum albumin, gelatin, and immunoglobulin, and amino acids such as glycine, glutamine, asparagine, arginine, and lysine. When the anti-NR10 antibody is prepared as an aqueous solution for injection, the anti-NR10 antibody may be dissolved in an isotonic solution containing, for example, physiological saline, dextrose, or other adjuvants. The adjuvants may include, for example, D-sorbitol, D-mannose, D-mannitol, and sodium chloride. In addition, appropriate solubilizing agents, for example, alcohols (for example, ethanol), polyalcohols (for example, propylene glycols and PEGs), and non-ionic detergents (polysorbate 80 and HCO-50) may be used concomitantly.

If necessary, anti-NR10 antibodies may be encapsulated in microcapsules (microcapsules made of hydroxymethylcellulose, gelatin, polymethylmethacrylate, and the like), and made into components of colloidal drug delivery systems (liposomes, albumin microspheres, microemulsions, nano-particles, and nano-capsules) (for example, see "Remington's Pharmaceutical Science 16th edition" &, Oslo Ed. (1980)). Moreover, methods for making sustained-release drugs are known, and these can be applied for anti-NR10 antibodies (Langer et al., J. Biomed. Mater. Res. (1981) 15,167-277; Langer, Chem. Tech. (1982) 12,98-105; US Patent No. 3,773,919; European Patent Application (EP) No. 58,481; Sidman et al., Biopolymers (1983) 22, 547-56; EP 133,988).

The pharmaceutical compositions of the present invention can be administered either orally or parenterally, but are preferably administered parenterally. Specifically, the agents are administered to patients by injection or percutaneous administration. Injections include, for example, intravenous injections, intramuscular injections, and subcutaneous injections, for systemic or local administration. The agents may be given to sites where inflammation is to be suppressed, or areas surrounding the sites by local infusion, intramuscular injection in particular. The administration methods can be properly selected according to the patient's age and condition. The single-administration dose can be selected, for example, from within the range of 0.0001 to 100 mg of the active ingredient per kg body weight. Alternatively, for example, when the agents are administered to human patients, the dose of the active ingredient can be selected from within the range of 0.001 to 1,000 mg/kg body weight. The single-administration dose preferably contains, for example, about 0.01 to 50 mg/kg body weight of the antibody of the present invention. However, the dose of an agent for preventing or treating inflammatory diseases of the present invention is not limited to these examples.

All prior-art documents cited in the present specification are herein incorporated by reference.

[Examples]

Herein below, the present invention will be specifically described with reference to Examples, but it is not to be construed as being limited thereto.

[Example 1] Preparation of hybridomas

1.1. Preparation of human and cynomolgus monkey NR10 plasmids for DNA immunization

1.1.1. Preparation of expression vectors for hNR10 and cynNR10

Human NR10 (nucleotide sequence, SEQ ID NO: 75; amino acid sequence, SEQ ID NO: 76) was inserted into the expression vector pMacII, which expresses a protein under the control of mouse β-actin promoter (WO2005/054467), to prepare an expression vector for hNR10. In the same manner, an expression vector for cynNR10 as constructed from cynomolgus monkey NR10 (nucleotide sequence, SEQ ID NO: 65; amino acid sequence, SEQ ID NO: 66).

1.1.2. Preparation of DNA cartridge

In order to use the hNR10 or cynNR10 expression vector prepared in 1.1.1 for DNA immunization of mice, the Helios Gene Gun Cartridge Kit (BIO-RAD) was used to produce a DNA cartridge for each DNA that allows immunization with 1 μg of DNA at one time.

1.2. Preparation of hybridomas producing anti-human NR10 antibody

1.2.1. Preparation of hybridomas using mice immunized with human or cynomolgus monkey NR10

Ten Balb/c mice (female; six weeks old at the beginning of immunization; Charles River Laboratories Japan) were immunized with human or cynomolgus monkey NR10 by the following procedure. For primary immunization, the mice were immunized with the DNA cartridge prepared with the hNR10 expression vector using the Helios Gene Gun System (BIO-RAD). One week later, secondary immunization was performed by the Helios Gene Gun System (BIO-RAD) using the DNA cartridge prepared with the cynNR10 expression vector. The third and subsequent immunizations were carried out at one-week intervals using the hNR10 and cynNR10 expression vectors alternately. After the titer of serum antibody against human NR10 was confirmed to be elevated, a human NR10 protein (extracellular domain) (Referential Example 4) diluted with PBS(-) was intravenously administered at 10 μg/head as the final immunization. Four days after the final immunization, mouse spleen cells were fused with mouse myeloma P3X63Ag8U.1 cells (abbreviated as P3U1; ATCC CRL-1597) by a conventional method using PEG1500 (Roche Diagnostics). The resulting fused cells, i.e., hybridomas, were cultured in RPMI1640 supplemented with 10% FBS (hereinafter abbreviated as 10% FBS/RPMI1640).

1.2.2. Selection of hybridomas

On the next day of fusion, the fused cells were suspended in a semisolid medium (StemCells), and cultured for selection as well as colonization of hybridomas.

After nine or ten days of fusion, hybridoma colonies were picked up and each colony was seeded into each well of 96-well plates containing the HAT selection medium (10% FBS/ RPMI1640, 2 vol% of HAT 50x concentrate (Dainippon Pharmaceutical), and 5 vol% of BM-Condimed H1 (Roche Diagnostics)). After three to four days of culture, the culture supernatant was collected from each well to determine the concentration of mouse IgG in the supernatant. The culture supernatants in which mouse IgG was detected were assessed for a neutralizing activity using a human IL-31-dependent cell line (hNR10/hOSMR/BaF3 cells; Referential Example 2), and several clones having a strong NR10-neutralizing activity were obtained (Fig. 3). Clones that suppress the human IL-31-induced growth of cells in a concentration-dependent manner and suppress the cynomolgus monkey IL-31-induced growth of cells (cynNR10/cynOSMR/BaF3 cells; Referential Example 2) in a concentration-dependent manner were obtained (Fig. 4).

[Example 2] Preparation of chimeric antibodies

Preparation of expression vectors for chimeric antibodies

Total RNAs were extracted from the hybridomas using RNeasy Mini Kits (QIAGEN), and cDNAs were synthesized from them using SMART RACE cDNA Amplification Kit (BD Biosciences). Antibody variable region genes were isolated by PCR using PrimeSTAR HS DNA polymerase (TaKaRa), 10x Universal Primer A Mix attached to SMART RACE cDNA Amplification Kit (BD Biosciences), and primers designed for each antibody constant region (H chain, mIgG1-rnot; L chain, mIgK-rnot). The nucleotide sequence of each isolated DNA fragment was determined with ABI PRISM 3730xL DNA Sequencer or ABI PRISM 3700 DNA Sequencer (Applied Biosystems), using BigDye Terminator Cycle Sequencing Kit (Applied Biosystems) according to the method described in the appended instruction manual. The determined amino acid sequences of H chain and L chain variable regions in the mouse antibodies NS18, NS22, NS23, and NS33 were shown in Figs. 1 and 2, respectively.

Each of the resulting H and L chain fragments was subjected to PCR using PrimeSTAR HS DNA Polymerase (TaKaRa) and the primer sets shown in Table 1. The resulting amplified fragments were ligated with the constant region (human $\gamma$1 or $\gamma$2, and human $\kappa$, respectively), and then inserted into an animal cell expression vector. The nucleotide sequence of each DNA fragment was determined with ABI PRISM 3730xL DNA Sequencer or ABI PRISM 3700 DNA Sequencer (Applied Biosystems), using BigDye Terminator Cycle Sequencing Kit (Applied Biosystems) according to the method described in the appended instruction manual.

Table 1

| | Sequence (5' →3') | SEQ ID NO: |
|---|---|---|
| mIgG1-rnot | TAATAGCGGCCGCTCATTATTTACCAGGAGAGTGGGAGAG | 90 |
| mIgK-rnot | TAATAGCGGCCGCTCATTAACACTCATTCCTGTTGAAGCT | 91 |
| mNS18H-feco | GACGAATTCCACCATGGGATGGAGCTGGATCTT | 92 |
| mNS18L-feco | GACGAATTCCACCATGAGTGTGCCCACTCAGGT | 93 |
| mNS33H-feco | GACGAATTCCACCATGGAATGTAACTGGATACT | 94 |
| mNS33L-feco | GACGAATTCCACCATGGATTTTCTGGTGCAGAT | 95 |

| | Forward primer | Reverse primer |
|---|---|---|
| NS18 H chain | mNS18H-feco | mIG1-rnot |
| NS18 L chain | mNS18L-feco | mIGK-rnot |
| NS22 H chain | Mns18H-feco | mIG1-rnot |
| NS22 L chain | mNS18L-feco | mIGK-rnot |
| NS23 H chain | mNS18H-feco | mIG1-rnot |
| NS23 L chain | mNS18L-feco | mIGK-rnot |
| NS33 H chain | mNS33H-feco | mIG1-rnot |
| NS33 L chain | mNS33L-feco | mIGK-rnot |

Preparation of chimeric antibodies

Human embryonic kidney cancer cell line HEK293H (Invitrogen) was suspended in DMEM (Invitrogen) supplemented with 10% fetal bovine serum (Invitrogen), and 10 ml of cells were seeded into dishes for adherent cells (10 cm in diameter; CORNING) at a cell density of 6 x $10^5$ cells/ml. The cells were incubated in a $CO_2$ incubator (37°C, 5% $CO_2$) for one whole day and night. Then, the medium was removed by aspiration, and 6.9 ml of CHO-S-SFMII medium (Invitrogen) was added. CHO-S-SFMII medium was added to the prepared plasmid DNA mixture (13.8 μg in total) to a volume of 700 μl. This was mixed with 20.7 μl of 1 μg/ml polyethyleneimine (Polysciences Inc.), and allowed to stand at room temperature for 10 minutes. The solution was added to the cells in each dish. The cells were incubated in a $CO_2$ incubator (37°C, 5% $CO_2$) for four to five hours. Then, 6.9 ml of CHO-S-SFMII medium (Invitrogen) was added, and the cells were incubated in a $CO_2$ incubator for three to four days. The culture supernatants were collected and then centrifuged (approx. 2000 g, five minutes, room temperature) to remove the cells. The supernatants were filtered through 0.22-μm filter MILLEX®-GV (Millipore). Each sample was stored at 4°C until use. Antibodies were purified from the supernatants using Protein G Sepharose (Amersham Biosciences). The purified antibodies were concentrated with Amicon Ultra 15 (Millipore), and then the solvent was replaced with PBS(-) containing 0.05% $NaN_3$ using PD-10 Desalting columns (Amersham Biosciences. The absorbance at 280 nm was measured with ND-1000 Spectrophotometer (NanoDrop), and the concentrations were determined by the method of Pace et al. (Protein Science (1995) 4: 2411-2423).

Assessment of the activity of chimeric NS22

The activity of neutralizing hIL-31 was assessed using the hNR10/hOSMR/BaF3 cell line, which grows in an hIL-31 dose-dependent manner, as described below.

hNR10/hOSMR/BaF3 cells were prepared at 1.5 x $10^5$ cells/ml using RPMI1640 medium (GIBCO) containing 10% FBS (MOREGATE) and 1% Penicillin-Streptomycin (Invitrogen). hIL-31 (R&D Systems) was added to an aliquot of the cells to a final concentration of 4 ng/ml (IL-31(+); final conc.: 2 ng/ml). The remaining cell suspension was used as IL-31 (-). The purified NS22 was adjusted to 2 μg/ml using the medium, and eight serial dilutions were prepared at a common dilution ratio of 3 (final conc.: 1 μg/ml or less). 50 μl each of the cell suspension and the dilution of chimeric NS22 (human γl, κ) was added to each well of 96-well flat-bottom plates (CORNING), and the cells were cultured in a 5% $CO_2$ incubator at 37°C for two days. After culture, 20 μl of a mixture of equal amounts of Cell Counting Kit-8 (Dojindo) and PBS was added to each well, and the absorbance (450 nm/620 nm) was measured (TECAN, SUNRISE CLASSIC). After the reaction was allowed to continue for two hours in a 5% $CO_2$ incubator at 37°C, the absorbance was measured again. The neutralizing activity of NS22 was presented as an inhibition rate using a value obtained by subtracting the 0-hour value from the 2-hour value. The result showed that NS22 suppressed the IL-31-induced growth of the hNR10/hOSMR/BaF3 cell line in a concentration-dependent manner. This demonstrates that NS22 has a neutralizing activity against the human IL-31 signaling (Fig. 5).

The IL-31-neutralizing activity was assessed as described below using the DU145 cell line (human prostate cancer cell line), in which IL-6 production is induced upon IL-31 stimulation.

DU145 cells were prepared at 2.5 x $10^5$ cells/ml in MEM (Invitrogen) containing 10% FBS (MOREGATE), 2 mmol/1 L-glutamine (Invitrogen), and 1 mmol/1 sodium pyruvate (SIGMA), and 200-μl aliquots were dispensed into each well of 48-well plates (CORNING). The cells were incubated at 37°C under 5% $CO_2$ overnight. The purified chimeric NS22 (human γ1, κ) was diluted to 100 μg/ml with MEM containing 10% FBS, 2 mmol/l L-glutamine, and sodium pyruvate. Using this solution, six serial dilutions were prepared at a common dilution ratio of 5. Each dilution was combined with 100 ng/ml human interleukin-31 (R&D systems) at a ratio of 1:1, and a 50-μl aliquot was added to each well. After two days of culture at 37°C under 5% $CO_2$, the concentration of IL-6 in the culture supernatant was determined using DuoSet ELISA Development kit (R&D systems). The neutralizing activity of NS22 was assessed by determining the inhibition rate (%). Specifically, assuming the IL-6 concentration in the absence of IL-31 (A) as the maximal inhibitory activity (100% inhibition) and the IL-6 concentration in the presence of IL-31 without NS22 (B) as no inhibitory activity (0% inhibition), the IL-6 concentration in the presence of IL-31 and NS22 (C) was determined according to the following formula:

$$\text{Inhibition rate (\%)} = (B-C)/(B-A) \times 100$$

The result showed that NS22 suppressed the IL-31-induced IL-6 production in the DU145 cell line in a concentration-dependent manner and thus demonstrated that NS22 had a neutralizing activity against the human IL-31 signaling (Fig. 6).

Assessment of competition of chimeric anti-NR10 antibody with IL-31

Human IL-31 (R&D Systems) was labeled with FMAT Blue Monofunctional Reactive Dye (Applied Biosystems). 100 μl of hIL-31 prepared at 0.5 mg/ml using 50 mM sodium phosphate buffer (pH 8.0) was mixed with 5.25 μl of 25 nmoles FMAT Blue dissolved in DMSO (Junsei). After vortexing, the mixture was allowed to stand at room temperature for 15 minutes. The FMAT Blue-conjugating reaction with hIL-31 was terminated by adding 5 μl of 1 M Tris-HCl (pH 7.4) and 1.1 μl of 10% Tween20, and then FMAT Blue-labeled hIL-31 and unreacted FMAT Blue were separated by gel filtration using Superdex 75 (GE Healthcare, 17-0771-01) column with 0.1% Tween20/PBS developing solution.

Antibodies were assessed for the activity of inhibiting the IL-31/NR10 binding by using hNR10-expressin CHO cells as described below.

NS22 and NA633 (the constant region of each is γ1, κ) were diluted at an appropriate concentration using Assay buffer (10 mM HEPES, 140 mM NaCl, 2.5 mM $CaCl_2$, 3 mM $MgCl_2$, 2% FBS, 0.01% $NaN_3$), and then seven serial dilutions were prepared at a common dilution ratio of 2. The dilutions were added at 40 μl/well to plates (96-Well FMAT Plates; Applied Biosystems). Then, FMAT Blue-labeled hIL-31 was diluted 400 times with Assay buffer and added at 20 μl/well. Finally, cell suspensions adjusted to 2.5 x $10^5$ cells/ml using Assay buffer were added at 40 μl/well (final 1x$10^4$ cells/well). Two hours after addition of cells, the fluorescence (FL1) was determined using the 8200 Cellular Detection System (Applied Biosystems). The result showed that NS22 inhibited the binding of hIL-31/hNR10 in a dose-dependent manner, and demonstrated that its activity was superior to that of NA633 (Fig. 7).

[Example 3] Competition of anti-NR10 antibody against NR10

The antibody NS22 purified from a hybridoma culture supernatant was labeled with FMAT Blue (Applied Biosystems, 4328853). 170 μl of NS22 prepared at 1 mg/ml in PBS was mixed with 17 μl of 1 M $NaHCO_3$ solution and 3.4 μl of FMAT Blue (17 nmoles) dissolved in DMSO. After vortexing, the mixture was allowed to stand at room temperature for 30 minutes. The FMAT Blue conjugating reaction with NS22 was terminated by adding 8 μl of 1 M Tris-HCl (pH 7.4) and 1.9 μl of 1% Tween 20, and then FMAT Blue-labeled NS22 (FMAT Blue-NS22) and unreacted FMAT Blue were separated by gel filtration using Superdex 75 (GE Healthcare, 17-0771-01) column with 0.01% Tween20/PBS developing solution.

Each antibody was examined for inhibition of the binding of the prepared FMAT Blue-NS22 to hNR10-expressing CHO cells (Referential Example 3) using the 8200 Cellular Detection System (Applied Biosystems, 4342920). The chimeric anti-NR10 antibodies (the constant region of each is γ1, κ) were added at various concentrations to each well containing 7500 cells and 8.8 x $10^{-2}$ μg/ml FMAT Blue-NS22. The cells were allowed to stand in the dark for four hours, and then the fluorescent signal from FMAT Blue bound to the cells was measured. The reaction was carried out in 10 mM Hepes-KOH containing 2.5 mM $CaCl_2$, 3 mM $MgCl_2$, 140 mM NaCl, 2% FBS, and 0.01% $NaNO_3$. The result is shown in Fig. 8. The fluorescence value FL1, which represents the binding of FMAT Blue-NS22 to NR10-expressing cells, was reduced with the increase in the concentration of antibody NS22 or NS23. On the other hand, FL1 was hardly reduced with the increase in the concentration of antibody NA633 (Referential Example 6) (Fig. 8).

[Example 4] Humanization of NS22 antibody

Selection of each framework sequence

The variable regions of mouse NS22 antibody were compared with human germline sequences. FR sequences used for humanization are summarized in Table 2. CDRs and FRs were determined based on the Kabat numbering. The humanized variable region sequences of H chain composed of FR1, FR2, FR3_1, and FR4, and composed of FR1, FR2, FR3_2, and FR4, which are listed in Table 2, are designated as H0-VH (SEQ ID NO: 50) and H1-VH (SEQ ID NO: 112), respectively. Meanwhile, the sequence of L chain composed of FR1, FR2, FR3, and FR4 is designated as L0 (SEQ ID NO: 52).

Preparation of variable region for humanized NS22 H0L0

Synthetic oligo DNAs were designed for each of the H and L chains to construct the variable regions of humanized NS22 in which the CDRs of NS22 are grafted onto the FRs used for humanization. The respective synthetic oligo DNAs were mixed, and then subjected to assembly PCR to construct a gene encoding the variable region of humanized NS22. The assembly PCR was carried out using KOD-Plus (TOYOBO) according to the following conditions. A reaction mixture containing 10 pmol synthetic oligo DNAs and the appended PCR Buffer, dNTPs, $MgSO_4$, and KOD-Plus was heated at 94°C for five minutes. The mixture was then subjected to two PCR cycles of 94°C for two minutes, 55°C for two minutes, and 68°C for two minutes. Then, 10 pmol each of a primer in which a restriction site and Kozak sequence has been added to the 5' end of the variable region, and a primer in which a restriction site has been added to the 3' end of the variable region, was added and subjected to 35 PCR cycles of 94°C for 30 seconds, 55°C for 30 seconds, and 68°C for one minute to yield a amplified fragment. The resulting amplified fragment was cloned into TOPO TA Cloning vector (TOYOBO), and its nucleotide sequence was determined by sequencing. The constructed variable regions were combined with the constant regions to prepare H0-SKSC (SEQ ID NO: 54) and L0 (SEQ ID NO: 56). The resulting construct was inserted into an expression vector capable of expressing the inserted gene in animal cells. The nucleotide sequence of each DNA fragment was determined using BigDye Terminator Cycle Sequencing Kit (Applied Biosystems) with ABI PRISM 3730xL DNA Sequencer or ABI PRISM 3700 DNA Sequencer (Applied Biosystems) according to the method described in the appended instruction manual.

Preparation of variable region for humanized NS22 H1

H1-SKSC (SEQ ID NO: 130) was generated by substituting the glutamine (E) at Kabat-numbering position 73 in FR3 of H0-SKSC (SEQ ID NO: 54) with lysine (K). The mutant was prepared using commercially available QuikChange Site-Directed Mutagenesis Kit (Stratagene) according to the appended instruction manual.

Expression of IgG-converted antibody

Antibody expression was performed by the method described below. Human fetal renal cancer cell-derived cell line HEK293H (Invitrogen) was suspended in DMEM (Invitrogen) containing 10% fetal bovine serum (Invitrogen), and 10 ml of cells at a density of 5-6 x $10^5$ cells/ml was seeded onto dishes for adherent cells (10 cm in diameter; CORNING). The cells were incubated in a $CO_2$ incubator (37°C, 5% $CO_2$) for one whole day and night. Then, the medium was removed by aspiration, and 6.9 ml of CHO-S-SFMII medium (Invitrogen) was added to the cells. The prepared plasmid DNA mixture (13.8 μg in total) was mixed with 20.7 μl of 1 μg/ml polyethyleneimine (Polysciences Inc.) and 690 μl of CHO-S-SFMII medium, and allowed to stand at room temperature for 10 minutes. The mixture was added to the cells in each dish, and the cells were incubated in a $CO_2$ incubator (5% $CO_2$, 37°C) for four to five hours. Then, 6.9 ml of CHO-S-SFMII medium (Invitrogen) was added, and the cells were incubated in a $CO_2$ incubator for three days. The culture supernatant was collected and centrifuged (approx. 2000 g, five minutes, room temperature) to remove the cells. The supernatant was then sterilized by filtration through 0.22-μm filter MILLEX®-GV (Millipore). Each sample was stored at 4°C until use.

Purification of IgG-converted antibody

50 μl of rProtein A Sepharose™ Fast Flow (Amersham Biosciences) suspended in TBS was added to the obtained culture supernatant, and mixed by inversion at 4°C for four hours or more. The solution was transferred to 0.22-μm filter cup of Ultrafree®-MC (Millipore). After three washes with 500 μl of TBS, rProtein A Sepharose™ resin was suspended in 100 μl of aqueous solution of 50 mM sodium acetate (pH 3.3), and allowed to stand for three minutes to elute the antibody. The solution was immediately neutralized by adding 6.7 μl of 1.5 M Tris-HCl (pH 7.8). The elution was performed twice and 200 μl of purified antibody was obtained. 2 μl of the antibody-containing solution was subjected to ND-1000 Spectrophotometer (NanoDrop)(Thermo Scientific NanoDrop™ 1000 Spectrophotometer (Thermo Scientific)) or 50 μl was subjected to Spectrophotometer DU-600 (BECKMAN) to measure absorbance at 280 nm, and the antibody concentration was calculated by the method of Pace et al. (Protein Science (1995) 4: 2411-2423).

Measurement of competition with IL-31 using FMAT

Antibodies were assessed for the activity of inhibiting the IL-31/NR10 binding by using hNR10-expressing CHO cells as described below. The chimeric NS22 antibody and NS22_H0L0 (H chain, H0-SKSC/SEQ ID NO: 54; L chain, L0/SEQ ID NO: 56) were diluted at an appropriate concentration using Assay buffer (10 mM HEPES, 140 mM NaCl, 2.5 mM $CaCl_2$, 3 mM $MgCl_2$, 2% FBS, 0.01% $NaN_3$, pH7.4), and further eight serial dilutions were prepared at a common dilution ration of 2. The dilutions were added at 40 μl/well to plates (96-Well FMAT Plates, Applied Biosystems). Then, FMAT Blue-labeled hIL-31 was diluted 400 times with Assay buffer, and added at 20 μl/well. Finally, a cell suspension adjusted to 2.5 x $10^5$ cells/ml using Assay buffer was added at 40 μl/well (final 1 x $10^4$ cells/well). Two hours after addition of cells, the fluorescence (FL1) was measured using the 8200 Cellular Detection System (Applied Biosystems).

The result showed that, as shown in Fig. 9, humanized NS22 antibodies H0L0 (H chain, H0-SKSC/SEQ ID NO: 54; L chain, L0/SEQ ID NO: 56), and H1L0 (H chain, H1-SKSC/SEQ ID NO: 130; L chain, L0/SEQ ID NO: 56) exhibited a competition activity comparable to that of the chimeric antibody, suggesting that both H0L0 and H1L0 are humanized anti-IL-31 receptor antibodies. In addition, it is considered that the FRs used for H0L0 and H1L0 can be used for humanization.

Accordingly, all of the mutations in CDRs described in the Examples hereinafter can be introduced into both H0 and H1.

Table 2

| H0 | Germline | Human FR sequence |
|---|---|---|
| FR1 | Germ line:hVH_1_46 (Access i on No. X92343) | QVQLVQSGAEVKKPGASVKVSCKASGYTFT (SEQ ID NO: 96) |
| FR2 | Germline:hVH_1_46 (Access i on No. X92343) | WVRQAPGQGLEWMG (SEQ ID NO:97) |
| FR3_1 | Germline:hVH_1_69 (Accession No. L22582) | RVTITADESTSTAYMELSSLRSEDTAVYYCAR (SEQ ID NO:98) |
| FR3_2 | Germ line:hVH_1_69 (Access i on No. Z27506) | RVTITADKSTSTAYMELSSLRSEDTAVYYCAR (SEQ ID NO: 131) |
| FR4 | Germline:JH1 | WGQGTLVTVSS (SEQ ID NO:99) |
| | | |
| L0 | Germline | Human FR sequence |
| FR1 | Germline:hVK_1_39 (Access i on No. X59315) | DIQMTQSPSSLSASVGDRVTITC (SEQ ID NO: 100) |
| FR2 | Germline:hVK_1_39 (Accession No. X59315) | WYQQKPGKAPKLLIY (SEQ ID NO:101) |
| FR3 | Germline:hVK_1_39 (Access i on No. X59315) | GVPSRFSGSGSGTDFTLTISSLQPEDFATYYC (SEQ ID NO:102) |
| FR4 | Germline:JK4 | FGGGTKVEIK (SEQ ID NO:103) |

[Example 5] Heterogeneity-reducing effect of novel constant regions M14 and M58 in humanized anti-IL31 receptor antibody

As shown in Referential Examples 7 to 9, it was demonstrated that the conversion of the constant region from IgG2 to M14 or M58 in the huPM1 antibody, a humanized anti-IL-6 receptor antibody, could reduce the heterogeneity derived from the IgG2 hinge region without loss of stability. Thus, humanized anti-IL-31 receptor antibodies were also tested to assess whether the heterogeneity can be reduced by converting their constant regions from the wild-type IgG2 to M14 or M58.

H0-M14, H0-M58, H0-IgG1, and H0-IgG2, which were generated by combining IgG1 (SEQ ID NO: 60), IgG2 (SEQ ID NO: 132), M14 (SEQ ID NO: 129) and M58 (SEQ ID NO: 128) generated in Referential Examples 8 and 9, with H chain variable region H0 (H0-VH/SEQ ID NO: 50) of humanized anti-IL-31 receptor antibody generated in Example 4, were used as H chains, and L0 (L0/SEQ ID NO: 56) produced in Example 4 was used as an L chain, to generate H0L0-IgG1 (H chain, H0-IgG1/SEQ ID NO: 133; L chain, L0/SEQ ID NO: 56), H0L0-IgG2 (H chain, H0-IgG2/SEQ ID NO: 134; L chain, L0/SEQ ID NO: 56), H0L0-M14 (H chain, H0-M14/SEQ ID NO: 135; L chain, L0/SEQ ID NO: 56), and H0L0-M58 (H chain, H0-M58/SEQ ID NO: 136; L chain, L0/SEQ ID NO: 56). Each antibody was expressed and purified by the method described in Example 4.

The heterogeneity was assessed by cation exchange chromatography. The prepared antibodies were assessed for heterogeneity using ProPac WCX-10 (Dionex) column, 20 mM sodium acetate (pH 5.0) as mobile phase A, and 20 mM sodium acetate/1M NaCl (pH 5.0) as mobile phase B, with an appropriate flow rate and gradient. The result of assessment by cation exchange chromatography (IEC) is shown in Fig. 10.

As shown in Fig. 10, the heterogeneity was increased by conversion of the constant region from IgG1 to IgG2 in the anti-IL-31 receptor antibody, and the heterogeneity can be reduced by conversion of the constant region to M14 or M58 in any antibody.

[Example 6] Pharmacokinetics improving effect of novel constant region M58 in anti-IL-31 receptor antibodies

As shown in Referential Example 9, conversion of the constant region from IgG1 to M58 in anti-IL-6 receptor antibody huPM1 was found to improve its human FcRn-binding activity and the pharmacokinetics in human FcRn transgenic mice. Thus, anti-IL-31 receptor antibodies were also tested to assess whether conversion of the constant region to M58 improves their pharmacokinetics.

H0L0-IgG1 (H chain: H0-IgG1/SEQ ID NO: 133; L chain: L0/SEQ ID NO: 56) and H0L0-M58 (H chain: H0-M58/SEQ ID NO: 136; L chain L0/SEQ ID NO: 56) prepared as described in Examples 4 and 5 were assessed for the human FcRn-binding activity by the method described in Referential Example 9. The result is shown in Table 3.

Table 3

| | KD($\mu$M) |
|---|---|
| H0L0-IgG1 | 1.07 |
| H0L0-M58 | 0.91 |

As shown in Table 3, conversion of the constant region from IgG1 to M58 also improved the human FcRn-binding activity of the anti-IL-31 receptor antibody H0L0 as in the anti-IL-6 receptor antibody hPM1. This suggests that conversion of the constant region from IgG1 to M58 may improve the pharmacokinetics of anti-IL-31 receptor antibody in human.

[Example 7] Identification of mutation sites reducing the isoelectric point

Production of mutants

Each mutant was produced by the method described in Example 4 or by assembly PCR. In the method using assembly PCR, oligo DNAs are synthesized based on forward and reverse sequences including an altered site. Forward oligo DNA including an altered site and reverse oligo DNA binding to the vector in which the gene to be altered was inserted were combined, and reverse oligo DNA including an altered site and forward oligo DNA binding to the vector in which the gene to be altered was inserted were combined. PCR was carried out using PrimeS TAR (Takara) to produce 5'-end and 3'-end fragments including the altered site. The two fragments were assembled by assembly PCR to produce each mutant. The produced mutant was inserted into an expression vector capable of expressing the insert gene in animal cells. The nucleotide sequence of the resulting expression vector was determined by a method known to those skilled in the art. Antibodies were produced and purified by the method described in Example 4.

Identification of mutation sites

To improve the pharmacokinetics of H0L0 (H chain, H0-SKSC/SEQ ID NO: 54; L chain, L0/SEQ ID NO: 56), altered sites capable of reducing the isoelectric point of the variable region were examined. Screening of mutation sites in the variable regions predicted from the three-dimensional structure model revealed mutation sites that would decrease the isoelectric point of the variable regions without significantly reducing its binding to NR10. These are summarized in Table 4 (Hp5-VH/SEQ ID NO: 137, Hp7-VH/SEQ ID NO: 138, Hp8-VH/SEQ ID NO: 139, Hp6-VH/SEQ ID NO: 140, Hp9-VH/SEQ ID NO: 141, Hp1-VH/SEQ ID NO: 142, Hp13-VH/SEQ ID NO: 143, Lp1-VL/SEQ ID NO: 144, Lp2-VL/SEQ ID NO: 145, Lp3-VL/SEQ ID NO: 146, Lp4-VL/SEQ ID NO: 147, Lp7-VL/SEQ ID NO: 148, Lp5-VL/SEQ ID NO: 149, Lp6-VL/SEQ ID NO: 150). Each variant was produced and purified by the method described in Example 4.

Each variant was tested for the activity of inhibiting the hIL-31/hNR10 binding by using FMAT. The test was carried out according to the method as described in Example 4. As shown in Fig. 11, the competition activity of each variant was not greatly reduced as compared to that of H0L0.

Table 4

| Name | Type | H0 sequence | Mutation site (kabat No) | H0 sequence | Amino acid after mutati | Sequence after on mutation |
|---|---|---|---|---|---|---|
| Hp5 | FR2 | WVRQAPGQGLEWMG (SEQ ID NO: 97) | 38<br>40 | R<br>*A | Q<br>S | WVQQSPGQGLEWMG (SEQ ID NO: 120) |
| Hp7 | CDR2 | LINPYNGGTSYNQKFKG (SEQ ID NO: 10) | 50 | L | E | EINPYNGGTSYNQKFKG (SEQ ID NO:113) |
| Hp8 | CDR2 | LINPYNGGTSYNGKFKG (SEQ ID NO:10) | 52 | N | D | LIDPYNGGTSYNQKFKG (SEQ ID NO:114) |

(continued)

| Name | Type | H0 sequence | Mutation site (kabat No) | H0 sequence | Amino acid after mutati | Sequence after on mutation |
|---|---|---|---|---|---|---|
| Hp6 | CDR2 | LINPYNGGTSYNQKFKG (SEQ ID NO:10) | 61 | Q | D | LINPYNGGTSYNDKFKG (SEQ ID NO:115) |
| Hp9 | CDR2 | LINPYNGGTSYNQKFKG (SEQ ID NO: 10) | 62 | K | Q | LINPYNGGTSYNQQFKG (SEQ ID NO:116) |
| Hp1 | CDR2 | LINPYNGGTSYNQKFKG (SEQ ID NO:10) | 64 | K | Q | LINPYNGGTSYNQKFQG (SEQ ID NO:117) |
| Hp13 | CDR2 | LINPYNGGTSYNQKFKG (SEQ ID NO:10) | 64<br>65 | K<br>G | Q<br>D | LINPYNGGTSYNQKFQD (SEQ ID NO:119) |

| Name | Type | L0 sequence | Mutation site (kabat No) | L0 sequence | Amino acid after mutation | Sequence after mutation |
|---|---|---|---|---|---|---|
| Lp1 | CDR1 | RTSENIYSFLA (SEQ ID NO: 13) | 24 | R | Q | QTSENIYSFLA (SEQ ID NO:121) |
| Lp2 | CDR1 | RTSENIYSFLA (SEQ ID NO: 13) | 28 | N | D | RTSEDIYSFLA (SEQ ID NO:122) |
| Lp3 | CDR2 | NAKTLAK (SEQ ID NO: 14) | 50 | N | D | DAKTLAK (SEQ ID NO:123) |
| Lp4 | CDR2 | NAKTLAK (SEQ ID NO: 14) | 52 | K | Q | NAQTLAK (SEQ ID NO:124) |
| Lp7 | CDR2 | NAKTLAK (SEQ ID NO: 14) | 54 | L | E | NAKTEAK (SEQ ID NO:125) |
| Lp5 | CDR2 | NAKTLAK (SEQ ID NO:14) | 56 | K | Q | NAKTLAQ (SEQ ID NO:126) |
| Lp6 | CDR2 | NAKTLAK (SEQ ID NO:14) | 56 | K | D | NAKTLAD (SEQ ID NO:127) |

Asterisk (*) in Table 4 above indicates a site that was not relevant to the isoelectric point but altered for conversion into a human sequence.

Examples of the humanized NS22 antibodies whose isoelectric point has been reduced by combining these alterations include Hp3Lp15 (H chain: Hp3-SKSC/SEQ ID NO: 151; L chain: Lp15/SEQ ID NO: 152). Affinity for NR10, isoelectric point, and plasma retention in mice were compared between Hp3Lp15 and H0L0.

Measurement of affinity

The affinity of each antibody for NR10 was determined by the method described in Referential Example 10.

The result of affinity measurement is shown in Table 5. The affinity of Hp3Lp15 was shown to be almost the same as that of H0L0.

Table 5

| | ka (1/Ms) | kd (1/s) | KD (M) |
|---|---|---|---|
| H0L0 | 3.7E+05 | 1.2E-03 | 3.3E-09 |
| Hp3Lp15 | 4.2E+05 | 1.6E-03 | 3.9E-09 |

Measurement of isoelectric point

Each antibody was analyzed by isoelectric focusing to assess changes in the isoelectric point of the whole antibody due to the amino acid alterations in its variable region. Isoelectric focusing was performed by the following method.

Phast-Gel Dry IEF gel (Amersham Biosciences) was swollen in Phastsystem Cassette (Amersham Biosciences) for about 30 minutes using the swelling solution shown below.

| | |
|---|---|
| MilliQ water | 1.5 ml |
| Pharmalyte 5-8 for IEF (Amersham Biosciences) | 100 μl |

Electrophoresis was carried out in PhastSystem (Amersham Biosciences) using the swollen gel according to the program indicated below. The samples were loaded onto the gel in Step 2. Calibration Kit for pI (Amersham Biosciences) was used as a pI marker.

| | | | | |
|---|---|---|---|---|
| Step 1: 2000 V | 2.5 mA | 3.5 W | 15°C | 75 Vh |
| Step 2: 200 V | 2.5 mA | 3.5 W | 15°C | 15 Vh |
| Step 3: 2000 V | 2.5 mA | 3.5 W | 15°C | 410 Vh |

After electrophoresis, the gel was fixed with 20% TCA, and then silver-stained using the Silver Staining Kit, Protein (Amersham Biosciences), according to the protocol attached to the kit. After staining, the isoelectric point of the sample (the whole antibody) was calculated from the known isoelectric points of the pI markers.

The result of isoelectric point measurement by isoelectric focusing showed that the isoelectric point of H0L0 was about 7.8, and the isoelectric point of Hp3Lp15 was about 5.5, showing that the isoelectric point of Hp3Lp15 was decreased by about 2.3 as compared to H0L0. When the theoretical isoelectric point of the variable region VH/VL was calculated by GENETYX (GENETYX CORPORATION), the theoretical isoelectric points of the variable regions of H0L0 and Hp3Lp15 were 7.76 and 4.63, respectively. Thus, the theoretical isoelectric point of Hp3Lp15 was decreased by 3.13 as compared to H0L0.

Assessment of pharmacokinetics of antibody with reduced isoelectric point using mice

In order to assess the plasma retention of Hp3Lp15, a modified antibody with reduced isoelectric point, the plasma retention of H0L0 and Hp3Lp15 was compared in normal mice. A single dose of H0L0 or Hp3Lp15 was intravenously administered at 1 mg/kg to mice (C57BL/6J, Charles River Japan, Inc.) to compare the time course of the plasma concentration. The plasma concentrations were determined by ELISA. Appropriate concentrations of a calibration sample and test plasma samples were dispensed into immunoplates (Nunc-Immuno Plate, MaxiSorp (Nalge Nunc International)) coated with anti-human IgG (Fc-specific) antibody (Sigma). The samples were allowed to stand at room temperature for one hour. After reaction with Goat Anti-Human IgG-ALP (Sigma) at room temperature for one hour, color developing reaction was carried out using BluePhos Microwell Phosphatase Substrates System (Kirkegaard & Perry Laboratories) as a substrate. The absorbance at 650 nm was measured with a microplate reader. The plasma concentrations were determined based on the absorbance of the calibration curve using the analytical software SOFTmax PRO (Molecular Devices).

Pharmacokinetic parameters (AUC and systemic clearance (CL)) were calculated from the obtained time-course data of the plasma concentration using the pharmacokinetics analysis software WinNonlin (Pharsight). The parameters are shown in Table 6. AUC and the clearance of Hp3Lp15 after the intravenous administration were increased by about 14% and reduced by about 12%, respectively, as compared to H0L0. Thus, it was demonstrated that Hp3Lp15, in which the isoelectric point of H0L0 has been reduced, had improved pharmacokinetics.

Table 6

| | AUC(μg·d/kg) | | CL(ml/d/kg) | |
|---|---|---|---|---|
| | Mean | SD | Mean | SD |
| H0L0 | 281.8 | 13.1 | 3.6 | 0.2 |
| Hp3Lp15 | 321.1 | 26.1 | 3.1 | 0.3 |

[Example 8] Effect of combinations of variable region and constant region on the biological activity

In order to assess the effects of different constant regions on the biological activity, the following variants were produced.

SKSC (SEQ ID NO: 62) and M58 (SEQ ID NO: 128), constant regions prepared in Referential Examples 7 and 9, were combined with Hp3 (Hp3-VH/SEQ ID NO: 167), a variable region prepared in Example 7, to produce Hp3-M58 (SEQ ID NO: 240) and Hp3-SKSC (SEQ ID NO: 151) as H chains. The prepared H chains were combined with Lp15 (Lp15/SEQ ID NO: 152), an L chain prepared in Example 7, to produce Hp3Lpl5-SKSC (H chain, Hp3-SKSC/SEQ ID NO: 151; L chain, Lp15/SEQ ID NO: 152) and Hp3Lp15-M58 (H chain, Hp3-M58/SEQ ID NO: 240; L chain, Lp15/SEQ ID NO: 152). Each antibody was expressed and purified by the method described in Example 4.

The antibodies produced as described above, H0L0-SKSC (H chain, H0-SKSC/SEQ ID NO: 54; L chain, L0/SEQ ID NO: 56) prepared using the constant region SKSC (SEQ ID NO: 62) described in Referential Example 7, and H0L0-M58 (H chain, H0-M58/SEQ ID NO: 136; L chain, L0/SEQ ID NO: 56) and H0L0-IgG2 (H chain, H0-IgG2/SEQ ID NO: 134; L chain, L0/SEQ ID NO: 56) prepared in Example 5, were assessed for the biological activity by the method described in Example 2 using BaF/NR10. The result is summarized in Fig. 18.

As shown in Fig. 18, no significant difference in the biological activity was detected between the constant regions. Since the biological activity was not affected when combining the two variable regions H0 and Hp3 with each constant region, combining variable regions created in future with any constant region would not result in alteration in the biological activity.

[Example 9] Identification of mutation sites suppressing degradation by thermal acceleration study

Antibodies used for pharmaceuticals have heterogeneity even though they are monoclonal antibodies obtained from clones derived from single antibody-producing cells. Such antibody heterogeneity is known to result from modification such as oxidation or deamidation, and to be increased during long-term storage or upon exposure to stress conditions, such as heat stress or light stress (see "Heterogeneity of Monoclonal Antibodies", Journal of pharmaceutical sciences, vol. 97, No. 7, 2426-2447). However, when an antibody is developed as a pharmaceutical, physical properties of the protein, particularly homogeneity and stability, are highly important. Thus, it is desired that the heterogeneity of desired/related substances be reduced and the substance be composed of a single substance as much as possible. In this context, the experiment described below was conducted to assess the antibody heterogeneity under stress conditions and to reduce the heterogeneity.

To assess degradation products, an accelerated sample of H0L0 (H chain, H0-SKSC/SEQ ID NO: 54; L chain, L0/SEQ ID NO: 56) was prepared by the method described below. The prepared accelerated sample and non-accelerated sample (initial) were analyzed by cation exchange chromatography using the method described below.

- <u>Method for preparing accelerated samples</u>
  Buffer: PBS

    Antibody concentration: 0.2 to 1.0 mg/ml
    Acceleration temperature: 60°C
    Acceleration period: one day

- <u>Method for analysis by cation exchange chromatography</u>
  Column: ProPac WCX-10,4 x 250 mm (Dionex)
  Mobile phase:

    (A) 25 mmol/l MES/NaOH, pH 6.1
    (B) 25 mmol/l MES/NaOH, 250 mmol/l NaCl, pH 6.1

  Flow rate: 0.5 ml/min
  Column temperature: 40°C
  Gradient: %B 0 to 0 (0-5 min)→0 to 30 (5-80 min)
  Detection: 280 nm

The resulting chromatograms for H0L0 samples before and after acceleration are shown in Fig. 19. The H0L0 sample after acceleration had a tendency to show an increased basic peak.

Then, screening was carried out to reduce this peak. As a result, Ha355, Ha356, Ha360, and Ha362 were found. These H chain variants were combined with L0 to produce Ha355L0 (H chain, Ha355-SKSC/SEQ ID NO: 242;

L chain, L0/SEQ ID NO: 56), Ha356L0 (H chain, Ha356-SKSC/SEQ ID NO: 243; L chain, L0/SEQ ID NO: 56), Ha360L0 (H chain, Ha360-SKSC/SEQ ID NO: 244; L chain, L0/SEQ ID NO: 56), and Ha362L0 (H chain, Ha362-SKSC/SEQ ID NO: 245; L chain, L0/SEQ ID NO: 56). The sequence of each variant is shown in Table 7.

Table 7

| Name | Type | H0 sequence | Mutation site (kabat No) | H0 sequence | Amino acid after mutation | Sequence after mutation |
|---|---|---|---|---|---|---|
| Ha355 | CDR3 | DGYDDGPYTMDY (SEQ ID NO: 265) | 100d<br>101<br>102 | M<br>D<br>Y | L<br>E<br>T | DGYDDGPYTLET (SEQ ID NO: 266) |
| Ha356 | CDR3 | DGYDDGPYTMDY (SEQ ID NO: 265) | 101<br>102 | D<br>Y | E<br>T | DGYDDGPYTMET (SEQ ID NO: 267) |
| Ha360 | CDR3 | DGYDDGPYTMDY (SEQ ID NO:265) | 97<br>101<br>102 | Y<br>D<br>Y | L<br>E<br>T | DGLDDGPYTMET (SEQ ID NO: 268) |
| Ha362 | CDR3 | DGYDDGPYTMDY (SEQ ID NO: 265) | 97<br>101<br>102 | Y<br>D<br>Y | L<br>E<br>S | DGLDDGPYTMES (SEQ ID NO: 269) |

Each of the identified antibodies was expressed and purified by the method described in Example 4. As with H0L0, a accelerated sample of each prepared antibody was prepared, and analyzed by cation exchange chromatography. The result is shown in Fig. 19.

The result showed that the generation of the basic peak increased after acceleration was reduced in the modified antibody containing a substitution of aspartic acid with glutamic acid at position 101 in the H chain, as compared to H0L0. The modified antibodies were assessed for the biological activity by the method described in Example 2 using BaF/NR10. The result is shown in Fig. 20. As shown in Fig. 20, the biological activities of the modified antibodies were comparable to or stronger than that of H0L0. These findings demonstrated that the modifications of Ha355, Ha356, Ha360, and Ha362 suppressed the generation of degradation products by acceleration, and therefore are effective in improving the stability of antibody.

[Example 10] Identification of mutation sites increasing the affinity

A library in which mutations were introduced into CDR sequences was constructed and examined to improve the affinity of H0L0 for NR10. As a result of screening of the library in which mutations were introduced into CDRs, mutations that improve the affinity for NR10 were found. The mutations are shown in Table 8. Each of the H chain variants Ha101-SKSC (SEQ ID NO: 246), Ha103-SKSC (SEQ ID NO: 247), Ha111-SKSC (SEQ ID NO: 248), Ha204-SKSC (SEQ ID NO: 249), and Ha219-SKSC (SEQ ID NO: 250) was combined with L0 (L0/SEQ ID NO: 56); and each of the modified L chains La134 (SEQ ID NO: 251), La130 (SEQ ID NO: 252), La303 (SEQ ID NO: 253), and La328 (SEQ ID NO: 254) was combined with H0 (H0-SKSC/SEQ ID NO: 54), to construct an antibody. Each variant was produced and purified by the method described in Example 4.

The affinity of each antibody for NR10 was assessed using Biacore. The result is shown in Table 9. The assay was carried out using the method described in Referential Example 10. As shown in Table 9, the KD value for each variant was found to be improved as compared to that of H0L0 (H chain, H0-SKSC/SEQ ID NO: 54; L chain, L0/SEQ ID NO: 56).

Table 8

| Name | Type | H0 sequence | Mutationsite (kabat No) | H0 sequence | Amino acid after mutation | Sequence after mutation |
|---|---|---|---|---|---|---|
| Ha101 | CDR1 | GYIMN (SEQ ID NO:270) | 33 | I | V | GYVMN (SEQ ID NO:272) |
| Ha103 | CDR1 | GY I MN (SEQ ID NO:270) | 34 | M | I | GYIIN (SEQ ID NO:273) |
| Ha111 | CDR1 | GYIMN (SEQ ID NO:270) | 34 | M | L | GYILN (SEQ ID NO: 274) |
| Ha204 | CDR2 | LINPYNGGTSYNQKFKG (SEQ ID NO: 271) | 58 | S | D | LINPYNGGTDYNQKFKG (SEQ ID NO:275) |
| Ha219 | CDR2 | LINPYNGGTSYNQKFKG (SEQ ID NO: 271) | 61 | Q | P | LINPYNGGTSYNPKFKG (SEQ ID NO: 276) |

| Name | Type | L0 sequence | Mutationsite (kabat No) | L0 sequence | Amino acid after mutation | Sequence after mutation |
|---|---|---|---|---|---|---|
| La134 | CDR1 | RTSENIYSFLA (SEQ ID NO:277) | 31 | S | R | RTSENIYRFLA (SEQ ID NO:279) |
| La130 | CDR1 | RTSENIYSFLA (SEQ ID NO:277) | 31 | S | R | RTSENIYRFVA (SEQ ID NO:280) |
| | | | 33 | L | V | |
| Ls303 | CDR3 | QHHYESPLT (SEQ ID NO:278) | 93 | E | D | QHHYDSPLT (SEQ ID NO:281) |
| La328 | CDR3 | QHHYESPLT (SEQ ID NO:278) | 94 | S | D | QHHYEDPLT (SEQ ID NO:282) |
| La326 | CDR3 | QHHYESPLT (SEQ ID NO:278) | 97 | T | F | QHHYESPLF (SEQ ID NO:283) |

Table 9

| Name | ka(1/Ms) | kd(1/s) | KD(M) |
|---|---|---|---|
| H0L0 | 1.9E+05 | 6.2E-04 | 3.2E-09 |
| Ha101L0 | 2.0E+05 | 3.1E-04 | 1.5E-09 |
| Ha103L0 | 2.2E+05 | 5.3E-04 | 2.4E-09 |
| Ha111L0 | 2.6E+05 | 5.6E-04 | 2.1E-09 |
| Ha204L0 | 3.7E+05 | 4.8E-04 | 1.3E-09 |
| Ha219L0 | 3.2E+05 | 9.6E-04 | 3.0E-09 |

| Name | ka(1/Ms) | kd(1/s) | KD(M) |
|---|---|---|---|
| H0L0 | 1.5E+05 | 7.4E-04 | 5.1E-09 |
| H0La134 | 2.5E+05 | 4.4E-04 | 1.8E-09 |
| H0La130 | 2.6E+05 | 4.0E-04 | 1.5E-09 |
| H0La303 | 2.2E+05 | 4.6E-04 | 2.1E-09 |
| H0La328 | 1.8E+05 | 5.2E-04 | 2.9E-09 |
| H0La326 | 1.4E+05 | 5.2E-04 | 3.7E-09 |

Examples of combinations of these affinity-improving mutations with the isoelectric point-lowering mutations generated in Example 7 include, for example, Ha401La402 (H chain, Ha401-SKSC/SEQ ID NO: 255; L chain, La402/SEQ ID NO: 256) and H17L11 (H chain, H17-M58/SEQ ID NO: 222; L chain, L11/SEQ ID NO: 236). Each variant was produced and purified by the method described in Example 4.

Ha401La402 (H chain, Ha401-SKSC/SEQ ID NO: 255; L chain, La402/SEQ ID NO: 256) was assessed for its affinity for NR10 and its biological activity by the method described in Referential Example 10 and the method using BaF/NR10 as described in Example 2, respectively, and they were compared to those of H0L0 (H chain, H0-SKSC/SEQ ID NO: 54; L chain, L0/SEQ ID NO: 56). The result of affinity measurement is shown in Table 10, and the biological activity determined using BaF/NR10 is shown in Fig. 21. Both affinity and biological activity were found to be improved as compared to those of H0L0 (H chain, H0-SKSC/SEQ ID NO: 54; L chain, L0/SEQ ID NO: 56).

Table 10

| | ka(1/Ms) | kd(1/s) | KD(M) |
|---|---|---|---|
| H0L0 | 2.9E+05 | 9.1E-04 | 3.2E-09 |
| Ha401La402 | 5.8E+05 | 2.9E-04 | 5.0E-10 |

Furthermore, H17L11 (H chain, H17-M58/SEQ ID NO: 222; L chain, L11/SEQ ID NO: 236) was assessed for its affinity for NR10 and its biological activity by the method described in Example 7 and the method using BaF/NR10 as described in Example 2, respectively, and they were compared to those of H0L0 (H chain, H0-M58/SEQ ID NO: 136; L chain, L0/SEQ ID NO: 56). The result of affinity measurement is shown in Table 11, and the biological activity determined using BaF/NR10 is shown in Fig. 22. Both affinity and biological activity were found to be improved as compared to those of H0L0 (H chain, H0-M58/SEQ ID NO: 136; L chain, L0/SEQ ID NO: 56).

Table 11

| | ka(1/Ms) | kd(1/s) | KD(M) |
|---|---|---|---|
| H0L0 | 1.4E+05 | 6.9E-04 | 4.8E-09 |
| H17L11 | 4.3E+05 | 2.6E-04 | 6.2E-10 |

[Example 11] Identification of mutation sites reducing immunogenicity risk Reduction of immunogenicity risk in H chain CDR1

T-cell epitopes present in the variable region sequence of H0L0 were analyzed using TEPITOPE (Methods 2004 Dec; 34(4): 468-75). As a result, CDR1 of the H chain was predicted to have many T-cell epitopes that bind to HLA (i.e. have sequences with a high immunogenicity risk). Then, TEPITOPE analysis was carried out to examine substitutions that would reduce the immunogenicity risk of the H chain CDR1. As a result, the immunogenicity risk was found to be greatly reduced by substituting isoleucine at position 33 in kabat numbering with alanine (A) (Table 12). Then, this alteration was added to H17 generated in Example 10 to produce H19 (H19-M58/SEQ ID NO: 223). The generated H19 was combined with L12 to produce H19L12 (H chain, H19-M58/SEQ ID NO: 223; L chain, L12/SEQ ID NO: 237). Each variant was produced and purified by the method described in Example 4.

The antibody was assessed for the affinity for NR10 and the biological activity by the method described in Referential Example 10 and the method using Bar/NR10 as described in Example 2, respectively, and they were compared to those of H0L0 (H chain, H0-M58/SEQ ID NO: 136; L chain, L0/SEQ ID NO: 56). The result of affinity measurement is shown in Table 13, and the biological activity determined using BaF/NR10 is shown in Fig. 23. Both affinity and biological activity were shown to be almost equal to those of H0L0.

Table 12

| Name | Type | H0 sequence | Mutation site (kabat No) | H0 sequence | Amino acid after mutation | Sequence after mutation |
|---|---|---|---|---|---|---|
| H19 | CDR1 | GYIMN (SEQ ID NO:270) | 33 | I | A | GYAMN (SEQ ID NO: 284) |

Table 13

| | ka(1/Ms) | kd(1/s) | KD(M) |
|---|---|---|---|
| H0L0 | 1.8E+05 | 8.7E-04 | 4.8E-09 |
| H19L12 | 2.3E+05 | 1.2E-03 | 5.1E-09 |

Reduction of immunogenicity risk in L chain CDR1

Threonine (T) present at kabat-numbering position 25 in CDR1 of the L chain corresponds to alanine (A) or serine (S) in the germline sequence. Thus, it is predicted that the immunogenicity risk is reduced by substituting threonine (T) at position 25 with alanine (A) or serine (S) (Table 14). Therefore, the above substitution was added to L12 to produce L17 (SEQ ID NO: 238). The produced L17 was combined with H0 to produce H0L17 (H chain, H0-M58/SEQ ID NO: 136; L chain, L17/SEQ ID NO: 238). Each variant was produced and purified by the method described in Example 4.

Each variant was assessed for the affinity for NR10 and the biological activity by the method described in Referential Example 10 and the method using BaF/NR10 as described in Example 2, respectively, and they were compared to those of H0L0 (H chain, H0-M58/SEQ ID NO: 136; L chain, L0/SEQ ID NO: 56) and H0L12 (H chain, H0-M58/SEQ ID NO: 136; L chain, L12/SEQ ID NO: 237). Since L12 contains a sequence that improves the affinity, it exhibits about two times higher affinity than H0L0. The result of affinity measurement is shown in Table 15, and the biological activity determined using BaF/NR10 is shown in Fig. 24. Both affinity and biological activity were shown to be almost equal to those of H0L12.

Table 14

| Name | Type | L0 sequence | Mutation site (kabat No) | L0 sequence | Amino acid after mutation | Sequence after mutation |
|---|---|---|---|---|---|---|
| Ld-1 | CDR1 | RTSENIYSFLA (SEQ ID NO:277) | 25 | T | A | RASENIYSFLA (SEQ ID NO:285) |
| Ld-2 | CDR1 | RTSENIYSFLA (SEQ ID NO:277) | 25 | T | S | RSSENIYSFLA (SEQ ID NO: 286) |

Table 15

| | ka(1/Ms) | kd(1/s) | KD(M) |
|---|---|---|---|
| H0L0 | 1.6E+05 | 7.8E-04 | 4.8E-09 |

(continued)

| | ka(1/Ms) | kd(1/s) | KD(M) |
|---|---|---|---|
| H0L12 | 3.8E+05 | 7.4E-04 | 2.0E-09 |
| H0L17 | 3.9E+05 | 8.1E-04 | 2.1E-09 |

[Example 12] Preparation of completely humanized NS22 antibody

Variable regions of NS22 variants were prepared by combining the multiple mutations that reduce the pI, increase the affinity, suppress the degradation of H chain, and reduce the immunogenicity risk, all of which were found in the above Examples, in H0 (H0-M58/SE ID NO: 136), H1 (H1-M58/SEQ ID NO: 257), or L0 (L0/SEQ ID NO: 56), and subjected to various screening procedures. As a result, H28L17 (H chain, H28-M58/SEQ ID NO: 224; L chain, L17/SEQ ID NO: 238), H30L17 (H chain, H30-M58/SEQ ID NO: 225; L chain, L17/SEQ ID NO: 238), H34L17 (H chain, H34-M58/SEQ ID NO: 226, L chain, L17/SEQ ID NO: 238), H42L17 (H chain, H42-M58/SEQ ID NO: 227; L chain, L17/SEQ ID NO: 238), H44L17 (H chain, H44-M58/SEQ ID NO: 228; L chain, L17/SEQ ID NO: 238), H46L17 (H chain, H46-M58/SEQ ID NO: 229; L chain, L17/SEQ ID NO: 238), H57L17 (H chain, H57-M58/SEQ ID NO: 230; L chain, L17/SEQ ID NO: 238), H71L17 (H chain, H71-M58/SEQ ID NO: 231; L chain, L17/SEQ ID NO: 238), H78L17 (H chain, H78-M58/SEQ ID NO: 232; L chain, L17/SEQ ID NO: 238), H92L17 (H chain, H92-M58/SEQ ID NO: 233; L chain, L17/SEQ ID NO: 238), H97L50 (H chain, H97-M58/SEQ ID NO: 234; L chain, L50/SEQ ID NO: 239), and H98L50 (H chain, H98-M58/SEQ ID NO: 235; L chain, L50/SEQ ID NO: 239) were found. Each variant was produced and purified by the method described in Example 4.

Each variant was assessed for the affinity for NR10 and the biological activity by the method described in Referential Example 10 and the method using BaF/NR10 as described in Example 2, respectively, and they were compared to those of H0L0 (H chain, H0-M58/SEQ ID NO: 136; L chain, L0/SEQ ID NO: 56). The result of affinity measurement is shown in Table 16, and the biological activity determined using BaF/NR10 is shown in Fig. 25-1 and 25-2. Both affinity and biological activity of each antibody were shown to be almost equal to or greater than those of H0L0.

Table 16

| Sample | ka(1/Ms) | kd(1/s) | KD(M) |
|---|---|---|---|
| H0L0 | 2.1E+05 | 8.8E-04 | 4.2E-09 |
| H28L17 | 6.4E+05 | 3.3E-04 | 5.2E-10 |
| H30L17 | 6.8E+05 | 5.7E-04 | 8.3E-10 |
| H34L17 | 3.4E+05 | 1.2E-03 | 3.6E-09 |
| H42L17 | 5.7E+05 | 3.7E-04 | 6.5E-10 |
| H44L17 | 6.1E+05 | 7.2E-04 | 1.2E-09 |
| H46L17 | 2.9E+05 | 1.3E-03 | 4.6E-09 |
| H57L17 | 7.1E+05 | 5.5E-04 | 7.7E-10 |
| H71L17 | 3.7E+05 | 1.2E-03 | 3.3E-09 |
| H78L17 | 6.1E+05 | 7.0E-04 | 1.1E-09 |
| H92L17 | 3.1E+05 | 1.3E-03 | 4.1E-09 |
| H97L50 | 3.6E+05 | 1.3E-03 | 3.5E-09 |
| H98L50 | 2.9E+05 | 1.3E-03 | 4.6E-09 |

[Example 13] Analysis of the binding domain of anti-NR10 neutralizing antibody (1) Preparation of human/mouse wild-type and chimeric antigens

The genes encoding human and mouse wild-type extracellular domains and chimeric extracellular domains of NR10 (hhh (SEQ ID NO: 258), mmm (SEQ ID NO: 259), hhm (SEQ ID NO: 260), mmh (SEQ ID NO: 261), hmm (SEQ ID NO: 262), mhm (SEQ ID NO: 263), and mhh (SEQ ID NO: 264)), were fused to His tag and Myc tag (HHHHHHEQKLISEEDL/SEQ ID NO: 287) at their C termini, inserted into an animal expression vector, and transiently expressed using FreeStyle 293 Expression System (Invitrogen™). Schematic diagrams for the human/mouse wild-type and chimeric NR10-ECDs are shown in Fig. 26.

The human/mouse wild-type and chimeric antigens (hhh, mmm, hhm, mmh, hmm, mhm, and mhh) were purified from culture supernatants by Ni-NTA Superflow column chromatography. Specifically, 1 ml of Ni-NTA Superflow (QIAGEN) was loaded onto Poly-Prep Empty Column (BioRad), and 30 ml of each culture supernatant was added thereto. After washing with D-PBS (Dulbecco's phosphate-buffered saline) containing 150 mM sodium chloride and 20 mM imidazole, the column was eluted with D-PBS containing 150 mM sodium chloride and 250 mM imidazole. The eluted fractions were buffer-exchanged with D-PBS and concentrated using Amicon-Ultra (Millipore) with a molecular weight cut-off of 10K.

(2) Detection of binding antigen by Western blotting

Each of the prepared human/mouse wild-type and chimeric antigens was electrophoresed at 0.5 μg/lane on three 4-20% polyacrylamide gels (Daiichi Pure Chemicals Co.). The proteins were electro-transferred onto PVDF membranes (Millipore) in a semi-dry blotting apparatus, and the membranes were blocked with TBS containing 5% skim milk. One membrane was incubated with 5 μg/ml of H44M58L17 (detection system for humanized anti-human NR10 antibody); another with 5 μg/ml of ND41 (detection system for mouse anti-human NR10 antibody); and the other one with anti-Myc antibody (SantaCruz, Cat.#sc-789) 500-times diluted with TBS containing 5% skim milk (detection system for Myc tag) at room temperature for one hour.

After washing three times with TBS containing 0.05% Tween™ 20, the secondary antibodies were incubated with the membranes. Alkaline phosphatase-labeled goat anti-human IgGγ (BIOSOURCE, Cat. #AHI0305) was used to detect humanized anti-human NR10 antibody; alkaline phosphatase-labeled goat anti-mouse IgG (SantaCruz, Cat. #sc-2008) was used to detect mouse anti-human NR10 antibody; and alkaline phosphatase-labeled goat anti-rabbit IgG (SantaCruz, Cat. #sc-2057) was used to detect Myc tag. The reaction was carried out at room temperature for one hour. After washing four times with TBS containing 0.05% Tween™ 20 for three minutes, color development was carried out using BCIP/NBT Phosphatase substrate, 1-Component System (KPL). TBS (Tris-buffered saline) used here was prepared by dissolving a pack of TBS (Tris buffered saline) powder (TaKaRa) in 1 L of distilled water. The result is shown in Fig. 27.

When the humanized antibody or mouse antibody was used, the binding was detected only for hhh, hhm, and hmm, which are NR10 extracellular domains.

[Referential Example 1] Isolation of cynomolgus monkey NR10, OSMR, and IL-31 genes

Since the cross-reactivity and neutralizing activity in cynomolgus monkeys were considered important for safety assessment at a pre-clinical stage, the cynomolgus monkey NR10 and OSMR genes were isolated. Primers were designed based on published information of Rhesus monkey genome and others, and the NR10 and OSMR genes were successfully amplified by PCR from cynomolgus monkey pancreatic cDNA. The sequences of the isolated cynomolgus monkey NR10, OSMR, and IL-31 genes are shown in SEQ ID NOs: 65, 69, and 67, respectively, and the amino acid sequences of cynomolgus monkey NR10, OSMR, and IL-31 are shown in SEQ ID NOs: 66, 70, and 68, respectively.

[Referential Example 2] Establishment of NR10- and OSMR-expressing Ba/F3 cell lines

The full-length human NR10 cDNA (SEQ ID NO: 75) was inserted into the expression vector pCOS1 (Biochem. Biophys. Res. Commun. 228, p838-45, 1996), and the resulting vector was named pCosNR10.3. An oncostatin M receptor cDNA (OSMR, GenBank accession No. NM003999) was isolated by PCR from a human placental library, and the expression vector pCos1-hOSMR was constructed in the same manner. 10 μg each of the vectors were simultaneously introduced into mouse IL-3-dependent pro-B cell-derived cell line Ba/F3 by electroporation (BioRad Gene Pulser, 960 μF, 0.33 kV). After introduction, human IL-31 (R&D Systems) was added, and the cells were cultured to obtain a cell line (hNR10/hOSMR/BaF3 cell) that proliferates in an IL-31-dependent manner. Furthermore, the cynomolgus monkey IL-31 gene (SEQ ID NO: 67) was inserted into a mammalian cell expression vector and introduced into CHO cell line DG44. The resulting culture supernatant was obtained as cynomolgus monkey IL-31. As with hNR10/hOSMR/BaF3, the full-length cynomolgus monkey NR10 and OSMR genes were inserted into the expression vector pCOS1 and expressed in Ba/F3 cells, and a cynomolgus monkey IL-31-dependent cell line (cynNR10/cynOSMR/BaF3 cell) was established using the culture supernatant described above.

[Referential Example 3] Establishment of NR10-expressing CHO cell lines

The genes for cytoplasmic domain-lacking human NR10 (SEQ ID NO: 73) and cytoplasmic domain-lacking cynomolgus monkey NR10 (SEQ ID NO: 71) were each inserted to a mammalian cell expression vector. The resulting vectors were linearized with a restriction enzyme, and then introduced into CHO cell line DG44 by electroporation (BioRad Gene Pulser, 25 μF, 1.5 kV). After drug selection, NR10-expressing cells were selected and established by FCM analysis using anti-human NR10 antibody. The amino acid sequence encoded by the nucleotide sequence of cytoplasmic domain-lacking human NR10 gene (SEQ ID NO: 73) is shown in SEQ ID NO: 74, and the amino acid sequence encoded by the nucleotide sequence of cytoplasmic domain-lacking cynomolgus monkey NR10 gene (SEQ ID NO: 71) is shown in SEQ ID NO: 72.

[Referential Example 4] Preparation of NR10 protein (extracellular domain)

The human NR10 cDNA was used as a template to amplify only the extracellular domain by PCR. The amplified region was then fused to a FLAG tag sequence at the C terminus and inserted to a mammalian cell expression vector. Ten μg of the linearized vector was introduced into Chinese hamster ovary cell line DG44 by electroporation (BioRad Gene PulserII, 25 μF, 1.5 kV). A cell line showing high level expression was obtained. The supernatant of the cell line cultured on a large scale was purified using anti-FLAG antibody column (Sigma) and gel filtration to obtain soluble NR10. The nucleotide sequence of soluble NR10 is shown in SEQ ID NO: 77, and the amino acid sequence is shown in SEQ ID NO: 78.

[Referential Example 5] Preparation of anti-human NR10 antibodies

Mice were immunized with human NR10 protein (extracellular domain) (described in Referential Example 4), and hybridomas were prepared by a conventional method. The culture supernatants of these hybridomas were assessed for the neutralizing activity using the human IL-31-dependent cell line (hNR10/hOSMR/BaF3 cell) described in Referential Example 2, and thereby NA633 which has an NR10-neuralizing activity was obtained.

Furthermore, DNA immunization was carried out by He gas-driven gene gun using a mammalian expression vector carrying the full-length human NR10 gene (SEQ ID NO: 75), and hybridomas were prepared by a conventional method. The culture supernatants of these hybridomas were assessed for the neutralizing activity using the human IL-31-dependent cell line (hNR10/hOSMR/BaF3 cell) described in Referential Example 2, and thereby ND41 which has an NR10-neuralizing activity was obtained.

[Referential Example 6] Preparation of human chimeric antibody

The amino acid sequences of heavy chain and light chain variable regions of NA633 are shown in SEQ ID NOs: 104 and 108, respectively. The amino acid sequences of CDR1, CDR2, and CDR3 of the heavy chain variable region of NA633 are shown in SEQ ID NOs: 105, 106, and 107, respectively, while those of CDR1, CDR2, and CDR3 of the light chain variable region are shown in SEQ ID NOs: 109, 110, and 111, respectively. Furthermore, a chimeric antibody between these mouse variable regions and human constant region (H chain, γl; L chain, κ) was produced by a conventional method.

[Referential Example 7] Preparation of huPM1-SKSC in which the heterogeneity of wild type IgG2 is reduced without loss of stability

Since the NS22 antibody is an NR10-neutralizing antibody, its binding to Fcγ receptor may be unfavorable in consideration of the immunogenicity and adverse effects. A possible method for reducing the binding to Fcγ receptor is to select IgG2 or IgG4 instead of IgG1 as the isotype of the constant region (Ann Hematol. 1998 Jun; 76(6): 231-48.). From the viewpoint of Fcγ receptor I and retention in plasma, IgG2 has been considered more desirable than IgG4 (Nat Biotechnol. 2007 Dec; 25(12): 1369-72). Meanwhile, when an antibody is developed as a pharmaceutical, properties of the protein, particularly homogeneity and stability, are highly important. The IgG2 isotype has been reported to have very high heterogeneity resulting from the disulfide bonds in the hinge region (J Biol Chem. 2008 Jun 6; 283(23): 16206-15.). It is not easy and would be more costly to manufacture it as pharmaceutical in a large scale while maintaining difference in the heterogeneity of desired/related substances among products resulting from the above. Accordingly, it is desired that the substance be composed of a single substance as much as possible. Thus, when antibodies of IgG2 isotype are developed as pharmaceuticals, it is preferred to reduce the heterogeneity resulting from disulfide bonds, without lowering the stability.

In order to reduce the heterogeneity of the wild type IgG2, cysteines in the hinge region and CH1 domain of IgG2 were substituted. As a result of examination of various variants, SKSC (SEQ ID NO: 62), which is a constant region obtained by altering cysteine at position 131 and arginine at position 133 in the EU numbering (Sequences of proteins of immunological interest, NIH Publication No.91-3242) within the H-chain CH1 domain of the wild type IgG2 constant region sequence to serine and lysine, respectively, and altering cysteine at EU-numbering position 219 in the H-chain upper hinge to serine could reduce the heterogeneity without decreasing the stability. Meanwhile, other possible methods for decreasing heterogeneity are to alter only cysteine at EU-numbering position 219 in the H-chain upper hinge to serine, and to alter only cysteine at EU-numbering position 220 to serine. Thus, constant region SC (SEQ ID NO: 153) in which cysteine at EU-numbering position 219 in IgG2 has been altered to serine, and constant region CS (SEQ ID NO: 154) in which cysteine at EU-numbering position 220 in IgG2 has been altered to serine, were produced.

huPM1-SC (SEQ ID NO: 157), huPM1-CS (SEQ ID NO: 158), huPM1-IgG1 (SEQ ID NO: 159), huPM1-IgG2 (SEQ ID NO: 160), and huPM1-SKSC (SEQ ID NO: 161), which were prepared by combining the constant regions produced as above, IgG1 (SEQ ID NO: 60), and IgG2 (SEQ ID NO: 132) with the variable region of the humanized anti-IL-6 receptor antibody (H chain variable region, huPM1-VH/SEQ ID NO: 155; L chain variable region huPM1-VL/SEQ ID NO: 156)(Cancer Res. 1993 Feb 15;53(4): 851-6.), were used as an H chain, and huPM1-L (SEQ ID NO: 162) was used as an L chain, to produce each antibody. Each antibody was expressed and purified by the method described in Example 4.

The antibodies were compared to each other in terms of the heterogeneity. The heterogeneity of huPM1-IgG1, huPM1-IgG2, huPM1-SC, huPM1-CS, and huPM1-SKSC was assessed by cation exchange chromatography. The chromatography was carried out using a ProPac WCX-10 (Dionex) column, 20 mM sodium acetate (pH 5.0) as mobile phase A, and 20 mM sodium acetate/1M NaCl (pH 5.0) as mobile phase B, with an appropriate flow rate and gradient. The result of assessment by cation exchange chromatography is shown in Fig. 12.

As shown in Fig. 12, conversion of the constant region from IgG1 into IgG2 increased the heterogeneity. In contrast, the heterogeneity was markedly reduced by converting the constant region into SKSC. While constant region SC resulted in considerable reduction of the heterogeneity as in SKSC, constant region CS did not sufficiently improve the heterogeneity.

When an antibody is developed as a pharmaceutical, it is generally desired that the antibody have high stability in addition to low heterogeneity for the production of stable preparations. Thus, to assess the stability, the thermal denaturation midpoint temperature (Tm value) was determined by differential scanning calorimetry (DSC) (VP-DSC; Microcal). The thermal denaturation midpoint temperature (Tm value) serves as an indicator of stability. In order to prepare stable preparations as pharmaceuticals, a higher thermal denaturation midpoint temperature (Tm value) is preferred (J Pharm Sci. 2008 Apr; 97(4): 1414-26.). Thus, huPM1-IgG1, huPM1-IgG2, huPM1-SC, huPM1-CS, and huPM1-SKSC were dialyzed against a solution of 20 mM sodium acetate/150 mM NaCl (pH 6.0) (EasySEP; TOMY), and DSC measurement was carried out using about 0.1 mg/ml of protein at a heating rate of 1°C/min between 40 and 100°C. The denaturation curves obtained by DSC are shown in Fig. 13. The Tm values of the Fab domains are listed in Table 17 below.

Table 17

| Name | Tm/°C |
|---|---|
| huPM1-IgG1 | 94.8 |
| huPM1-IgG2 | 93.9 |
| huPM1-SC | 86.7 |
| huPM1-CS | 86.4 |
| huPM1-SKSC | 93.7 |

The Tm values of huPM1-IgG1 and huPM1-IgG2 were almost the same, namely, about 94°C (IgG2 was lower by about 1°C). Meanwhile, the Tm values of huPM1-SC and huPM1-CS were about 86°C, which was significantly lower than those of huPM1-IgG1 and huPM1-IgG2. On the other hand, the Tm value of huPM1-SKSC was about 94°C, and almost the same as huPM1-IgG1 and huPM1-IgG2. Since the stability of huPM1-SC and huPM1-CS was markedly lower than that of IgG2, huPM1-SKSC in which cysteine in the CH1 domain have also been altered to serine may be more preferred in the development of pharmaceuticals. The significant decrease in Tm value of huPM1-SC and huPM1-CS as compared to IgG2 may be due to the disulfide-bonding pattern of huPM1-SC and huPM1-CS that is different from that of IgG2.

Furthermore, comparison of the DSC denaturation curves showed that the denaturation peak for the Fab domain was sharp in huPM1-IgG1 and huPM1-SKSC, while it was broader in huPM1-SC and huPM1-CS than the above two, and huPM1-IgG2 gave a shoulder peak on the lower temperature side of the Fab domain denaturation peak. The denaturation peak in DSC generally becomes sharp in the case of a single component, but may become broad when two or more components with different Tm values (namely, heterogeneity) are present. Thus, it was suggested that huPM1-IgG2, huPM1-SC, and huPM1-CS contained two or more components, and the heterogeneity of natural IgG2 was not reduced in huPM1-SC and huPM1-CS. This finding suggests that cysteines present in both the hinge region and the CH1 domain are involved in the heterogeneity of natural IgG2, and it is necessary to alter not only cysteine in the hinge region but also that in the CH1 domain to decrease the heterogeneity on DSC. Furthermore, as described above, it is only possible to attain stability equivalent to that of natural IgG2 by altering not only cysteine in the hinge region but also that in the CH1 domain.

As described above, as to the constant regions in which the heterogeneity resulting from the hinge region of IgG2 has been reduced, it was discovered that SC and CS, which are constant regions in which only cysteine in the hinge region has been substituted with serine, may be insufficient from the viewpoint of heterogeneity and stability, and that it is only possible to significantly reduce the heterogeneity while maintaining the stability comparable to IgG2 by additionally substituting cysteine at EU-numbering position 131 in the CH1 domain with serine. Such constant regions include SKSC.

[Referential Example 8] Production and assessment of optimized, non-Fcγ receptor-binding constant region M14

In the Fcγ receptor-binding domain of IgG2 constant region, the residues at EU-numbering positions 233, 234, 235, and 236 are of non-binding type, while the residues at EU-numbering positions 327, 330, and 331 are different from those of IgG4, which are of non-binding type. Thus, it is necessary to alter the amino acids at EU-numbering positions 327, 330, and 331 to the sequence of IgG4 (G2Δa in Eur J Immunol. 1999 Aug; 29(8):2613-24). However, since the amino acid at EU-numbering position 339 is alanine in IgG4 while it is threonine in IgG2, mere alteration of the amino acids at EU-numbering positions 327, 330, and 331 to the sequence of IgG4 will generate a novel non-naturally occurring 9-amino acid peptide sequence that could be a T-cell epitope peptide, thereby causing a risk of immunogenicity. Thus, it was found that the occurrence of the novel peptide sequence could be prevented by altering threonine at EU-numbering position 339 in IgG2 to alanine, in addition to the alterations described above. In addition to the mutations described above, methionine at EU-numbering position 397 was mutated into valine to improve the stability of IgG2 under acidic condition. Furthermore, in SKSC (SEQ ID NO: 62) produced in Referential Example 7, in which the heterogeneity resulting from the disulfide bonds in the hinge region has been improved, introduction of mutations at positions 131 and 133 will generate a novel non-naturally occurring 9-amino acid peptide sequence that could be a T-cell epitope peptide, thereby causing a risk of immunogenicity. Thus, the peptide sequence around positions 131 to 139 was converted into the same as IgG1 by mutating glutamic acid at EU-numbering position 137 into glycine and mutating serine at EU-numbering position 138 into glycine. The constant region sequence M14 (SEQ ID NO: 129) was produced by introducing all the above mutations.

The expression and purification of huPM1-M14, prepared by using huPM1-M14 as an H chain and huPM1-L (SEQ ID NO: 162) as an L chain, was carried out by the method described in Referential Example 7. The prepared huPM1-M14 (SEQ ID NO: 163), huPM1-IgG1, and huPM1-IgG2 were assessed for the heterogeneity using cation exchange chromatography by the method described in Referential Example 7.

As shown in Fig. 14, the heterogeneity was also reduced in huPM1-M14 as in huPM1-SKSC.

[Referential Example 9] Preparation of huPM1-M58 with reduced H-chain C-terminal heterogeneity and improved pharmacokinetics

Preparation of huPM1-M58 molecule

huPM1 is an IgG1 antibody. For the heterogeneity in the C-terminal sequence of the H chain of IgG antibody, the deletion of the C-terminal lysine residue and the amidation of the C-terminal amino group due to deletion of the two C-terminal amino acids, glycine and lysine, have been reported (Anal Biochem. 2007 Jan 1;360(1): 75-83). Also in huPM1, while the major component is a sequence in which the C-terminal lysine encoded by the nucleotide sequence has been deleted by post-translational modification, there are also a minor component in which the lysine remains and a minor component in which the C-terminal amino group is amidated due to deletion of both glycine and lysine, which contribute to heterogeneity. Producing a pharmaceutical in a large scale while maintaining the difference in the heterogeneity of desired/related substances between products is not easy but rather results in increase of cost, and it is thus desired that the substance be composed of a single substance as much as possible. When an antibody is developed as a pharmaceutical, reduction of the heterogeneity is desired. Thus, it is desired that the C-terminal of the H chain has no heterogeneity when developed as pharmaceuticals. It is also desirable to prolong the plasma half-life of the antibody in order to reduce the antibody dose.

Thus, the alterations described below were introduced to prepare a novel constant region in which the heterogeneity at C-terminal of the H chain has been reduced, the pharmacokinetics has been improved as compared to huPM1-IgG1, and the heterogeneity derived from wild-type IgG2 has also been reduced without loss of stability.

Specifically, in huPM1-SKSC, which has high stability and in which the above-mentioned heterogeneity related to antibodies with IgG2-isotype constant regions is reduced, glutamic acid at EU-numbering position 137 was substituted with glycine; serine at position 138 with glycine; histidine at position 268 with glutamine; arginine at position 355 with glutamine; and glutamine at position 419 with glutamic acid. In addition to the above substitutions, glycine and lysine at positions 446 and 447 were deleted to reduce the heterogeneity of the H-chain C terminus, thereby obtaining huPM1-M58 (SEQ ID NO: 164). huPM1-M58 prepared by using huPM1-M58 as an H chain and huPM1-L (SEQ ID NO: 162) as an L chain was expressed and purified by the method described in Example 4.

The huPM1-M58, huPMI-IgG1, and huPM1-IgG2 were assessed for the heterogeneity and stability by the methods described in Example 5 using cation exchange chromatography and DSC, respectively.

The result of DSC is shown in Table 18. As shown in Figs. 13 and 16, huPM1-M58 was found to show reduced heterogeneity without loss of stability as in huPM1-SKSC.

Table 18

| Name | Tm/°C |
|---|---|
| huPM1-IgG1 | 94.8 |
| nuPM1-IgG2 | 93.9 |
| huPM1-SKSC | 93.7 |
| huPM1-M58 | 93.7 |

Assessment of huPM1-M58 for plasma retention

The prolonged retention (slow elimination) of IgG molecule in plasma is due to the function of FcRn, which is known as a salvage receptor of IgG molecule (Nat Rev Immunol. 2007 Sep;7(9): 715-25). When incorporated into endosomes via pinocytosis, IgG molecules bind to FcRn expressed in endosomes under the acidic conditions within the endosome (approx. pH 6.0). While IgG molecules that are not bound to FcRn are transferred to and degraded in lysosomes, those bound to FcRn are translocated to the cell surface and then released from FcRn into plasma again under the neutral conditions in plasma (approx. pH 7.4).

IgG-type antibodies are known to include IgG1, IgG2, IgG3, and IgG4 isotypes. The plasma half-lives of these isotypes in human are reported to be about 36 days for IgG1 and IgG2; about 29 days for IgG3; and 16 days for IgG4 (Nat. Biotechnol. 2007 Dec; 25(12): 1369-72). Thus, the retention of IgG1 and IgG2 in plasma is believed to be the longest. In general, the isotypes of antibodies used as pharmaceutical agents are IgG1, IgG2, and IgG4. Reported methods for further improving the pharmacokinetics of these IgG antibodies include methods for improving the above-described binding activity to human FcRn by altering the sequence of IgG constant region (J. Biol. Chem. 2007 Jan 19;282(3): 1709-17; J. Immunol. 2006 Jan 1;176(1): 346-56).

There are species differences between mouse FcRn and human FcRn (Proc. Natl. Acad. Sci. USA. 2006 Dec 5;103(49): 18709-14). Therefore, to predict the retention of IgG antibodies having an altered constant region sequence in human plasma, it may be desirable to assess the binding to human FcRn and the plasma retention in human FcRn transgenic mice (Int. Immunol. 2006 Dec;18(12): 1759-69).

Assessment of the binding to human FcRn

FcRn is a complex of FcRn and β2-microglobulin. Oligo-DNA primers were prepared based on the published human FcRn gene sequence (J. Exp. Med. (1994) 180 (6), 2377-2381). A DNA fragment encoding the whole gene was prepared by PCR using human cDNA (Human Placenta Marathon-Ready cDNA, Clontech) as a template and the prepared primers. Using the obtained DNA fragment as a template, a DNA fragment encoding the extracellular domain containing the signal region (Met1-Leu290) was amplified by PCR, and inserted into an animal cell expression vector (the amino acid sequence of human FcRn/SEQ ID NO: 165). Likewise, oligo-DNA primers were prepared based on the published human β2-microglobulin gene sequence (Proc. Natl. Acad. Sci. U.S.A. 99 (26), 16899-16903 (2002)). A DNA fragment encoding the whole gene was prepared by PCR using human cDNA (Hu-Placenta Marathon-Ready cDNA, CLONTECH) as a template and the prepared primers. Using the obtained DNA fragment as a template, a DNA fragment encoding the whole β2-microglobulin containing the signal region (Met1-Met119) was amplified by PCR and inserted into an animal cell expression vector (the amino acid sequence of human β2-microglobulin/SEQ ID NO: 166).

Soluble human FcRn was expressed by the following procedure. The prepared plasmids for human FcRn and β2-microglobulin were introduced into the human embryonic kidney cancer-derived cell line HEK293H (Invitrogen) using 10% fetal bovine serum (Invitrogen) by lipofection. The resulting culture supernatant was collected and purified using IgG Sepharose 6 Fast Flow (Amersham Biosciences) by the method described in J. Immunol. 2002 Nov 1;169(9):5171-80. Then further purification was carried out using HiTrap Q HP (GE Healthcare).

The binding to human FcRn was assessed using Biacore 3000. An antibody was bound to Protein L or rabbit anti-human IgG Kappa chain antibody immobilized onto a sensor chip, human FcRn was added as an analyte for interaction with the antibody, and the affinity (KD) was calculated from the amount of bound human FcRn. Specifically, Protein L was immobilized onto sensor chip CM5 (BIACORE) by the amine coupling method using 50 mM Na-phosphate buffer (pH 6.0) containing 150 mM NaCl as the running buffer. Then, an antibody was diluted with the running buffer containing 0.02% Tween20, and injected and allowed to bind to the chip. Human FcRn was then injected to assess the binding activity of the antibody to the human FcRn.

The affinity was calculated using BIAevaluation software. The obtained sensorgram was used to calculate the amount of hFcRn bound to the antibody immediately before the end of human FcRn injection. This was fitted by the steady state affinity method to calculate the affinity of human FcRn for the antibody.

Predictive assessment of plasma retention of huPM1-IgG1 and huPM1-M58 in human using human FcRn

The binding activities of huPM1-IgG1 and huPM1-M58 to human FcRn were assessed using BIAcore. As shown in Table 19, the binding activity of huPM1-M58 was greater than that of huPM1-IgG1 by about 1.4 times.

Table 19

| | KD($\mu$M) |
|---|---|
| huPM1-IgG1 | 1.62 |
| huPM1-M58 | 1.17 |

Assessment of the plasma retention in human FcRn transgenic mice

The pharmacokinetics in human FcRn transgenic mice (B6.mFcRn-/-.hFcRn Tg line 276 +/+ mice; Jackson Laboratories) was assessed by the following procedure. An antibody was intravenously administered once at a dose of 1 mg/kg to mice, and blood was collected at appropriate time points. The collected blood was immediately centrifuged at 15,000 rpm for 15 minutes at 4°C to obtain plasma. The separated plasma was stored in a freezer at -20°C or below until use. The plasma concentration was determined by ELISA.

Predictive assessment of the plasma retention of huPM1-IgG1 and huPM1-M58 in human using human FcRn transgenic mice

The plasma retention of huPM1-IgG1 and huPM1-M58 in human FcRn transgenic mice was assessed. As shown in Fig.17, the result demonstrated that the pharmacokinetics of huPM1-M58 was improved as compared to huPM1-IgG1. It was suggested that the human FcRn-binding activity was correlated to the plasma retention in human FcRn transgenic mice.

[Referential Example 10] Measurement of the affinity in antigen-antibody reaction using Biacore

Kinetic analysis of the antigen-antibody reaction was carried out using Biacore T100 (GE Healthcare Biosciences). The antigen-antibody interaction was measured by immobilizing rec-Protein A (hereinafter Protein A) (ZYMED) onto a sensor chip, capturing an antibody on the immobilized Protein A, and then reacting the antigen as an analyte. Various concentrations of rhNR10 were used as the antigen. The kinetic parameters, association rate constant $k_a$ (1/Ms) and dissociation rate constant $k_d$ (1/s), were calculated from the sensorgrams obtained by the measurement. Then, $K_D$ (M) was determined based on the rate constants. Each parameter was determined using Biacore T100 Evaluation Software version 1.1 (GE Healthcare Biosciences).

Immobilization of Protein A onto sensor chip

Protein A was immobilized onto all flow cells of sensor chip CM5 (GE Healthcare Bioscinences) by the amine coupling method. The experiment was carried out using HBS-EP+ (10 mM HEPES, 0.15 M NaCl, 3 mM EDTA, 0.05% v/v Surfactant P20) as a running buffer at a flow rate of 10 $\mu$L/min. The carboxyl groups of carboxymethyl dextran on the sensor chip were activated with 100 $\mu$L of a 1:1 mixture of 75 mg/ml EDC (N-ethyl-N'-(3-dimethylaminopropyl)carbodiimide hydrochloride) and 11.5 mg/ml NHS (N-hydroxysuccinimide), and Protein A prepared at 50 $\mu$g/ml using 10 mM acetate buffer (pH 4.5) was allowed to flow for reaction. Then, 100 $\mu$L of 1 M ethanolamine hydrochloride (pH 8.5) was allowed to flow to inactivate the unreacted active groups. Ultimately, about 4000 to 5000 RU were immobilized. The experiment was carried out at 25°C at all times.

Measurement of affinity in antigen-antibody reaction between rhNR10 and antibody captured on Protein A

The running buffer used was HBS-EP+. Each antibody was prepared at 0.25 $\mu$g/ml, or prepared so that about 100 RU would bind to Protein A. rhNR10 used as an analyte was prepared at 0, 38.5, 77.0, and 154 nM, or at 0,19.25, and 77.01 nM using HBS-EP+. In the measurement, first, the antibody solution was captured on Protein A, and an analyte solution was reacted at a flow rate of 20 μL/min for three minutes. Then, the solution was switched to HBS-EP+, and the dissociation phase was measured for five minutes. After measurement of the dissociation phase, the sensor chip was regenerated by washing with 10 mM glycine-HCl (pH 1.5). The obtained sensorgrams were kinetically analyzed using the Biacore-specific data analysis software, Biacore T100 Evaluation Software Version 1.1.

Industrial Applicability

The anti-NR10 antibodies obtained by the present inventors exhibit an effective neutralizing activity against NR10, and are useful as, for example, therapeutic agents for inflammatory diseases.
Furthermore, the present invention relates to the following items:

1. An antibody or antibody variable region which is any one of:

(1) a heavy chain variable region comprising CDR1 of SEQ ID NO: 196, CDR2 of SEQ ID NO: 197, and CDR3 of SEQ ID NO: 11 (H17);
(2) a heavy chain variable region comprising CDR1 of SEQ ID NO: 176, CDR2 of SEQ ID NO: 197, and CDR3 of SEQ ID NO: 11 (H19);
(3) a heavy chain variable region comprising CDR1 of SEQ ID NO: 196, CDR2 of SEQ ID NO: 197, and CDR3 of SEQ ID NO: 184 (H28, H42);
(4) a heavy chain variable region comprising CDR1 of SEQ ID NO: 9, CDR2 of SEQ ID NO: 197, and CDR3 of SEQ ID NO: 184 (H30, H44);
(5) a heavy chain variable region comprising CDR1 of SEQ ID NO: 176, CDR2 of SEQ ID NO: 197, CDR3 of SEQ ID NO: 184 (H34, H46);
(6) a heavy chain variable region comprising CDR1 of SEQ ID NO: 9, CDR2 of SEQ ID NO: 198, and CDR3 of SEQ ID NO: 184 (H57, H78);
(7) a heavy chain variable region comprising CDR1 of SEQ ID NO: 176, CDR2 of SEQ ID NO: 198, and CDR3 of SEQ ID NO: 184 (H71, H92);
(8) a heavy chain variable region comprising CDR1 of SEQ ID NO: 9, CDR2 of SEQ ID NO: 199, and CDR3 of SEQ ID NO: 184 (H97, H98);
(9) a light chain variable region comprising CDR1 of SEQ ID NO: 200, CDR2 of SEQ ID NO: 170, and CDR3 of SEQ ID NO: 193 (L11);
(10) a light chain variable region comprising CDR1 of SEQ ID NO: 201, CDR2 of SEQ ID NO: 170, and CDR3 of SEQ ID NO: 193 (L12);
(11) a light chain variable region comprising CDR1 of SEQ ID NO: 202, CDR2 of SEQ ID NO: 170, and CDR3 of SEQ ID NO: 193 (L17);
(12) a light chain variable region comprising CDR1 of SEQ ID NO: 203, CDR2 of SEQ ID NO: 170, and CDR3 of SEQ ID NO: 193 (L50);
(13) an antibody comprising the heavy chain variable region of (3) and the light chain variable region of (11);
(14) an antibody comprising the heavy chain variable region of (4) and the light chain variable region of (11);
(15) an antibody comprising the heavy chain variable region of (5) and the light chain variable region of (11);
(16) an antibody comprising the heavy chain variable region of (6) and the light chain variable region of (11);
(17) an antibody comprising the heavy chain variable region of (7) and the light chain variable region of (11);
(18) an antibody comprising the heavy chain variable region of (8) and the light chain variable region of (12);
(19) an antibody in which one or more amino acids are substituted, deleted, added, and/or inserted in the antibody of any one of (13) to (18), which has an activity equivalent to that of the antibody of any one of (13) to (18); and
(20) an antibody which binds to the same epitope as an epitope bound by the antibody of any one of (13) to (18).

2. An antibody or antibody variable region which is any one of:

(1) a heavy chain variable region comprising the amino acid sequence of SEQ ID NO: 204 (H17);
(2) a heavy chain variable region comprising the amino acid sequence of SEQ ID NO: 205 (H19);
(3) a heavy chain variable region comprising the amino acid sequence of SEQ ID NO: 206 (H28);
(4) a heavy chain variable region comprising the amino acid sequence of SEQ ID NO: 207 (H30);
(5) a heavy chain variable region comprising the amino acid sequence of SEQ ID NO: 208 (H34),
(6) a heavy chain variable region comprising the amino acid sequence of SEQ ID NO: 209 (H42);

(7) a heavy chain variable region comprising the amino acid sequence of SEQ ID NO: 210 (H44);
(8) a heavy chain variable region comprising the amino acid sequence of SEQ ID NO: 211 (H46);
(9) a heavy chain variable region comprising the amino acid sequence of SEQ ID NO: 212 (H57);
(10) a heavy chain variable region comprising the amino acid sequence of SEQ ID NO: 213 (H71);
(11) a heavy chain variable region comprising the amino acid sequence of SEQ ID NO: 214 (H78);
(12) a heavy chain variable region comprising the amino acid sequence of SEQ ID NO: 215 (H92);
(13) a heavy chain variable region comprising the amino acid sequence of SEQ ID NO: 216 (H97);
(14) a heavy chain variable region comprising the amino acid sequence of SEQ ID NO: 217 (H98);
(15) a light chain variable region comprising the amino acid sequence of SEQ ID NO: 218 (L11);
(16) a light chain variable region comprising the amino acid sequence of SEQ ID NO: 219 (L12);
(17) a light chain variable region comprising the amino acid sequence of SEQ ID NO: 220 (L17);
(18) a light chain variable region comprising the amino acid sequence of SEQ ID NO: 221 (L50);
(19) an antibody comprising the heavy chain variable region of (3) and the light chain variable region of (17) (H28L17);
(20) an antibody comprising the heavy chain variable region of (4) and the light chain variable region of (17) (H30L17);
(21) an antibody comprising the heavy chain variable region of (5) and the light chain variable region of (17) (H34L17);
(22) an antibody comprising the heavy chain variable region of (6) and the light chain variable region of (17) (H42L17);
(23) an antibody comprising the heavy chain variable region of (7) and the light chain variable region of (17) (H44L17);
(24) an antibody comprising the heavy chain variable region of (8) and the light chain variable region of (17) (H46L17);
(25) an antibody comprising the heavy chain variable region of (9) and the light chain variable region of (17) (H57L17);
(26) an antibody comprising the heavy chain variable region of (10) and the light chain variable region of (17) (H71L17);
(27) an antibody comprising the heavy chain variable region of (11) and the light chain variable region of (17) (H78L17);
(28) an antibody comprising the heavy chain variable region of (12) and the light chain variable region of (17) (H92L17);
(29) an antibody comprising the heavy chain variable region of (13) and the light chain variable region of (18) (H97L50);
(30) an antibody comprising the heavy chain variable region of (14) and the light chain variable region of (18) (H98L50),
(31) an antibody in which one or more amino acids are substituted, deleted, added, and/or inserted in the antibody of any one of (19) to (30), which has an activity equivalent to that of the antibody of any one of (19) to (30); and
(32) an antibody which binds to the same epitope as an epitope bound by the antibody of any one of (19) to (30).

3. The anti-NR10 antibody of item 1 or 2, which is a humanized antibody.

4. An antibody, antibody heavy chain, or antibody light chain, which is any one of:

(1) a heavy chain comprising the amino acid sequence of SEQ ID NO: 222 (H17);
(2) a heavy chain comprising the amino acid sequence of SEQ ID NO: 223 (H19);
(3) a heavy chain comprising the amino acid sequence of SEQ ID NO: 224 (H28);
(4) a heavy chain comprising the amino acid sequence of SEQ ID NO: 225 (H30);
(5) a heavy chain comprising the amino acid sequence of SEQ ID NO: 226 (H34);
(6) a heavy chain comprising the amino acid sequence of SEQ ID NO: 227 (H42);
(7) a heavy chain comprising the amino acid sequence of SEQ ID NO: 228 (H44);
(8) a heavy chain comprising the amino acid sequence of SEQ ID NO: 229 (H46);
(9) a heavy chain comprising the amino acid sequence of SEQ ID NO: 230 (H57);
(10) a heavy chain comprising the amino acid sequence of SEQ ID NO: 231 (H71);
(11) a heavy chain comprising the amino acid sequence of SEQ ID NO: 232 (H78);
(12) a heavy chain comprising the amino acid sequence of SEQ ID NO: 233 (H92);
(13) a heavy chain comprising the amino acid sequence of SEQ ID NO: 234 (H97);

(14) a heavy chain comprising the amino acid sequence of SEQ ID NO: 235 (H98);
(15) a light chain comprising the amino acid sequence of SEQ ID NO: 236 (L11);
(16) a light chain comprising the amino acid sequence of SEQ ID NO: 237 (L12);
(17) a light chain comprising the amino acid sequence of SEQ ID NO: 238 (L17);
(18) a light chain comprising the amino acid sequence of SEQ ID NO: 239 (L50);
(19) an antibody comprising the heavy chain of (3) and the light chain of (17) (H28L17);
(20) an antibody comprising the heavy chain of (4) and the light chain of (17) (H30L17);
(21) an antibody comprising the heavy chain of (5) and the light chain of (17) (H34L17);
(22) an antibody comprising the heavy chain of (6) and the light chain of (17) (H42L17);
(23) an antibody comprising the heavy chain of (7) and the light chain of (17) (H44L17);
(24) an antibody comprising the heavy chain of (8) and the light chain of (17) (H46L17);
(25) an antibody comprising the heavy chain of (9) and the light chain of (17) (H57L17);
(26) an antibody comprising the heavy chain of (10) and the light chain of (17) (H71L17);
(27) an antibody comprising the heavy chain of (11) and the light chain of (17) (H78L 17);
(28) an antibody comprising the heavy chain of (12) and the light chain of (17) (H92L17);
(29) an antibody comprising the heavy chain of (13) and the light chain of (18) (H97L50);
(30) an antibody comprising the heavy chain of (14) and the light chain of (18) (H98L50);
(31) an antibody in which one or more amino acids are substituted, deleted, added, and/or inserted in the antibody of any one of (19) to (30), which has an activity equivalent to that of the antibody of any one of (19) to (30); and
(32) an antibody which binds to the same epitope as an epitope bound by the antibody of any one of (19) to (30).

5. A pharmaceutical composition comprising the antibody of any one of items 1 to 4.

6. The pharmaceutical composition of item 5, which is an agent for treating an inflammatory disease.

## Claims

1. A nucleic acid encoding an antibody or antibody variable region which is any one of:

(1) a heavy chain variable region comprising CDR1 of SEQ ID NO: 9, CDR2 of SEQ ID NO: 197, and CDR3 of SEQ ID NO: 184 (H30, H44);
(2) a heavy chain variable region comprising CDR1 of SEQ ID NO: 196, CDR2 of SEQ ID NO: 197, and CDR3 of SEQ ID NO: 11 (H17);
(3) a heavy chain variable region comprising CDR1 of SEQ ID NO: 176, CDR2 of SEQ ID NO: 197, and CDR3 of SEQ ID NO: 11 (H19);
(4) a heavy chain variable region comprising CDR1 of SEQ ID NO: 196, CDR2 of SEQ ID NO: 197, and CDR3 of SEQ ID NO: 184 (H28, H42);
(5) a heavy chain variable region comprising CDR1 of SEQ ID NO: 176, CDR2 of SEQ ID NO: 197, CDR3 of SEQ ID NO: 184 (H34, H46);
(6) a heavy chain variable region comprising CDR1 of SEQ ID NO: 9, CDR2 of SEQ ID NO: 198, and CDR3 of SEQ ID NO: 184 (H57, H78);
(7) a heavy chain variable region comprising CDR1 of SEQ ID NO: 176, CDR2 of SEQ ID NO: 198, and CDR3 of SEQ ID NO: 184 (H71, H92);
(8) a heavy chain variable region comprising CDR1 of SEQ ID NO: 9, CDR2 of SEQ ID NO: 199, and CDR3 of SEQ ID NO: 184 (H97, H98);
(9) a light chain variable region comprising CDR1 of SEQ ID NO: 200, CDR2 of SEQ ID NO: 170, and CDR3 of SEQ ID NO: 193 (L11);
(10) a light chain variable region comprising CDR1 of SEQ ID NO: 201, CDR2 of SEQ ID NO: 170, and CDR3 of SEQ ID NO: 193 (L12);
(11) a light chain variable region comprising CDR1 of SEQ ID NO: 202, CDR2 of SEQ ID NO: 170, and CDR3 of SEQ ID NO: 193 (L17);
(12) a light chain variable region comprising CDR1 of SEQ ID NO: 203, CDR2 of SEQ ID NO: 170, and CDR3 of SEQ ID NO: 193 (L50);
(13) an antibody comprising the heavy chain variable region of (1) and the light chain variable region of (11);
(14) an antibody comprising the heavy chain variable region of (4) and the light chain variable region of (11);
(15) an antibody comprising the heavy chain variable region of (5) and the light chain variable region of (11);

(16) an antibody comprising the heavy chain variable region of (6) and the light chain variable region of (11);
(17) an antibody comprising the heavy chain variable region of (7) and the light chain variable region of (11);
(18) an antibody comprising the heavy chain variable region of (8) and the light chain variable region of (12);
(19) an antibody in which one or more amino acids are substituted, deleted, added, and/or inserted in the antibody of any one of (13) to (18), which has an activity equivalent to that of the antibody of any one of (13) to (18); and
(20) an antibody which binds to the same epitope as an epitope bound by the antibody of any one of (13) to (18).

**2.** A nucleic acid encoding an antibody or antibody variable region which is any one of:

(1) a heavy chain variable region comprising the amino acid sequence of SEQ ID NO: 210 (H44);
(2) a heavy chain variable region comprising the amino acid sequence of SEQ ID NO: 204 (H 17);
(3) a heavy chain variable region comprising the amino acid sequence of SEQ ID NO: 205 (H 19);
(4) a heavy chain variable region comprising the amino acid sequence of SEQ ID NO: 206 (H28);
(5) a heavy chain variable region comprising the amino acid sequence of SEQ ID NO: 207 (H30);
(6) a heavy chain variable region comprising the amino acid sequence of SEQ ID NO: 208 (H34),
(7) a heavy chain variable region comprising the amino acid sequence of SEQ ID NO: 209 (H42);
(8) a heavy chain variable region comprising the amino acid sequence of SEQ ID NO: 211 (H46);
(9) a heavy chain variable region comprising the amino acid sequence of SEQ ID NO: 212 (H57);
(10) a heavy chain variable region comprising the amino acid sequence of SEQ ID NO: 213 (H71);
(11) a heavy chain variable region comprising the amino acid sequence of SEQ ID NO: 214 (H78);
(12) a heavy chain variable region comprising the amino acid sequence of SEQ ID NO: 215 (H92);
(13) a heavy chain variable region comprising the amino acid sequence of SEQ ID NO: 216 (H97);
(14) a heavy chain variable region comprising the amino acid sequence of SEQ ID NO: 217 (H98);
(15) a light chain variable region comprising the amino acid sequence of SEQ ID NO: 218 (L11);
(16) a light chain variable region comprising the amino acid sequence of SEQ ID NO: 219 (L12);
(17) a light chain variable region comprising the amino acid sequence of SEQ ID NO: 220 (L17);
(18) a light chain variable region comprising the amino acid sequence of SEQ ID NO: 221 (L50);
(19) an antibody comprising the heavy chain variable region of (4) and the light chain variable region of (17) (H28L17);
(20) an antibody comprising the heavy chain variable region of (5) and the light chain variable region of (17) (H30L17);
(21) an antibody comprising the heavy chain variable region of (6) and the light chain variable region of (17) (H34L17);
(22) an antibody comprising the heavy chain variable region of (7) and the light chain variable region of (17) (H42L17);
(23) an antibody comprising the heavy chain variable region of (1) and the light chain variable region of (17) (H44L17);
(24) an antibody comprising the heavy chain variable region of (8) and the light chain variable region of (17) (H46L17);
(25) an antibody comprising the heavy chain variable region of (9) and the light chain variable region of (17) (H57L17);
(26) an antibody comprising the heavy chain variable region of (10) and the light chain variable region of (17) (H71L17);
(27) an antibody comprising the heavy chain variable region of (11) and the light chain variable region of (17) (H78L17);
(28) an antibody comprising the heavy chain variable region of (12) and the light chain variable region of (17) (H92L17);
(29) an antibody comprising the heavy chain variable region of (13) and the light chain variable region of (18) (H97L50);
(30) an antibody comprising the heavy chain variable region of (14) and the light chain variable region of (18) (H98L50),
(31) an antibody in which one or more amino acids are substituted, deleted, added, and/or inserted in the antibody of any one of (19) to (30), which has an activity equivalent to that of the antibody of any one of (19) to (30); and
(32) an antibody which binds to the same epitope as an epitope bound by the antibody of any one of (19) to (30).

**3.** A nucleic acid encoding an antibody, antibody heavy chain, or antibody light chain, which is any one of:

(1) a heavy chain comprising the amino acid sequence of SEQ ID NO: 228 (H44);
(2) a heavy chain comprising the amino acid sequence of SEQ ID NO: 222 (H17);
(3) a heavy chain comprising the amino acid sequence of SEQ ID NO: 223 (H19);
(4) a heavy chain comprising the amino acid sequence of SEQ ID NO: 224 (H28);
(5) a heavy chain comprising the amino acid sequence of SEQ ID NO: 225 (H30);
(6) a heavy chain comprising the amino acid sequence of SEQ ID NO: 226 (H34);
(7) a heavy chain comprising the amino acid sequence of SEQ ID NO: 227 (H42);
(8) a heavy chain comprising the amino acid sequence of SEQ ID NO: 229 (H46);
(9) a heavy chain comprising the amino acid sequence of SEQ ID NO: 230 (H57);
(10) a heavy chain comprising the amino acid sequence of SEQ ID NO: 231 (H71);
(11) a heavy chain comprising the amino acid sequence of SEQ ID NO: 232 (H78);
(12) a heavy chain comprising the amino acid sequence of SEQ ID NO: 233 (H92);
(13) a heavy chain comprising the amino acid sequence of SEQ ID NO: 234 (H97);
(14) a heavy chain comprising the amino acid sequence of SEQ ID NO: 235 (H98);
(15) a light chain comprising the amino acid sequence of SEQ ID NO: 236 (L11);
(16) a light chain comprising the amino acid sequence of SEQ ID NO: 237 (L12);
(17) a light chain comprising the amino acid sequence of SEQ ID NO: 238 (L17);
(18) a light chain comprising the amino acid sequence of SEQ ID NO: 239 (L50);
(19) an antibody comprising the heavy chain of (4) and the light chain of (17) (H28L17);
(20) an antibody comprising the heavy chain of (5) and the light chain of (17) (H30L17);
(21) an antibody comprising the heavy chain of (6) and the light chain of (17) (H34L17);
(22) an antibody comprising the heavy chain of (7) and the light chain of (17) (H42L17);
(23) an antibody comprising the heavy chain of (1) and the light chain of (17) (H44L17);
(24) an antibody comprising the heavy chain of (8) and the light chain of (17) (H46L17);
(25) an antibody comprising the heavy chain of (9) and the light chain of (17) (H57L17);
(26) an antibody comprising the heavy chain of (10) and the light chain of (17) (H71L17);
(27) an antibody comprising the heavy chain of (11) and the light chain of (17) (H78L17);
(28) an antibody comprising the heavy chain of (12) and the light chain of (17) (H92L17);
(29) an antibody comprising the heavy chain of (13) and the light chain of (18) (H97L50);
(30) an antibody comprising the heavy chain of (14) and the light chain of (18) (H98L50);
(31) an antibody in which one or more amino acids are substituted, deleted, added, and/or inserted in the antibody of any one of (19) to (30), which has an activity equivalent to that of the antibody of any one of (19) to (30).

**4.** The nucleic acid of any one of claims 1 to 3, wherein the antibody is an anti-NR10 antibody.

**5.** The nucleic acid of any one of claims 1 to 4, which is a humanized antibody.

**6.** A vector comprising the nucleic acid of any one of claims 1 to 5.

**7.** A host cell containing the vector of claim 6.

**8.** A host cell transformed with the vector of claim 6.

**9.** A method for producing a variable region, a heavy chain, a light chain, or an antibody, which comprise the step of culturing the host cell of claim 7 or 8.

**10.** A method for producing a variable region, a heavy chain, a light chain, or an antibody, which comprise

(a) culturing a host cell containing a vector into which the nucleic acid of any one of claims 1 to 5 has been introduced; and
(b) obtaining a variable region, a heavy chain, a light chain, or an antibody encoded by the nucleic acid.

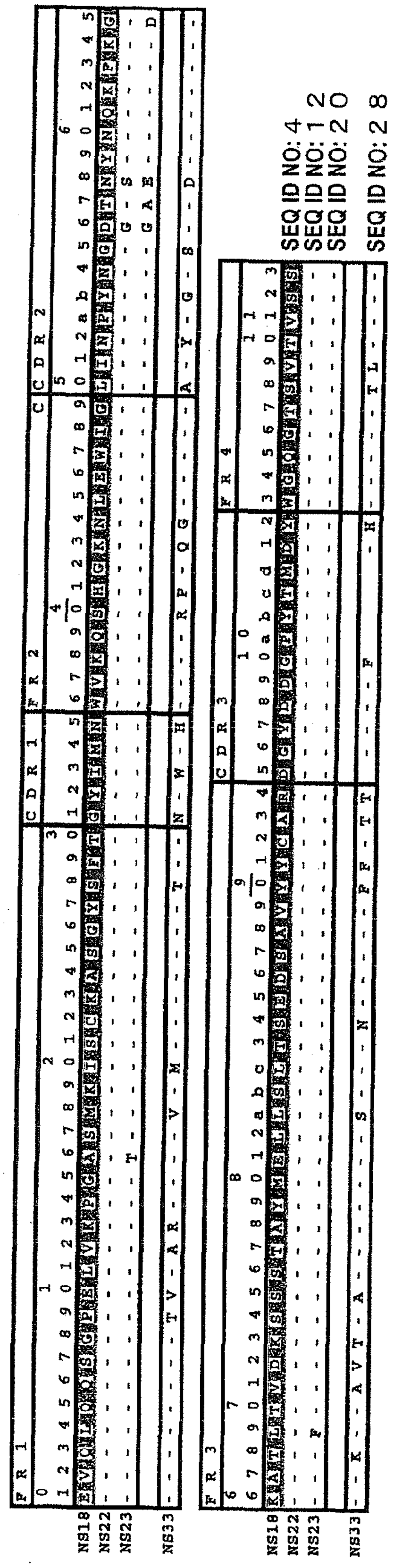
FR1
CDR1
FR2
CDR2
FR3
CDR3
FR4
NS18
NS22
NS23
NS33
SEQ ID NO: 4
SEQ ID NO: 12
SEQ ID NO: 20
SEQ ID NO: 28

FIG. 1

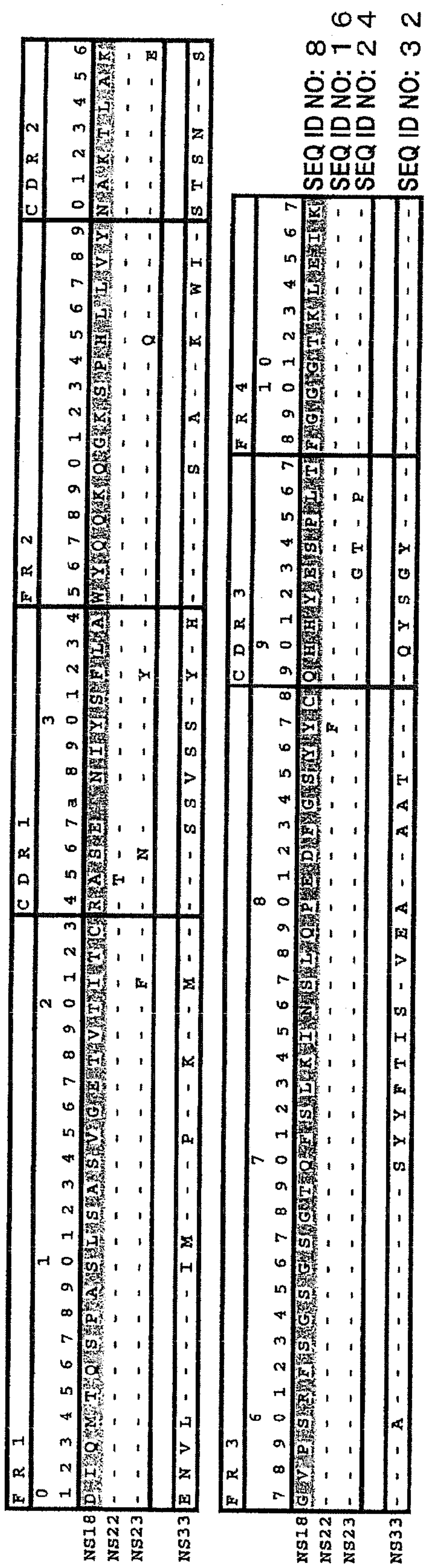

FIG. 2

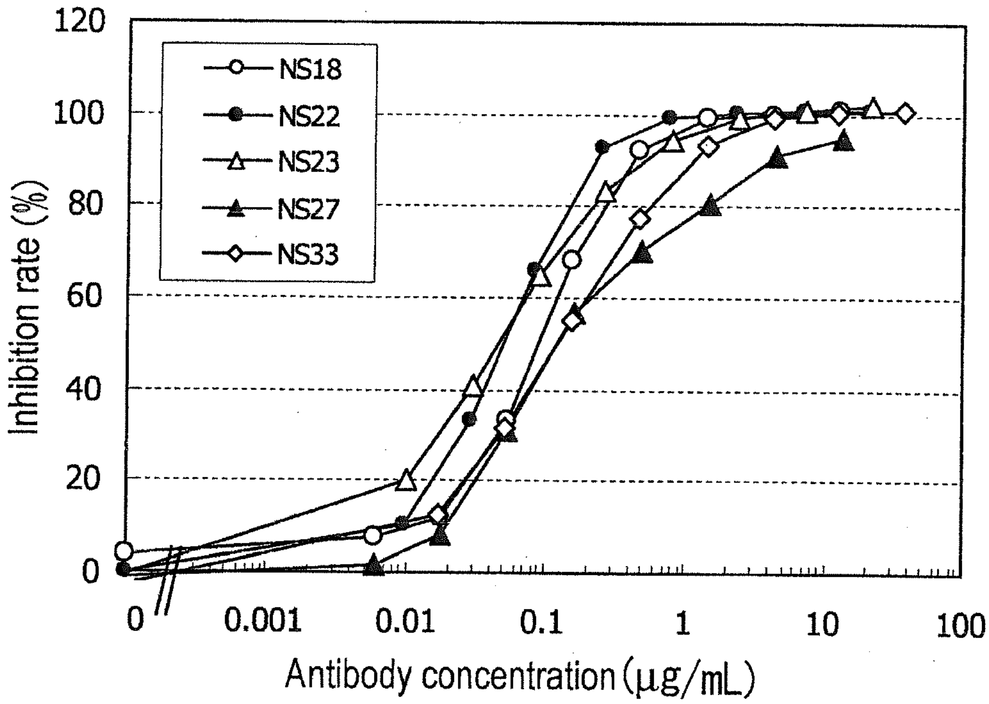
120
100
80
60
40
20
0
Inhibition rate (%)
NS18
NS22
NS23
NS27
NS33
0
0.001
0.01
0.1
1
10
100
Antibody concentration (μg/mL)

FIG. 3

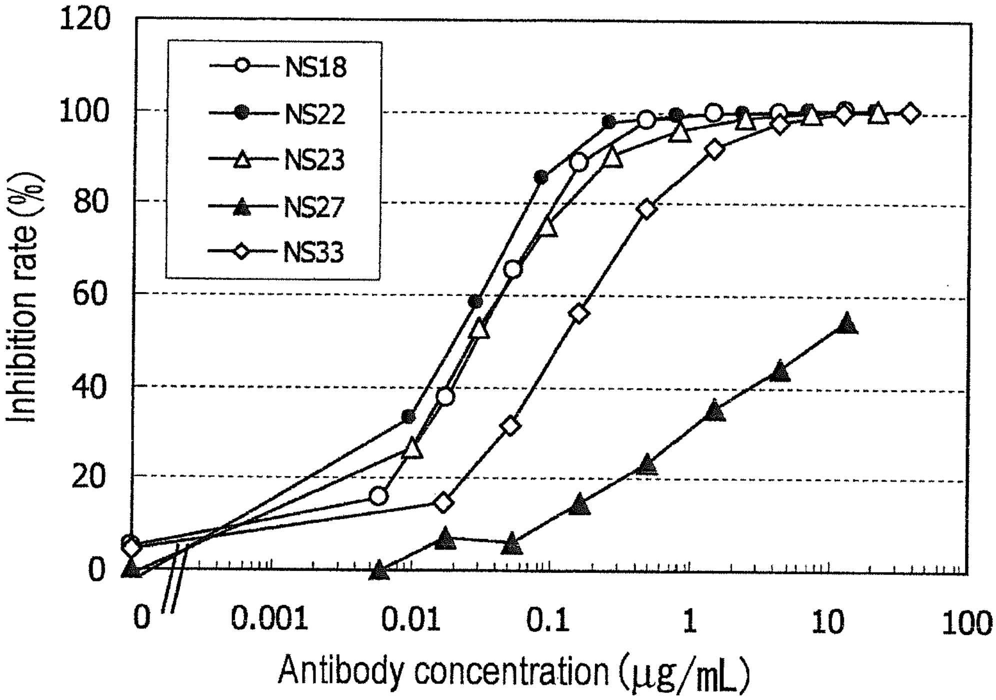
120
100
80
60
40
20
0
Inhibition rate (%)
NS18
NS22
NS23
NS27
NS33
0
0.001
0.01
0.1
1
10
100
Antibody concentration (μg/mL)

FIG. 4

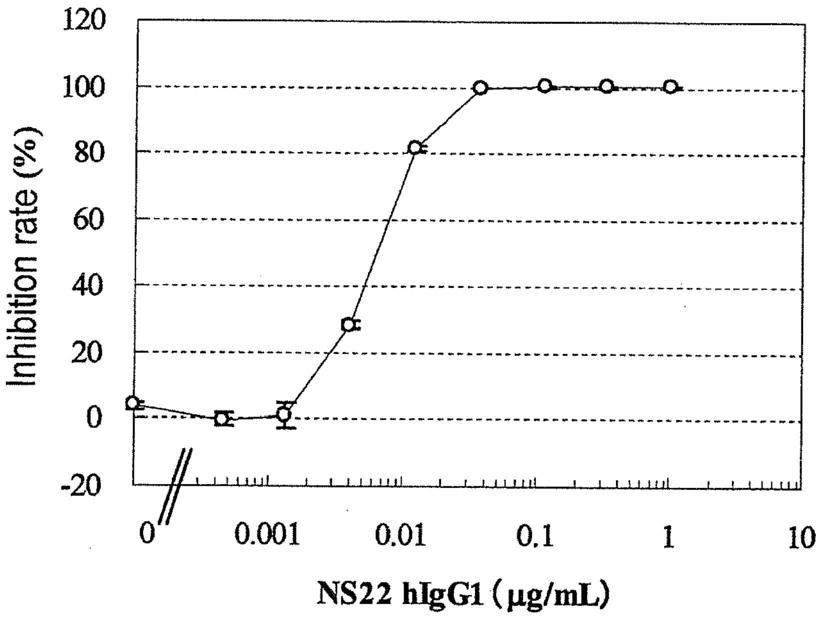
120
100
80
60
40
20
0
-20
Inhibition rate (%)
0
0.001
0.01
0.1
1
10
NS22 hIgG1 (μg/mL)

FIG. 5

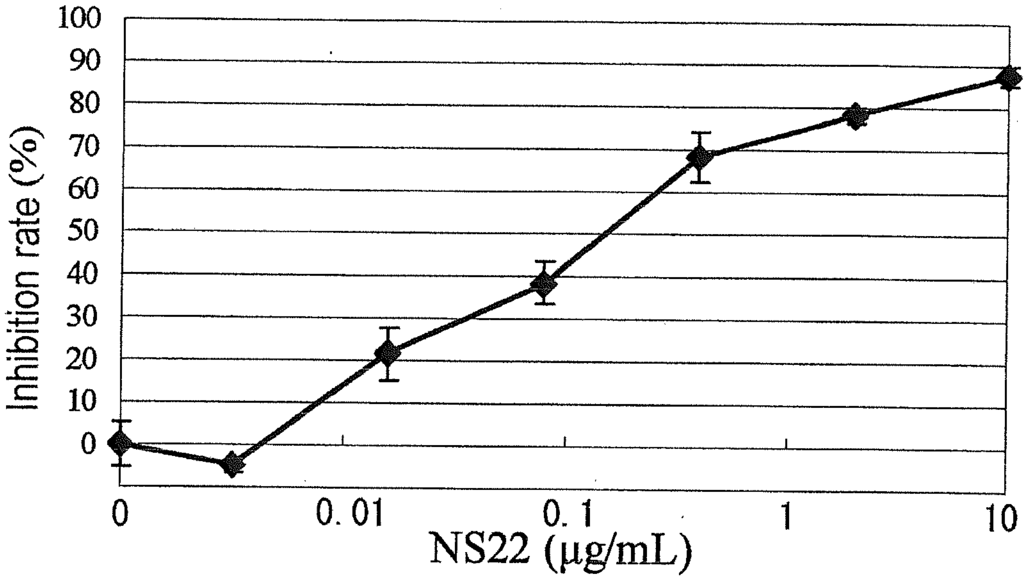
100
90
80
70
60
50
40
30
20
10
0
Inhibition rate (%)
0
0. 01
0. 1
1
10
NS22 (µg/mL)

FIG. 6

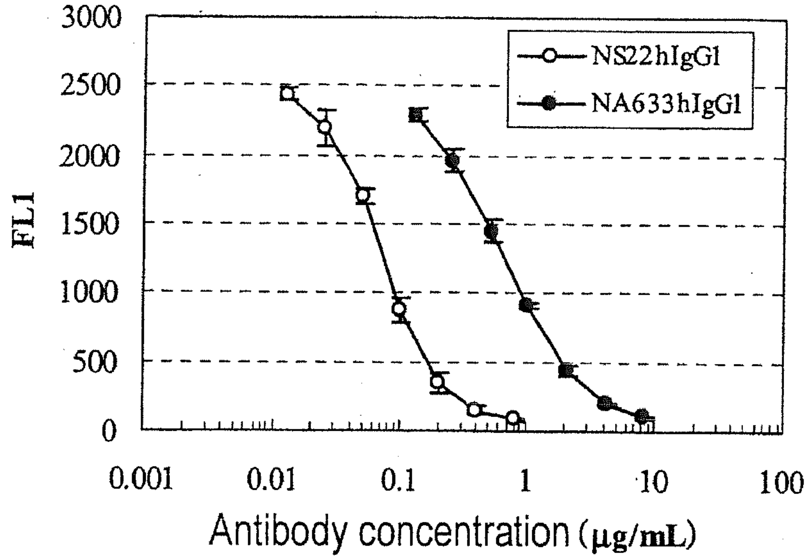
3000
2500
2000
1500
1000
500
0
FL1
NS22hIgG1
NA633hIgG1
0.001
0.01
0.1
1
10
100
Antibody concentration (μg/mL)

FIG. 7

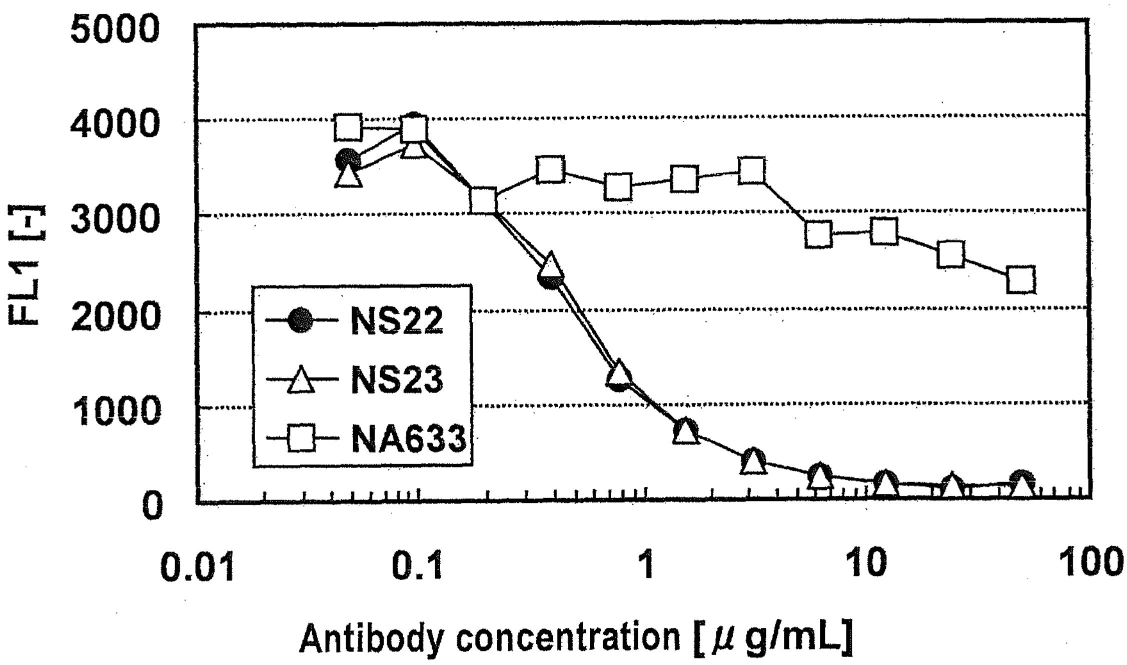
5000
4000
3000
2000
1000
0
FL1 [-]
NS22
NS23
NA633
0.01
0.1
1
10
100
Antibody concentration [μ g/mL]

FIG. 8

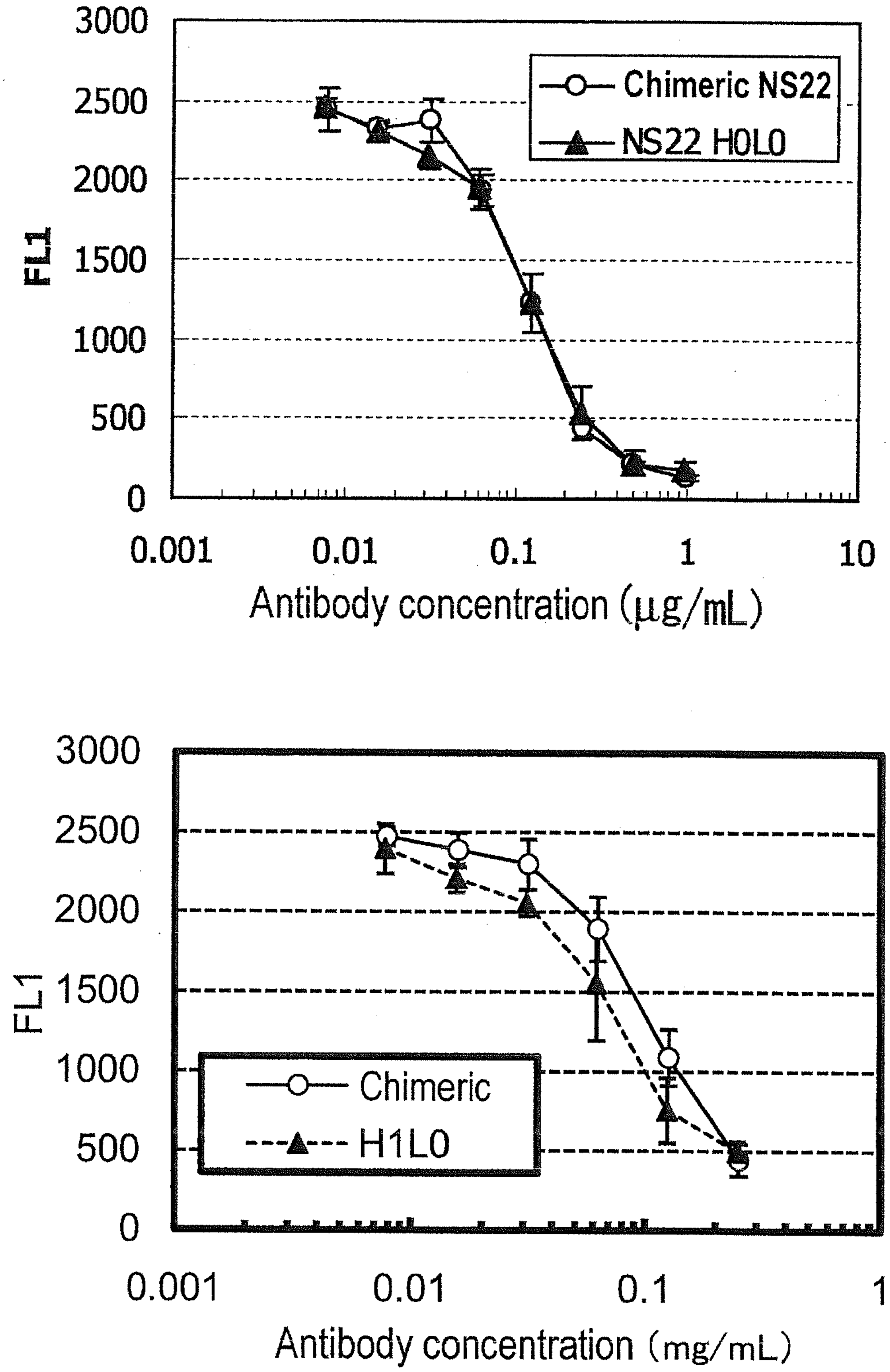
3000
2500
2000
1500
1000
500
0
FL1
Chimeric NS22
NS22 H0L0
0.001
0.01
0.1
1
10
Antibody concentration (μg/mL)
3000
2500
2000
1500
1000
500
0
FL1
Chimeric
H1L0
0.001
0.01
0.1
1
Antibody concentration (mg/mL)

FIG. 9

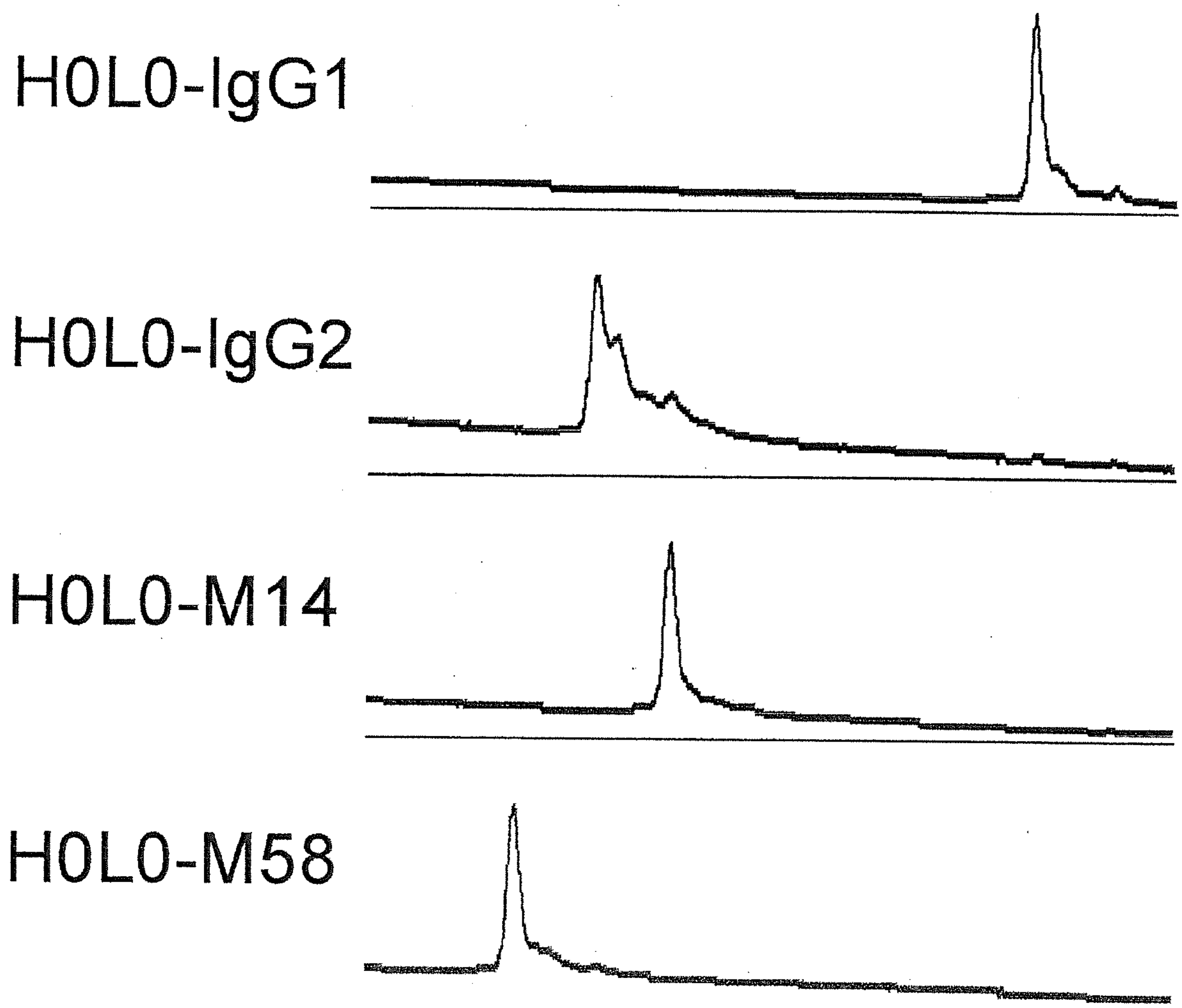


FIG. 10

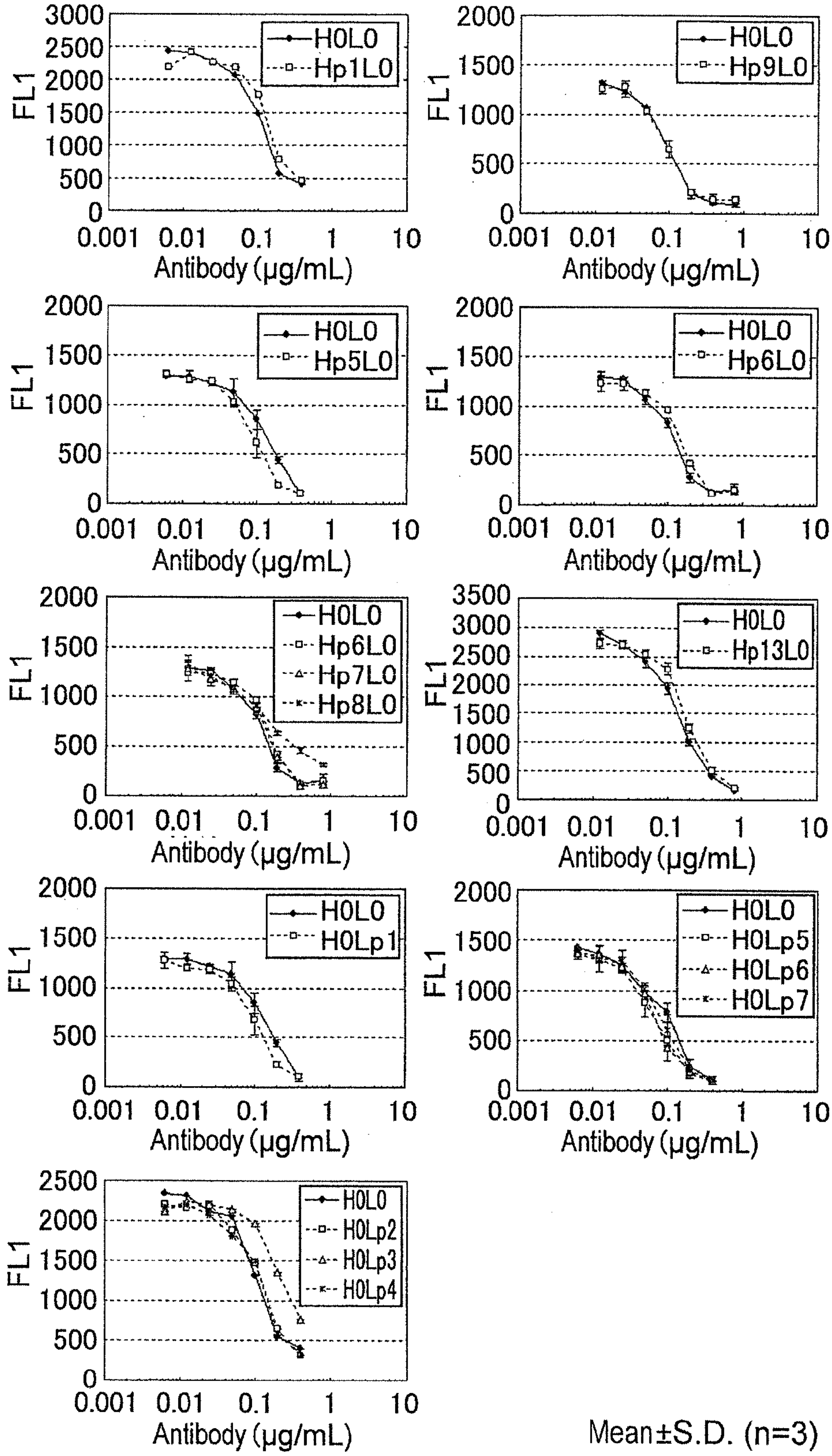
FL1
Antibody (μg/mL)
0.001
0.01
0.1
1
10
H0L0
Hp1L0
Hp9L0
Hp5L0
Hp6L0
Hp7L0
Hp8L0
Hp13L0
H0Lp1
H0Lp5
H0Lp6
H0Lp7
H0Lp2
H0Lp3
H0Lp4
Mean±S.D. (n=3)

FIG. 11

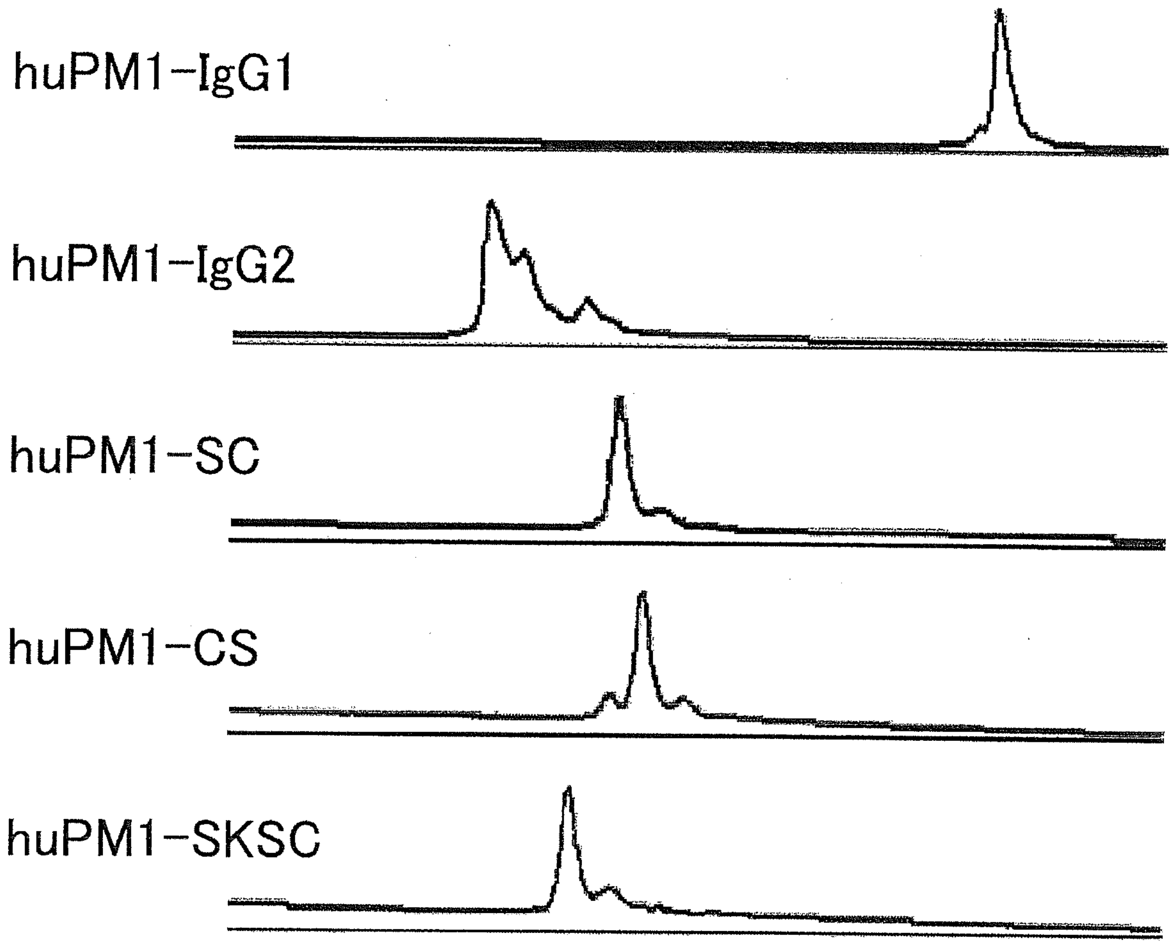


FIG. 12

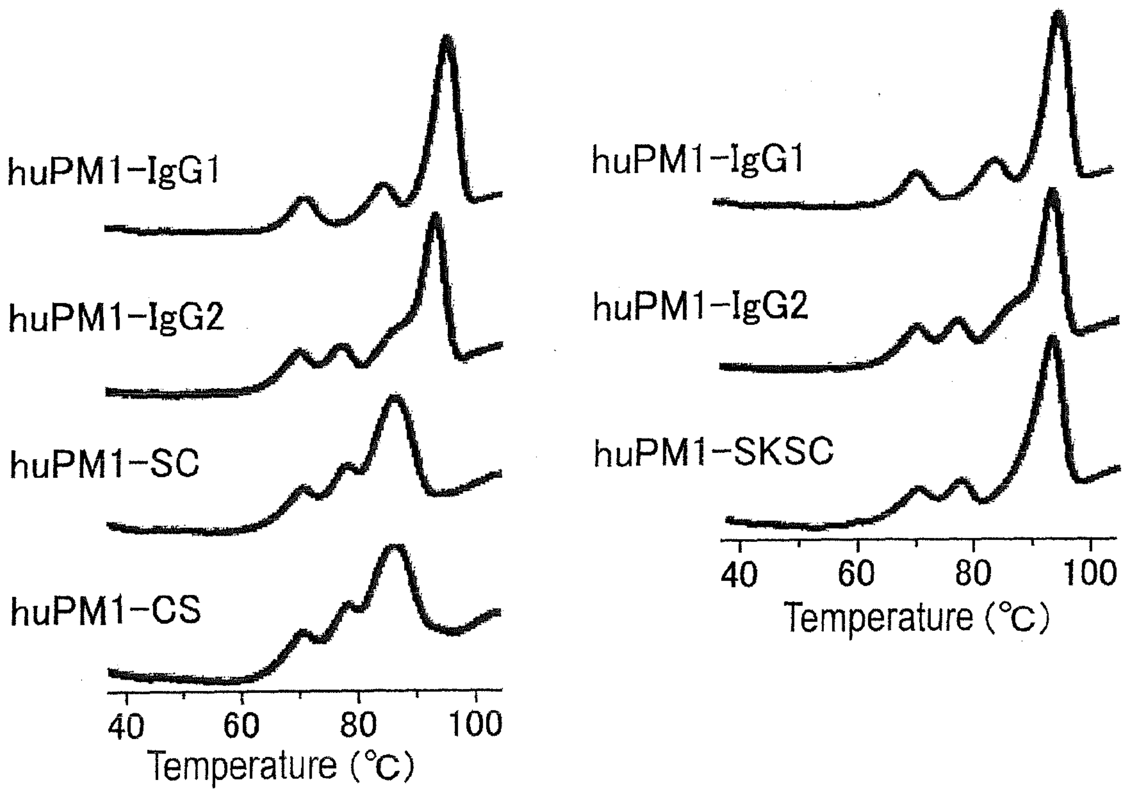
huPM1-IgG1
huPM1-IgG2
huPM1-SC
huPM1-CS
40
60
80
100
Temperature (°C)
huPM1-IgG1
huPM1-IgG2
huPM1-SKSC
40
60
80
100
Temperature (°C)

FIG. 13

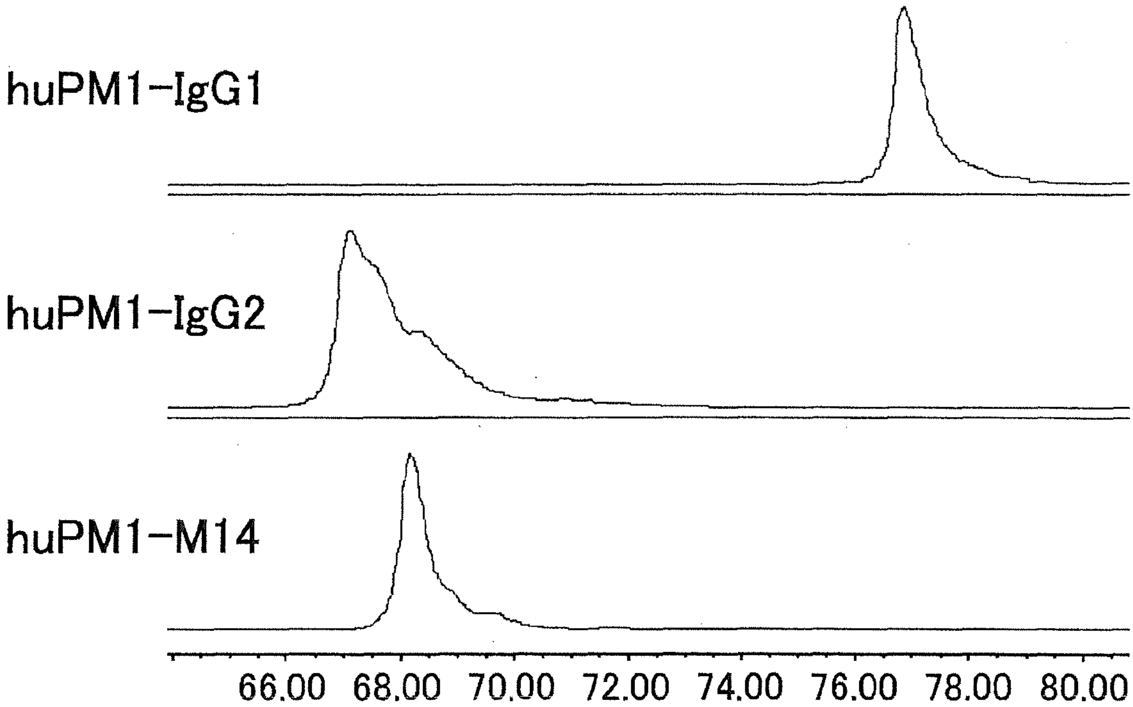
huPM1-IgG1
huPM1-IgG2
huPM1-M14
66.00
68.00
70.00
72.00
74.00
76.00
78.00
80.00

FIG. 14

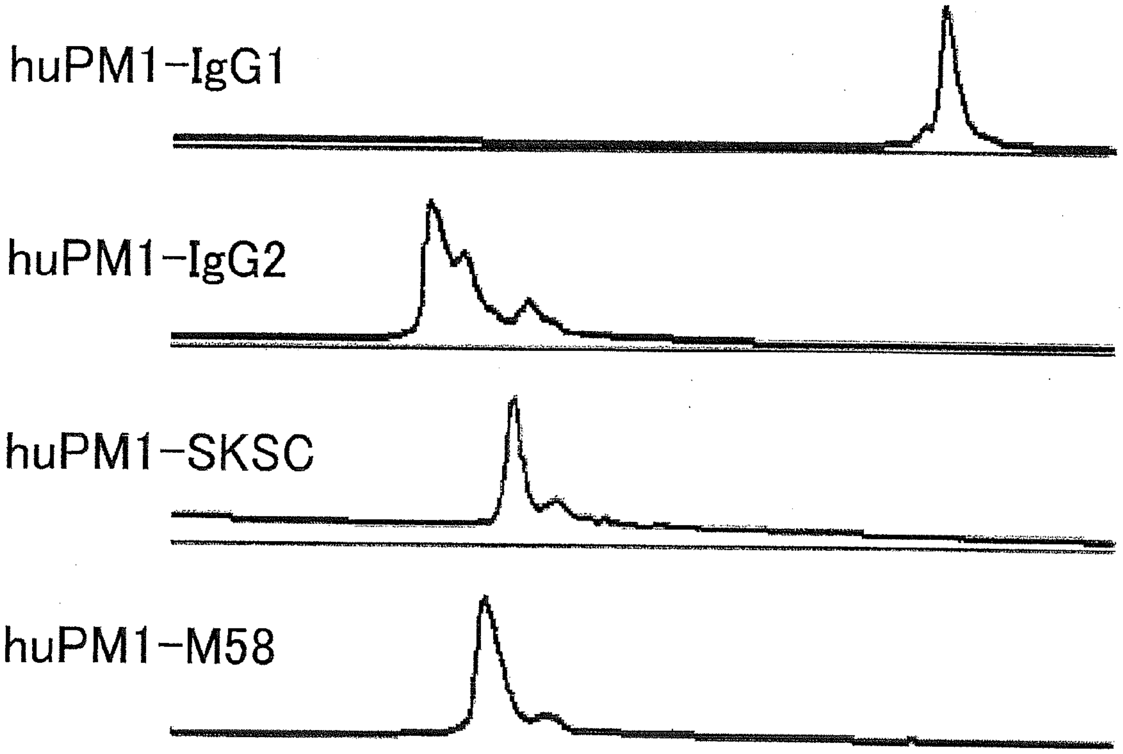


FIG. 15

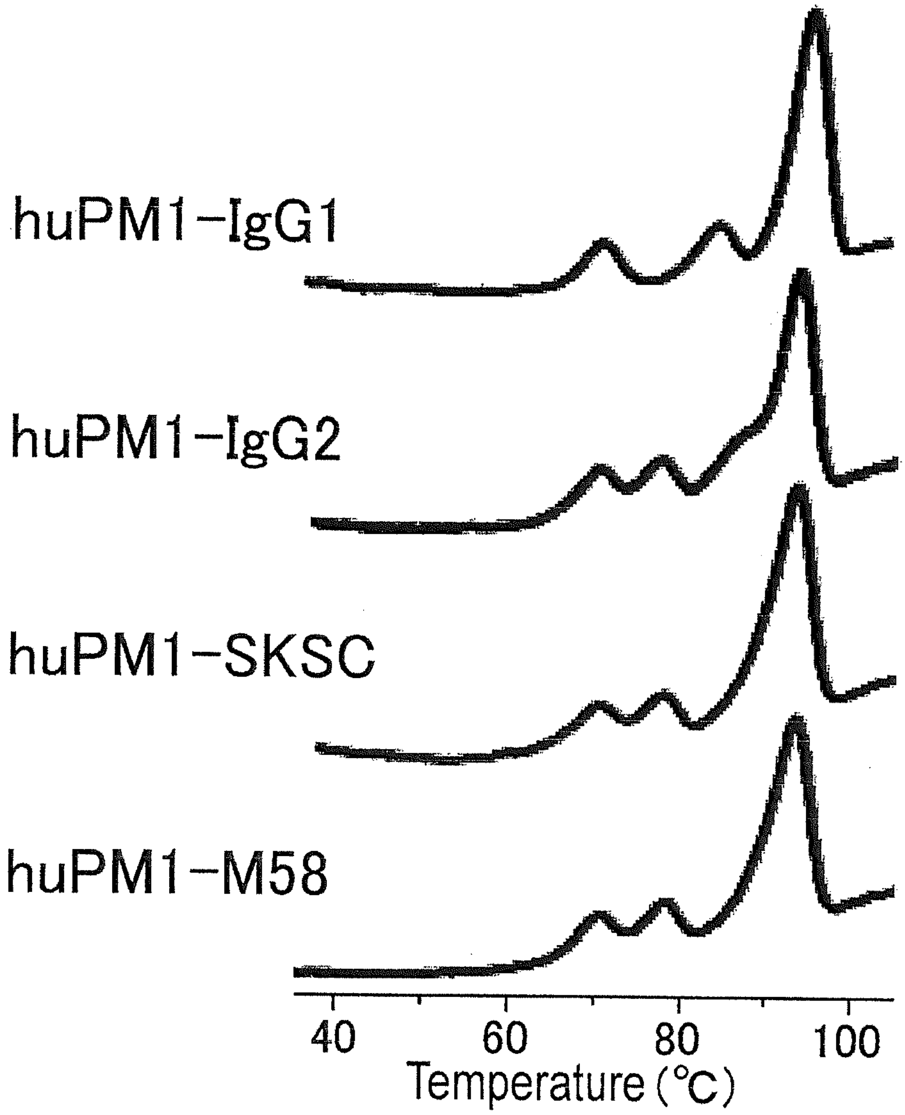
huPM1-IgG1
huPM1-IgG2
huPM1-SKSC
huPM1-M58
40
60
80
100
Temperature (°C)

FIG. 16

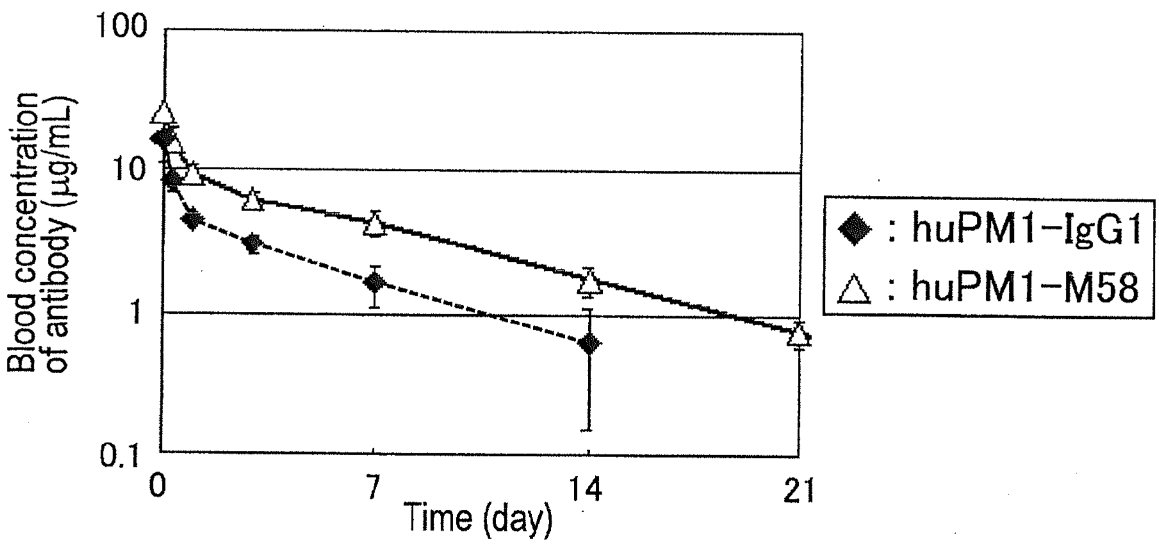
100
10
1
0.1
Blood concentration of antibody (μg/mL)
0
7
14
21
Time (day)
◆ : huPM1-IgG1
△ : huPM1-M58

FIG. 17

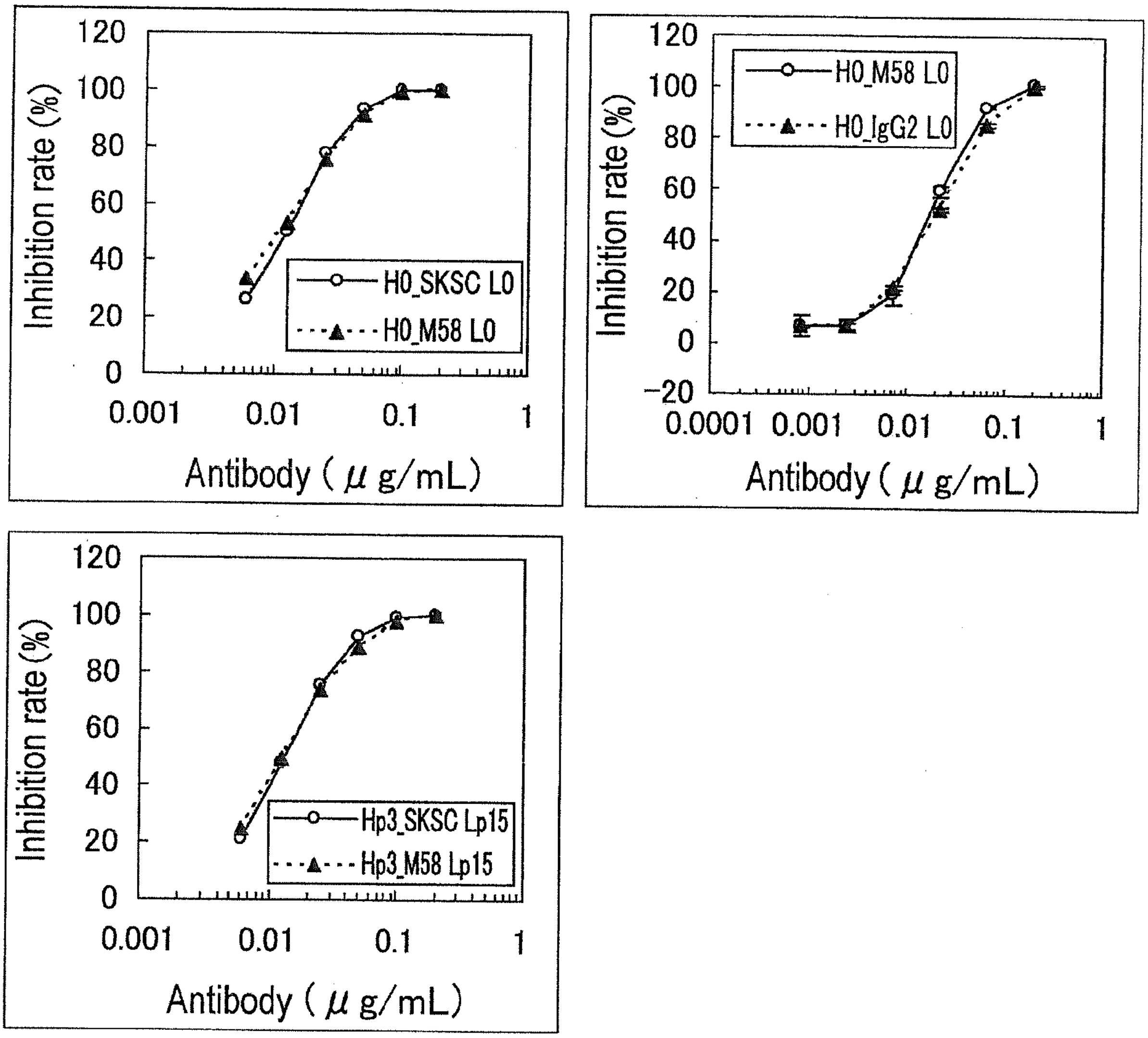

Inhibition rate (%)
Antibody ( μ g/mL)
H0_SKSC L0
H0_M58 L0
H0_IgG2 L0
Hp3_SKSC Lp15
Hp3_M58 Lp15
120
100
80
60
40
20
0
-20
0.0001
0.001
0.01
0.1
1


FIG. 18

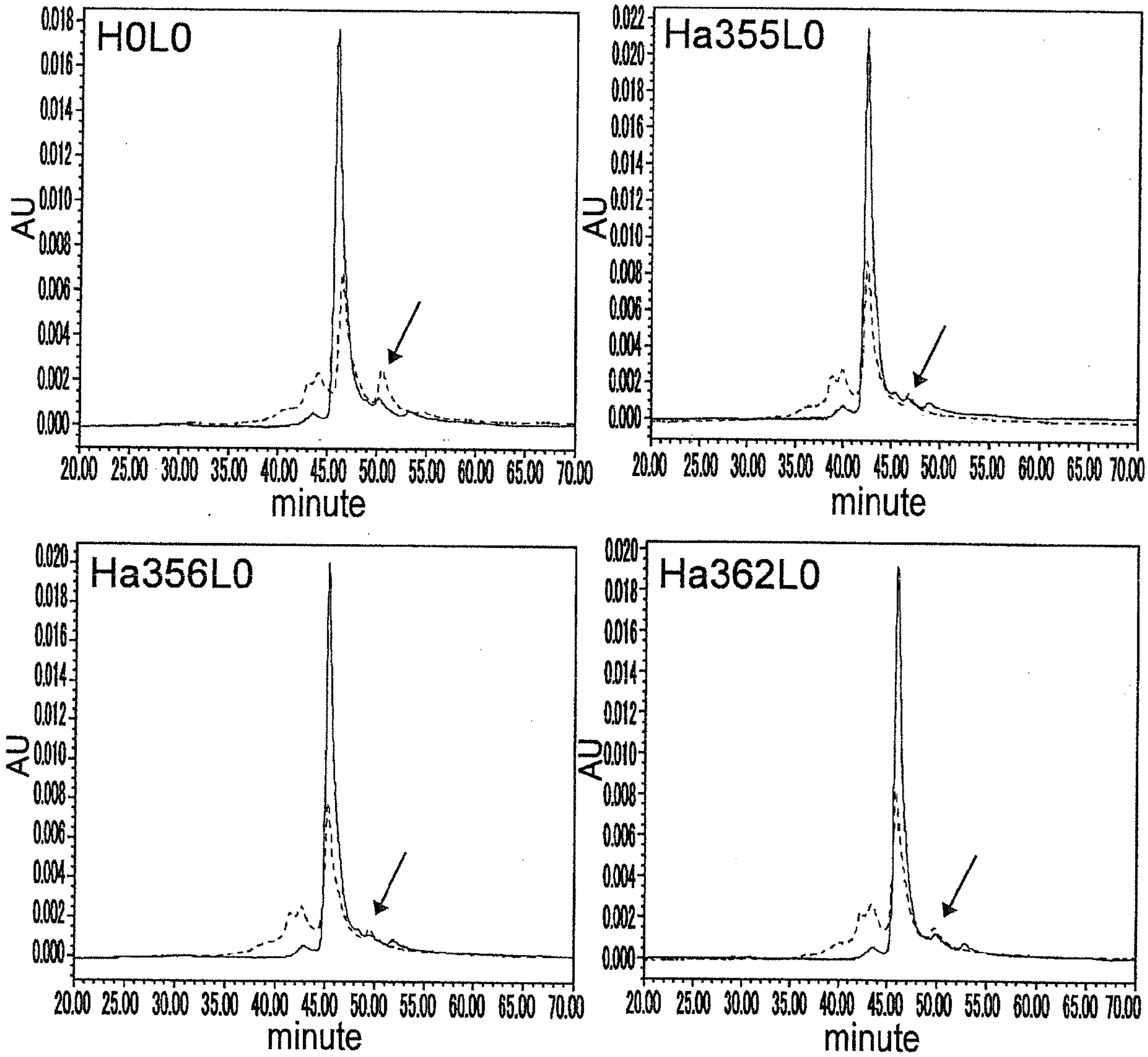


FIG. 19

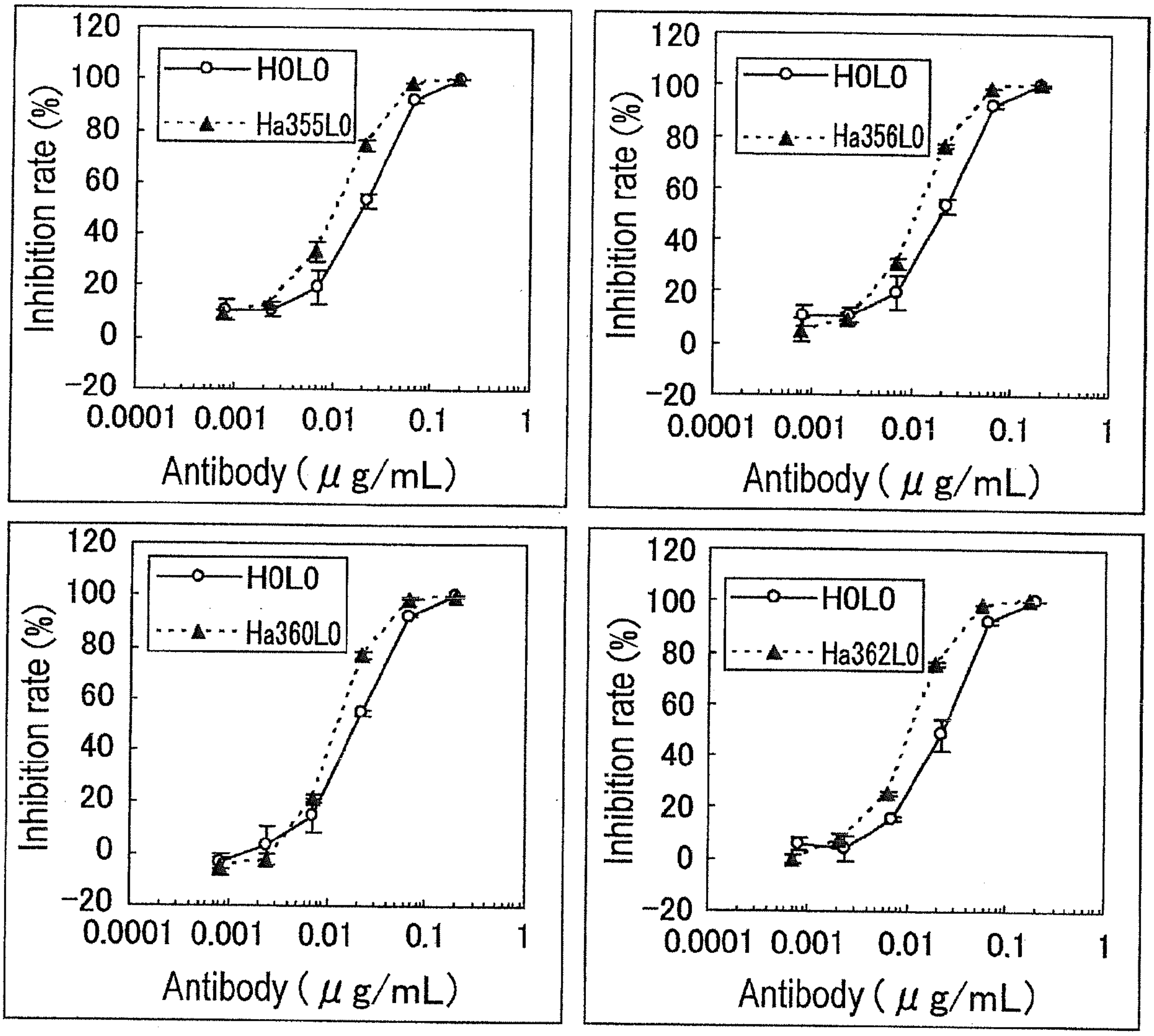
H0L0
Ha355L0
Ha356L0
Ha360L0
Ha362L0
Inhibition rate (%)
Antibody ( μ g/mL)
120
100
80
60
40
20
0
−20
0.0001
0.001
0.01
0.1
1

FIG. 20

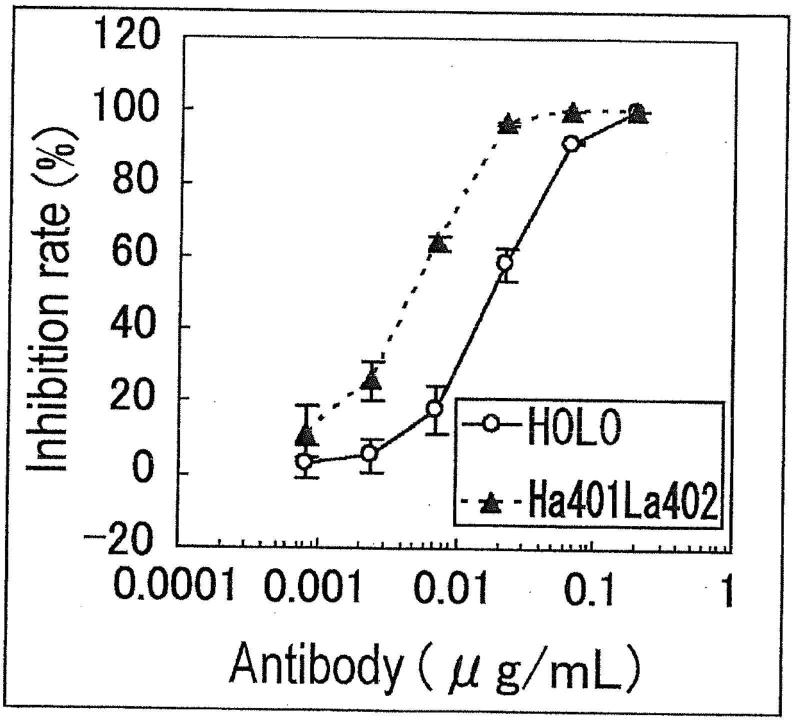

120
100
80
60
40
20
0
−20
Inhibition rate (%)
HOLO
Ha401La402
0.0001
0.001
0.01
0.1
1
Antibody ( μ g/mL)


FIG. 21

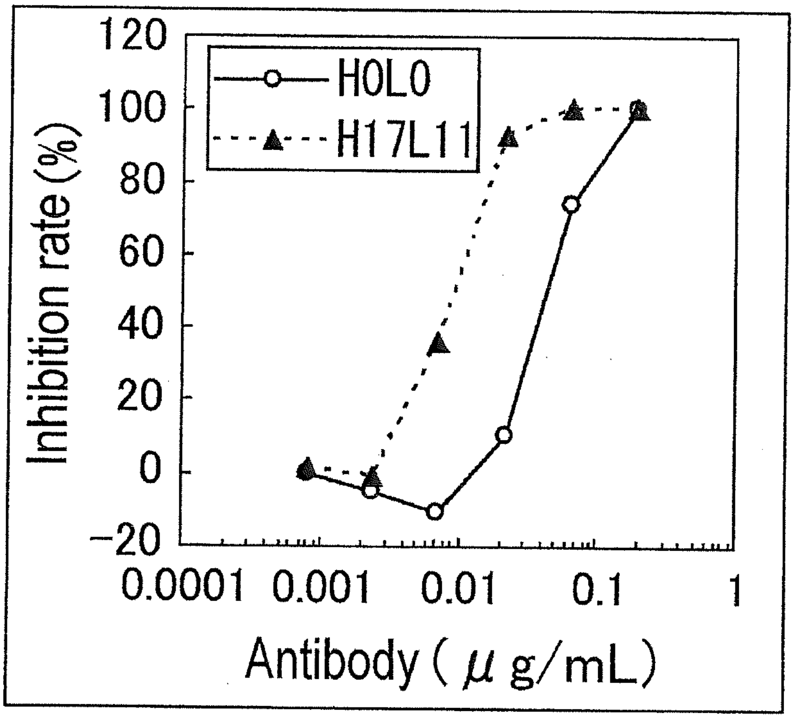

120
100
80
60
40
20
0
−20
Inhibition rate (%)
H0L0
H17L11
0.0001
0.001
0.01
0.1
1
Antibody ( μ g/mL)


FIG. 22

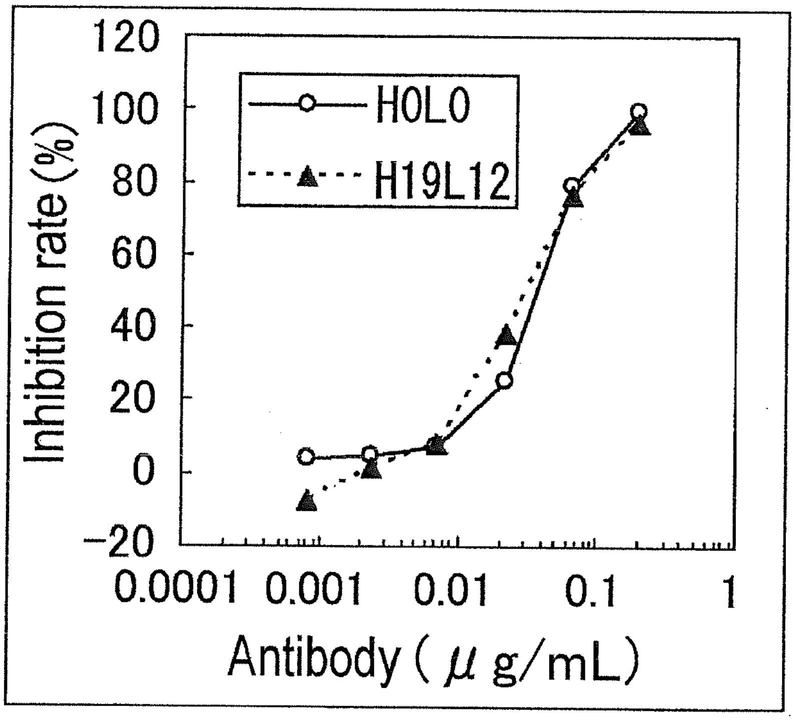
120
100
80
60
40
20
0
−20
Inhibition rate (%)
H0L0
H19L12
0.0001
0.001
0.01
0.1
1
Antibody ( μ g/mL)

FIG. 23

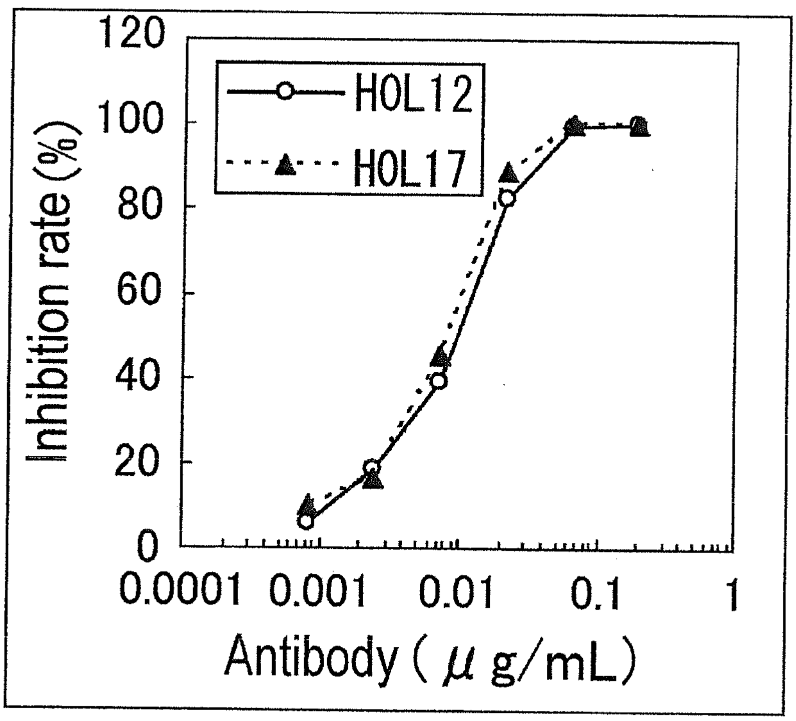

120
100
80
60
40
20
0
Inhibition rate (%)
HOL12
HOL17
0.0001
0.001
0.01
0.1
1
Antibody ( μ g/mL)


FIG. 24

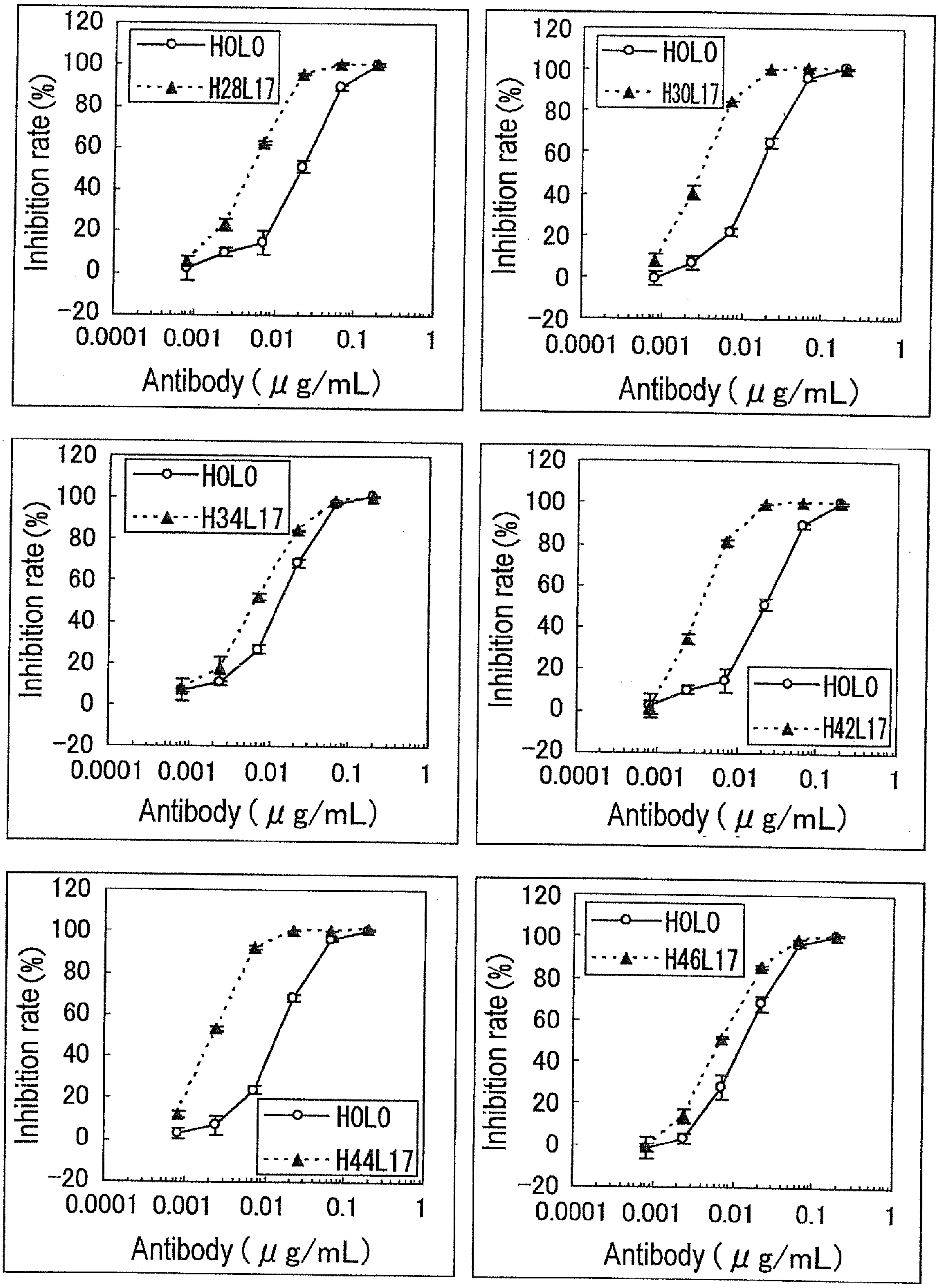

H0L0
H28L17
H30L17
H34L17
H42L17
H44L17
H46L17
Inhibition rate (%)
Antibody ( μ g/mL)
120
100
80
60
40
20
0
−20
0.0001
0.001
0.01
0.1
1


FIG. 25-1

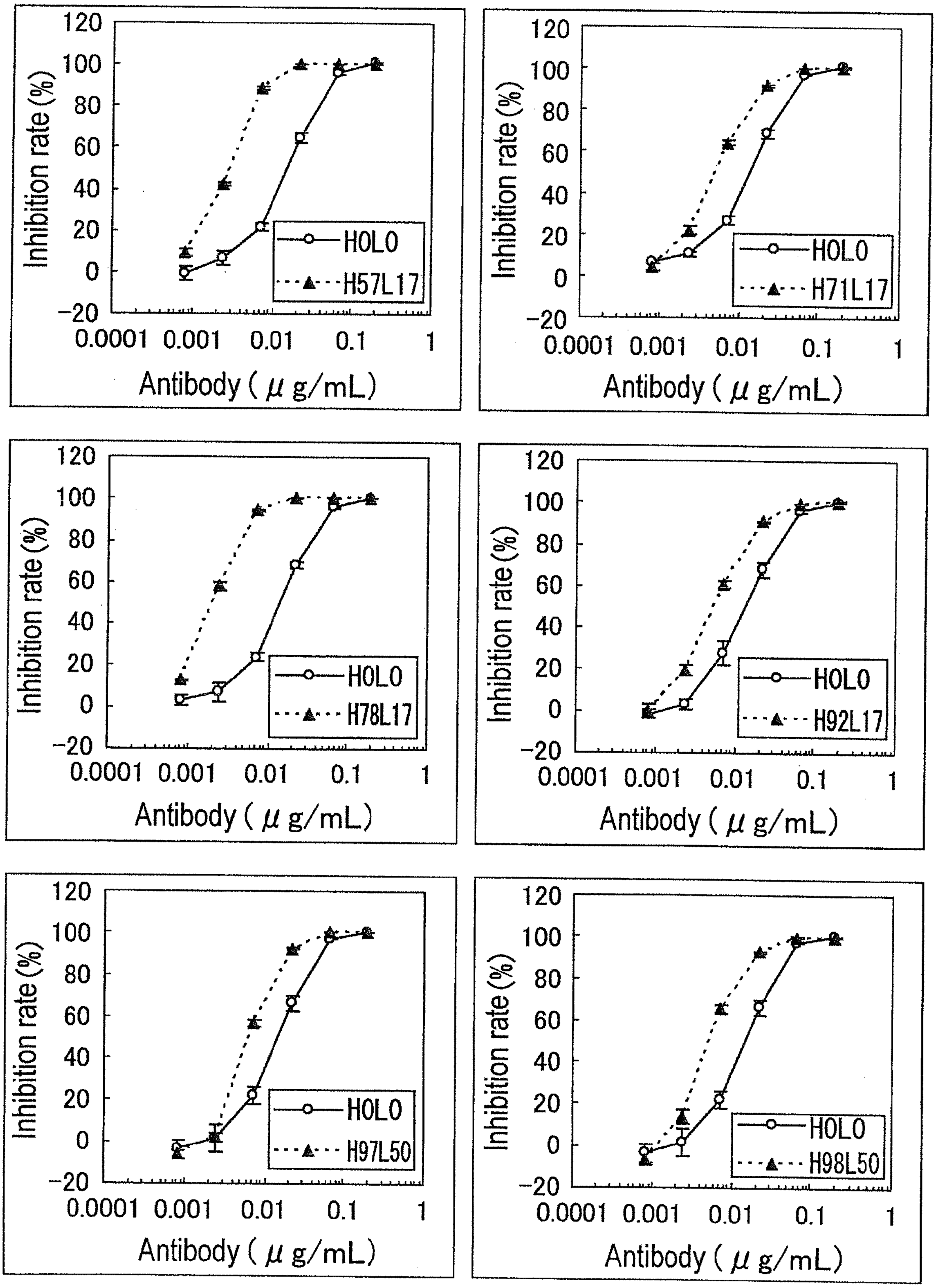
Inhibition rate (%)
Antibody ( μ g/mL)
120
100
80
60
40
20
0
−20
0.0001
0.001
0.01
0.1
1
H0L0
H57L17
H71L17
H78L17
H92L17
H97L50
H98L50

FIG. 25-2

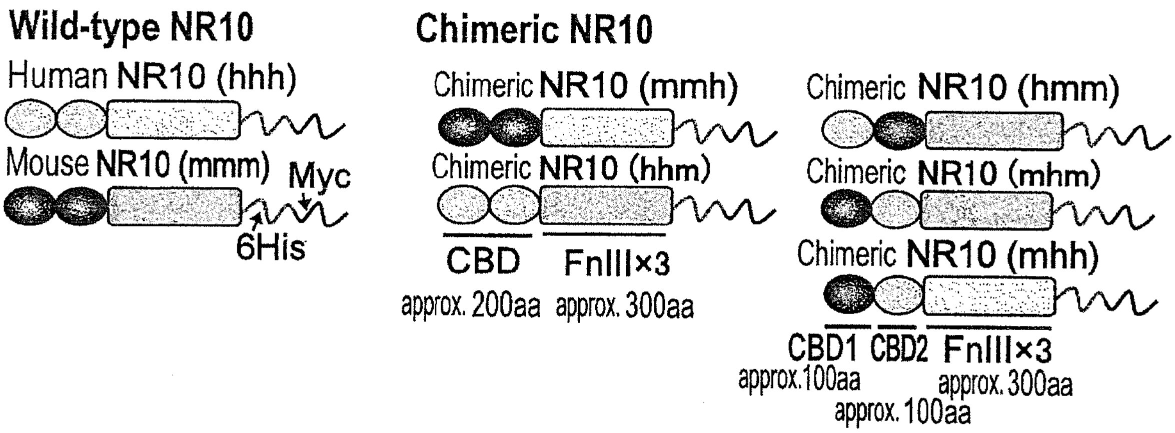
Wild-type NR10
Human NR10 (hhh)
Mouse NR10 (mmm)
Myc
6His
Chimeric NR10
Chimeric NR10 (mmh)
Chimeric NR10 (hhm)
CBD
FnIII×3
approx. 200aa
approx. 300aa
Chimeric NR10 (hmm)
Chimeric NR10 (mhm)
Chimeric NR10 (mhh)
CBD1
CBD2
FnIII×3
approx.100aa
approx. 300aa
approx. 100aa

FIG. 26

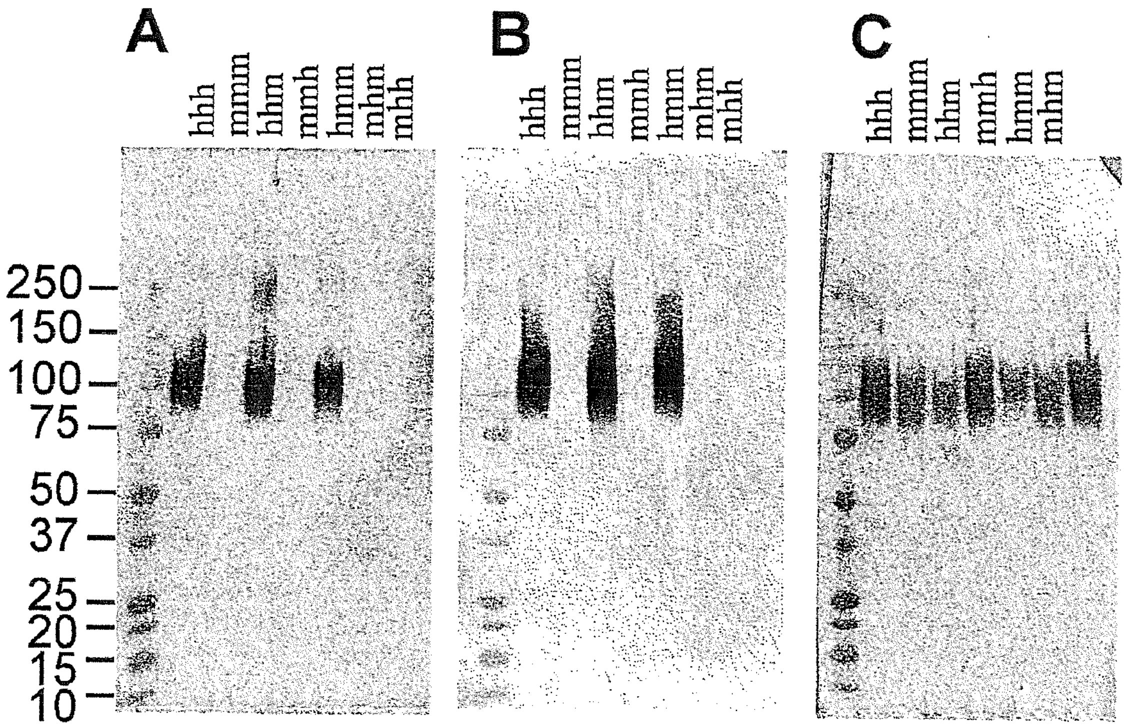


FIG. 27

Humanized

| | FR1 | CDR1 | FR2 | CDR2 | FR3 | CDR3 | FR4 | |
|---|---|---|---|---|---|---|---|---|
| | 0 1 2 3 | | 4 | 5 6 | 6 7 8 9 | 10 | 11 | |
| | 123456789012345678901234567890 | 12345 | 67890123456789 | 012ab456789012345 | 67890123456789012abc345678901234 | 567890abcd12 | 34567890123 | |
| H0 | QVQLVQSGAEVKKPGASVKVSCKASGYTFT | GYIMN | WVRQAPGQGLEWMG | LINPYNGGTSYNQKFKG | RVTITADESTSTAYMELSSLRSEDTAVYYCAR | DGYDDGPYTMDY | WGQGTLVTVSS | SEQ ID NO:50 |
| Hp5 | ------------------------------ | ----- | --Q-S--------- | ----------------- | -------------------------------- | ------------ | ----------- | |
| Hp7 | ------------------------------ | ----- | -------------- | E---------------- | -------------------------------- | ------------ | ----------- | |
| Hp8 | ------------------------------ | ----- | -------------- | --D-------------- | -------------------------------- | ------------ | ----------- | |
| Hp6 | ------------------------------ | ----- | -------------- | ------------D---- | -------------------------------- | ------------ | ----------- | |
| Hp9 | ------------------------------ | ----- | -------------- | -------------Q--- | -------------------------------- | ------------ | ----------- | |
| Hp1 | ------------------------------ | ----- | -------------- | ---------------Q- | -------------------------------- | ------------ | ----------- | |
| Hp13 | ------------------------------ | ----- | -------------- | ---------------QD | -------------------------------- | ------------ | ----------- | |
| Hp3 | ------------------------------ | ----- | -------------- | -------------Q-QD | -------------------------------- | ------------ | ----------- | |

FIG. 28-1

| | FR1 | CDR1 | FR2 | CDR2 | FR3 | CDR3 | FR4 | |
|---|---|---|---|---|---|---|---|---|
| | 0 1 2 3 | | 4 | 5 6 | 6 7 8 9 | 10 | 11 | |
| | 123456789012345678901234567890 | 12345 | 67890123456789 | 012ab4567890123456 | 67890123456789012abc345678901234 | 567890abcd12 | 34567890123 | |
| H0 | QVQLVQSGAEVKKPGASVKVSCKASGYTFT | GYIMN | WVRQAPGQGLEWMG | LINPYNGGTSYNQKFKG | RVTITADESTSTAYMELSSLRSEDTAVYYCAR | DGYDDGPYTMDY | WGQGTLVTVSS | SEQ ID NO:50 |
| Ha355 | ------------------------------ | ----- | -------------- | ----------------- | -------------------------------- | ---------LET | ----------- | |
| Ha356 | ------------------------------ | ----- | -------------- | ----------------- | -------------------------------- | ----------ET | ----------- | |
| Ha360 | ------------------------------ | ----- | -------------- | ----------------- | -------------------------------- | -L--------ET | ----------- | |
| Ha362 | ------------------------------ | ----- | -------------- | ----------------- | -------------------------------- | -L--------ES | ----------- | |
| Ha101 | ------------------------------ | -V--- | -------------- | ----------------- | -------------------------------- | ------------ | ----------- | |
| Ha103 | ------------------------------ | --I-- | -------------- | ----------------- | -------------------------------- | ------------ | ----------- | |
| Ha111 | ------------------------------ | --L-- | -------------- | ----------------- | -------------------------------- | ------------ | ----------- | |
| Ha204 | ------------------------------ | ----- | -------------- | ---------D------- | -------------------------------- | ------------ | ----------- | |
| Ha219 | ------------------------------ | ----- | -------------- | ------------P---- | -------------------------------- | ------------ | ----------- | |
| Ha401 | ------------------------------ | -VI-- | -------------- | ---------D------- | -------------------------------- | ------------ | ----------- | |
| H17 | ------------------------------ | -VL-- | -------------- | ---------D--PQ-QD | -------------------------------- | ------------ | ----------- | |
| H19 | ------------------------------ | -A--- | -------------- | ---------D--PQ-QD | -------------------------------- | ------------ | ----------- | |

FIG. 28-2

| | FR1 | CDR1 | FR2 | CDR2 | FR3 | CDR3 | FR4 | |
|---|---|---|---|---|---|---|---|---|
| | 0 1 2 3<br>123456789012345678901234567890 | <br>12345 | 4<br>67890123456789 | 5 6<br>012ab4567890123 45 | 6 7 8 9<br>67890123456789012abc34567890123 4 | 10<br>567890abcd12 | 11<br>34567890123 | |
| H0 | QVQLVQSGAEVKKPGASVKVSCKASGYTFT | GYIMN | WVRQAPGQGLEWMG | LINPYNGGTSYNQKFKG | RVTITADESTSTAYMELSSLRSEDTAVYYCAR | DGYDDGPYTMDY | WGQGTLVTVSS | SEQ ID NO:50 |
| H28 | ------------------------------ | --VL- | -------------- | ---------D--PQ-QD | -------------------------------- | ---------LET | ----------- | |
| H30 | ------------------------------ | ----- | -------------- | ---------D--PQ-QD | -------------------------------- | ---------LET | ----------- | |
| H34 | ------------------------------ | --A-- | -------------- | ---------D--PQ-QD | -------------------------------- | ---------LET | ----------- | |
| H42 | ------------------------------ | --VL- | -------------- | ---------D--PQ-QD | -------K------------------------ | ---------LET | ----------- | |
| H44 | ------------------------------ | ----- | -------------- | ---------D--PQ-QD | -------K------------------------ | ---------LET | ----------- | |
| H46 | ------------------------------ | --A-- | -------------- | ---------D--PQ-QD | -------K------------------------ | ---------LET | ----------- | |
| H57 | ------------------------------ | ----- | -------------- | ---------D--PQ-Q- | -------------------------------- | ---------LET | ----------- | |
| H71 | ------------------------------ | --A-- | -------------- | ---------D--PQ-Q- | -------------------------------- | ---------LET | ----------- | |
| H78 | ------------------------------ | ----- | -------------- | ---------D--PQ-Q- | -------K------------------------ | ---------LET | ----------- | |
| H92 | ------------------------------ | --A-- | -------------- | ---------D--PQ-Q- | -------K------------------------ | ---------LET | ----------- | |
| H97 | ------------------------------ | ----- | -------------- | -------------Q-QD | -------------------------------- | ---------LET | ----------- | |
| H98 | ------------------------------ | ----- | -------------- | -------------Q-QD | -------K------------------------ | ---------LET | ----------- | |

FIG. 28-3

Humanized

| | FR1 | CDR1 | FR2 | CDR2 | FR3 | CDR3 | |
|---|---|---|---|---|---|---|---|
| | 0 1 2 | 3 | 4 | 5 | 6 7 8 | 9 | |
| | 12345678901234567890123 | 4567ab01234 | 567890123456789 | 0123456 | 78901234567890123456789012345678 | 901234567 | |
| L0 | DIQMTQSPSSLSASVGDRVTITC | RTSENIYSFLA | WYQQKPGKAPKLLIY | NAKTLAK | GVPSRFSGSGSGTDFTLTISSLQPEDFATYYC | QHHYESPLT | SEQ ID NO:52 |
| Lp1 | ----------------------- | Q---------- | --------------- | ------- | -------------------------------- | --------- | |
| Lp2 | ----------------------- | ----D------ | --------------- | ------- | -------------------------------- | --------- | |
| Lp3 | ----------------------- | ----------- | --------------- | D------ | -------------------------------- | --------- | |
| Lp4 | ----------------------- | ----------- | --------------- | --Q---- | -------------------------------- | --------- | |
| Lp7 | ----------------------- | ----------- | --------------- | ----E-- | -------------------------------- | --------- | |
| Lp5 | ----------------------- | ----------- | --------------- | ------Q | -------------------------------- | --------- | |
| Lp6 | ----------------------- | ----------- | --------------- | ------D | -------------------------------- | --------- | |
| Lp15 | ----------------------- | Q---D------ | --------------- | --Q-E-Q | -------------------------------- | --------- | |

FIG. 29-1

| | FR1 | CDR1 | FR2 | CDR2 | FR3 | CDR3 | |
|---|---|---|---|---|---|---|---|
| | 0 1 2 | 3 | 4 | 5 | 6 7 8 | 9 | |
| | 12345678901234567890123 | 4567ab01234 | 567890123456789 | 0123456 | 78901234567890123456789012345678 | 901234567 | |
| L0 | DIQMTQSPSSLSASVGDRVTITC | RTSENIYSFLA | WYQQKPGKAPKLLIY | NAKTLAK | GVPSRFSGSGSGTDFTLTISSLQPEDFATYYC | QHHYESPLT | SEQ ID NO:52 |
| La134 | ----------------------- | -------R--- | --------------- | ------- | -------------------------------- | --------- | |
| La130 | ----------------------- | -------R-V- | --------------- | ------- | -------------------------------- | --------- | |
| La303 | ----------------------- | ----------- | --------------- | ------- | -------------------------------- | ----D---- | |
| La328 | ----------------------- | ----------- | --------------- | ------- | -------------------------------- | -----D--- | |
| La326 | ----------------------- | ----------- | --------------- | ------- | -------------------------------- | --------F | |
| La402 | ----------------------- | Q------R-V- | --------------- | ------- | -------------------------------- | ----D---- | |
| L11 | ----------------------- | Q--D---R-V- | --------------- | --Q-E-Q | -------------------------------- | ----D---- | |
| L12 | ----------------------- | Q--D-----V- | --------------- | --Q-E-Q | -------------------------------- | ----D---- | |
| L17 | ----------------------- | QA-D-----V- | --------------- | --Q-E-Q | -------------------------------- | ----D---- | |
| L50 | ----------------------- | QA-D------- | --------------- | --Q-E-Q | -------------------------------- | ----D---- | |

FIG. 29-2

Europäisches Patentamt
European Patent Office
Office européen des brevets

# EUROPEAN SEARCH REPORT

Application Number
EP 15 17 2595

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,P<br><br><br><br>E | WO 2009/072604 A1 (CHUGAI PHARMACEUTICAL CO LTD [JP]; KURAMOCHI TAICHI [JP]; KASUTANI KEI) 11 June 2009 (2009-06-11)<br>* the whole document *<br>& EP 2 236 604 A1 (CHUGAI PHARMACEUTICAL CO LTD [JP]) 6 October 2010 (2010-10-06)<br>* examples 1-13 *<br>----- | 1-10<br><br><br><br>1-10 | INV.<br>C07K16/28<br>A61K39/395<br>A61P29/00<br>C12N15/09 |
| A,P | WO 2009/072598 A1 (CHUGAI PHARMACEUTICAL CO LTD [JP]; HIGUCHI YOSHINOBU [JP]; KASUTANI KE) 11 June 2009 (2009-06-11)<br>* the whole document *<br>& EP 2 241 332 A1 (CHUGAI PHARMACEUTICAL CO LTD [JP]) 20 October 2010 (2010-10-20)<br>* paragraph [0079] - paragraph [0082]; example 2 *<br>----- | 1-10 | |
| X | WO 2007/142325 A1 (CHUGAI PHARMACEUTICAL CO LTD [JP]; HASEGAWA MASAKAZU [JP]; KITAMURA HI) 13 December 2007 (2007-12-13)<br>* paragraph [0050] - paragraph [0053]; example 3 *<br>& EP 2 047 863 A1 (CHUGAI PHARMACEUTICAL CO LTD [JP]) 15 April 2009 (2009-04-15)<br>----- | 1-10 | TECHNICAL FIELDS SEARCHED (IPC)<br>C07K<br>A61K<br>A61P |

The present search report has been drawn up for all claims

1

| Place of search | Date of completion of the search | Examiner |
|---|---|---|
| The Hague | 1 October 2015 | Siaterli, Maria |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 15 17 2595

This annex lists the patent family members relating to the patent documents cited in the above-mentioned European search report.
The members are as contained in the European Patent Office EDP file on
The European Patent Office is in no way liable for these particulars which are merely given for the purpose of information.

01-10-2015

| Patent document cited in search report | Publication date | Patent family member(s) | Publication date |
|---|---|---|---|
| WO 2009072604 A1 | 11-06-2009 | AU 2008332271 A1 | 11-06-2009 |
| | | AU 2009323249 A1 | 10-06-2010 |
| | | BR PI0821110 A2 | 07-07-2015 |
| | | CA 2708532 A1 | 11-06-2009 |
| | | CA 2710264 A1 | 10-06-2010 |
| | | CN 101939424 A | 05-01-2011 |
| | | CN 101952318 A | 19-01-2011 |
| | | EP 2236604 A1 | 06-10-2010 |
| | | JP 5682995 B2 | 11-03-2015 |
| | | KR 20100097721 A | 03-09-2010 |
| | | RU 2010127292 A | 10-01-2012 |
| | | TW 200932266 A | 01-08-2009 |
| | | US 2011129459 A1 | 02-06-2011 |
| | | US 2011229459 A1 | 22-09-2011 |
| | | US 2014039165 A1 | 06-02-2014 |
| | | US 2015175704 A1 | 25-06-2015 |
| | | WO 2009072604 A1 | 11-06-2009 |
| WO 2009072598 A1 | 11-06-2009 | AU 2008332276 A1 | 11-06-2009 |
| | | BR PI0821145 A2 | 16-06-2015 |
| | | CA 2708065 A1 | 11-06-2009 |
| | | CN 101939025 A | 05-01-2011 |
| | | CN 104162155 A | 26-11-2014 |
| | | EP 2241332 A1 | 20-10-2010 |
| | | HK 1200697 A1 | 14-08-2015 |
| | | JP 5475460 B2 | 16-04-2014 |
| | | KR 20100100935 A | 15-09-2010 |
| | | RU 2010127291 A | 10-01-2012 |
| | | RU 2014150548 A | 20-07-2015 |
| | | TW 200932265 A | 01-08-2009 |
| | | US 2010310556 A1 | 09-12-2010 |
| | | WO 2009072598 A1 | 11-06-2009 |
| WO 2007142325 A1 | 13-12-2007 | AR 061317 A1 | 20-08-2008 |
| | | AU 2007255753 A1 | 13-12-2007 |
| | | BR PI0712426 A2 | 17-07-2012 |
| | | CA 2654623 A1 | 13-12-2007 |
| | | CL 2007001665 A1 | 18-01-2008 |
| | | CN 101500608 A | 05-08-2009 |
| | | CN 103272232 A | 04-09-2013 |
| | | DK 2047863 T3 | 16-09-2013 |
| | | EP 2047863 A1 | 15-04-2009 |
| | | EP 2371393 A1 | 05-10-2011 |
| | | ES 2429407 T3 | 14-11-2013 |
| | | ES 2544888 T3 | 04-09-2015 |
| | | JP 5043008 B2 | 10-10-2012 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 15 17 2595

This annex lists the patent family members relating to the patent documents cited in the above-mentioned European search report.
The members are as contained in the European Patent Office EDP file on
The European Patent Office is in no way liable for these particulars which are merely given for the purpose of information.

01-10-2015

| Patent document cited in search report | Publication date | Patent family member(s) | Publication date |
|---|---|---|---|
| | | KR 20090024243 A | 06-03-2009 |
| | | KR 20140097495 A | 06-08-2014 |
| | | KR 20150091545 A | 11-08-2015 |
| | | NZ 572888 A | 24-02-2012 |
| | | PE 03332008 A1 | 15-06-2008 |
| | | PT 2047863 E | 21-10-2013 |
| | | RU 2008152827 A | 20-07-2010 |
| | | SI 2047863 T1 | 29-11-2013 |
| | | TW 200810778 A | 01-03-2008 |
| | | UA 100116 C2 | 26-11-2012 |
| | | US 2010055092 A1 | 04-03-2010 |
| | | WO 2007142325 A1 | 13-12-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0075314 A
- WO 03060090 A
- WO 00075314 A
- EP 125023 A
- WO 9602576 A
- WO 9813388 A
- EP 239400 A
- JP H0159878 B
- WO 9312227 A
- WO 9203918 A
- WO 9402602 A
- WO 9425585 A
- WO 9634096 A
- WO 9633735 A
- WO 9201047 A
- WO 9220791 A
- WO 9306213 A
- WO 9311236 A
- WO 9319172 A
- WO 9501438 A
- WO 9515388 A
- EP 404097 A
- WO 9311161 A
- US 5057313 A
- US 5156840 A
- WO 9846777 A
- WO 03104453 A
- JP H159878 B
- US 3773919 A
- EP 58481 A
- EP 133988 A
- WO 2005054467 A


### Non-patent literature cited in the description


- IL-31 is associated with cutaneous lymphocyte antigen-positive skin homing T cells in patients with atopic dermatitis. *J Allergy Clin Immunol.,* February 2006, vol. 117 (2), 418-25
- *J Biol Chem,* 2002, vol. 277, 16831-6
- *J Biol Chem,* 2003, vol. 278, 49850-9
- *Nat Immunol,* 2004, vol. 5, 752-60
- **HASHIMOTO-GOTOH, T ; MIZUNO, T ; OGASAHARA, Y ; NAKAGAWA, M.** An oligodeoxyribonucleotide-directed dual amber method for site-directed mutagenesis. *Gene,* 1995, vol. 152, 271-275
- **ZOLLER, MJ ; SMITH, M.** Oligonucleotide-directed mutagenesis of DNA fragments cloned into M13 vectors. *Methods Enzymol.,* 1983, vol. 100, 468-500
- **KRAMER, W ; DRUTSA, V ; JANSEN, HW ; KRAMER, B ; PFLUGFELDER, M ; FRITZ, HJ.** The gapped duplex DNA approach to oligonucleotide-directed mutation construction. *Nucleic Acids Res.,* 1984, vol. 12, 9441-9456
- **KRAMER W ; FRITZ HJ.** Oligonucleotide-directed construction of mutations via gapped duplex DNA Methods. *Enzymol.,* 1987, vol. 154, 350-367
- **KUNKEL, TA.** Rapid and efficient site-specific mutagenesis without phenotypic selection. *Proc Natl Acad Sci USA.,* 1985, vol. 82, 488-492
- **MARK, D. F et al.** *Proc. Natl. Acad. Sci. USA,* 1984, vol. 81, 5662-6
- **ZOLLER, M. J. ; SMITH, M.** *Nucleic Acids Res.,* 1982, vol. 10, 6487-500
- **WANG, A. et al.** *Science,* 1984, vol. 224, 1431-3
- **DALBADIE-MCFARLAND, G. et al.** *Proc. Natl. Acad. Sci. USA,* 1982, vol. 79, 6409-13
- **KARLIN ; ALTSCHUL.** *Proc. Natl. Acad. Sci. USA,* 1993, vol. 90, 5873-7
- **ALTSCHUL et al.** *J. Mol. Biol.,* 1990, vol. 215, 403-10
- **SATO, K. et al.** *Cancer Res.,* 1993, vol. 53, 851-856
- **CARL, A. K. BORREBAECK ; JAMES, W. LARRICK.** THERAPEUTIC MONOCLONAL ANTIBODIES. MACMILLAN PUBLISHERS LTD, 1990
- **CO, M.S. et al.** *J. Immunol.,* 1994, vol. 152, 2968-2976
- **BETTER, M. ; HORWITZ, A. H.** *Methods in Enzymology,* 1989, vol. 178, 476-496
- **PLUECKTHUN, A. ; SKERRA, A.** *Methods in Enzymology,* 1989, vol. 178, 476-496
- **LAMOYI, E.** *Methods in Enzymology,* 1989, vol. 121, 652-663
- **ROUSSEAUX, J. et al.** *Methods in Enzymology,* 1989, vol. 121, 663-669
- **BIRD, R. E. et al.** *TIBTECH,* 1991, vol. 9, 132-137
- **HOLLIGER P et al.** *Proc. Natl. Acad. Sci. USA,* 1993, vol. 90, 6444-6448

- **HUSTON, J. S. et al.** *Proc. Natl. Acad. Sci. U.S.A.,* 1988, vol. 85, 5879-5883
- **PLUCKTHUN.** The Pharmacology of Monoclonal Antibodies. Springer Verlag, 1994, vol. 113, 269-315
- **HUDSON et al.** *J Immunol. Methods,* 1999, vol. 231, 177-189
- *Protein Engineering,* 1996, vol. 9 (3), 299-305
- **HOPP, T. P. et al.** *BioTechnology,* 1988, vol. 6, 1204-1210
- **KOHLER, G. ; MILSTEIN, C.** *Methods Enzymol.,* 1981, vol. 73, 3-46
- **KOHLER. G. ; MILSTEIN, C.** *Methods Enzymol.,* 1981, vol. 73, 3-46
- **CO, M. S. et al.** *J. Immunol.,* 1994, vol. 152, 2968-2976
- **BETTER, M. ; HORWITZ, A. H.** *Methods Enzymol.,* 1989, vol. 178, 476-496
- **PLUCKTHUN, A. ; SKERRA, A.** *Methods Enzymol.,* 1989, vol. 178, 497-515
- **LAMOYI, E.** *Methods Enzymol.,* 1986, vol. 121, 652-663
- **ROUSSEAUX, J. et al.** *Methods Enzymol.,* 1986, vol. 121, 663-669
- **BIRD, R. E. ; WALKER, B. W.** *Trends Biotechnol.,* 1991, vol. 9, 132-137
- **WARD et al.** *Nature,* 1989, vol. 341, 544-546
- *FASEB J.,* 1992, vol. 6, 2422-2427
- **BETTER et al.** *Science,* 1988, vol. 240, 1041-1043
- **LEI, S. P. et al.** *J. Bacteriol.,* 1987, vol. 169, 4379
- *Nucleic Acids. Res.,* 1990, vol. 18 (17), 5322
- **MULLIGAN et al.** *Nature,* 1979, vol. 277, 108
- **MIZUSHIMA et al.** *Nucleic Acids Res.,* 1990, vol. 18, 5322
- Molecular Cloning. Cold Spring Harbor Laboratory Press, 1989
- Stedman's Medical Dictionary. MEDICAL VIEW CO, 2005
- Remington's Pharmaceutical Science. Mark Publishing Company
- Remington's Pharmaceutical Science. 1980
- **LANGER et al.** *J. Biomed. Mater. Res.,* vol. 15, 167-277
- **LANGER.** *Chem. Tech.,* 1982, vol. 12, 98-105
- **SIDMAN et al.** *Biopolymers,* 1983, vol. 22, 547-56
- **PACE et al.** *Protein Science,* 1995, vol. 4, 2411-2423
- Heterogeneity of Monoclonal Antibodies. *Journal of pharmaceutical sciences,* vol. 97 (7), 2426-2447
- *Biochem. Biophys. Res. Commun.,* 1996, vol. 228, 838-45
- *Ann Hematol.,* June 1998, vol. 76 (6), 231-48
- *Nat Biotechnol.,* December 2007, vol. 25 (12), 1369-72
- *J Biol Chem.,* 06 June 2008, vol. 283 (23), 16206-15
- *Cancer Res.,* 15 February 1993, vol. 53 (4), 851-6
- *J Pharm Sci.,* April 2008, vol. 97 (4), 1414-26
- *Eur J Immunol.,* August 1999, vol. 29 (8), 2613-24
- *Anal Biochem.,* 01 January 2007, vol. 360 (1), 75-83
- *Nat Rev Immunol.,* September 2007, vol. 7 (9), 715-25
- *Nat. Biotechnol.,* December 2007, vol. 25 (12), 1369-72
- *J. Biol. Chem.,* 19 January 2007, vol. 282 (3), 1709-17
- *J. Immunol.,* 01 January 2006, vol. 176 (1), 346-56
- *Proc. Natl. Acad. Sci. USA.,* 05 December 2006, vol. 103 (49), 18709-14
- *Int. Immunol.,* December 2006, vol. 18 (12), 1759-69
- *J. Exp. Med.,* 1994, vol. 180 (6), 2377-2381
- *Proc. Natl. Acad. Sci. U.S.A.,* 2002, vol. 99 (26), 16899-16903
- *J. Immunol.,* 01 November 2002, vol. 169 (9), 5171-80